(19) Europäisches Patentamt
European Patent Office
Office européen des brevets

(11) EP 1 218 537 B1

(12) **EUROPEAN PATENT SPECIFICATION**

(45) Date of publication and mention of the grant of the patent:
**10.09.2008 Bulletin 2008/37**

(21) Application number: **00912847.1**

(22) Date of filing: **24.03.2000**

(51) Int Cl.:
***C12Q 1/68*** *(2006.01)*
***G06F 19/00*** *(2006.01)*
***A01K 67/00*** *(2006.01)*
***C07K 16/18*** *(2006.01)*
***C12N 15/11*** *(2006.01)*
***C12N 5/10*** *(2006.01)*
***C07K 14/47*** *(2006.01)*

(86) International application number:
**PCT/IB2000/000403**

(87) International publication number:
**WO 2000/058508 (05.10.2000 Gazette 2000/40)**

(54) **BIALLELIC MARKERS RELATED TO GENES INVOLVED IN DRUG METABOLISM**

Biallelische Marker die im zusammenhang stehen mit Medikamentenmetabolismus beteiligten Genen

MARQUEURS BIALLELIQUES RELATIFS AUX GENES IMPLIQUES DANS LE METABOLISME DES MEDICAMENTS

(84) Designated Contracting States:
**AT BE CH CY DE DK ES FI FR GB GR IE IT LI LU MC NL PT SE**

(30) Priority: **25.03.1999 US 126269 P**
**30.04.1999 US 131961 P**

(43) Date of publication of application:
**03.07.2002 Bulletin 2002/27**

(73) Proprietor: **Serono Genetics Institute S.A.**
**91030 Evry Cedex (FR)**

(72) Inventors:
- **BLUMENFELD, Marta**
**F-75013 Paris (FR)**
- **BOUGUELERET, Lydie**
**F-92170 Vanves (FR)**
- **CHUMAKOV, Ilya**
**F-77000 Vaux-le-Penil (FR)**
- **COHEN-AKENINE, Annick**
**F-75012 Paris (FR)**

(74) Representative: **Brasnett, Adrian Hugh et al**
**Mewburn Ellis LLP**
**York House**
**23 Kingsway**
**London WC2B 6HP (GB)**

(56) References cited:
**EP-A- 0 723 021**
**WO-A-98/30883**

- **JAKOBSSON P-J ET AL: "IDENTIFICATION AND CHARACTERIZATION OF A NOVEL HUMAN MICROSOMAL GLUTATHIONE S-TRANSFERASE WITH LEUKOTRIENE C4 SYNTHASE ACTIVITY AND SIGNIFICANT SEQUENCE IDENTITY TO 5-LIPOXYGENASE-ACTIVATING PROTEIN AND LEUKOTRIENE C4 SYNTHASE" JOURNAL OF BIOLOGICAL CHEMISTRY, AMERICAN SOCIETY OF BIOLOGICAL CHEMISTS, BALTIMORE, MD, US, vol. 271, no. 36, 6 September 1996 (1996-09-06), pages 22203-22210, XP002044920 ISSN: 0021-9258 cited in the application**
- **RAUNIO H ET AL: "Diagnosis of polymorphisms in carcinogen-activating and inactivating enzymes and cancer susceptibility - a review" GENE, ELSEVIER BIOMEDICAL PRESS. AMSTERDAM, NL, vol. 159, no. 1, 14 June 1995 (1995-06-14), pages 113-121, XP004042243 ISSN: 0378-1119**
- **SANAK M ET AL: "leukotriene C4 synthase promotor polymophism and risk of aspirin-induced asthma" LANCET THE, LANCET LIMITED. LONDON, GB, vol. 350, 29 November 1997 (1997-11-29), pages 1599-1600, XP002086063 ISSN: 0140-6736**
- **DATABASE EMBL 19 January 1998 (1998-01-19), NCI-CGAP: "ny67e04.s1 NCI_CGAP_GCB1 Homo sapiens cDNA clone image: 1283358" retrieved from EBI Database accession no. AA744812**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE EMBL 25 January 1999 (1999-01-25), ZHAO ET AL: "use of BAC end sequences from library RPCI-11 for sequence ready map building" retrieved from EBI Database accession no. AQ345937
- DATABASE SWISS-PROT February 1991 (1991-02), "GSTM1_human" retrieved from EBI Database accession no. p09488
- DATABASE SWISS-PROT November 1997 (1997-11), "MGST2_human" retrieved from EBI Database accession no. q99735

SEQUENCE LISTING

<110> GENSET

<120> BIALLELIC MARKERS RELATED TO GENES INVOLVED IN DRUG METABOLISM

<130> 62.WO1

<150> US 60/126,269
<151> 1999-03-25

<150> US 60/131,961
<151> 1999-04-30

<160> 54

<170> Patent.pm

<210> 1
<211> 1001
<212> DNA
<213> Homo Sapiens

<220>
<221> allele
<222> 501
<223> 12-421-140 : polymorphic base A or G

<220>
<221> misc_binding
<222> 481..500
<223> 12-421-140.mis1, potential

<220>
<221> misc_binding
<222> 502..521
<223> 12-421-140.mis2, potential complement

<220>
<221> primer_bind
<222> 362..380
<223> upstream amplification primer

<220>
<221> primer_bind
<222> 792..812
<223> downstream amplification primer, complement

<220>
<221> misc_binding
<222> 489..513
<223> 12-421-140 potential probe

<400> 1

```
tgtctcattt tgatacagat gaacctagtg tcaaatgagt tggaggaagc atcctgttct      60
actttctcag ataattttaa taaggttgga agagtgtgtt ccttcaaggt tgtgtagaac     120
ttgccttaaa ataatctggg cctaatgttt ttctggtggg aaatttttaa attatgggat     180
ttgcattctt taatgattat tggactaggc aggctttttg ttacatcttg aatcaatttt     240
ggtaggcctt ggtccttgag atgataacat tggtctcttg attatccact tgctccaagg     300
agggttttac agactactcc ctaggcttga aagcgtgggg tgaataccac cgggagtaca     360
cgagataatt gtaagaggtg tgtagacttg gttttaaata acattgaact acatgatgag     420
aagtcattct catttcaatt atctttgaat ccttatggtt aagtcaagtg gaaagtattg     480

gtttggagcc agcatgtccc rcacctcaaa attaatgttt atttttatca aagtaattta     540
tgtacgtgat ctgaagataa agtgggatta caggttttat gacaacaaat aactcttcct     600
tgtccctacc tcccctctcc ctctatccct agagataacc ctttttgatt actactttgg     660
gtatttactt ctgtatttct aaacattata ctttcactgc tatttattga ttcacaaatt     720
ttagacattg tcagttggct tctttttatg gaagaaataa tttcatgctc ctttacttct     780
tcttttcctt acctcatcct tccagtaaca ttatatcact ctgttaggtt aaatcaatag     840
ccaatgttta taattattgt gattaagaaa ataatgttga aagcagggcc ttatagtata     900
ctgtggttac atttccttct tgtgcaattt tttttcccta gggttaataa ttgctcttta     960
tttctttgtt tgcttagttt catgcccaaa tcttctgcag a                        1001
```

<210> 2
<211> 999
<212> DNA
<213> Homo Sapiens

<220>
<221> allele
<222> 501
<223> 12-424-192 : polymorphic base A or G

<220>

<221> misc_binding
<222> 481..500
<223> 12-424-192.mis1, potential

<220>
<221> misc_binding
<222> 502..521
<223> 12-424-192.mis2, potential complement

<220>
<221> primer_bind
<222> 310..327
<223> upstream amplification primer

<220>
<221> primer_bind
<222> 751..771
<223> downstream amplification primer, complement

<220>
<221> misc_binding
<222> 489..513
<223> 12-424-192 potential probe

<220>
<221> misc_feature
<222> 521,541,635
<223> n=a, g, c or t

<400> 2

```
ggatgcaatc tgctggtctc tagacaatgt tccccttccc tctggtgcta ctggcttcca     60
gtgctgaaac tggcaactgc tagttcagca actccaaggc aggcaatgca gaaacatggc    120
gtgggggga tggggaattc ctctcttacc aaccctgcct catttcagtt tttgcttcag    180
aagcttgggc ttctcaccac tttctccatc tggctagctg gtctactcta agccctactg    240
tggggccact catccagggc tcacagaggg ccccatggta aggccaccag ctcttgattc    300
agcttccgca caatgtggga acttaggaca gctcaggaaa cctaactcag ggccttcctt    360
ctccttacca tactacttta ctctgctatg gctgtgtaaa agactgaatg ggacagagat    420
cttgtaccag gagagaaacg cccattagta atttccaagc atgcacacat gttataggtt    480
ctgagggctc aggaacagtt rcatgaggtc cagagttcga nttatgaagc tgaggaattt    540
nactaatgta ttcattcgtt gaatatttca gtgtattttg caagttggca ctgtgtgagt    600
gctacacttg gctctacagt ggttagcaaa tctgnacaca gtcctcatgg atcttacata    660
agaggaaaaa actgacaacc aaatgatcac acaaatgagt aactacacag tatgcaatgc    720

tccacaaaag gagggtcatg gtgctatctt cacgagaata taagcaccac agtggtttc     780
tttattttgt tcattgatgt acctagaatg gggcctcaca catattaagt gctaaataag    840
tatttgttga atgaatgaat aaataaatgc atgaaagcta tgagattact tctgagaggt    900
cagggatgaa ttcctgaggc agtgttattt tggctgatgt taaagagaaa aaaaaattct    960
tagttcgaat tctgctagat aaatttatat taaccaaac                           999
```

<210> 3
<211> 993
<212> DNA
<213> Homo Sapiens

<220>
<221> allele

<222> 501
<223> 12-424-198 : polymorphic base G or T

<220>
<221> misc_binding
<222> 481..500
<223> 12-424-198.mis1, potential

<220>
<221> misc_binding
<222> 502..521
<223> 12-424-198.mis2, potential complement

<220>
<221> primer_bind
<222> 304..321
<223> upstream amplification primer

<220>
<221> primer_bind
<222> 745..765
<223> downstream amplification primer, complement

<220>
<221> misc_binding
<222> 489..513
<223> 12-424-198 potential probe

<220>
<221> misc_feature
<222> 515,535,629
<223> n=a, g, c or t

<400> 3

```
aatctgctgg tctctagaca atgttcccct tccctctggt gctactggct tccagtgctg     60
aaactggcaa ctgctagttc agcaactcca aggcaggcaa tgcagaaaca tggcgtgggg    120
gggatgggga attcctctct taccaaccct gcctcatttc agtttttgct tcagaagctt    180
gggcttctca ccactttctc catctggcta gctggtctac tctaagccct actgtggggc    240
cactcatcca gggctcacag agggccccat ggtaaggcca ccagctcttg attcagcttc    300
cgcacaatgt gggaacttag gacagctcag gaaacctaac tcagggcctt ccttctcctt    360
accatactac tttactctgc tatggctgtg taaaagactg aatgggacag agatcttgta    420
ccaggagaga aacgcccatt agtaatttcc aagcatgcac acatgttata ggttctgagg    480
gctcaggaac agttgcatga kgtccagagt tcganttatg aagctgagga atttnactaa    540
tgtattcatt cgttgaatat ttcagtgtat tttgcaagtt ggcactgtgt gagtgctaca    600
cttggctcta cagtggttag caaatctgna cacagtcctc atggatctta cataagagga    660
aaaaactgac aaccaaatga tcacacaaat gagtaactac acagtatgca atgctccaca    720
aaaggagggt catggtgcta tcttcacgag aatataagca ccacagtggt tttctttatt    780
ttgttcattg atgtacctag aatggggcct cacacatatt aagtgctaaa taagtatttg    840
ttgaatgaat gaataaataa atgcatgaaa gctatgagat tacttctgag aggtcaggga    900
tgaattcctg aggcagtgtt attttggctg atgttaaaga gaaaaaaaaa ttcttagttc    960

gaattctgct agataaattt atattaacca aac                                 993
```

<210> 4
<211> 1001
<212> DNA

<213> Homo Sapiens

<220>
<221> allele
<222> 501
<223> 12-425-57 : polymorphic base G or A

<220>
<221> misc_binding
<222> 481..500
<223> 12-425-57.mis1, potential

<220>
<221> misc_binding
<222> 502..521
<223> 12-425-57.mis2, potential complement

<220>
<221> primer_bind
<222> 540..557
<223> upstream amplification primer, complement

<220>
<221> primer_bind
<222> 82..99
<223> downstream amplification primer

<220>
<221> misc_binding
<222> 489..513
<223> 12-425-57 potential probe

<400> 4

```
atctggtgga ggtcctggtt tctgagaaac aactcaagaa cttatgtgaa gacgttgtct     60
ttagtttcta tagggaaccg aacgtcttgt aactctgtct tatttgggtg actattattt    120
aaactattat tatcttctcg cttagcggga ctgaaggata gcaaggtgct tggaatttcc    180
cttgaaggaa ctcaagagtt tttatttccg tgcttgaggt ggctagcagg tccctaagag    240
tgttccttgc tctgtctcca tgccagaagt ttattatggg gtgaaaagag gaagttaagg    300
acaggaggca agacctactg aaggctcaaa aactgacaga ggaggaagta tggatgtgga    360
agtgaaagaa gtccacagag ctcatcacaa gatgaaggtg gtcccaggca tcagagttga    420
ttatctaatg aaataaagga gaagaaagtc atactgtttt ataacaggga aacgtcggca    480
ggtaggagcc atcatcccca ragttttctt gtacctgctg ggcaaggtgt cagagccctg    540
gcaatggaaa gtgattggcc cacagggagt aagaatttac cgacaacaat ataggtttga    600
aaaggaaagt tttattagat tgagagaaag ctgcagaaga gtgcagcggg ctgcctcagc    660
gagaggactg agcgcgccgg ggaggatttt ccttaggggt atttatggac cttaaagcaa    720
gagcttaggg gtaatttggt ccatattagc cacatatgtc atgataaatg attacatttg    780
tagacatttt ggtgtcgtaa tgtcagcaag ggttgcacaa tgagttttgt catgcatgca    840
ttccggagat gtatagaggt agagaaagtc tagttacata taaattttgg ggaaagaagc    900
ctggaaccgg ccttagatat agggaagatc aattatttct aaactcctca gataaggagt    960
tctgcctctg gatggtcggc ttcatggcca ccaggtggtc t                       1001
```

<210> 5
<211> 961
<212> DNA
<213> Homo Sapiens

<220>
<221> allele

<222> 461
<223> 12-426-154 : polymorphic base A or G

<220>
<221> misc_binding
<222> 441..460
<223> 12-426-154.mis1, potential

<220>
<221> misc_binding
<222> 462..481
<223> 12-426-154.mis2, potential complement

<220>
<221> primer_bind
<222> 308..325
<223> upstream amplification primer

<220>
<221> primer_bind
<222> 830..847
<223> downstream amplification primer, complement

<220>
<221> misc_binding
<222> 449..473
<223> 12-426-154 potential probe

<220>
<221> misc_feature
<222> 705,761..762
<223> n=a, g, c or t

<400> 5

```
tccctagcta gcaaaatcag tgactcctgg gaacttgtaa aaaatgcaga ttctcaagcc     60
ccgcttcaga tttactgaat cagaaacact gtgtatgttt taacaagctc tgctggtgct    120
ttcagtgcag gctaaagttt gagaactaca gatacaaagg agggctctgt atctgcaaca    180
acaactatgt aatgatgact agtccccact cttctgaagc tacccatggc aaaattagaa    240
acataattct tcaaaaacca ctggcactga tttagtgcac agaacacctg tggataactc    300
aattcctttt gtccttccct gtgtcaaggc caaaggatct aatgtgatat aagcattcca    360
gaggtggaag ggagtggtag ctactcaaga cccagtattt ttacaggtga tcttcaatgt    420
gatctgtcag taaatatcaa tggctttcag actgtaaact rcagctcaca gttcatgatc    480
taggatatat acatgtgggt ttaaaacaga aagaaaagtt tctgatatgc tctggtaatt    540
tctgttgtat ttcattttaa aaatgttggt tgtgacccac taagttgttt ccaataacct    600
tgtgtgttgc aatctgagaa gttaaaaatg ctaccttaga tcagaaaatt ttcacccagt    660
ctattgataa agttcaggga tacttgtgaa attatataaa agttntgtat atatttacat    720
attatatgtt tctctgcaag aggcttcata gctttttgca nnacacttaa aggaataggt    780
aacttttgag aagctaggac acattgatct agatcccaca aaatgggcag aggatgggat    840
taggatttct gctgctaggt gacccctgta gccccctgaa aactcctgtg gataagatta    900
ggaaggctcc tcaggctatt ttcttcaccc catataagac cctcaagggt cttttttttt    960
t                                                                    961
```

<210> 6
<211> 1001
<212> DNA
<213> Homo Sapiens

<220>

<221> allele
<222> 501
<223> 12-429-198 : polymorphic base C or T

<220>
<221> misc_binding
<222> 481..500
<223> 12-429-198.mis1, potential

<220>
<221> misc_binding
<222> 502..521
<223> 12-429-198.mis2, potential complement

<220>
<221> primer_bind
<222> 304..321
<223> upstream amplification primer

<220>
<221> primer_bind
<222> 803..823
<223> downstream amplification primer, complement

<220>
<221> misc_binding
<222> 489..513
<223> 12-429-198 potential probe

<220>
<221> misc_feature
<222> 44,561..562,954
<223> n=a, g, c or t

<400> 6

```
ggtcaggagt tcaagaccag cctggccaat atggtgaaac cccngtccgt actaaaaata     60
aaaaaattag ctgggcgtgg tggcaggcac ctgtagtctc agctactcag gaggctgagg    120
caggagaatc gcttgaaccc aggaggcgga ggttgcagtg agttgacagt gtaccactgc    180
actccagcat gggcgacaga ccaagattct gtcttaaaaa acaaaacaaa acaaaacaaa    240
aaactgggag gggaaaatat tttttattt tctaatatgt tagaaccctt tctcaatgtg    300
taactctcct cattcttctc cctctcatct ttaaccaaaa ttgtaaatgt tctacatgtg    360
catcatgtta gtctatcagg tgttatttag ataattggtg cctttgactt acattcttgt    420
aaacgttgct ttagttcata aactcataat attcccagac ctgaagcctg tcttaaaatt    480
gataatgtaa atcatctagg yctgaatgcc taaagtgatg gggagctcac cacctcttgt    540
ttctggtcat aggtaggtga nngatccctt ctgcagcaag aagtcagggg caggcctgtg    600
acactagctc tgattcctgc aaatgaccaa atgaggggca gggcctggtg gtctcccagt    660
cccagctgag gctcccatga cctgcaccct cctgccctcc caactctcag gttctcccaa    720
cagccctccc ccagctaatt tcataaaagg cactaaaaac ttttgatcag tcacaaaatc    780
atgggaagag aaagaaatac aagaagagag aaagaaggga aagtggcgtg aatcattaac    840
aaaggcacca catccctgat actgtactgt tctgggtgct tttttctcaa tgaattctca    900
caaaatcctg ttggtattag tgattcatat ttcttacata tagaaactga gtcngcaaag    960
aggttattac ttaacttgcc caagatcgta cagctaataa g                       1001
```

<210> 7
<211> 996
<212> DNA
<213> Homo Sapiens

<220>
<221> allele
<222> 501
<223> 12-430-80 : polymorphic base T or C

<220>
<221> misc_binding
<222> 502..521
<223> 12-430-80.mis1, potential complement

<220>
<221> misc_binding
<222> 482..500
<223> 12-430-80.mis2

<220>
<221> primer_bind
<222> 561..580
<223> upstream amplification primer, complement

<220>
<221> primer_bind
<222> 131..150
<223> downstream amplification primer

<220>
<221> misc binding
<222> 489..513
<223> 12-430-80 potential probe

<220>
<221> misc_feature
<222> 918,971,978
<223> n=a, g, c or t

<400> 7

```
gaatgcccta attctctgga gctgtgtcct gaggcattaa ggggtaaagt ggttggatgt     60
actttaaaat ggttctgcga aatatatatg tgaagcattt atggcaaagt gttaatgatt    120
acatctaatt ggtaagtgta taggtattcg ttgtattatt atttctactt tcctggttgt    180
ttaaaaatgt ttataataaa gagttaaagg atctgaagag aagttaaaac ctaaaatcag    240
attcttggta gccctgaaga ttttctgtgt tgagcattgt taagagatcc agtggtattg    300
atggatccac agtcatccat gctggcctgg cagctgtcct gggtgtggca cagatgcctg    360
ggccccacat gtgtgagagc tgcagtaatt acctccacct gttgcatgaa atgaggcagg    420
tgtctgtgcc agctactcca gaggacacta gtggaggaat ttaatgtcct tagtacttaa    480
aagagaattg aggacaaaat yggatttaca aaaagaggaa acaaatcaaa aagaaaatta    540
aaaggtaaag actggttacc caaagaggta gctctgttcc attttcagat aatttttctt    600
gttccacttt tcttttaggg gttggaggtg ggtggggtat ggccaaaaaa gtcattaata    660
aacaaaatca ggtcatttga ggttcaccct ttttctccag acacaatctt ccttgaaaag    720
aatggtcaga ggcagaaaca gctcattgaa tgctatggaa tgttccttaa tcaccagtga    780
aatacgtctt tctcactggg ggcagatgtc tagtgacttt ctagaaaaat gtttattctc    840
tcctctttct tcccccatca ccatacttac tgcactagga ttttataaag gtcagtaaga    900
ttacatgtaa gaactcantt tgaaaagaga aaggtattcc attcatccag tgccaaacga    960
gtataagtaa ngaatatngt ggcttgaatt gattgg                              996
```

<210> 8
<211> 1001
<212> DNA
<213> Homo Sapiens

<220>
<221> allele
<222> 501
<223> 12-433-215 : polymorphic base A or G

<220>
<221> misc_binding
<222> 481..500
<223> 12-433-215.mis1, potential

<220>
<221> misc_binding
<222> 502..521
<223> 12-433-215.mis2, potential complement

<220>
<221> primer_bind
<222> 287..304
<223> upstream amplification primer

<220>
<221> primer_bind
<222> 805..825
<223> downstream amplification primer, complement

<220>
<221> misc_binding
<222> 489..513
<223> 12-433-215 potential probe

<220>
<221> misc_feature
<222> 92,202
<223> n=a, g, c or t

<400> 8

```
cagctacccg aagggcacca tgtgagccca cgtgtaaggg ctctgggtga cagctccagc      60
tatggcccca gcacgcagcc ccacctgcat gngatatacg ttcatatgat tctagcccac     120
agctgtgggg gcactcccag ccattgtctc ctcagctgag gcctcagaca ttgaagagca     180
gagacatgcc agtcctaccg tngccctact gacccacata actcattaga gcctaattaa     240
atggttgtta taaaccccta aactttgggg tactttgtca cacctgagta gtcaccagaa     300
caacctcatt gcaaggtaac aactcagctt cccagggttc atcatttggc tccagtgata     360
ccaccccatg ccctgcaagt gtggcacttc agacaggagc cagggacacc aggagtatgg     420
ctggtggccc ttaggtggca ggattgcaga ggccccaccg gaagtcagct ggccacagca     480
gagccaaggt agagtgcatg rttgaaaatt caggagacag gatgagctcc agaaaattgg     540
gcgctgcact ggacgtgcct gataaagcaa agagggagag aaaagaggag ttaaggattt     600
ccacatttct ggtttctgaa cagctagata cattaatgat ggggcaattt actgccacag     660
aatttagacc aaaggagggg gctaggtttg aagtgcctgt gagtccatta gaaatgacaa     720
gaagtgggag cccaggtcac caaagatggt caaggtcatt gatcttgaga ccacagggcc     780
ccaagaaggc ttaagggtct gtccctgatt cttgtttcct gtgtgagaaa tattttgcag     840
gtgtcaggct gggactcttg accagctgaa ttacgtattt ccagaacagg gtgttaaaga     900
agacagaagc gaccccagga aaaagcgtgg cttccacaag agctgccata tacaaacaag     960
catgccccac aacgctggca ggagagatat catcagattt a                        1001
```

<210> 9
<211> 1001
<212> DNA
<213> Homo Sapiens

<220>
<221> allele
<222> 501
<223> 12-441-233 : polymorphic base G or A

<220>
<221> misc_binding
<222> 502..521
<223> 12-441-233.mis1, potential complement

<220>
<221> misc_binding
<222> 482..500
<223> 12-441-233.mis2

<220>
<221> primer bind
<222> 716..734
<223> upstream amplification primer, complement

<220>
<221> primer_bind
<222> 284..303
<223> downstream amplification.primer

<220>
<221> misc_binding
<222> 489..513
<223> 12-441-233 potential probe

<220>
<221> misc_feature
<222> 661,929,990
<223> n=a, g, c or t

<400> 9

```
gaagaactta gacaatgcct gaaatgcaaa ggcgacactg gagtcttctt tctctaacgt     60
gtagcgttga atgaatatct gcctggaacc aagagggctg ctctgatgtt tgggagtcgg    120
tttttgtga gccacatctg atatttctga tatccccagg aaggagtggc ctggaggtca    180
ctggttcagg ctccctttgg gcgaaatcct gggagtgatg ctctaaaaat ccacctttcc    240
catcatccct actcatcaga aagacaaata taaaatccca gagaggtgga ggagctaaaa    300
aagcaattgc tccaccttac aaatttggat agaaaggaga tgtagtttat ttcatatggg    360
caaagtagtc ctcttccaaa gtcctgtaca attgttctct gcaattgacg cacatctgcc    420
ctaagcgaaa tctgtcagaa ggaatcaaca aggctccttg cctcccctcc caatccccct    480
tttggaggac ttgtggcttc rgtgtcgtcc taagtgagag tggcgtgtgc tttttttcctg    540
tcccctcctc cctccgtgtc ctagacgctg gctgccttct gtgcactccc aggcagatca    600
ctacggaaga gtcggagcct gtggggttgg actggccaca ctcagtcctg agaaggcgag    660
nttgccatgg aaagctgggg gcagaggtgt tttggagag gaggcggcag gcaaacattg    720
cctttgactt gctctccgcg tacccggggt tgtagagctg ctcaggaagg ggcaggatgt    780
aaggccagag gtgcctggtg ggtgagaagc ccaggcaggg gctgggcgcc ctctccgaag    840
aggtggcagc agggtgaccc tgaactcccc aaatggggag tgatgccact ggggaaactg    900
agtggatcaa agagatgaaa ccaaaaaana agcaaacaaa caaatgagaa gacacaaaac    960
ataattacct tttcctgaaa ggtacaggan aataaatata t                        1001
```

<210> 10
<211> 1001
<212> DNA
<213> Homo Sapiens

<220>
<221> allele
<222> 501
<223> 12-441-343 : polymorphic base G or A

<220>
<221> misc_binding
<222> 481..500
<223> 12-441-343.mis1, potential

<220>
<221> misc_binding
<222> 502..521
<223> 12-441-343.mis2, potential complement

<220>
<221> primer_bind
<222> 826...844
<223> upstream amplification primer, complement

<220>
<221> primer_bind
<222> 394..413
<223> downstream amplification primer

<220>
<221> misc_binding
<222> 489..513
<223> 12-441-343 potential probe

<220>
<221> misc_feature
<222> 3,771
<223> n=a, g, c or t

<400> 10

```
tanttcctcc ttgcctccac accagcctcc ccttgcttct ccttacagac tatccaagaa     60
gtgaagctta tgtctttagg gagccttggg cagagtccac ataaatgcag gaagaactta    120
gacaatgcct gaaatgcaaa ggcgacactg gagtcttctt tctctaacgt gtagcgttga    180
atgaatatct gcctggaacc aagagggctg ctctgatgtt tgggagtcgg tttttgtga     240
gccacatctg atatttctga tatccccagg aaggagtggc ctggaggtca ctggttcagg    300
ctccctttgg gcgaaatcct gggagtgatg ctctaaaaat ccacctttcc catcatccct    360
actcatcaga aagacaaata taaaatccca gagaggtgga ggagctaaaa aagcaattgc    420
tccaccttac aaatttggat agaaaggaga tgtagtttat ttcatatggg caaagtagtc    480
ctcttccaaa gtcctgtaca rttgttctct gcaattgacg cacatctgcc ctaagcgaaa    540
tctgtcagaa ggaatcaaca aggctccttg cctcccctcc caatccccct tttggaggac    600
ttgtggcttc agtgtcgtcc taagtgagag tggcgtgtgc ttttttcctg tcccctcctc    660
cctccgtgtc ctagacgctg gctgccttct gtgcactccc aggcagatca ctacggaaga    720
gtcggagcct gtggggttgg actggccaca ctcagtcctg agaaggcgag nttgccatgg    780
aaagctgggg gcagaggtgt ttttggagag gaggcggcag gcaaacattg cctttgactt    840
gctctccgcg tacccggggt tgtagagctg ctcaggaagg ggcaggatgt aaggccagag    900
gtgcctggtg ggtgagaagc ccaggcaggg gctgggcgcc ctctccgaag aggtggcagc    960
agggtgaccc tgaactcccc aaatggggag tgatgccact g                       1001
```

<210> 11
<211> 1001
<212> DNA
<213> Homo Sapiens

<220>
<221> allele
<222> 501
<223> 12-442-221 : polymorphic base T or C

<220>
<221> misc_binding
<222> 481..500
<223> 12-442-221.mis1, potential

<220>
<221> misc_binding
<222> 502..521
<223> 12-442-221.mis2, potential complement

<220>
<221> primer_bind
<222> 704..721 .
<223> upstream amplification primer, complement

<220>
<221> primer_bind
<222> 270..289
<223> downstream amplification primer

<220>
<221> misc_binding
<222> 489..513
<223> 12-442-221 potential probe

<220>
<221> misc_feature
<222> 180,531..532,822
<223> n=a, g, c or t

<400> 11

```
attctaagcc catgttttat cctaaagtac ccctttacac agaaaaacga attcatggca     60
caaaatacac cagcttaaga ctagccttag aattcttttt ggcattaatc aaaactttac    120
agaggacata aacactgatt ttttcccccc attcatttaa ctatttgcac acagagacan    180
gaagccagaa atctgactgg gaagaaattc ctaccctttt gccagcatgc taagcttctg    240
ggttctcttt ccctgagtgg ccctagtgat ctggcttgtg gcacaactgc ctttgggggc    300
caagccgcat cataaaggaa aagtatttct ttttgttctg gccaaagcaa aatacgcgta    360
ataaaacata gatattaacc aggctgctta gcatccaata tcaaactggc aaggcttaaa    420
tttgccctca ggtgggccct gtcatcttta atctaacctc cgactaggag tttcaacatg    480
tggtctctgg gcaagatggt ygccctgagt aatagaaaag aaagagaaag nngagagaga    540
gaaaaacatt gcctgtggca gggcggggaa ggtgaaatga tcagggaggc agagaaagaa    600
ccacccattg cagcgacact aaaaagttca ggtggctgct gtcggtggag caaggatctt    660
ttccagtaat cctaccagct ctcaaatttc ccttgttagg gaggaaaaag ctccccatgt    720
cccaggatcc tgtacattcc taattctgtc acccatagcc atcagcaaag tacaagggag    780
attaatccaa agagaatagc agttaacatc ccatagtgcc gnaacctgtt cttagccgag    840
agggacttta ccgaagaggg gcctctaacc cgctaaatct tagaagggac tctaactctc    900
ctaagtcggg cctctaacca gaggtcagtc aagcatcctt gccttttatt aagggggagc    960
ctttaaccac ctctatctta ggagagactc taactccact a                       1001
```

<210> 12
<211> 1001
<212> DNA
<213> Homo Sapiens

<220>
<221> allele
<222> 501
<223> 12-447-58 : polymorphic base G or C

<220>
<221> misc_binding
<222> 482..500
<223> 12-447-58.mis1

<220>
<221> misc_binding
<222> 502..521
<223> 12-447-58.mis2, potential complement

<220>
<221> primer_bind
<222> 444..462
<223> upstream amplification primer

<220>
<221> primer_bind -
<222> 874..893
<223> downstream amplification primer, complement

<220>
<221> misc_binding
<222> 489..513
<223> 12-447-58 potential probe

<220>
<221> misc_feature
<222> 44,894
<223> n=a, g, c or t

<400> 12

```
tgggctgtag ttttctgact cctgatctat gtaatctcca attngcgcaa catcagtttg     60
tctctctctc tctcttttt tttttttttt tcagacaggg tctcactctg tggcccaggc     120
tggagtgcag tggcatgatc ttagcttatt gcagctttga tctcccaggc tcaaacgatc    180
ctcctgcctc agcctcctga gtagctggga ctacaggtgg gtgccactgg gcccagatag    240
tttttatttt tttgtagaga tagagttttg ccatgttgcc taggctggtt tcaaactcct    300
ggactcaagt tatctgccca ccttggcctc ccaaggtgct gggattacaa acgttagcca    360
ctgcccccag ctgtctccat tttttaaatt aaaatacata atgttctagt aaatattctt    420
gtgggcaatt cattggacct gtcttagatt acatgcccag agtaaattct agaaatgaaa    480
ttcctggtct tctccgaaga sggaattgag tggggaaaaa aaagaaaatg aaaaaagaaa    540
taaacacaag atattcttgg tcaaattgtg tctgcccttt ctcctccagt gagctacgat    600
gaagccacag taatccacat tcccagggga acacaggtgg tctgcctctc catgtgctca    660
gagtgccaat tcttggtaag gagatatagg gactggagtg aaacattgga ataatgactc    720
atattcatgt tttataaaca aatttaaaat attggatcag gttatttatc tggaaaccct    780
gaagtttggc tgtttaaagg gctattttaa atataggcaa cagaaaacaa aatacaaagt    840
cttatttctg acactttaaa atgttggcaa agtgtcttcc ttgttagtat ggtnggtgag    900
tatccccacc tgtcataaaa tggcaaatag gctgggtgca gtggctcatg cctgtaatcc    960
cagcactttg ggaggtcaag ttgggaggat catttgagtc c                        1001
```

<210> 13
<211> 1001
<212> DNA

<213> Homo Sapiens

<220>
<221> allele
<222> 501
<223> 12-453-429 : polymorphic base C or T

<220>
<221> misc_binding
<222> 481..500
<223> 12-453-429.mis1, potential

<220>
<221> misc_binding
<222> 502..520
<223> 12-453-429.mis2, complement

<220>
<221> primer_bind
<222> 73..91
<223> upstream amplification primer

<220>
<221> primer_bind
<222> 577..596
<223> downstream amplification primer, complement

<220>
<221> misc_binding
<222> 489..513
<223> 12-453-429 potential probe

<400> 13

```
aatcatgatt taccccctgga tgggaatcag ggtcaccttc aaagagcata tggacatgtg     60
aaggagcata aacacaatca caaaattggg gctcccacca tcggttgcag aggcagtcgg    120
cagtgtctgc tacaagtgac atcaagtagc atctatcaag tggcattact ttaacaaata    180

ttaacaatag ggtcctcatc tcagtttaga agaaaaatcc aaaatcaaag tagactgaag    240
ataaggtaaa cggtgatgtt tgccacacta gtggtgaggt tttcatagtt ttacccaaac    300
cagtttttat ttttagtgct tggttggggt cactgaaact tccaggttgt cctaacctgt    360
taaaatcttt taaaacatta tttgtgactg tttctctttg gacagtgctt tttgttaatc    420
acaaaaaaca gggtagaagg taataacaca tgcaaaggga tgtgtagcaa tcaaagggaa    480
atgttatcct tcctaggaga yaggatttat gggattcctg gatctttttt gtgtctgatt    540
gttaaggtct tgacagcctt tcaattgagg aatacggtcg tgtctgttaa actaattgta    600
gctgttcact ccactatata attttacaac atttcagctt caaatagaat ggcttcaata    660
aacataaagc agactgacac atcccagatt tctgcagttt attttacacc aagaaattac    720
atttagagat ttccagacac atactataaa tgaaaattca acaggaagtt tctttcattt    780
cctctagatc tgacctatat ttcaaaaata tatgagtggg ctataaccag gaacttcttc    840
tcttgctccc attattcaca ccgttttctg aagagttttt ttctcgtccg cgactctaat    900
ggccattaag ggaattgcag acattggaag actatacaac cctaagtgaa atgagtgaaa    960
tgataaatgt tatccacaag atggccaaat tgtatggttc t                       1001
```

<210> 14
<211> 929
<212> DNA

<213> Homo Sapiens

<220>
<221> allele
<222> 501
<223> 12-454-363 : polymorphic base A or G

<220>
<221> misc_binding
<222> 481..500
<223> 12-454-363.mis1, potential

<220>
<221> misc_binding
<222> 502..521
<223> 12-454-363.mis2, potential complement

<220>
<221> primer_bind
<222> 139..158
<223> upstream amplification primer

<220>
<221> primer_bind
<222> 634..652
<223> downstream amplification primer, complement

<220>
<221> misc_binding
<222> 489..513
<223> 12-454-363 potential probe

<220>
<221> misc_feature
<222> 674..679,881..882,892..893
<223> n=a, g, c or t

<400> 14

```
atcccagatg attactaatt taaaactact ttacatttta tttagtaatg tgtttggaac     60
agttacattt tgtagctact tacaatttta ctttaactga aataataaga caaggccata    120
aatattggcc tccctggctg gggagttgtg tgggtttcct ttccaggcag ttgaggtttt    180
ccatggagcc tactctacag tgtcagcagg agtataacat agaaggcttc caggtcaggt    240
ggcccagagt caaatcaaag ccccatccct ttttagcaaa tttctcagcc ttacttagct    300
gtgccggcct gcctctttct aaaatgagga aaataataat acccacttca ctggtttatt    360
gagaggatta aatgaggaca tgtgttattt cacaccatta ttgcttctgt tgttattatt    420

ttaaaatcta ggttggtgat tgcatcagtt tcttagggct gctctaaaga aagtaccaca    480
agctgagtga cttacatagc rgaaaagtgt tttcttacag gttaagaggc tggaagtctg    540
aaatcaaggt gtcagcaggg ccatgctccc actgaaaccc atagcgggga atcctttctt    600
gcttctaagt ttctagtgtt tccttggctt gtagatgtgt cacttcagtc acacggccat    660
cttcttcttt cttnnnnnnc ttcttcttct tcttcttctt cttcttcttc ttcttcttct    720
tcttcttctt cttcttcctt cttcttcttc ttcttcttct tcttcttctt cttcttcttc    780
ttcttcttct tcttcttctt cttcttcttc ttcttcttct tcttcttctt cttcttcttc    840
ttcttcttct tcttcttctt cttcttcttc tttgagatgg nntcgctgct gnngctcagg    900
ctggagtgca atggcgtgat cttggctca                                      929
```

<210> 15
<211> 827
<212> DNA
<213> Homo Sapiens

<220>
<221> allele
<222> 501
<223> 12-455-326 : polymorphic base T or C

<220>
<221> misc_binding
<222> 502..520
<223> 12-455-326.mis1, complement

<220>
<221> misc_binding
<222> 481..500
<223> 12-455-326.mis2, potential

<220>
<221> primer_bind
<222> 808..826
<223> upstream amplification primer, complement

<220>
<221> primer_bind
<222> 372..392
<223> downstream amplification primer

<220>
<221> misc_binding
<222> 489..513
<223> 12-455-326 potential probe

<220>
<221> misc_feature
<222> 798..799
<223> n=a, g, c or t

<400> 15

```
ttcataagcc gtaatatttc tagcttgaag aaaaaaataa taattcagag aactctgttt     60
cttagaggaa attgacagga agaggaattg agaagaattc tatagaatgg gtaatgaaca    120
atggatcagg aacaatagca gaataataag gcctgcagaa ctacctggaa acctgaactc    180
tgggtcacac acagggtgca aacttaagaa aactccccac agcttttaaa acagctcagg    240
aaatctgtga gttaaaggaa agcctaacac aaatacagct ttaccttaag catctttgtc    300
caccctcgcc ctataattcc tcagctttga gagaaagcct tccctgcaga ctgctactgt    360
agatttcaat ttgttttgtt tttagagatg cccatacctg acttagcatt atcctaggga    420
aaggcctgag gctgtccaca agagatcccc aggcggcttc tgtgagggtc tgcacagaga    480
atcttgcaca gcaggctctt ycttatagga ggtgtgaggt tgtcatataa agctgaaggc    540
ccaacataag tctgcctcac agttatcctt ctagaatatt cctgataggg tcagaatttt    600
atgaattttt tcttaaagaa atagagacgg ggtcgcacta tgttgaccag gctggtctcg    660
aacttctggc ctcaagcgat cctcccatct cagcctccca aagtgctagg attacagatg    720

tgagccacca cgccctgcca gaattttgca aatttactaa gcatccacca ccgtgtatcc    780
tgtgtaggac acgggccnng ggaggtccct attaaccaag atagcag                  827
```

<210> 16
<211> 884
<212> DNA
<213> Homo Sapiens

<220>
<221> allele
<222> 501
<223> 12-455-383 : polymorphic base G or A

<220>
<221> misc_binding
<222> 481..500
<223> 12-455-383.mis1, potential

<220>
<221> misc_binding
<222> 502..521
<223> 12-455-383.mis2, potential complement

<220>
<221> primer_bind
<222> 865..883
<223> upstream amplification primer, complement

<220>
<221> primer_bind
<222> 429..449
<223> downstream amplification primer

<220>
<221> misc_binding
<222> 489..513
<223> 12-455-383 potential probe

<220>
<221> misc_feature
<222> 855..856
<223> n=a, g, c or t

<400> 16

```
tgatttctgg tacaggggtc tgtgtaatca gcacatggcg gggactgtct cacatgcttc     60
ataagccgta atatttctag cttgaagaaa aaaataataa ttcagagaac tctgtttctt    120
agaggaaatt gacaggaaga ggaattgaga agaattctat agaatgggta atgaacaatg    180
gatcaggaac aatagcagaa taataaggcc tgcagaacta cctggaaacc tgaactctgg    240
gtcacacaca gggtgcaaac ttaagaaaac tccccacagc ttttaaaaca gctcaggaaa    300
tctgtgagtt aaaggaaagc ctaacacaaa tacagcttta ccttaagcat ctttgtccac    360
cctcgcccta taattcctca gctttgagag aaagcctccc ctgcagactg ctactgtaga    420
tttcaatttg ttttgttttt agagatgccc atacctgact tagcattatc ctagggaaag    480
gcctgaggct gtccacaaga ratccccagg cggcttctgt gagggtctgc acagagaatc    540
ttgcacagca ggctctttct tataggaggt gtgaggttgt catataaagc tgaaggccca    600
acataagtct gcctcacagt tatccttcta gaatattcct gataggtca gaattttatg    660
aatttttct taaagaaata gagacggggt cgcactatgt tgaccaggct ggtctcgaac    720
ttctggcctc aagcgatcct cccatctcag cctcccaaag tgctaggatt acagatgtga    780
gccaccacgc cctgccagaa ttttgcaaat ttactaagca tccaccaccg tgtatcctgt    840
gtaggacacg ggccnnggga ggtccctatt aaccaagata gcag                      884
```

<210> 17
<211> 1001
<212> DNA
<213> Homo Sapiens

<220>
<221> allele
<222> 501
<223> 12-456-269 : polymorphic base A or G

<220>
<221> misc_binding
<222> 481..500
<223> 12-456-269.mis1, potential

<220>
<221> misc_binding
<222> 502..521
<223> 12-456-269.mis2, potential complement

<220>
<221> primer_bind
<222> 233..252
<223> upstream amplification primer

<220>
<221> primer_bind
<222> 693..712
<223> downstream amplification primer, complement

<220>
<221> misc_binding
<222> 489..513
<223> 12-456-269 potential probe

<220>
<221> misc_feature
<222> 20,447
<223> n=a, g, c or t

<400> 17

```
catttcata gcttttttan tttattaaat atcaccattg ggttgactca gtagcatctc 60
atatacctac ctgatgggct gcagcagata ttggcactca gacaagccaa ttttaggcat 120
cagagccctg tagccgtggt aacctcaccc ctgtgctgga cgggaggcag gtcggctaag 180
cagaggtgct ggaagtgtgt gtggtaccag gactgggccg caggagctgc acagcctcac 240
agcacattag ccaatggccc catgccaacc cccttggctg ggttttctgg aacaggtacc 300
tccttaccat tccaagaaaa agtctcctcc tgccccccaa aagtgggggg caggtgatcc 360
aaaagatcac taagcaaatg cctgtttgtc ctttggaata ctcaaccctg atcagtggtg 420
aatactcagt caccaactat gtggaangag gcacaaccat ggcagacaac agatatggaa 480
agagcaggaa ttcttctagc rggaattctt atatcaaatg caatggtggc catgcccccca 540
acagctctgc agttggaaaa atgaagcctt caagtaaggc caaagtccta ctgtttgagg 600
gcaattcatc tgggccaaat ccctcaggag cagggccatg cgggggttac aactgttcat 660
gcaacacaac agattgggct taagctggcc agggcagtga cgggcttttc atcttaattg 720
tagttgtctg tgtcccaaga aaatgttatc agcttttcag aaaaactttc aagagactcg 780
agcaagtcaa acccaagctg ccctccttgt ctgcatatcc tgctctgttt tcccatttgg 840
gaaatgaaac cagcagcagc tgagagggag aaggttctac actcactggt tttctccact 900
cctaccactc cctccttccc cctcctctct gtcaccctga agaaatctcc ttctggaata 960
aggtgtcatt catccttgtc acctccagta tagttatgtc a 1001
```

<210> 18
<211> 1001
<212> DNA
<213> Homo Sapiens

<220>
<221> allele
<222> 501
<223> 12-456-380 : polymorphic base G or T

<220>
<221> misc_binding
<222> 481..500
<223> 12-456-380.mis1, potential

<220>
<221> misc_binding
<222> 502..521
<223> 12-456-380.mis2, potential complement

<220>
<221> primer_bind
<222> 122..141
<223> upstream amplification primer

<220>
<221> primer_bind
<222> 582..601
<223> downstream amplification primer, complement

<220>
<221> misc_binding
<222> 489..513
<223> 12-456-380 potential probe

<220>
<221> misc_feature
<222> 336
<223> n=a, g, c or t

<400> 18

```
tttaggcatc agagccctgt agccgtggta acctcacccc tgtgctggac gggaggcagg      60
tcggctaagc agaggtgctg gaagtgtgtg tggtaccagg actgggccgc aggagctgca     120
cagcctcaca gcacattagc caatggcccc atgccaaccc ccttggctgg gttttctgga     180
acaggtacct ccttaccatt ccaagaaaaa gtctcctcct gccccccaaa agtgggggc      240
aggtgatcca aaagatcact aagcaaatgc ctgtttgtcc tttggaatac tcaaccctga     300
tcagtggtga atactcagtc accaactatg tggaangagg cacaaccatg gcagacaaca     360
gatatggaaa gagcaggaat tcttctagca ggaattctta tatcaaatgc aatggtggcc     420
atgcccccaa cagctctgca gttggaaaaa tgaagccttc aagtaaggcc aaagtcctac     480
tgtttgaggg caattcatct kggccaaatc cctcaggagc agggccatgc gggggttaca     540
actgttcatg caacacaaca gattgggctt aagctggcca gggcagtgac gggcttttca     600
tcttaattgt agttgtctgt gtcccaagaa aatgttatca gcttttcaga aaaactttca     660
agagactcga gcaagtcaaa cccaagctgc cctccttgtc tgcatatcct gctctgtttt     720
cccatttggg aaatgaaacc agcagcagct gagagggaga aggttctaca ctcactggtt     780
ttctccactc ctaccactcc ctccttcccc ctcctctctg tcaccctgaa gaaatctcct     840
tctggaataa ggtgtcattc atccttgtca cctccagtat agttatgtca gatgcagtga     900
ggtgctggat ttagattaca agaagcggga ggattcttgc aaagagagga caacatttgc     960
ttaactatgc caagggtccc cttgtccccc gaccaagagg g                        1001
```

<210> 19
<211> 1001
<212> DNA
<213> Homo Sapiens

<220>
<221> allele
<222> 501
<223> 12-457-204 : polymorphic base A or G

<220>
<221> misc_binding
<222> 481..500
<223> 12-457-204.mis1, potential

<220>
<221> misc_binding
<222> 502..521
<223> 12-457-204.mis2, potential complement

<220>
<221> primer_bind
<222> 298..317
<223> upstream amplification primer

<220>
<221> primer_bind
<222> 772..792
<223> downstream amplification primer, complement

<220>
<221> misc_binding
<222> 489..513
<223> 12-457-204 potential probe

<400> 19

```
ttgagggcaa ttcatctggg ccaaatccct caggagcagg gccatgcggg ggttacaact      60
gttcatgcaa cacaacagat tgggcttaag ctggccaggg cagtgacggg cttttcatct     120
taattgtagt tgtctgtgtc ccaagaaaat gttatcagct tttcagaaaa actttcaaga     180
gactcgagca agtcaaaccc aagctgccct ccttgtctgc atatcctgct ctgttttccc     240
atttgggaaa tgaaaccagc agcagctgag agggagaagg ttctacactc actggttttc     300
tccactccta ccactccctc cttccccctc ctctctgtca ccctgaagaa atctccttct     360
ggaataaggt gtcattcatc cttgtcacct ccagtatagt tatgtcagat gcagtgaggt     420
gctggattta gattacaaga agcgggagga ttcttgcaaa gagaggacaa catttgctta     480
actatgccaa gggtcccctt rtcccccgac caagagggcc ttcatgctcc ctcagcttga     540
ctgaacttta ggcaggattc ttcctaacct cccttttttt tttagagcat ttcttgtcat     600
tgtacatttt ttctctgccc ctttgagatg tatgtaaatc tcttcaaaag cctcctgcca     660
gttttacaat ccaggaatgt ctttctcaag gacctgggag ccctcccttt gaaatgtaat     720
catcaaagga gatggcaccc ctgtctccca gtctttgtgg agagtttttg tgggagtcta     780
acttcagcag gacaccttcc tccaagtcat aaaaccatcc cttgtcaaaa gatagaagtt     840
tattagcaaa cacaaatggc ctacgacctc ttcacaacat cctctggcat gtttcactag     900
cctttaaaag cctttaaaaa ctttgcaccc tctgcatcag cagcattgag ttcacactga     960
gttctagtcc ctctctcctg ttgcaataac ttcggataaa c                        1001
```

<210> 20
<211> 1001
<212> DNA
<213> Homo Sapiens

<220>
<221> allele
<222> 501
<223> 12-457-206 : polymorphic base C or T

<220>
<221> misc_binding
<222> 481..500
<223> 12-457-206.mis1, potential

<220>
<221> misc_binding
<222> 502..521
<223> 12-457-206.mis2, potential complement

<220>
<221> primer_bind
<222> 296..315
<223> upstream amplification primer

<220>
<221> primer_bind
<222> 770..790
<223> downstream amplification primer, complement

<220>
<221> misc_binding
<222> 489..513
<223> 12-457-206 potential probe

<400> 20

```
gagggcaatt catctgggcc aaatccctca ggagcagggc catgcggggg ttacaactgt     60
tcatgcaaca caacagattg ggcttaagct ggccagggca gtgacgggct tttcatctta    120
attgtagttg tctgtgtccc aagaaaatgt tatcagcttt tcagaaaaac tttcaagaga    180
ctcgagcaag tcaaacccaa gctgccctcc ttgtctgcat atcctgctct gttttcccat    240
ttgggaaatg aaaccagcag cagctgagag ggagaaggtt ctacactcac tggttttctc    300
cactcctacc actccctcct tccccctcct ctctgtcacc ctgaagaaat ctccttctgg    360
aataaggtgt cattcatcct tgtcacctcc agtatagtta tgtcagatgc agtgaggtgc    420
tggatttaga ttacaagaag cgggaggatt cttgcaaaga gaggacaaca tttgcttaac    480
tatgccaagg gtccccttgt yccccgacca agagggcctt catgctccct cagcttgact    540
gaactttagg caggattctt cctaacctcc cttttttttt tagagcattt cttgtcattg    600
tacatttttt ctctgcccct ttgagatgta tgtaaatctc ttcaaaagcc tcctgccagt    660
tttacaatcc aggaatgtct ttctcaagga cctgggagcc ctccctttga aatgtaatca    720
tcaaaggaga tggcacccct gtctcccagt ctttgtggag agtttttgtg ggagtctaac    780
ttcagcagga caccttcctc caagtcataa aaccatccct tgtcaaaaga tagaagttta    840
ttagcaaaca caaatggcct acgacctctt cacaacatcc tctggcatgt ttcactagcc    900
tttaaaagcc tttaaaaact ttgcaccctc tgcatcagca gcattgagtt cacactgagt    960
tctagtccct ctctcctgtt gcaataactt cggataaact t                        1001
```

<210> 21
<211> 1001
<212> DNA
<213> Homo Sapiens

<220>
<221> allele
<222> 501
<223> 12-458-196 : polymorphic base T or A

<220>
<221> misc_binding
<222> 481..500
<223> 12-458-196.mis1, potential

<220>
<221> misc_binding
<222> 502..521
<223> 12-458-196.mis2, potential complement

<220>
<221> primer_bind
<222> 679..696
<223> upstream amplification primer, complement

<220>
<221> primer_bind
<222> 200..217
<223> downstream amplification primer

<220>
<221> misc_binding
<222> 489..513
<223> 12-458-196 potential probe

<220>
<221> misc_feature
<222> 33,792
<223> n=a, g, c or t

<400> 21

```
ctgctgcaac cactacaaga caggaagaag ggnaagggaa gggaagctga gtgctcaggg      60
catgctgtgg agggaaggcg agggcacttc ccgccgtcat ctccagaacc gccacagcct     120
tgggaggggg gctgagagag atcagtgctg actgcctgag aagaggcctt ccacaagctg     180
cctttctggt gtccccatgt tcttccttcc ttccttccag aggccgtgag agggagggcc     240
cgtgtcagcc tgctctgccg gtttcccgac tgctgaacct gtctgtggga agctgggttt     300
gactatgtga tgctgaggct ctgagaggag ggctaccctt ttccccaccc tcggccccca     360
ttcctatgag tagaagcctg cccagctgcg gtggtggggt ttgaggaggc taggacttag     420
agcagggaaa aacaaggaag cagaatgaaa aacagaaaca cttagaaatc tgggattggt     480
acttctcctt atgtaaagat wgtggctgac gaccagcttc ttagaggaag caagtctagg     540
agaagggctc atacctggtc cgtgagggcc tatgggggat cagggcataa gaccacctga     600
ccctgggcct tgcatccaca agaaccttga gtttagattt ttagacatat tctttgaaca     660
aggttttaca tatatagtca ctgagaaatc ctgagaggat tgaaaaatag catctatttt     720
acagctttaa attgtgccct gagctgagcg tggtggctca tgcctgtaat cccagcactt     780
tgggaggctg anggtgggag gattgcttga gttcagttca ggaccagcct gggcaacagc     840
aagacctcat ctctactaaa aataaaaaaa ttagctgggc atggtggtgt gcacctgtag     900
tcccaattac tcaggaggct gaggtgggag gattgcttga gcctgggagg ttgaggctgc     960
agtgagctgt gattgtgcca ctgcactcca gcttgggtga c                        1001
```

<210> 22
<211> 1001
<212> DNA
<213> Homo Sapiens

<220>
<221> allele
<222> 501
<223> 12-458-438 : polymorphic base T or C

<220>
<221> misc_binding
<222> 481..500
<223> 12-458-438.mis1, potential

<220>
<221> misc_binding
<222> 502..521
<223> 12-458-438.mis2, potential complement

<220>
<221> primer_bind
<222> 921..938
<223> upstream amplification primer, complement

<220>
<221> primer_bind
<222> 442..459
<223> downstream amplification primer

<220>
<221> misc_binding
<222> 489..513
<223> 12-458-438 potential probe

<220>
<221> misc_feature
<222> 275
<223> n=a, g, c or t

<400> 22

```
aaaataggga gggggagcag tctcagctgt cttcagctgc tagggagatt tttttccccc      60
tcctgagcta ctgtttcccc caacccgagc ctttctctct tattgtaccc accctttctg     120
atgaagtcat caaagcaaag attgcataac tgatgcatag gcctatcttg tgttatactg     180
ggagacaggc caatgtttcc attaatagac aagagcacca ccacgctgcc aaatggagct     240
ctctgctgca accactacaa gacaggaaga agggnaaggg aagggaagct gagtgctcag     300
ggcatgctgt ggagggaagg cgagggcact tcccgccgtc atctccagaa ccgccacagc     360
cttgggaggg gggctgagag agatcagtgc tgactgcctg agaagaggcc ttccacaagc     420
tgcctttctg gtgtccccat gttcttcctt ccttccttcc agaggccgtg agagggaggg     480
cccgtgtcag cctgctctgc yggtttcccg actgctgaac ctgtctgtgg gaagctgggt     540
ttgactatgt gatgctgagg ctctgagagg agggctaccc ttttccccac cctcggcccc     600
cattcctatg agtagaagcc tgcccagctg cggtggtggg gtttgaggag gctaggactt     660
agagcaggga aaaacaagga agcagaatga aaaacagaaa cacttagaaa tctgggattg     720
gtacttctcc ttatgtaaag attgtggctg acgaccagct tcttagagga agcaagtcta     780
ggagaagggc tcatacctgg tccgtgaggg cctatggggg atcagggcat aagaccacct     840
gaccctgggc cttgcatcca caagaacctt gagtttagat ttttagacat attctttgaa     900
caaggtttta catatatagt cactgagaaa tcctgagagg attgaaaaat agcatctatt     960
ttacagcttt aaattgtgcc ctgagctgag cgtggtggct c                         1001
```

<210> 23
<211> 1001
<212> DNA
<213> Homo Sapiens

<220>
<221> allele
<222> 501
<223> 12-460-274 : polymorphic base A or G

<220>
<221> misc_binding
<222> 481..500
<223> 12-460-274.mis1, potential

<220>
<221> misc_binding
<222> 502..521
<223> 12-460-274.mis2, potential complement

<220>
<221> primer_bind
<222> 228..245
<223> upstream amplification primer

<220>
<221> primer_bind
<222> 760..777
<223> downstream amplification primer, complement

<220>
<221> misc_binding
<222> 489..513
<223> 12-460-274 potential probe

<220>
<221> misc_feature
<222> 873
<223> n=a, g, c or t

<400> 23

```
gacataattg ccctctgcct ccttaggaaa acaccagacc tctgagaagg aggtgattaa     60
accgttcagt cacttgacaa agataatcta attaattaat gaaaggattt aacacccatg    120
tctggagcat ttgcaatgtg ctagacaccc ttctgcatat gccgtgacag ctctgacatg    180
acaaacaagg cctgtggaga aacgcagact aggtaacaga ataaacaaga atgtgtaagg    240
tggtgacaaa tgctatgatg aaaattaaca aagggaagta ttaaaccaga gaagtattca    300
atagaagtgt ggctgggtag ctacccttgg ttgggtggcc agggaagaca tctttgagga    360
agtgactttt atgctgagac caaaaaccag ctaagagaag ataaggggag gggaatccca    420
ggtaaggagg aactgtgaat gcaaaggtcc cgaggtagga cagaacaaaa tgcaaaggcc    480
ctgaggtagg atagaacaaa rcagtctcgg cctcccaaag tgctaggatt acaggcatga    540
gccaccactc ctggctacac tggaaggctt taagcagggc aaagccttat gtgatctgac    600
ttgaaaggcc actctcctac ccatgaagaa caggcaagag tagaggcaga agcacaaatg    660
aattagatga cagcagggat gctggtggcc actatgtagt gtacactcaa ccgtgtgcct    720
ggagctcctt aaagcccttt atctgcatta tccattaatc cacacaacct ctctatagag    780
taatttctag aatttccata ttacatatac agaaaataaa gtctagaaaa gtaatgtgcc    840
tccagctggt aaggtttttt tttttttaac aancctcttt ttataactat ataagtcaat    900
aatagtcatc ataagaaaca tttttaatg agcaaaaaga aaaaattaaa tcacttgaga     960
tcacatcatc ctgtgataat tactattaac attttggctt a                       1001
```

<210> 24
<211> 1001
<212> DNA
<213> Homo Sapiens

<220>
<221> allele
<222> 501
<223> 12-461-124 : polymorphic base A or C

<220>
<221> misc_binding
<222> 481..500
<223> 12-461-124.mis1, potential

<220>
<221> misc_binding
<222> 502..521
<223> 12-461-124.mis2, potential complement

<220>
<221> primer_bind
<222> 378..396
<223> upstream amplification primer

<220>
<221> primer_bind
<222> 911..928
<223> downstream amplification primer, complement

<220>
<221> misc_binding
<222> 489..513
<223> 12-461-124 potential probe

<220>
<221> misc_feature
<222> 850
<223> n=a, g, c or t

<400> 24

```
aatggaaatt tatttaaatc tcctgtgact attaggctct gagtgtttaa aaattttact     60
ctggattaag ttatcatgac aattagtagt agtagatcat tggaaaaact aacataagta    120
tatatacatt ttgtgaaatc attattttaa aaagtaaaat tttattgaaa atatatctta    180
ataagatagg actagttttt cttccaaata cttcttggtg tgtttttttg tttgtttgct    240
ttttggttaa tatttctaat tactagaatg agacaggttt ctatatgaat tctattccta    300
cttttatttt ttatagacac aagcaaggaa tattgttgac ataagtagaa atgaatcttg    360
taattgcaat tttacttaat tttctgaatg ccttccaagt tctcaatcaa caatcacagt    420
gatctgacat ccaaaattat ttttaaaatg ttgatactac cagctaaaaa cttgatgaga    480
ttctccttta atacagttga magcagagaa ttagaaacca cctggcttgc aaaaaggtct    540
gttacccaaa catttgagtc attgtctatt tcactattaa catcattatt tcaaattctc    600
ctttggttgc tgcactggag gttttgcttt atttgtttgc tggtttgggg tttttgttgt    660
tgttgttgtt ttctgtcttt ttaactatat aggacagggg ttggcaaact aaggccagtt    720
tgtactgatc aaatcttgcc tagcttgctt tcatgcagcc ctcaggttaa gaatggtttt    780
catatttttg gtttgttttt ttttttaaaa ggaagtacta cttcttattg atcaaataaa    840
ttctgctttn cttattatca ttattttgta tttgctcaag gaaagctact taaagactaa    900
gtaaagaaca gagttgggag aggtaaagta ccaacttctt gaccacatgt aaggagttct    960
tagactggcc ttcctgtgtg tatacttagg tctcaattca c                       1001
```

<210> 25
<211> 1001
<212> DNA
<213> Homo Sapiens

<220>
<221> allele
<222> 501
<223> 12-461-299 : polymorphic base C or T

<220>
<221> misc_binding
<222> 481..500
<223> 12-461-299.mis1, potential

<220>
<221> misc_binding
<222> 502..520
<223> 12-461-299.mis2, complement

<220>
<221> primer_bind
<222> 203..221
<223> upstream amplification primer

<220>
<221> primer_bind
<222> 736..753
<223> downstream amplification primer, complement

<220>
<221> misc_binding
<222> 489..513
<223> 12-461-299 potential probe

<220>
<221> misc_feature
<222> 675,831,997

<223> n=a, g, c or t

<400> 25

```
tcttaataag ataggactag tttttcttcc aaatacttct tggtgtgttt ttttgtttgt     60
ttgcttttttg gttaatattt ctaattacta gaatgagaca ggtttctata tgaattctat    120
tcctactttt atttttatta gacacaagca aggaatattg ttgacataag tagaaatgaa    180
tcttgtaatt gcaattttac ttaattttct gaatgccttc caagttctca atcaacaatc    240
```

```
acagtgatct gacatccaaa attattttta aaatgttgat actaccagct aaaaacttga    300
tgagattctc ctttaataca gttgaaagca gagaattaga aaccacctgg cttgcaaaaa    360
ggtctgttac ccaaacattt gagtcattgt ctatttcact attaacatca ttatttcaaa    420
ttctcctttg gttgctgcac tggaggtttt gctttatttg tttgctggtt tggggttttt    480
gttgttgttg ttgttttctg ycttttttaac tatataggac aggggttggc aaactaaggc    540
cagtttgtac tgatcaaatc ttgcctagct tgctttcatg cagccctcag gttaagaatg    600
gttttcatat ttttggtttg tttttttttt taaaaggaag tactacttct tattgatcaa    660
ataaattctg ctttncttat tatcattatt ttgtatttgc tcaaggaaag ctacttaaag    720
actaagtaaa gaacagagtt gggagaggta aagtaccaac ttcttgacca catgtaagga    780
gttcttagac tggccttcct gtgtgtatac ttaggtctca attcaccacc natgataagg    840
cctgctttgg catatactct aaactcaggg atgatctcaa aattatatct tctttttttt    900
ttttttttgag acggagtctc actctgtcac ccaggctgga gtgcagtggt gtgatctcag    960
ctcactgcaa gctcccccctc ccgggttcat gccattnctc c                        1001
```

<210> 26
<211> 985
<212> DNA
<213> Homo Sapiens

<220>
<221> allele
<222> 501
<223> 12-461-465 : polymorphic base C or T

<220>
<221> misc_binding
<222> 481..500
<223> 12-461-465.mis1, potential

<220>
<221> misc_binding
<222> 502..521
<223> 12-461-465.mis2, potential complement

<220>
<221> primer_bind
<222> 37..55
<223> upstream amplification primer

<220>
<221> primer_bind
<222> 570..587
<223> downstream amplification primer, complement

<220>
<221> misc_binding
<222> 489..513
<223> 12-461-465 potential probe

<220>
<221> misc_feature
<222> 509,665,831
<223> n=a, g, c or t

<400> 26

```
taagtagaaa tgaatcttgt aattgcaatt ttacttaatt ttctgaatgc cttccaagtt      60
ctcaatcaac aatcacagtg atctgacatc caaaattatt tttaaaatgt tgatactacc     120
agctaaaac ttgatgagat tctcctttaa tacagttgaa agcagagaat tagaaaccac      180
ctggcttgca aaaaggtctg ttacccaaac atttgagtca ttgtctattt cactattaac     240
atcattattt caaattctcc tttggttgct gcactggagg ttttgcttta tttgtttgct     300
ggtttggggt ttttgttgtt gttgttgttt tctgtctttt taactatata ggacaggggt     360
tggcaaacta aggccagttt gtactgatca aatcttgcct agcttgcttt catgcagccc     420
tcaggttaag aatggttttc atatttttgg tttgtttttt tttttaaaag gaagtactac     480

ttcttattga tcaaataaat yctgctttnc ttattatcat tattttgtat ttgctcaagg     540
aaagctactt aaagactaag taaagaacag agttgggaga ggtaaagtac caacttcttg     600
accacatgta aggagttctt agactggcct tcctgtgtgt atacttaggt ctcaattcac     660
caccnatgat aaggcctgct ttggcatata ctctaaactc agggatgatc tcaaaattat     720
atcttctttt tttttttttt tgagacggag tctcactctg tcacccaggc tggagtgcag     780
tggtgtgatc tcagctcact gcaagctccc cctcccgggt tcatgccatt nctcctacct     840
cagcctcccg agtagctggg actacaggca cctgccacca tacctggcta atttttttgt     900
atttttagta gagacggggt ttcactgtgt tagccaggat ggtctcgatc tcctgacctc     960
gtgatccgcc cgcctcggcc tccca                                           985
```

<210> 27
<211> 1001
<212> DNA
<213> Homo Sapiens

<220>
<221> allele
<222> 501
<223> 12-462-280 : polymorphic base C or T

<220>
<221> misc_binding
<222> 481..500
<223> 12-462-280.mis1, potential

<220>
<221> misc_binding
<222> 502..521
<223> 12-462-280.mis2, potential complement

<220>
<221> primer_bind
<222> 222..241
<223> upstream amplification primer

<220>
<221> primer_bind
<222> 655..675
<223> downstream amplification primer, complement

<220>

<221> misc_binding
<222> 489..513
<223> 12-462-280 potential probe

<220>
<221> misc_feature
<222> 174
<223> n=a, g, c or t

<400> 27

```
agggttgctc aggccagtga tgtttgcaat gcaaacccaa gaattctgaa aggagcccat     60
ggctgaaagg agccaattct tccaggacgg actactccag ttgcaactgt gctgtggagg    120
ctgaggagca tccatgactg aggaagggcg agtcccctat aatgcccttg ctangggag     180
catcagacat ccctagactt acaagtccct cgtgtaataa ggtgaactat ggaaagctat    240
gatttgagga aagttccctc tgattgaaag agttcttcaa atatgaagaa tgtaacactt    300
atggggcacc tactcattgc aaaacactct actaggggct ttcacctgta tgtttaactc    360
attttgttga tttttttttt tttaagccca aatttctgag aggttaagga acttgtctgg    420
tggtggcgtc tctgggattc tgacttttcc actgaccata acatgcatct ggtctaaatt    480
gagcggacca cccaggagga ygttggaata aaaagaaatg ggaagggggt ggggagagga    540
atgggcatag aataaaaagg tagaaaccct tgggatcaaa ggatgcaact gaaagagcac    600
caaaatatgc atcatttttt ttaatatgtt gtctggtacc caaaattggg atgcctctgt    660
ggtatcaact ttgacattta aagaaagggc ataagattag atcattcaac cataagtgga    720

aggaagaaac aaaataaaat aaaaccttac aattcccttt gaagttgatc aggtgtcaag    780
gcagtgattt ggttttcttt tacaaacttt ctttttccag aaaaattttt actgtgggtt    840
tggagaggtg tttacaataa attcatagca gagctccaaa agggcccacc ccatctaata    900
tcctctgtct cagcagatta ttgaagaaga ttggattaaa aggtttgttt tgtgatgtgt    960
gatggttgtg tccccaagat gattgagttt gaggcagagt t                       1001
```

<210> 28
<211> 1001
<212> DNA
<213> Homo Sapiens

<220>
<221> allele
<222> 501
<223> 12-464-66 : polymorphic base G or T

<220>
<221> misc_binding
<222> 481..500
<223> 12-464-66.mis1, potential

<220>
<221> misc_binding
<222> 502..521
<223> 12-464-66.mis2, potential complement

<220>
-<221> primer_bind
<222> 436..455
<223> upstream amplification primer

<220>
<221> primer_bind

<222> 880..900
<223> downstream amplification primer, complement

<220>
<221> misc_binding
<222> 489..513
<223> 12-464-66 potential probe

<220>
<221> misc_feature
<222> 121,163..164
<223> n=a, g, c or t

<400> 28

```
aatcttaact ccctgaattc cctttagtta atggagactc tagtacacca tcctgcagtg     60
gggccatttc catagctggt gaagaaattt tctttcccac agtcttcctc ctctcttttt    120
ncatactttt ccctcttttg ctttctggcc atatgccttc ttnnttgctt tcctcccctt    180
ccacctctaa ctcacagcaa ttctcttccc tctaccaacc agcctgttcc aaaaaaaaaa    240
aaaaaaaaaa aaaaggtccc cttttattcc ttagggtagc acttgttttt gtttttaac     300
atccaaatcc ttcttggagc cccttcgtat tctctctctg aggaagacgg agggggcttc    360
cctcatcctc ccttgttacc tagcaacttg tcttcttcct gacaagtcac caccctgcca    420
ggatgccagc tgatgcacac acaataaagg gaaagtgagc agacaccacc actccagtcc    480
actcatagag gaccctctgc kcattagcac tccgatgagt ggcttgctat gggcaggtcc    540
cccagacgcc tctcccccag taagctggcc acactgctcc tccagggatc tcagagagat    600
ggcagcttgt caagttcctg agtcgctggg tatgtactgg gaatttcaga tttaatccct    660
aattacatcc agctcatcac ctttgtaata ggagccataa tcatcttttc attgttgtct    720
ttgagtcact ggggctgtgt ggaacactat atccatattc atgcatgtgt tttctatttt    780
gctactacat cctcttcctt agacttcaaa atgaaggggg attttctttt ctcctgcaaa    840
agcctttatg aaagaaggtg tttgcttaac actttcaagc ttccccattg acatcctttg    900
ttctttctct acacattcat ggattaacta ctttgggtcc agccgtggta accaacttca    960

atttgtcaca gaaaatgtag atgtttttag gtagggctta t                       1001
```

<210> 29
<211> 1001
<212> DNA
<213> Homo Sapiens

<220>
<221> allele
<222> 501
<223> 12-465-26 : polymorphic base C or T

<220>
<221> misc_binding
<222> 481..500
<223> 12-465-26.mis1, potential

<220>
<221> misc_binding
<222> 502..521
<223> 12-465-26.mis2, potential complement

<220>
<221> primer_bind
<222> 476..493

<223> upstream amplification primer

<220>
<221> primer_bind
<222> 945..962
<223> downstream amplification primer, complement

<220>
<221> misc_binding
<222> 489..513
<223> 12-465-26 potential probe

<220>
<221> misc_feature
<222> 556,630,796
<223> n=a, g, c or t

<400> 29

```
ctttctactt tttaatgtgg ttaccagaaa atttttaatt acatattaat tagtgacttg      60
ctttgtggct tgcgttgtat ttctattgaa cagtgctggt ttagagttta atgtttacta     120
gttcttttgt tctactaaat taccacaaaa cttcctgctt catttgtatt tatgaggtat     180
atgagttgtg gaacgtggac tggtttgaaa gcaatatttc tgcacagaca aattctaggg     240
ttggcttttt gcatactata ataactctag acaaatcatg gaaattgtga cttccccagg     300
aagatgggga ctgggggagg tgaagtatgg aggaagggaa aggaggcttg gagaggaaag     360
gaagaaggcc atatactcca gaggcgatag gatcactgaa aaaaactaaa tgtgtggctt     420
gcaagaagag gaggtggcct ccttctaaga tgtcccttgt cattctgaag aagacttcct     480
cccaaaccaa ctctttccca ytgggcagtc agcttgatct cttgaggttg aggaaggccc     540
tgctattaat tgtccntagg agaaagagca tatccactga gctatgagtc aatgatctgt     600
cttagggtgg gatttattat tttcattatn ttgtgtgttt gattttcttg ttagagttct     660
ttccttagga ctgtttgttg cttttaacga ggtggatggg ggtggtagtg tgctgagcag     720
agcagtgctg atgctgacaa caacaccaca gcaaacagca aacaggacat gcaaagcagg     780
aggcaaactc gtcttnccca accaacattt agctgaggac tagtcagggt gtgtggctat     840
tatgttctag tagctaggag agtcccagca tgtgctctta gaggccattt tggagagggt     900
gaaatagaga aactagaaac attattcatc tcagagactc tctggacctg agagagaaca     960
gtaactgtat tcaggcctaa tccctcagat ggctgccttg g                        1001
```

<210> 30
<211> 1001
<212> DNA
<213> Homo Sapiens

<220>
<221> allele
<222> 501
<223> 12-465-234 : polymorphic base G or T

<220>
<221> misc_binding
<222> 481..500
<223> 12-465-234.mis1, potential

<220>
<221> misc_binding
<222> 502..521
<223> 12-465-234.mis2, potential complement

<220>
<221> primer_bind

<222> 266..283
<223> upstream amplification primer

<220>
<221> primer_bind
<222> 735..752
<223> downstream amplification primer, complement

<220>
<221> misc_binding
<222> 489..513
<223> 12-465-234 potential probe

<220>
<221> misc_feature
<222> 346,420,586,869..870,876..877
<223> n=a, g, c or t

<400> 30

```
gcaatatttc tgcacagaca aattctaggg ttggcttttt gcatactata ataactctag     60
acaaatcatg gaaattgtga cttccccagg aagatgggga ctgggggag tgaagtatgg    120
aggaagggaa aggaggcttg gagaggaaag gaagaaggcc atatactcca gaggcgatag    180
gatcactgaa aaaaactaaa tgtgtggctt gcaagaagag gaggtggcct ccttctaaga    240
tgtcccttgt cattctgaag aagacttcct cccaaaccaa ctctttccca ttgggcagtc    300
agcttgatct cttgaggttg aggaaggccc tgctattaat tgtccntagg agaaagagca    360
tatccactga gctatgagtc aatgatctgt cttagggtgg gatttattat tttcattatn    420
ttgtgtgttt gattttcttg ttagagttct ttccttagga ctgtttgttg cttttaacga    480
ggtggatggg ggtggtagtg kgctgagcag agcagtgctg atgctgacaa caacaccaca    540
gcaaacagca aacaggacat gcaaagcagg aggcaaactc gtcttnccca accaacattt    600
agctgaggac tagtcagggt gtgtggctat tatgttctag tagctaggag agtcccagca    660
tgtgctctta gaggccattt tggagagggt gaaatagaga aactagaaac attattcatc    720
tcagagactc tctggacctg agagagaaca gtaactgtat tcaggcctaa tccctcagat    780
ggctgccttg gcggccactc tctttaccat gaaaattgcc ccccagatgg aaacctcttg    840
aacttggaaa aacctcttta tttccaaann cagaannaaa aaaaaatcac tatgtatttc    900
ttcttaagaa aaaaaaattg ttattcaact actgtgtccc attttcccaa taaacattaa    960
ttgaactgct agtgggcacc attcatccat ttaacaaata c                        1001
```

<210> 436
<211> 1001
<212> DNA
<213> Homo Sapiens

<220>
<221> allele
<222> 501
<223> 12-421-135 : insertion T

<220>
<221> misc_binding
<222> 481..500
<223> 12-421-135.mis1, potential

<220>
<221> primer_bind
<222> 367..385
<223> upstream amplification primer

<220>
<221> primer_bind
<222> 797..817
<223> downstream amplification primer, complement

<400> 436

```
gttcttgtct cattttgata cagatgaacc tagtgtcaaa tgagttggag gaagcatcct     60
gttctacttt ctcagataat tttaataagg ttggaagagt gtgttccttc aaggttgtgt    120
agaacttgcc ttaaaataat ctgggcctaa tgtttttctg gtgggaaatt tttaaattat    180
gggatttgca ttctttaatg attattggac taggcaggct tttgttaca tcttgaatca     240
attttggtag gccttggtcc ttgagatgat aacattggtc tcttgattat ccacttgctc    300
caaggagggt tttacagact actccctagg cttgaaagcg tggggtgaat accaccggga    360
gtacacgaga taattgtaag aggtgtgtag acttggtttt aaataacatt gaactacatg    420
atgagaagtc attctcattt caattatctt tgaatcctta tggttaagtc aagtggaaag    480
tattggtttg gagccagcat gtcccacacc tcaaaattaa tgtttatttt tatcaaagta    540
atttatgtac gtgatctgaa gataaagtgg gattacaggt tttatgacaa caaataactc    600
ttccttgtcc ctacctcccc tctccctcta tccctagaga taaccctttt tgattactac    660
tttgggtatt tacttctgta tttctaaaca ttatactttc actgctattt attgattcac    720
aaattttaga cattgtcagt tggcttcttt ttatggaaga aataatttca tgctccttta    780
cttcttcttt tccttacctc atccttccag taacattata tcactctgtt aggttaaatc    840
aatagccaat gtttataatt attgtgatta agaaaataat gttgaaagca gggccttata    900
gtatactgtg gttacatttc cttcttgtgc aatttttttt ccctagggtt aataattgct    960
ctttatttct ttgtttgctt agtttcatgc ccaaatcttc t                      1001
```

<210> 437
<211> 1001
<212> DNA
<213> Homo Sapiens

<220>
<221> allele
<222> 501
<223> 12-442-133 : insertion C

<220>
<221> misc_binding
<222> 502..520
<223> 12-442-133.mis1, complement

<220>
<221> primer_bind
<222> 616..633
<223> upstream amplification primer, complement

<220>
<221> primer_bind
<222> 184..203
<223> downstream amplification primer

<220> .
<221> misc_feature
<222> 94,734
<223> n=a, g, c or t

<400> 437

```
ttagaattct ttttggcatt aatcaaaact ttacagagga cataaacact gatttttttcc   60
ccccattcat ttaactattt gcacacagag acangaagcc agaaatctga ctgggaagaa   120
attcctaccc ttttgccagc atgctaagct tctgggttct ctttccctga gtggccctag   180
tgatctggct tgtggcacaa ctgcctttgg gggccaagcc gcatcataaa ggaaaagtat   240
ttctttttgt tctggccaaa gcaaaatacg cgtaataaaa catagatatt aaccaggctg   300
cttagcatcc aatatcaaac tggcaaggct taaatttgcc ctcaggtggg ccctgtcatc   360
tttaatctaa cctccgacta ggagtttcaa catgtggtct ctgggcaaga tggtcgccct   420
gagtaataga aaagaaagag aaaggagaga gagaaaaaca ttgcctgtgg cagggcgggg   480
aaggtgaaat gatcagggag gcagagaaag aaccacccat tgcagcgaca ctaaaaagtt   540
caggtggctg ctgtcggtgg agcaaggatc ttttccagta atcctaccag ctctcaaatt   600
tcccttgtta gggaggaaaa agctccccat gtcccaggat cctgtacatt cctaattctg   660
tcacccatag ccatcagcaa agtacaaggg agattaatcc aaagagaata gcagttaaca   720
tcccatagtg ccgnaacctg ttcttagccg agagggactt taccgaagag gggcctctaa   780
cccgctaaat cttagaaggg actctaactc tcctaagtcg ggcctctaac cagaggtcag   840
tcaagcatcc ttgcctttta ttaaggggga gcctttaacc acctctatct taggagagac   900
tctaactcca ctaagctggg cctctaaccc aatcccattc tttacccagg taccccacca   960
cttacccaaa gtcttccaat cagtgctgca gtctatttcc t                      1001
```

<210> 438
<211> 756
<212> DNA
<213> Homo Sapiens

<220>
<221> allele
<222> 501
<223> 12-449-63 : insertion AT

<220>
<221> misc_binding
<222> 481..500
<223> 12-449-63.mis1, potential

<220>
<221> primer_bind
<222> 546..563
<223> upstream amplification primer, complement

<220>
<221> primer_bind
<222> 86..106
<223> downstream amplification primer

<220>
<221> misc_feature
<222> 123,277
<223> n=a, g, c or t

<400> 438

```
ggggacggaa gaaaggaaaa gaaaaaatag tttatatgta tatacatttg tatcaaaata    60
agaaataaat actcataatt attacaattt tcctctgaaa gtagtcacat ggttatacct   120
ggntatttat ttattttta ttattattat tattatttga gacagggtct caccctgtca    180
cacaggctgg agtgcagtgg tgcaattatg gctcactgct acctctgcca cctgggctca   240
agccatcctc tcaccttagc cttctgaaca gctgggnata catgtgcact aattttttt    300
tttttgaaac aaaaatattt gtgtagaagg cacaaaagct acaatcacag actccactgt   360
gcaaaggcgc aacctgcctc attgatctct agtgtacacc aacccagctt ccctttccat   420
tcagcctgtg aaaggagata gtgcttgggc catttggtaa aagaaggga tgggagatga    480
tcaaaacccc aagtaaggtt catccaatat ggtgtctaag cagcaaatga ctaattgctg   540
aagaaggaga ctagacagag gattagaggc agccatgggg ccagtgcagc tgtggagagc   600
tctgagcaaa gaaacaaggt tggcaggtga ggaggcctag gatagaggcc agaaggccaa   660
gcctggggct gcatgtgcac taatttttgt attttttgc tttttgata gagatgagat     720
ttcatcatgt tgcacaggtg ggtcttgaag tcctgg                             756
```

<210> 439
<211> 1001
<212> DNA
<213> Homo Sapiens

<220>
<221> allele
<222> 501
<223> 12-454-242 : deletion AT

<220>
<221> misc_binding
<222> 481..500
<223> 12-454-242.mis1, potential

<220>
<221> primer_bind
<222> 260..279
<223> upstream amplification primer

<220>
<221> primer_bind
<222> 755..773
<223> downstream amplification primer, complement

<220>
<221> misc_feature
<222> 795..800
<223> n=a, g, c or t

<400> 439

```
caaatggaaa aaatgaaaac tgtcacactt cttacataac ttcttttatt atctgtattg      60
taaatacaga gtctcaatta ttgactaatt tatactaata gtgggaagca gtgtctagat     120
aatcccagat gattactaat ttaaaactac tttacatttt atttagtaat gtgtttggaa     180
cagttacatt ttgtagctac ttacaatttt actttaactg aaataataag acaaggccat     240
aaatattggc ctccctggct ggggagttgt gtgggtttcc tttccaggca gttgaggttt     300
tccatggagc ctactctaca gtgtcagcag gagtataaca tagaaggctt ccaggtcagg     360
tggcccagag tcaaatcaaa gccccatccc tttttagcaa atttctcagc cttacttagc     420
tgtgccggcc tgcctctttc taaaatgagg aaaataataa tacccacttc actggtttat     480
tgagaggatt aaatgaggac atgtgttatt tcacaccatt attgcttctg ttgttattat     540
tttaaaatct aggttggtga ttgcatcagt ttcttagggc tgctctaaag aaagtaccac     600
aagctgagtg acttacatag cagaaaagtg ttttcttaca ggttaagagg ctggaagtct     660
gaaatcaagg tgtcagcagg gccatgctcc cactgaaacc catagcgggg aatcctttct     720
tgcttctaag tttctagtgt ttccttggct tgtagatgtg tcacttcagt cacacggcca     780
tcttcttctt tcttnnnnnn cttcttcttc ttcttcttct tcttcttctt cttcttcttc     840
ttcttcttct tcttcttcct tcttcttctt cttcttcttc ttcttcttct tcttcttctt     900
cttcttcttc ttcttcttct tcttcttctt cttcttcttc ttcttcttct tcttcttctt     960
cttcttcttc ttcttcttct tcttcttctt ctttgagatg g                        1001
```

<210> 440
<211> 1001
<212> DNA
<213> Homo Sapiens

<220>
<221> allele
<222> 501
<223> 12-463-230 : deletion CAT

<220>
<221> misc_binding
<222> 481..500
<223> 12-463-230.mis1, potential

<220>
<221> primer_bind
<222> 255..272
<223> upstream amplification primer

<220>
<221> primer_bind
<222> 773..790
<223> downstream amplification primer, complement

<220>
<221> misc_feature
<222> 136,400,713,730
<223> n=a, g, c or t

<400> 440

```
aatcaatgca taaacaaaaa ggatgcccca gaaaacctgt gcttttcaga cacacacaca      60
catacacaca cactcactca ctcactcact ctgaattggg tcaagtaaaa aaaaattttt     120
tttaaaggaa ggaatncttt agcccaactg ggatttcgac gaaattgtat atatcaaaag     180
atagctacat ccaacctctc accagaatcc agaggctttc tcctgcaaag atactattga     240
aagttttatt agtaagtttg agaaggcgaa agtatgtatc agaggtgaaa gctaacatga     300
caattgcttg gccaaagagc gcagcagttt tatgttattg agcagcacag gatgaatcga     360
acacccacga tggctagaca gaggcgcctg gctgtgactn ccagaggttc aggcaagcca     420
tctgacctct ctcagtcccc acccccctgaa atgaaaacca tactcataag actccttgac     480
aatggaaatt ttggaggtga catagttaat gaagtgtcat agaaaggaaa tagctaagaa     540
tactctaaaa cctccccatt cccagtatgg gcaatttggg aaaaatgagt aactaaaaat     600
taggggttct caaagtgtgg ttccaggatc aaatgcatta gtccccacct gggagcttgt     660
tagaaatgca aattcccagg ctcccctcta ggcctcctga attagaactt ttngggggtg     720
ttttaacaan gctctccagg tggttacgat gtcccataga gaatcattgg ggcaaataat     780
cctgtgccct gattagattg caaagctgca ctttcaaaag caagtcaggg tgctgagttg     840
cttagtttct attgcaaaat ttcctgcttt ggaacaaaat tttcaaaatt ttcagagggt     900
tgctcaggcc agtgatgttt gcaatgcaaa cccaagaatt ctgaaaggag cccatggctg     960
aaaggagcca attcttccag gacggactac tccagttgca a                        1001
```

<210> 441
<211> 1001
<212> DNA
<213> Homo Sapiens

<220>
<221> allele
<222> 501
<223> 12-462-199 : deletion ATGTCCCACAGCTG

<220>
<221> misc_binding
<222> 481..500
<223> 12-462-199.mis1, potential

<220>
<221> primer_bind
<222> 303..322
<223> upstream amplification primer

<220>
<221> primer_bind
<222> 736..756
<223> downstream amplification primer, complement

<220>
<221> misc_feature
<222> 255
<223> n=a, g, c or t

<400> 441

```
caaaagcaag tcagggtgct gagttgctta gtttctattg caaaatttcc tgctttggaa     60
caaaattttc aaaattttca gagggttgct caggccagtg atgtttgcaa tgcaaaccca    120
agaattctga aaggagccca tggctgaaag gagccaattc ttccaggacg gactactcca    180
gttgcaactg tgctgtggag gctgaggagc atccatgact gaggaagggc gagtccccta    240
taatgccctt gctanggga gcatcagaca tccctagact tacaagtccc tcgtgtaata     300
aggtgaacta tggaaagcta tgatttgagg aaagttccct ctgattgaaa gagttcttca    360
aatatgaaga atgtaacact tatggggcac ctactcattg caaaacactc tactaggggc    420
tttcacctgt atgtttaact cattttgttg attttttttt ttttaagccc aaatttctga    480
gaggttaagg aacttgtctg gtggtggcgt ctctgggatt ctgacttttc cactgaccat    540
aacatgcatc tggtctaaat tgagcggacc acccaggagg acgttggaat aaaaagaaat    600
gggaaggggg tggggagagg aatgggcata gaataaaaag gtagaaaccc ttgggatcaa    660
aggatgcaac tgaaagagca ccaaaatatg catcattttt tttaatatgt tgtctggtac    720
ccaaaattgg gatgcctctg tggtatcaac tttgacattt aaagaaaggg cataagatta    780
gatcattcaa ccataagtgg aaggaagaaa caaaataaaa taaaacctta caattccctt    840
tgaagttgat caggtgtcaa ggcagtgatt tggttttctt ttacaaactt tctttttcca    900
gaaaaatttt tactgtgggt ttggagaggt gtttacaata aattcatagc agagctccaa    960
aagggcccac cccatctaat atcctctgtc tcagcagatt a                       1001
```

<210> 469
<211> 1000
<212> DNA
<213> Homo Sapiens

<220>
<221> allele
<222> 501
<223> 10-290-326 : polymorphic base A or G

<220>
<221> misc_binding
<222> 481..500
<223> 10-290-326.mis1, potential

<220>
<221> misc_binding
<222> 502..521
<223> 10-290-326.mis2, potential complement

<220>
<221> primer_bind
<222> 177..196
<223> upstream amplification primer

<220>
<221> primer_bind
<222> 576..595
<223> downstream amplification primer, complement

<220>
<221> misc_binding
<222> 489..513
<223> 10-290-326 potential probe

<400> 469

```
atgctttgaa tgttcaaaga aaccaagtga tctctatggc taggcattcc ctttaactta     60
tgtgatgagc taaaattttc aaattgcaaa aatatagaag ttctggacag tgttaacgat    120
aggacagcct acctcaggac tctcagcttc cttattacag tccagtttgt cacttttctc    180
cctcatgtcc actctgcagg atcaccggtt tccgactgag tctggggatt ttggccttgt    240
tgaccctcct aggtgccctg ggaattgcaa acagctttct ggatgaatat ctggacctca    300
atattgccaa gaaactgagg cggcaattct aactttttct cttcccttta atgcttgcag    360
aagctgttcc caccatgaag gtaatatggt atcatttgtt aaataaaaat aaagtcttta    420

ttctgttttt cttgaaatgg ctttgtagaa acacacactt tagagaatac atttcctgtt    480
taagaactga aatttgcttg rggtaggtac atccatatca tcagtggaag tgtgttgtgc    540
cctagtttta gaaatgcatg ctgaagtcat gaccggttgt gtagggacac agcagcctga    600
cccgtgtgct ggccgttccg aggactactg acatatcctg ccactctgac tgcttctctc    660
ttgatggaaa gtttcagaca gaagctgata aagcagctga gccctttcat tggttatagg    720
aaaacaacag ataggaaggc atgtggggga catcaggcaa acacatagag tagccagaga    780
agggagtcat caactgatag cacaaaacaa aacaggttat agaaggctgg gcgtggtggc    840
tcatgcctgt aatcccagca ctttgagaag ccgaggcggg cagatcacaa ggtcaggaga    900
tcgagaccat cctggctaac acagtgaaac cctgtctcta ctaaagatac aaaaaattag    960
ccgggcatgg tggcgggtgc ctgtagtcct agctactcgg                         1000
```

<210> 470
<211> 1000
<212> DNA
<213> Homo Sapiens

<220>
<221> allele
<222> 501
<223> 10-290-37 : polymorphic base C or T

<220>
<221> misc_binding
<222> 481..500
<223> 10-290-37.mis1. potential

<220>
<221> misc_binding
<222> 502..521
<223> 10-290-37.mis2, potential complement

<220>
<221> primer_bind
<222> 465..484
<223> upstream amplification primer

<220>
<221> primer_bind
<222> 864..883
<223> downstream amplification primer, complement

<220>
<221> misc_binding
<222> 489..513
<223> 10-290-37 potential probe

<400> 470

```
aaaaaacggt aaggagaacc cagtaatttt gtatttatgc aaaaagtagc aggataaggt     60
ctgggtcagt ttccttctcc tgagagatat taacagcact gtgagttgtg gtggcaaaag    120
taatccatac tgtctgaggt caagtcttgc cagttcatac acaattaggc atgagatggg    180
aaaatgcaag tataaaactg tgcactgaat gtggactcta gttccttgca aaaattttgc    240
attgtctcca gaaagttacc gaggaaataa tcctgcctag ccttgataat gctttgaatg    300
ttcaaagaaa ccaagtgatc tctatggcta ggcattccct ttaacttatg tgatgagcta    360
aaattttcaa attgcaaaaa tatagaagtt ctggacagtg ttaacgatag gacagcctac    420
ctcaggactc tcagcttcct tattacagtc cagtttgtca cttttctccc tcatgtccac    480
tttgcaggat caccggtttc ygactgagtc tggggatttt ggccttgttg accctcctag    540
gtgccctggg aattgcaaac agctttctgg atgaatatct ggacctcaat attgccaaga    600
aactgaggcg gcaattctaa ctttttctct tccctttaat gcttgcagaa gctgttccca    660
ccatgaaggt aatatggtat catttgttaa ataaaaataa agtctttatt ctgtttttct    720
tgaaatggct ttgtagaaac acacacttta gagaatacat ttcctgttta agaactgaaa    780
tttgcttggg gtaggtacat ccatatcatc agtggaagtg tgttgtgccc tagttttaga    840
aatgcatgct gaagtcatga ccggttgtgt agggacacag cagcctgacc cgtgtgctgg    900
ccgttccgag gactactgac atatcctgcc actctgactg cttctctctt gatggaaagt    960

ttcagacaga agctgataaa gcagctgagc cctttcattg                        1000
```

<210> 471
<211> 1000
<212> DNA
<213> Homo Sapiens

<220>
<221> allele
<222> 501
<223> 10-523-232 : polymorphic base C or T

<220>
<221> misc_binding
<222> 481..500
<223> 10-523-232.mis1, potential

<220>
<221> misc_binding
<222> 502..521
<223> 10-523-232.mis2, potential complement

<220>
<221> primer_bind
<222> 270..288
<223> upstream amplification primer

<220>
<221> primer_bind
<222> 527..545
<223> downstream amplification primer, complement

<220>
<221> misc_binding
<222> 489..513
<223> 10-523-232 potential probe

<400> 471

```
acttcaggag attttaaggg aattatttaa agaccatttt gtaatgttct gggtaactca     60
agtggaaggc aatggatgtg agggaatttt taaaataaga aatctcttga atatgaattg    120
agtaatctct taagttcttt agataagata tggagtgatc tttaggctca ggaggcctgt    180
cttttgggaa agtgctataa ctcacttcta ctaccggcaa cttaatacgt aagagaatat    240
tcttggccct gctctgaggt ttaaacaatg ttcaaccttg aaagattgat tttaaatgta    300
aaggtcattt aaactctttt gtaagttctt atattttgtt aaaataggct gaacatttac    360
ctctagatga aaaatcaatg cttcagtgta tacttttctc ataaaataac ctatgtcttt    420
atttttttct gcagacaaaa ctgtgtggag ttttatccta tattcataat tacattgtgg    480
atggctgggt ggtatttcaa ycaaggtaat gttaaaatac agtcaactgt gttctaatga    540
tgactatgat gcttaaacga ttaaggagta gatatatggc caggcacggt ggctcacacc    600
tatattccca gcactttggg aggcccaggt gggtggatca cctgaggtca ggagttcgag    660
accagcctgg ccaaaatggt gaaaccctgt ctctactaaa aatacaaaaa ttagctaagt    720
gtggtggcgc atgcctgtaa tcccagctac ttgggaggct gagacaggag aatcacttga    780
acccaggagg tggaggctgc agtgagctga gatcgtgcca ctacactcca gtctgggtga    840
cagagactcc atctcaaaaa aacaaacaaa aaaaggagca gatatgcaac tatatagcta    900
ccaatcctgg agactaaaca aaataaacag tgtgtgcatc agagtgctat gttgcaggta    960
tatgaacttt ggcttcattc taatttaatt caataatgaa                         1000
```

<210> 472
<211> 1000
<212> DNA
<213> Homo Sapiens

<220>
<221> allele.
<222> 501
<223> 12-449-300 : polymorphic base T or C

<220>
<221> misc_binding
<222> 481..500
<223> 12-449-300.mis1, potential

<220>
<221> misc_binding
<222> 502..521
<223> 12-449-300.mis2, potential complement

<220>
<221> primer_bind
<222> 783..800
<223> upstream amplification primer, complement

<220>
<221> primer_bind
<222> 325..345
<223> downstream amplification primer

<220>
<221> misc_binding
<222> 489..513
<223> 12-449-300 potential-probe

<400> 472

```
ccaccagagt tcataccagc atggaatctg tacacatctt taaatgatac ttagatttac      60
ttccaaataa agagctcttt catctcataa tgcaactgtc cattatataa ccaaacccat     120
actaactctt tctccaaaag aggatccaaa gtccctttat ccctctttgg gccatgatta     180
tttgtcttct aacagagtca cattgtctcc ttgagacgta aagatgcatt tagatgcagg     240
gggacggaag aaaggaaaag aaaaaatagt ttatatgtat atacatttgt atcaaaataa     300
gaaataaata ctcataatta ttacaatttt cctctgaaag tagtcacatg gttatacctg     360
gtatttattt atttttattt attattatta ttatttgaga cagggtctca ccctgtcaca     420
caggctggag tgcagtggtg caattatggc tcactgctac ctctgccacc tgggctcaag     480
ccatcctctc accttagcct yctgaacagc tgggatacat gtgcactaat tttttttttt     540
ttgaaacaaa aatatttgtg tagaaggcac aaaagctaca atcacagact ccactgtgca     600
aaggcgcaac ctgcctcatt gatctctagt gtacaccaac ccagcttccc tttccattca     660
gcctgtgaaa ggagatagtg cttgggccat ttggtaaaag aaggggatgg gagatgatca     720
aaaccccaag taaggttcat ccaatatggt gtctaagcag caaatgacta attgctgaag     780
aaggagacta gacagaggat tagaggcagc catggggcca gtgcagctgt ggagagctct     840
gagcaaagaa acaaggttgg caggtgagga ggcctaggat agaggccaga aggccaagcc     900
tggggctgca tgtgcactaa tttttgtatt tttttgcttt tttgatagag atgagatttc     960
atcatgttgc acaggtgggt cttgaagtcc tgggctcaag                          1000
```

<210> 474
<211> 1000
<212> DNA
<213> Homo Sapiens

<220>
<221> allele
<222> 501
<223> 10-286-289 : polymorphic base G or C

<220>
<221> misc_binding
<222> 481..500
<223> 10-286-289.mis1, potential

<220>
<221> misc_binding
<222> 502..521
<223> 10-286-289.mis2, potential complement

<220>
<221> primer_bind
<222> 213..231
<223> upstream amplification primer

<220>
<221> primer_bind
<222> 613..631
<223> downstream amplification primer, complement

<220>
<221> misc_binding
<222> 489..513
<223> 10-286-289 potential probe

<400> 474

```
aaaaaaaaaa gtatgattat tatcagagtc tctaggtgac agtgagaggc agcctggtgc     60
aggggatagg gcacagttgt aatctgctag actgggttca aatcctaaat cggccattca    120
gagcgtgaat acattagaac tggcttaaat tagcatttaa aatgtcaaca ggattaagta    180
agtagttcct tctcactttt tccacaggag gtgagaaagg tctcagcaag gccctaggag    240
ggaagggcgt ggggatgaaa gggatccaga gagtctgtgc attggcagag aagatagtgt    300
aactggcact gcggctggag ggctggtccc acagtgagct acagccctgc ccgctggccg    360
tgggagaggc ttaaaacaaa cgccggaagc aactcccagc cccataaaga tctgtgaccg    420
gcagccccag acctgcctgc cttcctgact tctgttccag agcaaaggtc attcagccgc    480
ttgaatcagc cttttccccc sacccggtcc ccaactttgt ttacccgata aggaaggtca    540
gcattcaaag tcaagaagcg ccatttatct tcccgtgcgc tctacaaata gttccgtgag    600
aaagatggcc gggaactcga tcctgctggc tgctgtctct attctctcgg cctgtcagca    660
aagtaagagg catgggaagt tcgtgtgtgt gcgcgtgtgt gcgtgtgtgt gtgtgtgtga    720
caaggcttgc gggagagaga gggagggagg gagatgggtc cggtgttttg tttcctactt    780
gcccttgcag gtagctctgg gtcctcagag cacagtcgcc tcagggtcac ccatgccgcc    840
tgctaccctc cttcccaggg gcaagcagag actgagaaca ttccagagat tagttctccc    900
aactggaacg ctgtggggcc tcagagctca gcgattctgc atcatctgtg attacgaccc    960
acagcccgtt caaacgagcg ttagtagcct gctaacctgc                          1000
```

<210> 475
<211> 1000
<212> DNA
<213> Homo Sapiens

<220>
<221> allele
<222> 501
<223> 10-286-345 : polymorphic base A or T

<220>
<221> misc_binding
<222> 481..500
<223> 10-286-345.mis1, potential

<220>
<221> misc_binding
<222> 502..521
<223> 10-286-345.mis2, potential complement

<220>
<221> primer_bind
<222> 158..176
<223> upstream amplification primer

<220>
<221> primer_bind
<222> 558..576
<223> downstream amplification primer, complement

<220>
<221> misc_binding
<222> 489..513
<223> 10-286-345 potential probe

<400> 475

```
ggtgcagggg atagggcaca gttgtaatct gctagactgg gttcaaatcc taaatcggcc      60
attcagagcg tgaatacatt agaactggct taaattagca tttaaaatgt caacaggatt     120
aagtaagtag ttccttctca ctttttccac aggaggtgag aaaggtctca gcaaggccct     180
aggagggaag ggcgtgggga tgaaagggat ccagagagtc tgtgcattgg cagagaagat     240
agtgtaactg gcactgcggc tggagggctg gtcccacagt gagctacagc cctgcccgct     300
ggccgtggga gaggcttaaa acaaacgccg gaagcaactc ccagccccat aaagatctgt     360
gaccggcagc cccagacctg cctgccttcc tgacttctgt tccagagcaa aggtcattca     420
```

```
gccgcttgaa tcagcctttt ccccccaccc ggtccccaac tttgtttacc cgataaggaa     480
ggtcagcatt caaagtcaag wagcgccatt tatcttcccg tgcgctctac aaatagttcc     540
gtgagaaaga tggccgggaa ctcgatcctg ctggctgctg tctctattct ctcggcctgt     600
cagcaaagta agaggcatgg gaagttcgtg tgtgtgcgcg tgtgtgcgtg tgtgtgtgtg     660
tgtgacaagg cttgcgggag agagagggag ggagggagat gggtccggtg ttttgtttcc     720
tacttgccct tgcaggtagc tctgggtcct cagagcacag tcgcctcagg gtcacccatg     780
ccgcctgcta ccctccttcc caggggcaag cagagactga gaacattcca gagattagtt     840
ctcccaactg gaacgctgtg ggcctcaga gctcagcgat tctgcatcat ctgtgattac      900
gacccacagc ccgttcaaac gagcgttagt agcctgctaa cctgcaggaa gtggtgtgaa     960
tattaattac aagtgttcca aaggaaacgt gcctgcttct                          1000
```

<210> 476
<211> 1000
<212> DNA
<213> Homo Sapiens

<220>
<221> allele
<222> 501
<223> 10-286-375 : polymorphic base A or G

<220>
<221> misc_binding
<222> 481..500
<223> 10-286-375.mis1, potential

<220>
<221> misc_binding
<222> 502..521
<223> 10-286-375.mis2, potential complement

<220>
<221> primer_bind
<222> 128..146
<223> upstream amplification primer

<220>
<221> primer_bind
<222> 528..546
<223> downstream amplification primer, complement

<220>
<221> misc_binding
<222> 489..513
<223> 10-286-375 potential probe

<400> 476

gctagactgg gttcaaatcc taaatcggcc attcagagcg tgaatacatt agaactggct 60
taaattagca tttaaaatgt caacaggatt aagtaagtag ttccttctca ctttttccac 120
aggaggtgag aaaggtctca gcaaggccct aggagggaag ggcgtgggga tgaaagggat 180
ccagagagtc tgtgcattgg cagagaagat agtgtaactg gcactgcggc tggagggctg 240
gtcccacagt gagctacagc cctgcccgct ggccgtggga gaggcttaaa acaaacgccg 300
gaagcaactc ccagccccat aaagatctgt gaccggcagc cccagacctg cctgccttcc 360
tgacttctgt tccagagcaa aggtcattca gccgcttgaa tcagcctttt ccccccaccc 420
ggtccccaac tttgtttacc cgataaggaa ggtcagcatt caaagtcaag aagcgccatt 480
tatcttcccg tgcgctctac raatagttcc gtgagaaaga tggccgggaa ctcgatcctg 540
ctggctgctg tctctattct ctcggcctgt cagcaaagta agaggcatgg gaagttcgtg 600
tgtgtgcgcg tgtgtgcgtg tgtgtgtgtg tgtgacaagg cttgcgggag agagagggag 660
ggagggagat gggtccggtg ttttgtttcc tacttgccct tgcaggtagc tctgggtcct 720
cagagcacag tcgcctcagg gtcacccatg ccgcctgcta ccctccttcc caggggcaag 780
cagagactga gaacattcca gagattagtt ctcccaactg gaacgctgtg gggcctcaga 840
gctcagcgat tctgcatcat ctgtgattac gacccacagc ccgttcaaac gagcgttagt 900
agcctgctaa cctgcaggaa gtggtgtgaa tattaattac aagtgttcca aaggaaacgt 960

gcctgcttct aaacctggtt gtgatttctt gaacgttgat 1000

<210> 477
<211> 1000
<212> DNA
<213> Homo Sapiens

<220>
<221> allele
<222> 501
<223> 10-289-201 : polymorphic base C or T

<220>
<221> misc_binding
<222> 481..500
<223> 10-289-201.mis1, potential

<220>
<221> misc_binding
<222> 502..521
<223> 10-289-201.mis2, potential complement

<220>
<221> primer_bind
<222> 307..324
<223> upstream amplification primer

<220>
<221> primer_bind
<222> 700..719
<223> downstream amplification primer, complement

<220>
<221> misc_binding
<222> 489..513
<223> 10-289-201 potential probe

<400> 477

```
tgcaacctcc acctcctggg ttcaaacgat tctcctgcct cagcttcctg agtagctggg      60
actgcaggtg cccgccacca tgcccagcta atttttgtat ttttagtaga gacgaggttt     120
cacaatgttg gccaggctgg tcttgaactc ctgacctcaa gtgattcacc ccccctcggc     180
ctctcaaagt gttgggaata caggcctgag ctaccacgtc cagcctcatt atagatttaa     240
gtgggaattt gttgatgttg aatcatgttg aaacattttt cttgagatta ttggcacatt     300
tgtttccttc ctatgtggat tctctctata ttctgaatat aagtatcttg tccaatatat     360
gttttgtgaa tattttctca gtcgtcgtac aacatcttaa gagactgctg ttgtattttg     420
tgccttttct tttttttccc caccccccta tagtttttgc tacttgtctg ggtctggtgt     480
acatatatgg ccgtcaccta yacttctggg gatattcaga agctgctaaa aaacggtaag     540
gagaacccag taattttgta tttatgcaaa aagtagcagg ataaggtctg ggtcagtttc     600
cttctcctga gagatattaa cagcactgtg agttgtggtg gcaaaagtaa tccatactgt     660
ctgaggtcaa gtcttgccag ttcatacaca attaggcatg agatgggaaa atgcaagtat     720
aaaactgtgc actgaatgtg gactctagtt ccttgcaaaa attttgcatt gtctccagaa     780
agttaccgag gaaataatcc tgcctagcct tgataatgct ttgaatgttc aaagaaacca     840
agtgatctct atggctaggc attcccttta acttatgtga tgagctaaaa ttttcaaatt     900
gcaaaaatat agaagttctg gacagtgtta acgataggac agcctacctc aggactctca     960
gcttccttat tacagtccag tttgtcactt ttctccctca                          1000
```

<210> 484
<211> 1000
<212> DNA
<213> Homo Sapiens

<220>
<221> allele
<222> 500
<223> 10-290-328 : deletion of G

<220>
<221> misc_binding
<222> 481..499
<223> 10-290-328.mis1, potential

<220>
<221> primer_bind
<222> 174..193
<223> upstream amplification primer

<220>
<221> primer bind
<222> 573..592
<223> downstream amplification primer, complement

<400> 484

```
ctttgaatgt tcaaagaaac caagtgatct ctatggctag gcattccctt taacttatgt     60
gatgagctaa aattttcaaa ttgcaaaaat atagaagttc tggacagtgt taacgatagg    120
acagcctacc tcaggactct cagcttcctt attacagtcc agtttgtcac ttttctccct    180
catgtccact ctgcaggatc accggtttcc gactgagtct ggggatttg gccttgttga     240
ccctcctagg tgccctggga attgcaaaca gctttctgga tgaatatctg gacctcaata    300
ttgccaagaa actgaggcgg caattctaac tttttctctt ccctttaatg cttgcagaag    360
ctgttcccac catgaaggta atatggtatc atttgttaaa taaaaataaa gtctttattc    420
tgtttttctt gaaatggctt tgtagaaaca cacactttag agaatacatt tcctgtttaa    480
gaactgaaat ttgcttgggg taggtacatc catatcatca gtggaagtgt gttgtgccct    540
agttttagaa atgcatgctg aagtcatgac cggttgtgta gggacacagc agcctgaccc    600
gtgtgctggc cgttccgagg actactgaca tatcctgcca ctctgactgc ttctctcttg    660
atggaaagtt tcagacagaa gctgataaag cagctgagcc ctttcattgg ttataggaaa    720
acaacagata ggaaggcatg tgggggacat caggcaaaca catagagtag ccagagaagg    780
gagtcatcaa ctgatagcac aaaacaaaac aggttataga aggctgggcg tggtggctca    840
tgcctgtaat cccagcactt tgagaagccg aggcgggcag atcacaaggt caggagatcg    900
agaccatcct ggctaacaca gtgaaaccct gtctctacta aagatacaaa aaattagccg    960
ggcatggtgg cgggtgcctg tagtcctagc tactcgggag                          1000
```

<210> 485
<211> 49312
<212> DNA
<213> Homo sapiens

<220>
<221> misc_feature
<222> 5466..7466
<223> 5'regulatory region

<220>
<221> exon
<222> 7467..7725
<223> exon 1

<220>
<221> exon
<222> 20256..20355
<223> exon 2

<220>
<221> exon
<222> 36905..36975
<223> exon 3

<220>
<221> exon
<222> 45167..45248
<223> exon 4

<220>
<221> exon
<222> 45728..45965
<223> exon 5

<220>
<221> misc_feature
<222> 45966..49312
<223> 3'regulatory region

<220>

<221> allele
<222> 7564
<223> 10-286-289 : polymorphic base G or C

<220>
<221> allele
<222> 7619
<223> 10-286-345 : polymorphic base A or T

<220>
<221> allele
<222> 7649
<223> 10-286-375 : polymorphic base A or G

<220>
<221> allele
<222> 17258
<223> 12-425-57 : polymorphic base A or G

<220>
<221> allele
<222> 21590
<223> 12-421-135 : insertion of T

<220>
<221> allele
<222> 21595
<223> 12-421-140 : polymorphic base A or G

<220>
<221> allele
<222> 36971
<223> 10-523-232 : polymorphic base C or T

<220>
<221> allele
<222> 45214
<223> 10-289-201 : polymorphic base C or T

<220>
<221> allele
<222> 45741
<223> 10-290-37 : polymorphic base C or T

<220>
<221> allele
<222> 46029
<223> 10-290-326 : polymorphic base A or G

<220>
<221> allele
<222> 46032
<223> 10-290-328 : deletion of G

<220>
<221> primer_bind
<222> 7276..7294
<223> 10-286.pu

<220>
<221> primer_bind
<222> 7676..7694
<223> 10-286.rp complement

<220>
<221> primer_bind
<222> 16839..16856
<223> 12-425.rp

<220>
<221> primer_bind
<222> 17297..17314
<223> 12-425.pu complement

<220>
<221> primer_bind
<222> 21456..21474
<223> 12-421.pu

<220>
<221> primer_bind
<222> 21886..21906
<223> 12-421.rp complement

<220>
<221> primer_bind
<222> 36740..36758
<223> 10-523.pu

<220>
<221> primer_bind
<222> 36997..37015
<223> 10-523.rp complement

<220>
<221> primer_bind
<222> 45020..45037
<223> 10-289.pu

<220>
<221> primer_bind
<222> 45413..45432
<223> 10-289.rp complement

<220>
<221> primer_bind
<222> 45705..45724
<223> 10-290.pu

<220>
<221> primer_bind
<222> 46104..46123
<223> 10-290.rp complement

<220>
<221> primer_bind
<222> 7545..7563

<223> 10-286-289.mis

<220>
<221> primer_bind
<222> 7565..7583
<223> 10-286-289.mis complement

<220>
<221> primer_bind
<222> 7600..7618
<223> 10-286-345.mis

<220>
<221> primer_bind
<222> 7620..7638
<223> 10-286-345.mis complement

<220>
<221> primer_bind
<222> 7630..7648
<223> 10-286-375.mis

<220>
<221> primer_bind
<222> 7650..7668
<223> 10-286-375.mis complement

<220>
<221> primer_bind
<222> 17239..17257
<223> 12-425-57.mis

<220>
<221> primer_bind
<222> 17259..17277
<223> 12-425-57.mis complement

<220>
<221> primer_bind
<222> 21576..21594
<223> 12-421-140.mis

<220>
<221> primer_bind
<222> 21596..21614
<223> 12-421-140.mis complement

<220>
<221> primer_bind
<222> 36952..36970
<223> 10-523-232.mis

<220>
<221> primer_bind
<222> 36972..36990
<223> 10-523-232.mis complement

<220>

<221> primer_bind
<222> 45195..45213
<223> 10-289-201.mis

<220>
<221> primer_bind
<222> 45215..45233
<223> 10-289-201.mis complement

<220>
<221> primer_bind
<222> 45722..45740
<223> 10-290-37.mis

<220>
<221> primer_bind
<222> 45742..45760
<223> 10-290-37.mis complement

<220>
<221> primer_bind
<222> 46010..46028
<223> 10-290-326.mis

<220>
<221> primer_bind
<222> 46030..46048
<223> 10-290-326.mis complement

<220>
<221> misc_binding
<222> 7552..7576
<223> 10-286-289.probe

<220>
<221> misc_binding
<222> 7607..7631
<223> 10-286-345.probe

<220>
<221> misc_binding
<222> 7637..7661
<223> 10-286-375.probe

<220>
<221> misc_binding
<222> 17246..17270
<223> 12-425-57.probe

<220>
<221> misc_binding
<222> 21583..21607
<223> 12-421-140.probe

<220>
<221> misc_binding
<222> 36959..36983
<223> 10-523-232.probe

<220>
<221> misc_binding
<222> 45202..45226
<223> 10-289-201.probe

<220>
<221> misc_binding
<222> 45729..45753
<223> 10-290-37.probe

<220>
<221> misc_binding
<222> 46017..46041
<223> 10-290-326.probe

<400> 485

```
cagtgggcgg ggaagtactg ggcccaatca gctgagactc ggctccagcc acacttacct     60
ttgctggtgc ctcagagcct gttgccccat ctcagcctca gatttccctc ccatgtctgt    120
ctgtccttct ctcttccacc caggcatact gtaaactcct ccagtggaaa ccagcatttc    180
tcctgggctg cacccgtgag gcctacaggt cccatctatt cccagcctca cctcttccct    240
tctgccactg cagaaccttc cacacccctt tctcctctat cctggcacca ctgatgctca    300
ctttgggcac ctaccagatt attcaccccc tcaacagccc tgccccatct tccctctttt    360
ggatgcagtc aaatggagtg atttgtcaag ctcttgaagc tttctaggtc tcagtttcag    420
tttccttatc tataaaatgg gaataattta caggctcctg ggagttttgt gcaagttaca    480
tgagaccatg tgtgctttgc tggcgtgtca taatggatcc tggatgaggc ccagacatct    540
gtgtgtttct gtcacctcca gtgtggtagc agtggtatgg gctatgctga gaaacattgg    600
ttaagacagt ggttctccac ctggctgtgc attaggatta cctgggaagc tttaaaacgt    660
tcaggtgcct aggctgaacc tgagctaatt aaatctgaag ttctgtaatt tttaatgacc    720
cctcctcaca tgattccaat gtgcagccaa agttaagagt aaaggctctg gggcagcagg    780
actgggttgt actgactctg ggccagtcac taacctctca gcctcagttt ctccatctga    840
agaatgggaa taatatatgc actttactgt ttgactatgc agattagttg agataattct    900
gtccagtggt tggcataggg cctagtacag tgccttctgt tgactggatt tgggagctgg    960
cagggtgggc atgagaggcc cccatcagag tgaggccttc tcagcagccc tgggctcatc   1020
cagaacacct caggcctctg ggctccaggc tgtcccgtct cttgctgctc tatcccagca   1080
agttcaacag tcgagctctg agcatggcct acaggagcca gggcacgttc acgccagtga   1140
ccctgtttta aagtatctaa tacaaagtac tattcagttt aaaaagtttg taatgattcc   1200
atgaaaaagg caaaccaata gtattagttg tttttgttgt tgcttaattg ccaatatgca   1260
gtatgttacc taatttatgg gttcaaattt gatttaaaac acaaaagcaa aaaaaaaaaa   1320
aaaagaatag aaagttttgt ttcatgtgtg gtaacctgga aacatggcca ccatttatat   1380
tattattatt attttttgag acggagtctg gctctgtcgt ccaggctgca gtgcagtggc   1440
acgatcacag ttcactgcaa cctccgcctc ctgggttcaa gtgattctcc tgcctcagca   1500
tcctgagtag agtagctggg actacaggca cgtgacacca tgcctggcta atttttctat   1560
ttttagtaga gacagggttt caccatgttg gccaggctgg tcctgaactc ctgacctcaa   1620
gtgatccgcc cacctcggct tcccaaagag ttgggattag aggcgtgagc caccgctcct   1680
ggccataata ttattttgt gaaaatatgt gttctgaggt ctaaacattc agcttacaaa    1740
tttccttttg gaacgtaagt ttttgtagag tatgtaagtg tatgagtgtg tgttttgaat   1800
gtgtgatggg agtacatgaa tgtgaatgtg tgagtgcaca agtgtgtgtg cgtgtgtgtg   1860
ggtgtttatg agtgtgggtg tgtgtgcgta ggaatgtgta tgaatctgga tgtgtgtgtg   1920
tgtgactttg tgtgtgtggg gagggctgca tgtgctgaga gctgtcaaga atggggactt   1980
ctatttctcc tcaccaatct ctagatcctg ctttatttta tcctctccta agccttatgc   2040
aaacttaaga caactcaggt tgagaccctc tattactgtc aaaagccatt ttgaagcaat   2100
tcaaatgttc tgtttctatt cttctgggaa atctgtgctg tctcagaaat tctaatggca   2160
ttctaaaatc atctgttgct ttgtaatatg gccttttcaa caaataggcc tagtctcttc   2220
tgctttcgcc cagggttgga gggcatcagg atggcagaga gaatgggcag ggtggtgaca   2280
cctgtggaca gccagcatca tgccgggttc tgtgctattt gcttccttca catgacttcc   2340
tttattcatc agaggaaccc tgcaaggtca gtattattat ttccctttga aaacacatgt   2400
```

ccaggaagtt tagcaacttt ccaccgtgac acagagatag gagcttaaac cttgtactct 2460
ttttttctac actacattgg taactgatct ttattttcct tgcctttctt ctctttttct 2520
gtctttatct ttgagccttg atatgtcatt actggtgctg ggtagtgggc agtttctgct 2580
ggaaggtagc tggcattcaa ctgtaaccct ttgccctgtc tggtccaggg caaagtctcc 2640
tcaatgggaa taaaatgcct ttgccttctt tagctcattt cttcacactc agggctacaa 2700
agcaaaatgc tgcagtttct gaaatgggag aaaatattcc gtaggatgac taaaatttcc 2760
taaattttg caacatgtag caaaatggag cataatttca aattgtgttt atctgagctt 2820
tgcttaaaaa cttactgtgt taaagccact attaaaccca gctactcagg agactgaggt 2880
gggagggttg cttgaaccct ggaagttgag gctgcagtga gccatgatca tgtcactgca 2940
ctccagccag ggtgatagag taagaccctg tctcaaaaca aaacaaaaca cagaaaaact 3000
gctgtgtagt tacaagattc ttcaactagt caggacttct agcaagataa tgtaatgaac 3060
tctgggtaca tggagagtta tgtggtcatt gataatcatg atcactctga tagttcctgg 3120
gaaagaggtg tatttctgt ctctttctgc tacctggcct gatggcagtg aggacagccc 3180
tgctgtagtg ctcagcagaa acctggatga tgtgctgtca attttggaag ggtagcaagt 3240
gaacagagag ggtgagacca gggaataatt tgcgggggtt gcaattggtt tgaggaaaaa 3300
cctttttttt tttttttttt tttttttgag atggagacag agcttggcag agcaagctct 3360
gtcacccagg ctggagtgtt gtagcgcatt ctcggctcac tgcaacttcc acctcttggg 3420
ttcaagtgat tctcatgtct cagcctcccc agtagctggg attacagatg tgcaccatca 3480
tgcctggcta atttttgtat ttttagtaga gatggggttt caccatgttg gctaggctgg 3540
tcttgaactc ctgacctcag gtgatccacc caccttggcc tcccaaagtt ctgggattac 3600
aagtgtgagc caccatgccc ggctgaggaa aaacatttct atagtttaat atatcatttc 3660
ttttcatcta ttaccaaaat tatactgaaa gagttacatt tagagttcac atttcatgaa 3720
agcagttctt ttacatggtt caggtttgtt tttatgtgat caaatcttgg cctctctgtt 3780
tcaccttacc tagagtatag ttttggtaac agtgattcag tagtaaccaa agtcctcaga 3840
aagtctttta ggtttctttt gagtttacat agataataaa aatattttaa aaacatttta 3900
ctatcattac tgtcttattc cattttgttg ctataacaga ataccacaca ctgggtaatt 3960
tataaagaaa agaaatttat ttctcacagt tctggaggtt aggaggttta atatcaaggt 4020
gctggcatct ggtaaggacc tttgtgctat gtcatcccaa agcggggggg caagagaggg 4080
ccaagctcac atttataaca atccactcct ataacaacat taatccattc atgggagcag 4140
agctctcatg gcctaatcac cccttattgt cctacctctt aataccatta taatggaagt 4200
taaatttaag catgagtttt ggaggggaca aacattcaga ccatagcaat taccagtatg 4260
ctgtttgtcc tcttattttc cttttcatag aaaaatgtaa taaatggaac atactagaag 4320
gatctatggg aacaacattc agggaaaatg ccattcctct tcaaccaatc cagggaaaac 4380
gtacagagaa actctaattt ttgtgagttt ttgttaatgc tatggcagta tttcagctgt 4440
gggtcacctg gaaattttat cactgcagaa ggtgataaac atttcattat acaatgctct 4500
attcttttat actttcctag aggcaataac tacatatacc gacaatgaag atctttttaa 4560
atagacacgt ggggttttga agcacttgag attttattga ttaattgatt ttttattttt 4620
ttactttgtg tcaaccatct cttagaatct ggatttgggg gactgactta agattggctg 4680
ggagaagtca gtagggaacc tcaggatttg tccagaagct gggaagtttc attttttttt 4740
tttttttgag acgaagtctc gctctgtcac ccaggctgga gtgcagtggc acgatctcag 4800
ctcactgcaa gctccgcttt cgaggttcac gccattctcc tgcctcagcc tcccgagtag 4860
ctgggactac aggcgcccgc caacacgccc ggctaatttt tttgtatttt tagtagagac 4920
ggggtttcac cgtgttagcc aggatggtct ggaactccca acctcgggtg attcgcccac 4980
ctcggcctcc caaagtgtgg gattacaggc gtgagccacc acgcccggcc ggaagttgta 5040
gattttaaac cagttgacta gtacaggtgt acccaagagt ttctcaaaat aagagttacc 5100
aacacccaaa agttacagtc atcagccagg gccacaattc tctgctcagc tcctcctgct 5160
gttgttcctc tcttcccgag ccgttctctg actttgagag cctctgcctg tccccaggcc 5220
taatgtagac ctctccttcg agatctgtca tttgggaggt tattatagta gatggtgata 5280
tgggttcagg gcagctagtt tgcagtttag aatctctgcc atgtactgaa gcccaaaact 5340
attaaactga gtatacaaga atgttttggt gctgccccag cttcttggct ctactttctt 5400
atctaatctt agttgtcaat gatttcaaga gcaaacagcc aaggaagcct gaagaccaac 5460
agaaactttg agcagacctg gcttgcaact atttataaat taattttgtt attccttctt 5520
tcctgtatat gtgtagttcc atgaattcta ctttgaactg gttataacat cttactggtt 5580
ccctcatgaa tgagttacat aaaaatttg atatgttttc attttatgtt gtatgaaagg 5640
catgattttt gcctctgaaa gtatttttta acattaggta cattatatgc taagtacttg 5700
tctagatact ttatgtgttt attttgcaga cacacagaac acatttgtat atgccaggtg 5760
ctgttttaag tgctctataa gtattaacta attcaatcct tagaatagga ttatgtcatt 5820
taaacttctc ttcagctctt attagcccta tcttattaaa tggaggctca gagaggccca 5880
aggttgtgtt attaaattgt agggtctgtg tgaggctgag gcaactgccc tccccagaag 5940
acactgggag ctccctgcta gggactaaat gtttgtgtcc cctctgcaat tcatatgttg 6000
agaccctaac ccccaacgtg atgttatcag gcatttggca gataattagc tacaggtgag 6060
gttatgagca taggccccca taatgggatc tgtgccctta taagaagtga ccaggaagct 6120
tactcttttct ctgtctccaa catgaaggtg tccttctgca aaccaggaag agggccctca 6180

ccaggaactg attggccagc actgtcatct tggacttccc agccttcagg actgtgacga 6240
ataaattgct attgtaagcc acccagtcta tggtatcttt gttatagcag cttgagccaa 6300
gatataccct ctaatggtgc tgtaggatgg ggtgaggaaa ggccccccagc tctggttttt 6360
gggaagctgc atctttattt ttattatttg tttgagacag ggtctcactt tgtcacccag 6420
gctgaagtgc agtggaacga ccttggctca ctgcagtctc aggctcctgg gttcaagcaa 6480
tccccttgcc tcagcccccct aagtagctgg gactacaggc acgtgccacc atgccctgct 6540
aatttttgta tttctttaag agatggggtt ttgccatgtt acccaggctg gtctcgaact 6600
cctgagctca aaccatctgc ccatttcagc ctcccaaact gttgggatta caggcatgag 6660
ccatccttcc aggccagaat ctgcatctct aaagagcaga ttctcctgcc tcagcctcct 6720
gcgtagctgg gactacaggt tcgtgccacc atgcccagct gatttttttgt atttttagta 6780
gagaaggggt tttactgtgt tagccaggat agtcttgatc tcctgacctc gtgattcacc 6840
ctccttggcc tcttaaagtg ctgggattac aagtgtgagc catcgcgccc ggccggttta 6900
tttttttaaa aatggctggg cacagtgact tgtggctgta gttctagcta cttgggaggc 6960
tgaggcagga ggattactta agcccaagag ttggaggctg cagtgagcta tgatcgtgcc 7020
accacacact ccagcctggg tgacagatca agaccctgtc ttaaaaaaaa aaagtatgat 7080
tattatcaga gtctctaggt gacagtgaga ggcagcctgg tgcaggggat agggcacagt 7140
tgtaatctgc tagactgggt tcaaatccta aatcggccat tcagagcgtg aatacattag 7200
aactggctta aattagcatt taaaatgtca acaggattaa gtaagtagtt ccttctcact 7260
ttttccacag gaggtgagaa aggtctcagc aaggccctag gagggaaggg cgtggggatg 7320
aaagggatcc agagagtctg tgcattggca gagaagatag tgtaactggc actgcggctg 7380
gagggctggt cccacagtga gctacagccc tgcccgctgg ccgtgggaga ggcttaaaac 7440
aaacgccgga agcaactccc agccccataa agatctgtga ccggcagccc cagacctgcc 7500
tgccttcctg acttctgttc cagagcaaag gtcattcagc cgcttgaatc agccttttcc 7560
cccsacccgg tccccaactt tgtttacccg ataaggaagg tcagcattca aagtcaagwa 7620
gcgccattta tcttcccgtg cgctctacra atagttccgt gagaaagatg gccgggaact 7680
cgatcctgct ggctgctgtc tctattctct cggcctgtca gcaaagtaag aggcatggga 7740
agttcgtgtg tgtgcgcgtg tgtgcgtgtg tgtgtgtgtg tgacaaggct tgcgggagag 7800
agagggaggg agggagatgg gtccggtgtt ttgtttccta cttgcccttg caggtagctc 7860
tgggtcctca gagcacagtc gcctcagggt cacccatgcc gcctgctacc ctccttccca 7920
ggggcaagca gagactgaga acattccaga gattagttct cccaactgga acgctgtggg 7980
gcctcagagc tcagcgattc tgcatcatct gtgattacga cccacagccc gttcaaacga 8040
gcgttagtag cctgctaacc tgcaggaagt ggtgtgaata ttaattacaa gtgttccaaa 8100
ggaaacgtgc ctgcttctaa acctggttgt gatttcttga acgttgatgt tttaattaat 8160
gtgttttctt aaataaactg cctatggtgg tatgattatc agattgaaaa aaacttcctt 8220
cagaaatatt agctttagat taagtaatta gctctaaatt ttaaaacagc ttcccactag 8280
gattattcaa tatctcgact gcctggttaa atagagggct tttactccat gggagtcaca 8340
tttgctcaat tcatattatc ttacctacaa tgtcagctgg ggaaaggggt ccgagtgcaa 8400
gagtgcaaga cttcttacca agcatgcata tttctttata tttccaagta tgcctgtacc 8460
aggaaatagg gccaagccgt agactgctca ggttacacaa acagccagaa gcaggaaggc 8520
ctcacagctg aggatccagt ccaggcctcc gtgcatgctc aatgtcttgc ctagccagtc 8580
tgttttgaga ttgtatgaag atcttgaacc ttcagactca cgttttgtgg tttgattcac 8640
acaatctgtt gtggagaaga gcaggatatt tgtttctaat taaagaagca cttagatctg 8700
ttaaaaggaa aaccctagac aaattacatt taactgagtt taattgagca aagaacgatt 8760
cacagttggg catggggaac cggaataggt tcagagagac tctggggctg ccacgtggta 8820
gaagaagatt tatggacaga aaaaggaaag cgactcccag aaaacggaag catatagttc 8880
acgatgtacg gagaaacctt taggctgaac ttaaaatatg tcagaaggca gctttaggct 8940
aaacctaatt tagcaggtct aaggctcact gttggtagca tcctgactca caggtgctac 9000
caaacctgca tgcgccttta tcctaggcct gcaggtccac agatgtccca aagctgacag 9060
aggtgctcat cctccctgtt tgctgcttgt gcagggggct gtgtgaactg gggccagaat 9120
gttaggggc tggatggagt gggggtaggg gtggggagag aggagaggag actgggtgaa 9180
tgatgattaa tggtggacag ggaatgttta atgacaaggt gttagggccg ggcacggtgg 9240
ctcacccctg taatccgagt actttgggag gccaaggcag gtggatcacg aggtcaagag 9300
atcgagacca tcctggccaa catggtgaaa ctccgtctct acttaaaata cacaaatttg 9360
ctggacatgg tggcacgctc ctgtagtccc agctactagg gaggctgagg caggagaatc 9420
gcttgaacct gggaggggga ggttgcagtg gactgagatc acagcactac actccagcct 9480
ggcaacagag cgagactcca tctcaaagaa aaaaaaaaga tgttgatgaa gagttgaact 9540
ttgtaaaata attgaaaagc tgtattctga gccaaacatg agggacgagt ggccggtgac 9600
acagcccaag gagattctaa gaacatctgc ccaaaggggt ctggctacag attggtttta 9660
tacattttag ggagacataa gacatcaacc aattcatgta agatgtacat tagtttggtc 9720
ccaaaaggcg ggacgggaga cataagacat caaccaattc atgtaagatg tacattagtt 9780
tggtcccaaa aggcgggaca actggaagtg gggcatgggc ttccaggtca taggtggatt 9840
taaagatttt ctgattggca attgattaaa agagtttatc tacagacctc aaatccatag 9900
aagggagtgc ctgggttaag ataaggggtt gtggaggcca aggttcttac tatgcaaatg 9960

aagcttccag gtagcagcgt ttggagagaa tagattgtaa atgtttctta tgagacttga 10020
aaaggtgcca gactctgtta attctctcct ggatcagaga aaagacctgg caagggaaag 10080
ggattttcta cagaatgtag actttcccca caagagacaa ctttgcagga ccatttcaaa 10140
atatgacaaa gaaatatatt ttagggtaaa atacttcaat ttctttcaaa ggcctgctat 10200
ctgtcatgtg atgctatact ggagtcaggc tggaatttgg tgtcttattt ctacaaaaag 10260
tcttaagtgt tagatttttt aaagggaagg tggggttcaa gaaagacaca gagagagaga 10320
tagagagagg agggggaggg ggtagagggg gagagggaga gagagagagg ctttacaaca 10380
acacaggttt attacaaaaa cctgtggagg tggggggacca gcttaatgcc agagcccgcc 10440
attgcttaca ggctggggta tgtacaggta tgggcaggag ggttttgggc agtatggctt 10500
gctgccctgg aggatattga taagatgttc ttatgttcag gtggtttggc cagggatatt 10560
ccttgggctc tgtcaggatt tgatagggat atttcttcag ttgggtcttt gcttggcagg 10620
gtatgataaa gatgttcctg tgtcttgtgg tcaggtggct aggcaggatt tctcagggcc 10680
cgaaccccca tggaatgttt cactttgaca aaggcctgtg aaatggcagg gggcttacaa 10740
aatggtgtag tttggactaa cattaaggtc tgtttcaatg ttaatgctgg tcagttatgc 10800
ctgaattcca aagggcggag gatataatga ggcacatctg acacctttttc cccatcatgg 10860
cctgaactcg ttttttaggt taactttgga atgcccttgg ctgagaggag gactccattt 10920
agatggttgg ggggcttaga attttatttt tggtttacaa aggagagaga gagtgtgtaa 10980
gagggagaca gagagaggaa gacacaactg tttagctggc attaggaacc agaatttgct 11040
ccttttgcaa gtctgatgtt catggagtcg ggacaccatg tgccatggtt gaagcagctt 11100
aggttttgaa gacacagagc tgtatctgtt ggtctagcca cttcccagct gggtgtcccc 11160
gttcaggata cactgccctt caatgttacc atcaaagagg gtttaatggg acgctgggga 11220
ggaaatccca ccatagttcc tggccccatg taggcgctca gcactgtcag ttcccatcct 11280
aaggtccttc ttcgaagaga gaagtttctg ctgcatggag aggtagagac agggtgaagg 11340
tacccagctg tggctgggtg ggcatcacag gggtaaggg gcagagaaag cttccccttc 11400
accctctgaa ggttcactga acgtcaactg acaaaaggca gattaacagg agaaaggctg 11460
tgacaattta ttttaatatg cacagcatga gagaatggtt gcccaataat ccaaaggggt 11520
acagcagttt gtataccatt cttcacaggg gagggaggaa atggggaatg cagacaattc 11580
ttttgagtgg tagtaaacaa ttattaggga gaatgaatgg actgggagac aaagtaactt 11640
gtcaatgatt ctctggaatt tgattggcca gaggggtagg cttaatctgg tgggaaagtt 11700
cgtccaggtg tgtgcattcc tcggtcttct tttccacatg agataatgag gtctgaggga 11760
ggagacggca ggcaattgtg tttgtcttag gggtctggt ttctaggtag ataagggagt 11820
ttcagaggac tgcccagctg catcctgtgc cttggtagag accacaggtt gagacaggag 11880
gcagtggaga ggtcacaaag accttgaggc agcttcttta gttcagtcat attttgaggt 11940
gtaatattct gatttccttc ggtggggggc gggggggggg gcggttaaca gaaataccaa 12000
actctgtgaa atcttttaaa gaggtttatt ctgagccaat atgagtgacc atggctcgga 12060
gaaaacacaa acccaagaag ccttcagtgg gtggtcccaa ggcaattggg ttacagtttg 12120
gttttataca ttttaaggag gcaggaatta taggcaaaga cataaatcaa tacatggaag 12180
gtatctattg gttcggcccc aaaaggtggg atatcttaaa gccagggggc ttacagatta 12240
taagtggact cagagattct cagatttgca gttgttaaag gagtaaagct ttgtttaaag 12300
acttgaattc agcacaaaga aatgcttaca ttaagagaag gggtggccag gtgtggtggt 12360
tcatgccttt aatcaagcac tttgggtgac caaggtgggt agatcactta agcccaggag 12420
ttcaagacca gcctgggcaa catgatgaaa tgctgtctct attaaaaata caaaaaatta 12480
gctgagtgtt gtggcactca cctgtaatcc cagccacttg ggaggctgag gtgtgagaat 12540
tgcttgagtc catgggttgc agtgagccga gatcatgcca ctgcactcca gactgggtga 12600
gagtgagacc ctatctcaaa aaaaaaaaaa aaaagataaa gaggtcatgt gatgctatac 12660
cagagtcagt tggaaagtaa gccacatact gggttaattt aaaagaactg gcctaatgag 12720
atttgatggt ttgtagggcg tgacttaacc cttgccttgt aaggccttag gtcttgtcta 12780
caatttgatg tcttcttgct gcagccggct ttgttagtct tatgatcccc attttaacct 12840
taatcctggg cagttgtgcc taaactccaa aagggagggg gcacaaccag gtgtgtccta 12900
cctcccttcc cctcacggca gggaattcag tttttcaggt ctctctgggg ttccctcggc 12960
caagagtggg tctgttcagt tgtttggggg ttaggatttc atttttggtt tctcctcagc 13020
acatgtgtat ctctggagaa agctagggaa agaggaaaga caggcaaagc aaaaggagat 13080
tattatctag aggttttaat caactcataa gggaaataaa gttgaaactc tacagaaaag 13140
gaggtgaaat agcaccccac tgtcatcata tattcatcat atatacgtat atatattttc 13200
agacctactg ctctgtcgcc cgggctggag tgcagtggtg tgatctcagc tcaactgcag 13260
cctctgcctc ccaaggtcaa gcaattctcc tgcctcagcc tcccgagtag ctgggactac 13320
aggtgcgcca ccatgcccgg ctaatttttg ttgttgttct ttttgttttt ttgagaggga 13380
gtctcactct gtggcccagg ctggagtgca gtggtgtgat ctcggctcac tgcaacctct 13440
gcctcccagg ttcaagtgat tctcccggct cagcctcctg agtatctggg actacaggca 13500
cacgacactg cacctggcta atttttgtat ttttagtaga gatggggttt caccgtgtgg 13560
gccaggctgg tcttgatcac ctgacctcaa gtgatccacc cgcctcaacc tcccaaagtg 13620
ctgggattac aggcacgaac taccgcacct ggccccactc tcatcatatt attccaaaaa 13680
tagccatggt ctctctgagg cccctcacct gagagaacag aagggcttct atccccttggg 13740

gctggaggtg agcccagctg aatcctcttt cttggggctg gactgggagt aggtgggata 13800
tctcagtacc tcttccattt actgaagctc attcctcaca cttgctagtg tcacaaacag 13860
atgccccattg ggtgcaatta acgtaagaaa agatagcact tttaaggctt gcttactccc 13920
agcctcattt tgaagctcta ggctcctgtg cattactaat tcataagtct aggagcacat 13980
acctttcatt atttccattt tataggtgga aacaagaaga cttgccaagt attttctgca 14040
aggctgtgaa gcatgttaga agcaatggta atagcaggtg cagagtggcc aagtcctcat 14100
ttcccctca atggacaaat ccctttagct gagcccatga gtcattggcc tttccagatt 14160
gatgctgccc tggagggatg gagcccaggg ttggagacag tcagggccca tctgtacatt 14220
ccttctcctc actctgctct ctcaggctgc agtatgaatg agaaaactcc aatcccaagc 14280
atttacacac tttttttttt ttttttgact cggagtacag tggtgcgatc tcagctcact 14340
gcaacctccg cctcctgggt ttcagcaaat ctcctgtctc agcctcctga gtagctggga 14400
ctacaggtgt gcaccaccaa actcagctaa tttttgtatt tttagtagag atggggtttc 14460
accatgttgg ccgggcttgt ctcaaactcc tgacctcaag tgatccacta gcctcagcct 14520
cccaaagtgt tgggattaca ggcgtgagct actactgggc ccggacagca tttacacact 14580
ttagatattc agtgacaatg tttaaatcct aaacaacagc ttatttgtag cagaagggga 14640
ttagtggaag ggaagagcct gctttgagag cccttgctac accaggatca atattgactt 14700
cttgtgagag gggagatca tatcctgttc cagtaggaaa cacattgcct ctaatactct 14760
taacacaggg agagacccaa actatacact tggtgtggaa ttttaaaagg aaacattttt 14820
aaaatattag caggtaggaa acagatattg aggtactaaa acaagtcaga caaggagata 14880
gtgggggaga caactggtaa ggagctaaaa ctgaaataaa taatgtgaag cgaaataata 14940
gttactgtaa cccaactgaa tgtgggtcca ttcacccagc atacagaaac aaacacaagc 15000
accaggattt gcagtgacag aaagggaagc attgattgca gggtgccaag caaggagaac 15060
tgggcagcta atgcttaaga ccctaactcc ccaatggctt tcaggcaagg gttttaaaat 15120
tttatttatt ttattgttat ttgaaacagg gtcttgctct gtcacccagg ctggagtgca 15180
ttgatcctgg ctcactgcag ccttgtcctc ccaggctcaa gcgatcctgc ctactcagcc 15240
tcctaagtag ctggaactac aggcgtgcac catcaccaca cctggttaat ttttgtattt 15300
ttattttcta gacatgggtt ctcactatgt tgtccaggct ggtctcaaac tcctgggctc 15360
aagcagtcct cctgcctcgg cctcccagaa tgctggaatt acaggtgtga accaccatgc 15420
ctaaatgggt ttttaaaatt tgagataggg tcacaccatg ttgcccaggc tggtcttgaa 15480
ctcctagtct caagtgatcc tcccaccttg gcctcctgaa attctggatt acaggagtga 15540
gccaacagac ttggcccaag caagggtttt taaaggcagg ggtatatttc aggaaagcag 15600
aaggtatagg aaagataata aatcaataca tggaggttat accttggttt ggcctcaaaa 15660
ggcaggatat cttgacacag gggcttacag gccacaggtg gattcagaga ttctttggtt 15720
tgcagttggt taagggagca aggctttgtc taaaaacttg ggtcagcaga aagaaatatt 15780
aaggttctaa gctaaaaagt atctgagaca atttagaaaa tttattttgc cagggttaag 15840
gacatgcccg tgacacagcc tcagggggctc ctgacaacat gtgcccaagg tggtcagggc 15900
acagcctggt tttatacatt ttagggagac atgagacatc aattcattta tgtcagatgt 15960
acattggttc agttcggaaa ggcgagacaa ctcaaagtct atatccaggt cataggtaga 16020
taggagacat tcttttgagt ttctgattag ccttttactg aatcaaagga agcaatcaga 16080
tatgtatttg tctcaagtga gcagaggaat cactttgagt tctgtctgtc ctttgtccac 16140
aaggaatttc cttgtgggta aattgtgagg gagctatgta gttttttttt tttttctttt 16200
tttatctggt gtctaggcaa agtggctcac ttctgtaatc ccagcacttt tggaggctaa 16260
ggcgggtaga tcacttgagg tcaggagttc aagaccagcc tggccaacat ggtgaaaccc 16320
catctctgct aaaaatacaa aaaactagct gggcatggtg gcacatgcct gtaatcccag 16380
ctactcagga ggttgaggca ggagaattgc ttgaacccag gaggtgaagg ttgcagtgag 16440
ctgagatcat gccactgcac tccagcctgg gtgacagaat gagactctgt ctcttaaaaa 16500
aacaaaatct ttatagctat cttatttagg tatagaatgg gaggcaggtt tgtccaatgc 16560
agttcccagc ttgacttttc cctttggctt agtgatttgg gggtcctaag atttattttc 16620
ctttcatgag gtttaacccg tgtgtgtgat tctctgcagg cccctcagga agaaatttag 16680
aacagggaat ggtggtcaga attcagtcct cagttcccgc ttagctgagg tctgtgtgac 16740
agtggtcagc attttccatc tggtggaggt cctggtttct gagaaacaac tcaagaactt 16800
atgtgaagac gttgtcttta gtttctatag ggaaccgaac gtcttgtaac tctgtcttat 16860
ttgggtgact attatttaaa ctattattat cttctcgctt agcgggactg aaggatagca 16920
aggtgcttgg aatttccctt gaaggaactc aagagttttt atttccgtgc ttgaggtggc 16980
tagcaggtcc ctaagagtgt tccttgctct gtctccatgc cagaagttta ttatggggtg 17040
aaaagaggaa gttaaggaca ggaggcaaga cctactgaag gctcaaaaac tgacagagga 17100
ggaagtatgg atgtggaagt gaaagaagtc cacagagctc atcacaagat gaaggtggtc 17160
ccaggcatca gagttgatta tctaatgaaa taaaggagaa gaaagtcata ctgttttata 17220
acagggaaac gtcggcaggt aggagccatc atccccarag ttttcttgta cctgctgggc 17280
aaggtgtcag agccctggca atggaaagtg attggcccac agggagtaag aatttaccga 17340
caacaatata ggtttgaaaa ggaaagtttt attagattga gagaaagctg cagaagagtg 17400
cagcgggctg cctcagcgag aggactgagc gcgccgggga ggattttcct taggggtatt 17460
tatggacctt aaagcaagag cttaggggta atttggtcca tattagccac atatgtcatg 17520

ataaatgatt acatttgtag acattttggt gtcgtaatgt cagcaagggt tgcacaatga 17580
gttttgtcat gcatgcattc cggagatgta tagaggtaga gaaagtctag ttacatataa 17640
attttgggga aagaagcctg gaaccggcct tagatatagg gaagatcaat tatttctaaa 17700
ctcctcagat aaggagttct gcctctggat ggtcggcttc atggccacca ggtggtcttt 17760
gctctcttta gtggcccctc cctcctggcc tcctcatctc caggcaatct ttatgtcact 17820
atagattcat ttgcattttg agaattttat ataagtgaaa ttattcagta gtactctttt 17880
ttaaaatcct tgctttcatt cagcataatt attttgagat tcactcattt ttgggggta 17940
tcaatagttt attccttttt cttattgtta agtagtatcc cattgtatgg ctctatcatg 18000
atttatttat ctactcacct gttgatggat actcttgtga gtcagggttc tccagagaaa 18060
cagaatcaat acacacactc acacatagac acacacacgg agaattattt taaggaattg 18120
gctcacacag ttgtgggggc tggcaagtct gaaatctgca ggaaaggctg gtaggcaaaa 18180
gttgatgtta caatctggtg ttgaaggcag tctggaggca gaagtccttt ctctttgggg 18240
gacctcagtc ttacctccta gggccttcaa ctgattggat gaggcccacc cacattaagg 18300
agggtaatct gctttactcc aagtcaattg atttcaatat taatcacatc taaaaaaaca 18360
ccttcacagc aacatttagg ctggtgtttg accaaacaac taggcaccgt atgctagcca 18420
agctgatgca taaaattagc catcacagag ttgttcctgt tacaaacaaa gctgctgtga 18480
atatttgtgt acaattcttt gtactgaagg aaataaaaat atttacccca agatagattt 18540
ctttgacaga ttttcaaatg gccctgcaaa gctgtttttt gtgggggaac tttgcattgt 18600
ggagaatctc catgaatgca gcaaggcctt cccttgtcca gatctgggag agattaatgg 18660
agtctgacac ctgtaaagat ctggaagaaa cacttaccat ctatttttctc taagggggctg 18720
ctatctgtga gatttcgtct gtataacatg actcccttgg ctagctgagc ctcctcttct 18780
tcccctccca tatcctgtct tgccactaaa acctgactta ccaccataac ctgttttttt 18840
ggccatgttc tgagcctgca ttgcttctgt gaacccaaaa tatctgatag gtctcaacca 18900
atttagaaag tttattttgt caaggttaag gacatgcccg tgacataaaa ggacatgccc 18960
gtgacatagc ctcaggaggt cctgatgaca tgtgcccaag atggtcaggg tacaacttgc 19020
ttttatacat ttcagaggga catgagacat caatcagtat gtgtaacatg tacattggtt 19080
tggtctaggt aaggcaggac aaatcaaagc aggggcttcc aggtcataga tagataagag 19140
ataaaggtta cattcttttg agtccttgat aagcctttca ctgaatacac aatttagtct 19200
ggctcagtga atctgcattt tttttttttt tgagacagag tttcactctt gttgtccagg 19260
ctggagtgca atggcatgat ctcggctcac tgcaacctct gtctcccagg ttcaagtgat 19320
tatcctgcct tagcctcctg agtagctggg attacaggca tgcaccacca cgtccagcaa 19380
attttttgta ttttagtag agacagggtt tctccatgtt ggtcaggctg gtctcgaact 19440
cccgaccttg ggtaatccac ccgtctcagc ctcccaaagt gctgggatta caggcgtgag 19500
ccaccgtgat cggcacattt ttacataagc aatcagatat gcgtttgtct cagatgagca 19560
gagggtgact ttctgtccag cacctgtgaa gataagcttt tccttgtggg caaattgtgc 19620
tttgtagcta tcttatttag gaattaaaat gggaggcagg tttgcctgct ggagttctca 19680
gcttgacttt tcccttggct tagtgatttt ggggtcctga gatttatttt cctttcacat 19740
ttctgtagcc tcaagatggc atatgaactt ctgtacctca ctgtggagcg gggtctttgt 19800
tctgaaggct gctatgtata catatgaaat aaatgtgtgt gatctttctt ttaatgtctg 19860
ttaattaatc tgcctcatgt cagtgatttt cagtgaacct ccagggggcc aagggctttg 19920
gcccctacag tatggacata caattttgtt tctcttaggg aaatacctat gaatggaatg 19980
actgggttat acattggagg tatgtctaac taaaagactc ccttccaaac tgtttttcaa 20040
agtggttgta ccgttttaca ttcccaccag cattgtatga aagtttcagt tgctctcata 20100
tcctctcctg tcttttttaa ttgctatcat ctgtcttttt gattttagac attgtaatag 20160
gtatgtagta ctatctaatt gtgatttaaa tttgcattcc actaatgact aatgacgtgc 20220
cccatcttta acgcaacatt tccctttact tgcaggttat tttgctttgc aagttggaaa 20280
ggcaagatta aaatacaaag ttacgccccc agcagtcact gggtcaccag agtttgagag 20340
agtatttcgg gcacagtaag taatgcttct tccacccttc atggatctgt gcctgccata 20400
cagacacaat tcctgactag gggaaaggga tatggagaag accctaagtt catgtgcaat 20460
atctagttat aacaagtcac tcttcacagc caagaccgtt ctcagaatcc agaaagtttg 20520
aagggttcgg ggcatgaagt ctctgcccac atctattacc aattcagcag ggtatattta 20580
cagaaaaggc tgggatctca ggagggactg ctgtgtctcc caaggagggt cactgggtca 20640
aagaggcccc ctggagcctt gtgagaccac aatagtctga agttggattc gttatgctac 20700
aattttctat cttatttttt cctccctact tcaaaacaga ggtagatcag gattaatctt 20760
ttgccagttt ctctgtgaac catttcttaa aatcagaaac ctgtaagaat gagaaagctt 20820
gccagctcac atgcatgcgc catgtttttt gatcatcttg tgctccttgt tttttcagac 20880
ccacacaaaa tgttccacca taaacaccgt gaacttgcct gctgcaggaa tgctagggcg 20940
tactttatat ttcatccccc aaaatacgtg ctttaagaaa catttcatgc gtcaccactg 21000
atatatcatt gggttctttt gttaccatct tgggattttt catttatgtt tacaagtgag 21060
attggcctac actttgcctt tcttgtctgg ttcttgtctc atttgatac agatgaacct 21120
agtgtcaaat gagttggagg aagcatcctg ttctactttc tcagataatt ttaataaggt 21180
tggaagagtg tgttccttca aggttgtgta gaacttgcct taaaataatc tgggcctaat 21240
gtttttctgg tgggaaattt ttaaattatg ggatttgcat tctttaatga ttattggact 21300

aggcaggctt tttgttacat cttgaatcaa ttttggtagg ccttggtcct tgagatgata 21360
acattggtct cttgattatc cacttgctcc aaggagggtt ttacagacta ctccctaggc 21420
ttgaaagcgt ggggtgaata ccaccgggag tacacgagat aattgtaaga ggtgtgtaga 21480
cttggtttta aataacattg aactacatga tgagaagtca ttctcatttc aattatcttt 21540
gaatccttat ggttaagtca agtggaaagt attggtttgg agccagcatg tcccrcacct 21600
caaaattaat gtttattttt atcaaagtaa tttatgtacg tgatctgaag ataaagtggg 21660
attacaggtt ttatgacaac aaataactct tccttgtccc tacctcccct ctccctctat 21720
ccctagagat aacccttttt gattactact ttgggtattt acttctgtat ttctaaacat 21780
tatactttca ctgctattta ttgattcaca aattttagac attgtcagtt ggcttctttt 21840
tatggaagaa ataatttcat gctcctttac ttcttctttt ccttacctca tccttccagt 21900
aacattatat cactctgtta ggttaaatca atagccaatg tttataatta ttgtgattaa 21960
gaaaataatg ttgaaagcag ggccttatag tatactgtgg ttacatttcc ttcttgtgca 22020
atttttttc cctagggtta ataattgctc tttatttctt tgtttgctta gtttcatgcc 22080
caaatcttct gcagaactga aaatctcctt tcgttacatt caaatgcatt agataatcta 22140
gtaatacatt tttctgtctt ggagtcattt ctgcctgagt cctccacttt tcgcttcaag 22200
ttgcgggatg ctcttggtgc ttgctgcaca gctgctatcc tgggacttcc tgtcaccatc 22260
ctagaaattt cccttatact gtcctgattt ggatttcctg gacctcctgt tttcctgttt 22320
attggtttc tctctctttt tagtggagta caccccttcag aagcttcctg agaaagggtg 22380
aatgtttggt aaagttttga gacttcacat atctgaaaat gcacctttat cttgcctcac 22440
agttaaggga tggcttggtt aaatgcattt ctgtattaga aatccttacc cttagagttt 22500
ttgaaaaaat ttgctccatg gcttcctagt tcccactgat attgttgaga tgttctgtgc 22560
tattctgatt ctggaccttt tgtttggaaa ctatggaagc ttcaggatct tgcctggcct 22620
actgtcccga gatgttaata gcattgtgct tgttgagagg ctttttcttc actgtgctca 22680
accccaggtg aggcctttc acagcctgaa aactcatgtc cttctgtttt agaattttt 22740
ttttgttttg agacagggtc tcagtgttgc cttggctgaa gtacagtggc acaatctcac 22800
ctcactgcag ccttgacctg ccagcctcag gcaatcctcc tacctctgcc tcctgagtat 22860
ctaggactac agatctgtgc tacaatgccc tgctaatatt tgtatttttt ggtagagata 22920
aggtttcacc atgttgccca gtctggtctc aaacatctgg gctcaagcaa tctgcccacc 22980
tcagcctccc aaagtgctgg gaatacaggt gttagccact actcctggcc cagaatattt 23040
tttaatatta tttcttcccc cagatttcct ctccttcatt ttctctgttc tttcattctt 23100
aaaacgcata ctagttgtgt gttaacctcc aggactgatc ctctagtttt cttttatctt 23160
caggttttca taattttgga tttttgttct ttcagaggaa tttcctcaac ttctaacttt 23220
ttatctgaaa tttgagatat cataatttta atttccaata acttattttt attctctgat 23280
tgctttttag ttgttggtgc tgatgttaaa ccgtcacttt tttatttcat tgatgtagtt 23340
tattctctta tatttgagga ttttaattat aattttaaaa attctctgta acatttctgt 23400
ttcttccaag tatctttgtg tctttttgg cctttgtctt tcatttgtat ttattattta 23460
cccattaagt atttattgag cacttctgtg taccgggcat tgttataata gcttggccta 23520
gatccatgaa caaatcccac aaaaatcctt gccctcttgg agtttgctga ggaagccaga 23580
aaataaacaa taagcagatg atattataag aaaataatat aagctgggca tagtagctca 23640
cacctgtaat cccagggact cagtaggccc aggcgggaga actgcttgag accagaagtt 23700
caagagcagc ctgggcaaca tagcaagagc cccctctctc tattaaaaaa aaattaaaaa 23760
attagccaca tgtgggtaca tacctgcagt cctagctact tgggaggctg aggttggagg 23820
attggttgag cccaggagtt caaggcttca gtgagctatg atcatgccac tatactccag 23880
cctgggcaac agcgaaagac cccaactcaa aatgaaagag aaaatcatat attatgttag 23940
aaggtaattg gtgctaaaaa aaaaaaaaca aaaacaaaaa ggatagcgtt gtaagagggt 24000
atgggatggg ttcctggggg gtaagttggg tggtctgtga tatacaataa agctatgggt 24060
gtaggtcccg ctgagaaggt ggcatttgtg aacatgtaga gaaagagctc ttgggtcaga 24120
gacaatagtc agagaaaagc ccttagctga ggatacaccc agtgggttag tcaggaggtt 24180
agggggctgc agggagcctg gcagggagtg ggaggaggtc aactcagaga ggtcatagac 24240
ggctgggtca cactgggcct taaaggccac tgtaggggac tttggctttt ctctgagtga 24300
aatggaaagt catgacaggg ttttaagcaa aagagtgaca tgatcttata ttttaaaagg 24360
agaatcctgt ttgctttgct gagaagagcc tgtatagagg cattggtaga agcagaaaat 24420
gcatttagaa ttccaggcaa gagatgatgg tggctgtgtt gcagaatttt tgctccttag 24480
ttcagctaaa actgggttct tgtcacatgg ccaggaaaga ttaggcgtgc tgacacattg 24540
aagggtgagt agagcagaat ttattggatg taaaggaaaa aaagaaaaaa aaaaaaagaa 24600
actcaacaaa gcgggagggg gtcctactca caggcccctg cctcgcagat taattccagg 24660
tcaccaccca cgagctggag accaggctcc tccctctgca caaggttcga acttcctgtg 24720
gctccaccct gttcctccag tgagcatgca ggctttattc agaaagaatc agtttggaaa 24780
gggcaggcaa gcgggggcag ttctccctca gggtggcaag tttcatcctg gaccagcagt 24840
ccaggctttc agccttcaag ctttttaga cttgagggtg gggtttcacc agggaccctt 24900
ggctgtctcc tgtctctgtc agctagagcc agggagatag cagtgaaatg atgggaagtg 24960
gtcaggttct ggatatattt tgagggtaga ctcatagaat tttctgacat aatgaatgtg 25020
aagtgtgaga acagtcaaga atgattcttg gattattggc ctgagcatcc atcccaaagg 25080

atggccttgc gatgatctga gatggagaag gctgcaggag aactggtttt ggggaagtat 25140
taggaggttc tgtcttgaca tgttgtgttt gagatgtgac ttaaggagag gccttaagca 25200
ttatgatcta ctaagctatt tggattgttt atgattgcat cataaactga caaatacatt 25260
gttggttaca cctccaagta gagaggcagc tggataaaca aatctgcatt ttaggagaga 25320
gagctgagct ggaaatataa attcaggggt cattggcaca ggaagatgat ttacagccat 25380
gagactggat aaaatcactt aggggagtgt gtgtaggtgt attagtccgt tttcacactg 25440
ctgataaaga catacccgag actgagcaat ttacaaaaca aagaggttta attggactta 25500
cagttccatg tggctgggga agcctcacag tcatggagaa aggcaagtca cattttacat 25560
ggatggcagc aggcaaaaaa tgagagagct tgtgcagggg aatgcctctt ttaaaaacca 25620
tcagttttgt gttttttttt tttttttttt tttgagatgg agtctcaccc tgtcacccag 25680
gctggagtgc agtggccaca atcttggctc actgcaacct ccgcctccca ggttcaagca 25740
attcttgtgc ctcagcctcc tgcgtactgg gactacaggt gcctgccacc atgcctagct 25800
aatttttttgt atttttaatg gagacagggt ttcaccatgt tgactgggct ggtcttgaat 25860
gcctaaactc aggtgatctg actgccttgg cctcccaaag tgctgggatt acaggtatga 25920
accaccgtac ccggcctaaa accatcagat cttgtgagac atattcactg tcacaagaat 25980
agcatgggaa agacttgcct ccatgattca attacctccc accaggtccc tcctacaaca 26040
cgtgggaatt caagatgaga ctggggtggg gacacagcca aaccatatca gtaggtaaag 26100
aacaaaagag gactgagccc tgatggcatc aggaggtcaa gatgatgaca agggtcctgc 26160
agagagatct gaataaagga gatcttagag gttttaccca aacatctggt attaataatt 26220
ctcaactggc tgttaagaaa gaggcattag agagtgacgt ggaagctcca tgtgcatggc 26280
tggggcttat gattggtcag gaggcagctt tcttctggag aaccctcaaa gtatctgtag 26340
gtcatctttc tggtaccact tagtttctct agagaaaact ctttaaacct ccttcagggg 26400
ttgaaggaaa gaggggtgga gataggataa taggcctgac ttttgagagt gcagtagtaa 26460
agacaggaag gtttttcgac tgtgcagtgt gcagcatgca gattctgact gttttcagga 26520
cagaatttta cccccaccct ctattattcc tgaagtccct aacagtctct ggttctgttt 26580
ctccaggcaa taagcctccc acctcctgtg atggggagca ggctggggct caggagtggg 26640
aagcagaggt ggagggcagc taagccctcc ttattcagtt tttcaaccac tcttaagttc 26700
cagcctttgg cctctaaggt acctggggtg cccaattcct gagcttcttt tggttctggg 26760
gtaaggatgg tctcagtctt cattgcttct tccttcagca ggcacatagg tttcagcttt 26820
ctgtactcta ccagtccagc cactgttcct ccatgtgctg tctttcccgc agaattttgc 26880
taatggctct cttccactct tgagagattg tttggaggtt ccagcagggg agcatagcta 26940
ctcatatacc cttgaccgaa aactggtcct cctctatgga ggatggtcgt cctctttgac 27000
tgagcatgca gctttgggag ggacgcacat ggagtgggga gggaggaagg gaacacccgc 27060
ctagccagcc agatcagccg aatcaaccct ggcaattgat ggggtgacag atgttgcagc 27120
cagattaccc tcacttcctc tcttttactc ttgtgtctgc tctcattctc tttgttcttg 27180
attactcata tctatttttt tttctttaac tagcatttta gtgaagcttc agaagtgaga 27240
agaaatgagc atgggttcaa tctgccatgt ttaacaggaa gtcatcatga tacatttttg 27300
atatcctgtg cacacccatt ttcccttgtc aacaaaaaga gtcaaactct ataaatact 27360
taaagagatt tatgtatgta tttatttatt tttagacaga atctcactct gtcgcccata 27420
ctggagtgca atggcgcaat cttggctcac tgcaacctcc ccttcctggg ttcaagcgat 27480
tctcttgcct cagcctcccc agtagctggg actacaggca tgtgccacca caccccggcta 27540
atttttgtat ttttagtaga gacagggttt caccatcttg gccaggctgg tctcaaagtc 27600
ctgacctcaa gtgatccacc ctccttggcc tcccaaagtg ctgggattac aggcatgagc 27660
caccacaccc agcctgaaga gatttattct gagccaaata tgagtgatca tagcccatgc 27720
cacagccctc aggaggtcct gagaacacgt gcccagtttg tttggggtgc aacttggcgt 27780
tatacatttt agggagatat gaaacttcaa tcaaatacat ttaagaaata cattggtttt 27840
gtccagaaaa gtgggacaat gtgaagtgtg gtggggtggg agcagggggc ttccagctta 27900
taggtagatt taaaattttt cctggttgac aactggttga gtttatctaa agacctggga 27960
tcaacagaag ggaatgtctg ggttaagata aaggattatg gagaccaaag ttcttgtttg 28020
cagaggaagc cttcagatgg taggcttcag agagaatagt ttgtaaaacg tttcttatca 28080
gccttaaagt ctgtgttgct gtgaatgctg aagaggtata agggggtatg tctgactctc 28140
actttccatc atggcctgaa acagcctctc aggttaaatt tttaaaagag ccctggctga 28200
ggaggaagtc cattcagatg gttgggggggc cttagaattt aatttttggt ttacacccta 28260
ttcaacagag aaagaatctc atggtcagag ccgtcagcaa tcattagtat ctaactagaa 28320
tttagtaact ttttgctttc attggattta tttttatact taacctttat ttagaaagct 28380
acactctttc tgctcttgta atgatgtaaa atttattttt tcttaattta agcaaaaaag 28440
ctgatttgta gaaagatagt gatacattaa aaggctaaaa ctttcacagt gttagattaa 28500
tgcttgaagt ttgggagaca ctatgctatt tataaaaatc atcacgggag gaaattccag 28560
atcttcccac tttgtctttc atgatctgaa aacttgttgg gaactactgg gtatcaagga 28620
ggctaagtcc ctgggccact tcttggttct atttttattc tcatagtttc cacaaatttg 28680
actttctttg aagttatttt gctctccctc ctcttctata tctggaatcc tcttgtccca 28740
ggaataaaac tgaacaccat ttccaagtat ttctctcaag ttactattta taaagtaagt 28800
actgtataca aacccagttt ccttgttaga gatgtttttgc tttgttggat taatcatggt 28860

agaacagaga gtagttaaaa aatgacgatc aataatcagg acagatttgg agccaggaaa 28920
tactttgagc aacaacacag tagttgttcc cctggtttct cactgaagag tgaatgggga 28980
tggctccact ccatactttg aatttcttct tgaattttcc cagccctagc ccaatttttg 29040
ccttagattc taaggcgtag gataaatcgg gccaaaccaa atcaagagtc attgcttgca 29100
aagccattta gactttatct gaagtagtta gagcagaatt ccctgggata accagttcag 29160
tccaaagcct acttggttat ctgttttttt ccctggagga ggaatgcact gtcttttctg 29220
gctctcctgg accagaagca ctggtttccc tttgcctctt tggaaggact tagagcttag 29280
aggtccagcc ttttccctac ccttttctcc tgctgatttt gccctcagca tagccaagaa 29340
gcccaaagtt atgactaatt ctatctttag gtggacctgg acatctcatc agcgtcactt 29400
caaggttcac tgatcctaaa ggactgtttc ttgttggaag gtcatctcag ggtgggtatc 29460
tatttgtgac cagagctgat atggagtcca gcctttctga gttactagcc ctgggctcct 29520
taaaatctcc attcacttcc tcttcccttc cttacaggag aggacttaca gatgacagtc 29580
tctagagtct tgccgtcttt tgccagaata catttttttaa aattgtgaaa atagcaacta 29640
taatttcacc aaggaccatt cctttacgtt attactgaat ttagaaaatt ctatctcatc 29700
taatctgagc accttgatcc ctttagagta aagtcatttt ataaaatcta aaaagtagat 29760
ccaagtaata aattgatttt cttgcaattt aaatacattt cctctgattc taccttcact 29820
ggaaggggag aatagctgct gacctcactc cttttcatgt gctgaaagac cattattaag 29880
catctttcat cttctggatt ttgaggctag ggagcactaa ttcttcagct ttttttctcc 29940
aaccatttag aaattttggt tctctttaga caaaaatttt taatattatt tctaaacctg 30000
gagtctagat gtatatctag tatactaata atggtttctt tggtgctggt taagacagaa 30060
gaattgcttc ttagttcttg tgaatgcact cctatttgct taatgctgaa ttattcagtt 30120
cctgttccaa aattaacctg ccacaaggca caggtaagcc tgatcatcgt gaatctcggt 30180
acccttttaga aatattagcc tcttctcatt ctactgtgat tctgtatttt ttttttttaca 30240
ttttattaac tttttaatgt cattttgaat tctgattctg aaatttggaa attaaaacta 30300
gtgaccttaa tgcatgtcta aaaactatgg agaaaaagaa aatttacaag aatagtgatt 30360
tttcaggtat tgtgcaacta taagattaca gtaatgtggg caactggctt ttgtaataag 30420
tggaaaaaaa agagacagta aacatattcc tgttttctta caagtgtaac catcatggac 30480
aatagcagaa agcactagat gcccttatcc actgaattat tataaagggc tgggggctgg 30540
gcgcagtggc ccgtgcctgt aatcacagca ctttgggagg ccaaggtggg tggatcactt 30600
gaggtcagga gttcaggacc agcctggcca acgtgatgaa acctcgtctc tactaaaaat 30660
tacaaaaatt agctgggcgt ggtggcacat gcctgtaatc ccagctactt gggagctgag 30720
gcaggagaat catttgaacc caggaggcgg aggttgcagt gagctgagat cacaccactg 30780
cactccagcc tgggtgacag agtgagacgc cattcaaaaa aaaaaaaaaa aaaaaaggga 30840
tctcccaaaa tgacagagtg actctgtgtg atgagctcct caaactccag tgtccttacc 30900
aataccaagc agcatctgtc acataatccg ggagattctc caggtggtca ggggacaaga 30960
gggagctggg aggtggagga actgcaaatc tcctttccct gcatgctctt tgtgttccct 31020
acaagcagca ccactgaagg gcagtggtgg gactcatgct ctcctgccac cagccgccac 31080
ctccctattg tgtcccactc agagttactt gacaaggagc aatttagaca gttacacgtc 31140
tttataaaaa gattaactcc agcaacctct catccccaag ccttttctga gcatattaac 31200
ctgttgctct gatcccagtc agtgtaatca tccaaaagac cgcccagggt ccaaagtcag 31260
aagaccaagg ccgggtcatc cattcagagt gcaaatggct catggcagtg tacagcccac 31320
tggttcagga tgagtcacct aaacaagaag agaaaaaatc agaagcaaac agaaaacctg 31380
caagaagcaa acttcctttg aattgctttg tctgttttta tttagaaaaa gatctactgt 31440
gaacctggca ttagaaatgg ctcatgaggt tttccttagg agaatgtttc atatttctg 31500
aacaaagttg ttgcaagcaa agttgcatat tgtgttcgtt ggacacggtc aaatagagaa 31560
atattcataa aaagtttttag gccagaagtc tcccaagtga tgtgcccaca ttgaaggaat 31620
acactaagta atccactgtg agggtgagga aaatattgga gcatctgttt atacttattc 31680
ttaaaatcct atcctttaat taaaaaaaat tttttttgag acagagcctt gctctgttgc 31740
ccaggctgga gtgcagtggt gcgatcttgg ctcactgcag cctccgcctc ctgggttcaa 31800
gcgattcttc tgcctcagcc tcctgagtag ctgggactac aggcacgcac caccatgctc 31860
agttaatttt tgtatttgta atagagtcag ggtttcacca tattgtccag gctggtcttg 31920
aactcctgac ctcaagtgat ccacctgcct cggcctccca aactgctagg attacaggtg 31980
tgagccaccg ctcctggcct taaatttttt taaaaaaatt ttattttttt atagagatga 32040
ggtctcactg tgttgcccag gctggtctca aactcctggc tcaaatgatc cttctgtctc 32100
tgcctcccaa aggcccggga ttacaggtgt gaaccagctt cctgatcccc catcctttaa 32160
tttttatgat gtttgtttta taatgaatat gatattcttt tccagttgaa tgtattagct 32220
gtgctaatgg ataaatccca gattctccat ggtttaacac agtggaagtt catgtcttgc 32280
tcaagtctgg tcctatgtgg gagctcctgg gtgtgggcct cctctgctgc aggaggatgt 32340
gtgcatattt ttatggggct gtgcaactgt tagagctatg caatcagaaa agtttggaga 32400
ctagtgttga aggaactgcc acatttggag ctgccaggcc acagtttgat tctttttccc 32460
acccccaaat aacataaact ttgtagtata cttattgact gagacaaggc ccaaatagca 32520
cttagtttgg gccatcctta ctgctcctgg ttaccagatc caggtgggaa tgtgcagtag 32580
tcctgagccc ccagggccca ggctccatgg tactgggatg aacaacaagg ggtctcagca 32640

gctttaacta ctcaatctcc acagtgccca ccagactctt tccagttctt ctgggtctta 32700
cccagtttta gcacttaaag ctccatgtcc ccctaaattc ctcagtccca ggcaaaccaa 32760
gatagcgttc atgggaagct tcaatatcaa aggagcaggg gattgttcaa agagggctct 32820
cgccagggaa tgagtataca cagcctgttt tcccatggac agcctgtttt tccagtatgg 32880
acatgcagag aggagacagt ggtcataatc aactctaccc cctgttctca caggaaggga 32940
agattgacag ctactgcagg ctgtttgctg ttccatttgg gaaattttcc tgaggatccc 33000
ctgtaataag aggatagaat atatctgcct tcatgactta aaagaggcta taaattattt 33060
tcatgtcaat gaaactgtaa ttagccactg acaggattgt caagaagtaa ttttcagagt 33120
ttggtcccct tgagttaaac atgcctgctg acagcttact gaggacgtgt tattgtggga 33180
caccatccag gcctggaggg acccagaatc aggcagaagg acatggtcac atggtggagg 33240
ggctggagga ggtgacaatt agcaggtaga atgggattcc aggtatttca aggccaaacc 33300
aatacttggt gctttcacca ggcaggcagc agggaagact gtcagcaaca aacagggcac 33360
cccactggca gtcagatgat gatgatgatg atgatgatga ttattattat tattattatt 33420
atttgagacg gaatctcact ctgtcaccca ggctggagtg cagtggcgtg atctcggctc 33480
actgcaagct ccgcctcctg ggctcacacc attctcctgc cttagtctcc cgagtaactg 33540
ggactacagg cacccgccac cacgcccggc taattttta tattttttaa gtagagacgg 33600
ggtttcaccg tgttagccag gacggtctcg atctcctgac ctcgtgattt gcccacctcg 33660
gcctcccaaa gtgctgggat tacaggcatg agccactgcg cccagcaatt attttcaatc 33720
tctgagagat gatagcaaga aatgggcaaa gtctcatttc caagggacta aggcttgagg 33780
caggtcagca aaagagggac cagggtgaaa gtacaatgag cacagctggg acgtgaggtt 33840
ggggtgagct cagcagtgac cagccacttg taggggacaa aggcctatt tcttgagaaa 33900
gaaaggtaca gagatatggt gctgagctgc tccagtactc caggtgttgc catcctgtga 33960
gcacaaacgc agctgcaatc acatccttgc ctaaatctca gtggcttcca gccacctgct 34020
gctcacccag gatcccagca ccccggttcc ccatcttccc aattccttct gggaatacag 34080
gggatgtgcc cagggacaca gctcctgctg gcaagcagct gaagcaaggg gctatgccac 34140
tgtcctgctc cccagtcctc tgtgagtaca ataccctccc caggttctgg agtgccttcc 34200
accaccacca ggagctgctc tgggtctgtg gcagagggag attaagggtg tgactctccc 34260
cgactttgtc tcttctgtca tctgaggcat ggcaccactt gtatctggaa tctcaccaag 34320
tttttcatga ggacgatccg tcagcatctg ttctggtctc tggtttccca ttaggtgtga 34380
aactagttcc ctagcttgtg agggcagcag gggaactcca aaacatcccc tctattttta 34440
tttcctttct ccttgctcaa aggccttctt cctctttcag ataagagaca caaaagcaca 34500
catgcctatc aatttatgtg gacagacacc caaagggctt ctgccctctg cagtgcagtt 34560
tggacttagg acattcttac acaatattat ctctttgtcc ccagatgcca agccaaggcc 34620
actagaaaaa cctctaaata attagacttt tatgaaggct aacctgggct atttttgaag 34680
gtgcttttaa aacaatctta tcacacctaa ttttgaataa agatatgttg tggaaaaact 34740
tgatgcttac atttggaata atttaaggtg tattacaaaa caataaatga tgggacaatt 34800
gtgttatgtt tccaaaaaag ttcatataaa atttcagtga gtgggctggg cgcggtggct 34860
cacgcctgta atcccagcac tttgggaggc cgaggtgggc ggatcacgag gtcaggagat 34920
cgagaccacg atgaaacccc gtctctacta aaaatacaaa aaattagccg ggcgcggtgg 34980
caggtgcctg tagtcccagc tactcgggag gctgggggcag gagaatggcg tgaacccagg 35040
aggcggagct tgtagtgagc cgagatcgtg ccactgcact ccagcctggg cgacagagca 35100
agactacgtc tcaaaaaaaa aaaaaaaaaa aagaaatttt cagtgagtgt ggaaggacta 35160
gagccttcct ctgctactac tacattttac ctcgtgggaa acaggcctat ggatttagcc 35220
aaggaaaaag aaacgtggga atgctgttta ttccttgcat agccgtaata taaagtatga 35280
attttatcaa gtatcttttc actcaaccta tcatagccac tcaaaaagat gttaatggaa 35340
agacaaccat cagaagcaaa atcatgccaa gaacgagaag cagcaggagg aaaaactttg 35400
gttacttctg tgagttctag aaagttgtag ttacggtttg caagagtatc agtgttaggc 35460
tttagacaag acattccttg ggtcaccagt ggcctttttgg gtggtaatgg ccacagtcca 35520
aaaaggaaga agaaagcaat tttgcattca tattatccat ttccaatttt ttaggggact 35580
gatggtcact ctcctggccc ttagagatgt gtgtgggtaa cctcgctggt gtctttgctg 35640
gtggggttaa aattttccat acgtgcttca ggactctacc tcagccatgt ctttctgtga 35700
gcattgagct tgttaatgat gcatgtttct tacccagctt taaaagatga ttgagttatt 35760
actgggggagt tattattggg gagtttttc ccatgtagtt gaggcataag ttgataaggt 35820
tgagctgacc tagacgcagt tttctgtggg attatggaga atgagggaag aattacgctt 35880
tgtaattatg cttttttttt tttttaaatt cagagaaaaa agatcactta tggtgaccca 35940
ggctaaaaga cctaggtctt caacaaccag cgtcatctat ttctagatca attgccttgt 36000
aatattaagt atttaagctt tgaggcttac tgaaggaaag atatggggtc atttacaatc 36060
ctgtagacta tgaccttcca ccaggatcta ctcaagacct catcacacat tatgttaaat 36120
tctttctggt ggtgaaattg tcagcaaaga gctcggccaa cctaaccaag atcacttgga 36180
gttatacctt aggagggagg atgaatataa gtcatctttt tccccttttgt tagatgcaga 36240
tgaattaatt atagttccca gagaactatt cccttttgct taaattagtg tcagattttc 36300
catccacgtc ttctatgaat agaggcaata tgacttgaca ttaggacaga gctttggaat 36360
ctagagatgg atgctgtcta tctttgattc tctgcaatgc atgtcagtct gggctctcag 36420

gccttagttt cttcctccat aaaatgggaa tgaaatatgc tcactgattt acttcaggag 36480
attttaaggg aattatttaa agaccatttt gtaatgttct gggtaactca agtggaaggc 36540
aatggatgtg agggaatttt taaaataaga aatctcttga atatgaattg agtaatctct 36600
taagttcttt agataagata tggagtgatc tttaggctca ggaggcctgt cttttgggaa 36660
agtgctataa ctcacttcta ctaccggcaa cttaatacgt aagagaatat tcttggccct 36720
gctctgaggt ttaaacaatg ttcaaccttg aaagattgat tttaaatgta aaggtcattt 36780
aaactctttt gtaagttctt atattttgtt aaaataggct gaacatttac ctctagatga 36840
aaaatcaatg cttcagtgta tacttttctc ataaaataac ctatgtcttt atttttttct 36900
gcagacaaaa ctgtgtggag ttttatccta tattcataat tacattgtgg atggctgggt 36960
ggtatttcaa ycaaggtaat gttaaaatac agtcaactgt gttctaatga tgactatgat 37020
gcttaaacga ttaaggagta gatatatggc caggcacggt ggctcacacc tatattccca 37080
gcactttggg aggcccaggt gggtggatca cctgaggtca ggagttcgag accagcctgg 37140
ccaaaatggt gaaaccctgt ctctactaaa aatacaaaaa ttagctaagt gtggtggcgc 37200
atgcctgtaa tcccagctac ttgggaggct gagacaggag aatcacttga acccaggagg 37260
tggaggctgc agtgagctga gatcgtgcca ctacactcca gtctgggtga cagagactcc 37320
atctcaaaaa aacaaacaaa aaaaggagca gatatgcaac tatatagcta ccaatcctgg 37380
agactaaaca aaataaacag tgtgtgcatc agagtgctat gttgcaggta tatgaacttt 37440
ggcttcattc taatttaatt caataatgaa aaaaatattg agacaaacta tatgctagat 37500
actgaagaat gagaacgaca tgagctttgt cttcaaatct aataatctac agttgactcg 37560
aacaatggag gggttggggt gctgaccccc cgccccacac acacacagta gaaaatctgc 37620
atgtaacttg ataacccaaa acttgactac tgatatccta ttggtctgga agccttactg 37680
agtacagtct actaataaat attttgcatg ttttatgtat catatactgt attcttgcaa 37740
taaagcaagc tagggaaaag aaaatgttat taaaattata agtaagaaca aatatttact 37800
atttattaag tggaagtgga ccatcataaa ggtcttcatc ctcatcattt tcacgttgag 37860
taagctgagg aagaggaaga agaggggttg gttttgctgc ctcaagggtg gcagagtcag 37920
aaaaggtgaa ggaggtggaa gcggagacag aattggcagg cgcagtaggt ataaatttat 37980
agaaatatca tttctgacgt ttttgctttt tttctctaaa aatgtttcta tatagtacca 38040
atccttcttc cactgttacg tttagtttca gtgcttgtat catagaaggg tccatgtcat 38100
aaaagaagta aaaaagcaga cttgaatcat cagaacccttc ctgccagatt gtctaatgtc 38160
aatttgtttt ctggtactgc ctcttctgtc ttcttcctta tcatctggca gtggttcaga 38220
gcactcgtct ctaccaagtc atcttctgtt aattcatctg gcgtggtgtc tattagctct 38280
tgaatttctc caagattcat atcttgagac ccttcatccc ccacattctt tgccacagcc 38340
actatctctt tcatgatttc cttgattggc tctgttgtaa atcctgtgag tgtcatgcac 38400
aacgcctgta cacagtgttc ttcagcaaga atttattgtt tcaggtttga cggctttcac 38460
aaccttccta taacaatgct agcatcttca atggtgtaat ccttctaggc attcataatg 38520
ctgtttctat cagggttctc ttccacaaca ttgacgatca tatccataga gtaccttgta 38580
tcatgagcct taaaggtcct gataaccccc tgatttagag gctgaattaa agatgttgtg 38640
tttgggggca agtagactac ttttatgcct tccacgttga actcatgggg ttctgggtgg 38700
tcagggacag tgtccaatat caaaagaatg ttagaaggta atcccgtcct gacttcaggg 38760
acaaagcatt gatggaacca atccagagaa ggggttcttg ttgtccaagc tttttttttg 38820
tacaaccaaa tgactggcag gtgctgttta tctttttcct taaggctcag ggggttagca 38880
actttataga taagggcagt cctgattata aacctgactg catttacaca aaacagtaga 38940
gttagcctat cccttcctgc cttaaatcct ggagcttgct tctcttcttt actaataaat 39000
gtcctttgtg actttttttt tctcccccgg gacagggcac ttttgtttgc attaaaaacc 39060
tgttcaggca gatatccttt ctccctcaatg atttcttaa tgtgcctgg gaactcatgt 39120
attgcttctt ggttggtaga agctgcttct cctgttatct tgatatttta aaagctaaac 39180
ttctttctaa aattatcaaa ccatcctttg ctggcattaa attcttcagc tttagatcct 39240
tcatcttcct tttgcttaaa gttgtcatac aaggattttg ctttttctcg aatcctatta 39300
gagtctacaa gtatgtgttt ctttttttctt ttaatagttt aatgtatttt aatagcaaac 39360
ttacaggaac agcacagaag acagacaaca ttaaaaacat gtacttgcat gtaggacaac 39420
tcagttagaa aagtatagtg aatggatgga atctactgta tgataaaaat gctacaagca 39480
ccatttagtt gccatcaata agaaatttac ttgttttaaa aaaatccaaa tgctggcatt 39540
gtccagaaaa atttaacagg tttatttata attattataa agttgaaccg ctgaaacttg 39600
ttcactgaaa cattttaact tgcattaacg ctttacgtct ccgcatttat attaaaaatt 39660
cacacacaaa tgaaaatgga aaaactgcca atacctgatt tctgtcccct atttttccac 39720
tcgcaatcat atacttaggt accttttgac cccatggaaa aaaattatct aacgttcaga 39780
actaccaata acaggaagaa gagaaatttt tttttttttt gagaatgaaa tgtttcccat 39840
catagtggat tcttaagcac gttctccacg tatgctgtgt gctagctgga tgtcttttgg 39900
cataattgtt acacgtttgg catggatagc acacaggttg gtgtcttcaa aaaggccaac 39960
cagataggcc tcacttgcct cctgcaaagc accgatagct gcgctctgga agcgcagatc 40020
tgttttaaag tcctgagcaa tttctcgcac cagacgctgg aagggaagtt tgcgaaccag 40080
aagttcagtg gacttctgat aacgtctaat ttcacggagc gccacagtac caggcctgta 40140
acgatgaggt ttcttcaccc ctccagtaga gggcgcactc ttgcgagcgg cttttgtagc 40200

cagttgcttc ctgggtgctt taccaccggt cgatttgcgg gcagtctgct ttgtacgagc 40260
catggtacag agacctcctt acttaccccc cttctccttc ggctggagct cggcgagcga 40320
gaggcggcgc tggcgttgga gagccaagta tgtgtttctt atagcaatcc tgctcccaca 40380
taaaagctgc atttcaatat gagataaaaa gacattttgg aaaaagtgca aggtttttcc 40440
ccctgctggt gtagctgcaa gtgacagctt tacaaatcac tttttctttc tttttctttt 40500
tcttatcttt tcctttcttc tttcttcatc ttcttttttt tttttccac tggtccttgc 40560
actgaattca tttatcttgc aatggtggtc aaccacagct cacagctgca gacctcaatc 40620
tatgacacat attaagccat tcaatttttt cttgtaatat cataactttt ctctgctttt 40680
tgggagcact ttcagcacca ctagtagcaa tctgtatggg tatcatggca ttattcaagg 40740
tttatggtat tgcactaaac attgatgaaa aataggcaaa aaccctgaga gatcactttt 40800
tactgcgata cgcaatttttc tggaaagaag aactgctcag ttggagataa ttagcatcac 40860
cttttaagca gatacttgca acacttgagc tcactgcaat agcaacagga ggtggctaca 40920
aaatatttac agtagtacag tatttctata gttaatttta tgcagttatg atttaatact 40980
gcatctttac acttgtttac atttctctca actacaagtg gcatcatgca ttatggtctg 41040
tgtttgtatg ctttgataaa ttttaacttt gcataaaatt catatatata tctatatagt 41100
ctttctgctg aagctttgta aaaatttgcg tatattttat gatagtaaat gataaaatag 41160
accagtatct acatctgttt tgtgcatttg tgacagacct ttttcctaat ttttcagtat 41220
ttctgggcta tgtgattcat ttgtgagttt tgtcacattg tcacaaatct ccaaaatgtt 41280
ttccaatata tttatgttta tttttgtaaa gccacctcct gcacaatcca atatatttat 41340
ttgaaaatat ccatgcataa atggacctgt gcagttcaaa cccatgttgt tcaagggtca 41400
cctataattc taattaactg aaggaggagg acacagaatg aaaaattcac attaataatt 41460
atagctggcg aggcccagaa gcttatgcct gtaatcccag caatttggga ggctaaggtg 41520
gagggattgc ttgaggccag aaattcacga ccagcctggg caacaaagga gaccctgtgt 41580
ctacaaaatt tttttaaaa aactatctgg gcacacatgt agtcccagct acttgggagg 41640
ctgaggtgag aggagctctc aagcccaaga gtttcaggct gcagtgaatc atgacagtgc 41700
cactgcactc cagcatgggt gacagagtga gaccctgtct caaaaagaaa ataataataa 41760
ttatcattat agctattatt tattgagccc ttactatgtg ccaggtactg tttttgtttt 41820
ttggtctttg gttttgcttt tttttttttt tttttttgag atggagtctc gctttgttgc 41880
ccaggctgga gtgcagtggt gcgatctcgg ctcactgcaa gctccgcctc ccgggttcac 41940
gccattctcc tgcctcagcc tccggagtag ctgggactat aggtgcctgc caccacgccc 42000
agctaatttt tcgtattttt agtagagatg cggtttcact gtgttagcca ggatggtctc 42060
gatctcctga tctcgtgatc cgccagtctc ggcctcccaa agtgctggga ttacaggcgt 42120
gagccaccac gcccggccac caggtactgt ttttaagttt tatgcataca ttgtcccatg 42180
tgtaagattt acaggattta aagccttcat atgagggagg gtctataatt aatttatact 42240
ttacagatga ggaagctgag gtacagagat gttaagtaat ttttccaact tgacattcac 42300
caatgtgggg aaatttttcc tttcaagtgc agagccgggt ctgtaagtat ttgtaccaac 42360
aacccatgca cctttttctta aaggtctcca agtatgtagt gctgccgttt gtatgtagcg 42420
actctacaaa catccacttt ctatttgact ggggtatcct tggtgagctg agggctttct 42480
ccaaacttgg cttatgcgtt taacagtgag tcagaccagc ccctgctacc ttcgttggag 42540
aaattcaatc tcctttttct ggtaactttc actgtctcat tttggtctat acttgattgc 42600
caagatttaa ataaaccaaa atccagctgc tttcaaaggc aaccccatca ctctctttct 42660
tttagttttt actgtgaact gagtaatttc aggtacaatg tcctttagag aaagacttct 42720
tcactttatt acaactatac ttgtgtcgct taagggcatg aatgaaaccg ctgttccttg 42780
tcacgtaaga gcacgttaac aactaataag aaaagcgcct tttggtaatg ggactctgtc 42840
tctgccattt gaccctttgg atttctctgt gataggctga atgaggcaga ctgcctgtca 42900
gaaaggattg cttgagtatc ttgtcagaaa tttcttttca ttctttctca tccaaagtaa 42960
gtcacctact aactgttcct gaactgttct cattggcttt ctgggagtaa ctccctagac 43020
agggagtctc aaaactgaga accatgttca gaactctttg gcttcctcag accctgggaa 43080
gggtccacca gctcttcctt cccagctctt tgcttggtcc gctcgttcct gtcctgtggt 43140
gccgatacag atgtcatccc ctccagaggc cttcctatac cccattttgg caacctcctc 43200
cagttcctct ctctcccatc tctcttcatt tctttcacag cactttcaag tttgcacttt 43260
tcttctcatt tttttctttg ttgtctgtct ttgctgagag aatgtatact ctctgaagtc 43320
agggagcttg tatctttttc actgccgacc tcaatcctta aagtaatacc tggtttatgg 43380
tagatacggg gctgggtgtg gtggctcaca cctgcaatcc cagcactttg ggaggccaag 43440
acaggaggat ggcttgagtc caggagttca agaccagcct aggcaacata gggaggcctt 43500
ttctctgaaa aaaaaaatta aaaaattggc caggcctggt ggcatacacc agtggtccca 43560
gctacttagg aggctgaggt gggaggattg cttgagccca ggaggttaaa gctgtgctga 43620
gctgtgatca tgccacacat tcctgcctgg gtgacagagc aagaccctgt ctcaagaaaa 43680
aaaaaaaaat catgcggcca agtaaaataa taaatttaat ttaatctagc atatcgcagt 43740
cccacacata gactccaaat gtttacagta gggctctggc aacttgcatc tgtgaggtgc 43800
tgaggaagag agtggggccc taacataggg aagacattga tatgtattga tatgtttcac 43860
tggatgcgat gatagaacat catcatactg tatttcataa ttctttttga gataaatttt 43920
gtatgtaata aaatgcataa atcttcaatg aatgttgaga agtacacaca cctgtgtggt 43980

ccagatctct atcaagatct agaacatgac aaccacccag aaagtgtcct cctgcttctc 44040
ccagtcagtc cccattctca ctcccagatg caagcacttc tgattgtttt catcagttag 44100
gtttgtccat tctagaactt cgtataattg gaatcataca gtatgtagcc tgtataatgc 44160
ttcattcact cagcaaaatg ttgtgttgca tagatctgtt tttttcatt gcaaaatgtt 44220
gtgttgcata gatctgtttc ttttaattga atagactcta tcacagcttg gttatccatt 44280
ctcctgttga taggcttctg gactgtttcc agtttttgtc tataatgaat aaatctgcaa 44340
tgaacattct taaacaagtc cttttgtgaa cacatgcttt catttctctt gggtaaatac 44400
ataggaatga gattgctgag tcaaatgtta cgttcgattt tataagaaac taccagaact 44460
tttcccaaag tggctgtact attttgcatt cctgccaatg atgtgtgtgg attccagttg 44520
cttcacatcc tcaccaacat tttgtgttac tggtttttt ttttttttt ttttttttt 44580
ttacaatttt agccattctg ttggatgtgt ggtggtgcca tctcattata gtttttattt 44640
tatttattta tttttcagat ggagtctcaa tttgtcaccc aggctggagt tcagtggcat 44700
gatcttggct cactgcaacc tccacctcct gggttcaaac gattctcctg cctcagcttc 44760
ctgagtagct gggactgcag gtgcccgcca ccatgcccag ctaattttg tatttttagt 44820
agagacgagg tttcacaatg ttggccaggc tggtcttgaa ctcctgacct caagtgattc 44880
acccccctc ggcctctcaa agtgttggga atacaggcct gagctaccac gtccagcctc 44940
attatagatt taagtgggaa tttgttgatg ttgaatcatg ttgaaacatt tttcttgaga 45000
ttattggcac atttgtttcc ttcctatgtg gattctctct atattctgaa tataagtatc 45060
ttgtccaata tatgttttgt gaatattttc tcagtcgtcg tacaacatct taagagactg 45120
ctgttgtatt ttgtgccttt tcttttttt tcccaccccc ctatagtttt tgctacttgt 45180
ctgggtctgg tgtacatata tggccgtcac ctayacttct ggggatattc agaagctgct 45240
aaaaaacggt aaggagaacc cagtaatttt gtatttatgc aaaaagtagc aggataaggt 45300
ctgggtcagt ttccttctcc tgagagatat taacagcact gtgagttgtg gtggcaaaag 45360
taatccatac tgtctgaggt caagtcttgc cagttcatac acaattaggc atgagatggg 45420
aaaatgcaag tataaaactg tgcactgaat gtggactcta gttccttgca aaaatttgc 45480
attgtctcca gaaagttacc gaggaaataa tcctgcctag ccttgataat gctttgaatg 45540
ttcaaagaaa ccaagtgatc tctatggcta ggcattccct ttaacttatg tgatgagcta 45600
aaattttcaa attgcaaaaa tatagaagtt ctggacagtg ttaacgatag gacagcctac 45660
ctcaggactc tcagcttcct tattacagtc cagtttgtca cttttctccc tcatgtccac 45720
tctgcaggat caccggtttc ygactgagtc tggggatttt ggccttgttg accctcctag 45780
gtgccctggg aattgcaaac agctttctgg atgaatatct ggacctcaat attgccaaga 45840
aactgaggcg gcaattctaa ctttttctct tccctttaat gcttgcagaa gctgttccca 45900
ccatgaaggt aatatggtat catttgttaa ataaaaataa agtctttatt ctgtttttct 45960
tgaaatggct ttgtagaaac acacacttta gagaatacat ttcctgttta agaactgaaa 46020
tttgcttgrg gtaggtacat ccatatcatc agtggaagtg tgttgtgccc tagttttaga 46080
aatgcatgct gaagtcatga ccggttgtgt agggacacag cagcctgacc cgtgtgctgg 46140
ccgttccgag gactactgac atatcctgcc actctgactg cttctctctt gatggaaagt 46200
ttcagacaga agctgataaa gcagctgagc cctttcattg gttataggaa aacaacagat 46260
aggaaggcat gtgggggaca tcaggcaaac acatagagta gccagagaag ggagtcatca 46320
actgatagca caaaacaaaa caggttatag aaggctgggc gtggtggctc atgcctgtaa 46380
tcccagcact ttgagaagcc gaggcgggca gatcacaagg tcaggagatc gagaccatcc 46440
tggctaacac agtgaaaccc tgtctctact aaagatacaa aaaattagcc gggcatggtg 46500
gcgggtgcct gtagtcctag ctactcggga ggctgaggca ggagaatggt gtgaacctgg 46560
gaggcggagc ttgcagtgag ccaagatcgc gccactgcac tccagcctgg gtgacagact 46620
ctgtctcaaa ccaacaaaaa acccaaagaa aaacaaaaac aggttataaa aatgtacttt 46680
ataaagaaaa aagtgtttcc aggccaaaag agcttgctgc ctgatgccca tactgtaaaa 46740
ccaggttttt gggaaaagaa agaaatttaa ttttaaaaaa gagacggtct tgctgtcacc 46800
caggccggag cctccaacac atgggctcaa gcgagcctcc tgcttcagcc tcccaaagtg 46860
ctgggattca ggtgtgagcc actctgcccg gccaacaaaa gctttatatt gcaagtcaac 46920
tcacaggaag ataggagtca agctcaaatc tgtctccctc tgctggcttc caagcagtat 46980
ttttattaga aaatgtcagg gggtaaattc tgagattagt aggtgattgg tggaaagaaa 47040
ggggaggtct gaaaagtcct tgggcatgtc cagttatctc tttatgctgc ctcagaggta 47100
gcatgtgcaa attccagagg agttagtatg aaatgtcatg gaaattcagg ctgtgacatc 47160
agcaagcttg ttctgtgcag actccagtta gccatcttgg ttccaacaaa tttcagccag 47220
ttttttttta atctcataag gagagagagt ttcagtgttt cagtaagtta tttcttttct 47280
tatctgccat cctccaaatt caagaatttc ctttagttat tggtttcttg aactctttgg 47340
ggcacaattt caagaggaag aagtaaaaaa aaggatacca tttcaaggaa ttgcccaggg 47400
acaagtgcga agagaatatt ctttctcttg gctgaggaag gtgggtcatg ctcacggctg 47460
tagaacgtgg gttgggttct gctgcatgcc ctgtgtcatg tggggtttgg agggtgatac 47520
gggaagtaca gcggaacccc caaagctgtt ctgaccctga agtgcagtta ttgtctggca 47580
aaatgcagat gacttttcct catttcttc atctgtaaaa tgagaacgat tctacctcac 47640
agctttcaaa taaaaggcaa aatgaattat gagcatacat aacacatttt gacgtcagga 47700
aaagggcttg agtctggtgt gggcatcttc aaaaattgtt tagtagtact tttcttttt 47760

```
tgtggataca atagaagtta aaaaggccgg gaaaaatatt aatagaaaaa gaaagagaga   47820
cacttaaata ctagtgttcc cataatacat aacctcctgc atcccaaata gcagtacagt   47880
attaaatacc ccaaagcaca ctgagaatat actggcctgg gagtgggcac atttgcatac   47940
tagctcaggt tcccctactc attggtcatt tgaatttgga caaggcaccc catcagtagg   48000
aggttgtgaa tcaaagaagt gaagactcca gaaccagacc accctatcct gtcctatcac   48060
ttgctggctg tgctacccca gcactttgta gctctctgcc ttggttttct tttaggtaaa   48120
atagggcaaa tatgctaaaa agctatgatt ctatggtctt tttacttaat attcttttca   48180
gttatgatcg tgacttggat tgtgacattt taattgtttt atacattgag accaatgaag   48240
gatgagaatg caaatttttg atactaaggc ataattactt cataaccatg tcctttgcaa   48300
gttgaaaaga aactttccaa atcttacaac tgaagatttt taagcaattt ctgttttgca   48360
cttaaattgg ctctgggcag ggtaaaacac acacatacac acacacacac acactttggt   48420
taaacacgtg gaaataagct atgggtgtag cagactatca aggccttgtc actttaaaca   48480
ttcaaacgat agattttcct ttgttctaat tgcattgtca ccaggcaacc ttttgattat   48540
tttcattcct attagatttg gctacagaga gcaaccattt ctggccacag agatgtgggt   48600
ttctgatcgg tttgacaatt acatgtacat tttttgtata ttactaaatt gatcaatttt   48660
atttttagaa atcagattca ttgccttaaa aagtcagtgt caggaagtgg gggatagaga   48720
agaacttttc tgcattaaga gtctttttta aattttttta tttttatttt attattatta   48780
tactttaagt tttagggtac atgtgcacaa tgtgcaggtt tgttacatat gtatacatgt   48840
gccatgttgg tgtgctgcac ccattaactc gtcatttagc attaggtata tctcctaatg   48900
ctatacctcc cccctctccc caccccacaa cagtccccgg agtgtgatgt tccccttcct   48960
gtgtccatgt gttctcattg ttcaattccc acctatgagt gagaacatga ggtgtttggt   49020
tttttgtcct tgtgatagtt tgctgagaat gatggtttcc agtttcatcc atgtccctac   49080
aaaggacatg aactcttcat ttttatggc tgcatagtat tccatggtgt atatgtgcca   49140
cattttctta atccagtcta tcattgttgg acatttgggt tggttccaag tctttgctat   49200
tgtgaataat gccgcaataa acatacgtgt gcatgtgtct ttatagcaag cagcatgatt   49260
tataatcctt tgggtatata cccagtaatg ggatggctgg gtcaaatggt at           49312
```

<210> 486
<211> 750
<212> DNA
<213> Homo sapiens

<220>
<221> 5'UTR
<222> 1..201

<220>
<221> CDS
<222> 202..645

<220>
<221> 3'UTR
<222> 646..750

<220>
<221> polyA_signal
<222> 721..726

<220>
<221> allele
<222> 98
<223> 10-286-289 : polymorphic base G or C

<220>
<221> allele
<222> 153
<223> 10-286-345 : polymorphic base A or T

<220>

<221> allele
<222> 183
<223> 10-286-375 : polymorphic base A or G

<220>
<221> allele
<222> 426
<223> 10-523-232 : polymorphic base C or T

<220>
<221> allele
<222> 478
<223> 10-289-201 : polymorphic base C or T

<220>
<221> allele
<222> 526
<223> 10-290-37 : polymorphic base C or T

<220>
<221> misc_feature
<222> 57
<223> n=a, g, c or t

<400> 486

```
ataaagatct gtgaccggca gccccagacc tgcctgcctt cctgacttct gttccanagc    60
aaaggtcatt cagccgcttg aatcagcctt ttcccccsac ccggtcccca actttgttta   120
cccgataagg aaggtcagca ttcaaagtca agwagcgcca tttatcttcc cgtgcgctct   180
acraatagtt ccgtgagaaa g atg gcc ggg aac tcg atc ctg ctg gct gct     231
                        Met Ala Gly Asn Ser Ile Leu Leu Ala Ala
                        1               5                   10
gtc tct att ctc tcg gcc tgt cag caa agt tat ttt gct ttg caa gtt     279
Val Ser Ile Leu Ser Ala Cys Gln Gln Ser Tyr Phe Ala Leu Gln Val
                15                  20                  25
gga aag gca aga tta aaa tac aaa gtt acg ccc cca gca gtc act ggg     327
Gly Lys Ala Arg Leu Lys Tyr Lys Val Thr Pro Pro Ala Val Thr Gly
            30                  35                  40
tca cca gag ttt gag aga gta ttt cgg gca caa caa aac tgt gtg gag     375
Ser Pro Glu Phe Glu Arg Val Phe Arg Ala Gln Gln Asn Cys Val Glu
        45                  50                  55
ttt tat cct ata ttc ata att aca ttg tgg atg gct ggg tgg tat ttc     423
Phe Tyr Pro Ile Phe Ile Ile Thr Leu Trp Met Ala Gly Trp Tyr Phe
    60                  65                  70
aay caa gtt ttt gct act tgt ctg ggt ctg gtg tac ata tat ggc cgt     471
Asn Gln Val Phe Ala Thr Cys Leu Gly Leu Val Tyr Ile Tyr Gly Arg
75                  80                  85                  90
cac cta yac ttc tgg gga tat tca gaa gct gct aaa aaa cgg atc acc     519
His Leu Xaa Phe Trp Gly Tyr Ser Glu Ala Ala Lys Lys Arg Ile Thr
                95                  100                 105
ggt ttc yga ctg agt ctg ggg att ttg gcc ttg ttg acc ctc cta ggt     567
Gly Phe Xaa Leu Ser Leu Gly Ile Leu Ala Leu Leu Thr Leu Leu Gly
            110                 115                 120
gcc ctg gga att gca aac agc ttt ctg gat gaa tat ctg gac ctc aat     615
Ala Leu Gly Ile Ala Asn Ser Phe Leu Asp Glu Tyr Leu Asp Leu Asn
        125                 130                 135
att gcc aag aaa ctg agg cgg caa ttc taa ctttttctct tccctttaat       665
Ile Ala Lys Lys Leu Arg Arg Gln Phe  *
    140                 145
gcttgcagaa gctgttccca ccatgaaggt aatatggtat catttgttaa ataaaaataa   725
agtctttatt ctgtttttct tgaaa                                         750
```

<210> 487
<211> 650
<212> DNA
<213> Homo sapiens

<220>
<221> 5'UTR
<222> 1..201

<220>
<221> CDS
<222> 202..294

<220>
<221> 3'UTR
<222> 295..650

<220>
<221> polyA_signal
<222> 621..626

<220>
<221> allele
<222> 98
<223> 10-286-289 : polymorphic base G or C

<220>
<221> allele
<222> 153
<223> 10-286-345 : polymorphic base A or T

<220>
<221> allele
<222> 183
<223> 10-286-375 : polymorphic base A or G

<220>
<221> allele
<222> 326
<223> 10-523-232 : polymorphic base C or T

<220>
<221> allele
<222> 378
<223> 10-289-201 : polymorphic base C or T

<220>
<221> allele
<222> 426
<223> 10-290-37 : polymorphic base C or T

<220>
<221> misc_feature
<222> 57
<223> n=a, g, c or t

<400> 487

```
ataaagatct gtgaccggca gccccagacc tgcctgcctt cctgacttct gttccanagc     60
aaaggtcatt cagccgcttg aatcagcctt ttcccccsac ccggtcccca actttgttta    120
cccgataagg aaggtcagca ttcaaagtca agwagcgcca tttatcttcc cgtgcgctct    180
acraatagtt ccgtgagaaa g atg gcc ggg aac tcg atc ctg ctg gct gct      231
                        Met Ala Gly Asn Ser Ile Leu Leu Ala Ala
                        1               5                   10
gtc tct att ctc tcg gcc tgt cag caa aac aaa act gtg tgg agt ttt      279
Val Ser Ile Leu Ser Ala Cys Gln Gln Asn Lys Thr Val Trp Ser Phe
                15                  20                  25
atc cta tat tca taa ttacattgtg gatggctggg tggtatttca aycaagtttt      334
Ile Leu Tyr Ser  *
            30

tgctacttgt ctgggtctgg tgtacatata tggccgtcac ctayacttct ggggatattc    394
agaagctgct aaaaaacgga tcaccggttt cygactgagt ctggggattt tggccttgtt    454
gaccctccta ggtgccctgg gaattgcaaa cagctttctg gatgaatatc tggacctcaa    514
tattgccaag aaactgaggc ggcaattcta acttttctc ttccctttaa tgcttgcaga     574
agctgttccc accatgaagg taatatggta tcatttgtta aataaaaata aagtctttat    634
tctgtttttc ttgaaa                                                    650
```

<210> 488
<211> 147
<212> PRT
<213> Homo sapiens

<220>
<221> VARIANT
<222> 93
<223> Xaa=Tyr or His

<220>
<221> VARIANT
<222> 109
<223> Xaa=Arg or Stop

<400> 488

```
Met Ala Gly Asn Ser Ile Leu Leu Ala Ala Val Ser Ile Leu Ser Ala
1               5                   10                  15
Cys Gln Gln Ser Tyr Phe Ala Leu Gln Val Gly Lys Ala Arg Leu Lys
            20                  25                  30
Tyr Lys Val Thr Pro Pro Ala Val Thr Gly Ser Pro Glu Phe Glu Arg
        35                  40                  45
Val Phe Arg Ala Gln Gln Asn Cys Val Glu Phe Tyr Pro Ile Phe Ile
    50                  55                  60
Ile Thr Leu Trp Met Ala Gly Trp Tyr Phe Asn Gln Val Phe Ala Thr
65                  70                  75                  80
Cys Leu Gly Leu Val Tyr Ile Tyr Gly Arg His Leu Xaa Phe Trp Gly
                85                  90                  95
Tyr Ser Glu Ala Ala Lys Lys Arg Ile Thr Gly Phe Xaa Leu Ser Leu
            100                 105                 110
Gly Ile Leu Ala Leu Leu Thr Leu Leu Gly Ala Leu Gly Ile Ala Asn
        115                 120                 125
Ser Phe Leu Asp Glu Tyr Leu Asp Leu Asn Ile Ala Lys Lys Leu Arg
    130                 135                 140
Arg Gln Phe
145
```

<210> 489
<211> 30
<212> PRT
<213> Homo sapiens

<400> 489

Met Ala Gly Asn Ser Ile Leu Leu Ala Ala Val Ser Ile Leu Ser Ala
1 5 10 15
Cys Gln Gln Asn Lys Thr Val Trp Ser Phe Ile Leu Tyr Ser
20 25 30

<210> 490
<211> 357
<212> DNA
<213> Homo sapiens

<400> 490

ataaagatct gtgaccggca gccccagacc tgcctgcctt cctgacttct gttccagagc 60
aaaggtcatt cagccgcttg aatcagcctt ttccccccac ccggtcccca actttgttta 120

cccgataagg aaggtcagca ttcaaagtca agaagcgcca tttatcttcc cgtgcgctct 180
acaaatagtt ccgtgagaaa gatggccggg aactcgatcc tgctggctgc tgtctctatt 240
ctctcggcct gtcagcaaag ttattttgct ttgcaagttg gaaaggcaag attaaratac 300
aaagttacgc ccccagcagt cactgggtca ccagagtttg agagagtatt tcgggca 357

<210> 491
<211> 274
<212> DNA
<213> Homo sapiens

<400> 491

ccccaacttt gtttacccga taaggaaggt cagcattcaa agtcaagaag cgccatttat 60
cttcccgtgc gctctacaaa tagttccgtg agaaagatgg ccgggaactc gatcctgctg 120
gctgctgtct ctattctctc ggcctgtcag caaaacaaaa ctgtgtggag ttttatccta 180
tattcataat tacattgtgg atggctgggt ggtatttcaa ccaagttttt gctacttgtc 240
tgggtctggt gtacatatat ggccgtcact tggg 274

<210> 492
<211> 18
<212> DNA
<213> Artificial Sequence

<220>
<223> sequencing oligonucleotide PrimerPU

<400> 492
tgtaaaacga cggccagt 18

<210> 493
<211> 18

<212> DNA
<213> Artificial Sequence

<220>
<223> sequencing oligonucleotide PrimerRP

<400> 493
caggaaacag ctatgacc 18

## Description

### FIELD OF THE INVENTION

The present invention is in the field of pharmacogenomics, and is primarily directed to biallelic markers that are located in or in the vicinity of genes, which have an impact on the metabolism of xenobiotics such as drugs and the uses of these markers. The present invention encompasses methods of establishing associations between these markers and a phenotype such as drug response, toxicity and susceptibility to disease. The present invention also provides means to determine the genetic predisposition of individuals to such drug responses, toxicity and diseases.

### BACKGROUND OF THE INVENTION

To assess the origins of individual variations in drug response, pharmacogenomics uses the genomics technologies to identify polymorphisms within genes associated with drug response. In this respect, there are three main categories of genes that may theoretically be expected to be associated with drug response, namely genes linked with the targeted disease, genes related to the drug's mode of action and genes involved in the drug's metabolism. Among these genes of pharmacogenomic importance, genes coding for drug-metabolizing enzymes have a central role.

#### Drug Metabolism

Drug-metabolizing enzymes are important determinants of drug disposition, safety and efficacy. The enzyme systems involved in the metabolism and the subsequent elimination from the body of environmental chemicals, food toxins and drugs are mainly localized in the liver, although every tissue examined has some metabolic activity.

In order to produce its characteristic effects, a given drug must be present in appropriate concentrations at its sites of action. The absorption, distribution, biotransformation and excretion of a drug all involve its passage across cell membranes. The lipophilic characteristics of drugs that promote their passage through biological membranes and subsequent access to their site of action reduce their elimination from the body. Renal excretion of unchanged drug plays only a modest role in the overall elimination of most therapeutic agents, since lipophilic compounds filtered through the glomerulus are largely reabsorbed through the tubular membranes. Biotransformation of drugs into more hydrophilic metabolites plays a major role in the termination of their biological activity and their elimination from the body. In general, biotransformation reactions generate more polar, inactive metabolites that are readily excreted from the body. However in some cases, metabolites with potent biological activity or toxic properties are generated and may result in adverse side effects. Metabolic biotransformation of drugs can be classified as either Phase I functionalization reactions or Phase II biosynthetic reactions. Phase I reactions introduce or expose a functional group on the parent compound, and generally result in the loss of pharmacological activity although there are some examples of retention or enhancement of activity. Phase II conjugation reactions lead to the formation of a covalent linkage between a functional group on the parent compound with glucuronic acid, sulfate, glutathione, amino acids or acetate. These highly polar conjugates are generally inactive and are excreted rapidly in the urine and feces. Within a given cell, most drug metabolizing Phase I enzymes are located primarily in the endoplasmic reticulum, while the Phase II conjugation enzyme systems are mainly cytosolic. In some cases, drugs biotransformed through a Phase I reaction in the endoplasmic reticulum are further metabolized by conjugation in the cytosolic fraction of the same cell (Hardman J.G., Goodman, Gilman A., Limbird L.E.; Goodman & Gilman's The Pharmacological Basis of Therapeutics, 9th edition, McGraw-Hill, N.Y., 1996).

#### Enzymes Involved in the Biotransformation of Xenobiotics

Besides being involved in the biotransformation of drugs, drug-metabolizing enzymes are also involved in the metabolism of xenobiotics (foreign compounds) as well as in the metabolism of endogenous compounds including steroids, vitamins and fatty acids. Foreign compounds include therapeutic agents, carcinogens, plant metabolites, environmental pollutants, foodstuffs and other dietary components as well as industrial chemicals. The biotransformation of foreign compounds (xenobiotics) is often regarded as detoxification because it usually converts compounds into more water-soluble, readily excreted substances. This tends to decrease the exposure of the organism to the compound and therefore tends to decrease toxicity. However, in some cases the reverse occurs and a metabolite is produced which is more toxic than the parent compound. For example, drug-metabolizing enzymes may activate some carcinogens, and interindividual differences in cancer susceptibilities, have been linked to polymorphisms in drug-metabolizing enzymes. There are many factors, which affect biotransformation and toxicity, such as the dose, availability of cofactors and the relative activity of the various drug-metabolizing enzymes. There may also be several competing pathways of metabolism - some leading to detoxification others to toxicity. Factors, such as genetic factors or environmental factors, which influence the balance between these competing pathways, will also determine the eventual toxicity.

As mentioned above, the metabolic conversion of drugs and other xenobiotics is enzymatic in nature. The enzyme systems involved in the biotransformation of drugs are localized in the liver, although every tissue examined has some metabolic activity. Other organs with significant metabolic capacity include the kidneys, gastrointestinal tract, skin and lungs. Following non-parenteral administration of a drug, a significant portion of the dose may be metabolically inactivated in either the liver or intestines before it reaches the systemic circulation. This first-pass metabolism significantly limits the oral availability of highly metabolized drugs.

**Glutathione conjugation and further metabolism**

Glutathione is a tripepetide (γ-glutamylcysteinylglycine, GSH) found in high concentrations in most mammalian tissues, but especially in the liver. Glutathione has several functions including roles in metabolism, transport and catalysis. Glutathione is also important for the maintenance of the thiol moieties of proteins and for the maintenance of the reduced form of other molecules such as cysteine, coenzyme A, and antioxidants such as ascorbic acid; it is also used in the formation of deoxyribonucleic acids (Anderson M.E., Advances in Pharmacology, 38: 65-74, 1997). Glutathione has a major protective role in the body, as it is the major cellular antioxidant. GSH can react non-enzymatically with reactive oxygen species (ROS) and thereby protect the cell from oxidative damage. ROS have been widely implicated in the pathology of numerous diseases such as arteriosclerosis, rheumatoid arthritis, cancer, AIDS, adult respiratory distress syndrome and Parkinson’s disease.

Moreover, conjugation with the tripeptide glutathione represents a major detoxification pathway for xenobiotics including drugs and carcinogens. Glutathione may react either chemically or in enzyme catalyzed reactions with a variety of compounds, which are reactive electrophilic metabolites produced in Phase I reactions. The glutathione S-transferase enzymes (GSTs) that catalyze these reactions are members of a multigene family and are expressed in virtually all tissues. Glutathione conjugates are cleaved to cysteine derivatives and subsequently are acetylated by a series of enzymes located primarily in the kidney to give N-acetylcysteine conjugates collectively referred to as mercapturic acids. Mercapturic acid derivatives are the ultimate metabolites excreted in the urine. This is a particularly important route of Phase II metabolism from the toxicological point of view, as it is often involved in the removal of reactive intermediates. Xenobiotics that act as substrates for the glutathione S-transferases (GSTs) fall into four broad categories: electropilic carbon, nitrogen, sulfur and oxygen. Examples of substrates for glutathione S-transferases include aromatic, heterocyclic, alicyclic and aliphatic epoxides; aromatic halogen and nitro compounds; alkyl halides; and unsaturated aliphatic compounds (Ballantyne, B., Marrs T. and Turner P., General & Applied Toxicology, Stockton Press, New York, 1993). The GSTs are also involved in the metabolism of endogenous molecules such as the leukotrienes. As mentioned above, many of the enzymes involved in xenobiotic metabolism are also involved in specific aspects of the metabolism of normal cellular biochemical constituents. Leukotrienes are important mediators and modulators of the inflammatory reaction and contribute to a number of physiological and pathological processes. Moreover, the GSTs are also capable of directly binding hydrophobic compounds such as heme, bilirubin, and steroids, which may enable them to serve as intracellular storage and transport proteins for biological substances with limited water solubility. By their catalytic activity and their capacity for binding, the GSTs provide the cell with mechanism to protect itself from the noxious effects of various xenobiotics and endogenous substances. Further, GSTs may undergo amplification in tumors and may thereby be implicated in drug resistance in cancer chemotherapy. GSTs are mostly cytosolic although, more recently, microsomal GSTs have been identified. Human microsomal GST II (MGST II) is a member of the microsomal glutathione S-transferase family. This enzyme catalyzes the production of LTC4 (leukotriene C4) from LTA4 (leukotriene A4) and reduced glutathione. Leukotrienes are derived from arachidonic acid and related fatty acids. Metabolites of arachidonic acid have been collectively termed eicosanoids, the principal eicosanoids are prostaglandins, thromboxanes and leukotrienes (LT). Eicosanoids are among the most important chemical mediators and modulators of the inflammatory reaction and contribute to a number of physiological and pathological processes (see Hardman J.G., Goodman, Gilman A., Limbird L.E.; Goodman & Gilman’s The Pharmacological Basis of Therapeutics, 9th edition, McGraw-Hill, N.Y., 1996). The ability to mount an inflammatory response is essential for survival in the face of environmental pathogens and injury, although in some situations and diseases the inflammatory response may be exaggerated and sustained for no apparent beneficial reason. This is the case in numerous chronic inflammatory diseases and allergic inflammation. Acute allergic inflammation is characterized by increased blood flow, extravasation of plasma and recruitment of leukocytes. These events are triggered by locally released inflammatory mediators including eicosanoids and more particularly leukotrienes. The participation of arachidonic acid metabolism in inflammatory diseases such as rheumatoid arthritis, asthma and acute allergy is well established. Pathological actions of leukotrienes are best understood in terms of their roles in immediate hypersensitivity and asthma. LTC4 and LTD4 are potent bronchoconstrictors, they act principally on smooth muscle in peripheral airways and are a 1000 times more potent than histamine both *in vitro* and *in vivo*. They also stimulate bronchial mucus secretion and cause mucosal edema. A complex mixture of chemical messengers is released when sensitized lung tissue is challenged by the appropriate antigen. Various prostaglandins and leukotrienes are prominent components of this mixture. Response to the leukotrienes probably dominates during allergic constriction of the airway.

A particularly important role for the cysteinyl-leukotrienes (LTC4, LTD4, and LTE4) has been suggested in pathogenesis of asthma, which is now recognized as a chronic inflammatory condition. They are potent spasmogens causing a contraction of bronchiolar muscle and an increase in mucus secretion. An increased LTC4 formation has also been reported in leukocytes from patients with chronic myelogenous leukemia (Stenke et al., Acta Oncologica, 27:803-805, 1987) and in experimental glomerulonephritis (Petric et al., Biochim. Biophys. Acta, 1254:207-215, 1995).

Moreover, MGST II has the capacity to conjugate other compounds such as 1-chloro-2, 4 dinitrobenzene with glutathione and may be involved in a general metabolic system for detoxifying fatty acid epoxides (Jakobsson et al., Journal of Biological Chemistry, 271:22203-22210, 1996).

The resulting glutathione conjugate usually undergoes further metabolism, which involves first a removal of the glutamyl residue, catalyzed by γ-glutamyltransferase (GGT). In addition to catalyzing the initial step in the conversion of glutathione-conjugated compounds to mercapturic acids, GGT also converts LTC4 to LTD4. Interestingly, expression of GGT is often increased in cancerous tissues.

Renal dipeptidase is also implicated in the renal metabolism of glutathione and its conjugates including conjugated xenobiotics and endogenous molecules such as Leukotriene D4. Pharmacologically it is an important enzyme, for it is responsible for hydrolysis of some β-lactam antibiotics such as penem and carbapenem.

The effectiveness of the GSTs and therefore of detoxification by glutathione conjugation in general as well as the ability of the cell to resist to oxidative stress, are strongly influenced by the availability of reduced glutathione. Reduction of oxidized glutathione and *de novo* synthesis of glutathione, are both completely dependent on NADPH. Glutathione reductase (GSHR) maintains high levels of reduced glutathione in the cytosol in an NADPH dependent reaction. Reduced glutathione is synthesized *de novo* in the cytosol of most cells via the γ-glutamyl cycle; a series of tightly controlled, enzyme catalyzed reactions. The first and second step in the *de novo* glutathione biosynthesis are catalyzed by γ-glutamylcysteine synthetase (GLCL) and glutathione synthase (GSHS) respectively. Deficiencies in γ-glutamylcysteine synthetase and GSH synthetase are associated with hemolytic anemia and impaired central nervous system function.

**Genetic Polymorphisms in Drug Metabolizing Enzymes and Pharmacogenomics**

Genetic, environmental, and physiological factors are involved in the regulation of drug biotransformation reactions. Results obtained from epidemiological studies and experimental animal model systems have shown a wide range of phenotypic variation in the ability of individuals to metabolize drugs and environmental chemicals. While some of this variation can be attributed to different environmental exposures, it has become clear that genetic factors also play an important role in determining the response of the individual to exogenous agents. Certain allelic forms of drug-metabolizing enzymes can render the individual either more sensitive or resistant to the toxic or therapeutic effects of exogenous drugs and chemicals. Genetic factors seem to be the major determinants of the variability of drug effects and are responsible for a number of striking quantitative and qualitative differences in pharmacological activity. Genetic differences in the ability of individuals to metabolize a drug through a given pathway are an important contributor to the large interindividual differences of drug efficacy and adverse effects within a population. There are many diverse examples of xenobiotics whose toxicity is directly dependent on the activity of drug-metabolizing enzymes. Often impaired metabolism of a drug through a genetically polymorphic pathway has been associated with an increased incidence of adverse effects in the slow metabolizer population (Weber W.W., Pharmacogenetics, Oxford University Press, N.Y., 1997). Moreover, genetic differences in the regulation, expression and activity of genes coding for Phase I and Phase II drug-metabolizing enzymes can be crucial factors in defining cancer susceptibility and the toxic or carcinogenic power of environmental chemicals and xenobiotics. In addition, the majority of serious cases of drug-drug interactions are a result of the interference of the metabolic clearance of one drug by a coadministered drug. The interference usually occurs via inhibition or induction of drug-metabolizing enzymes. Interindividual differences in susceptibility to severe drug-drug interactions also involve drug-metabolizing enzyme polymorphism. In some cases the design of the drug takes into account the activity of drug-metabolizing enzymes. For example, prodrugs require activation by drug-metabolizing enzymes to exhibit their therapeutic activity. The activation and efficiency of such prodrugs depends on interindividual polymorphism in drug-metabolizing enzymes.

Individual differences in metabolism of therapeutics can lead to severe toxicity or therapeutic failure. Therapeutic management and drug development can be markedly improved by the identification of specific genetic polymorphisms that determine and predict patient susceptibility to diseases or patient responses to drugs. Assessing individual risk rather than population risk will lead to better targeted therapeutic strategies defining individual drug usage based on a benefit/risk prognosis. To assess the origins of individual variations in disease susceptibility or drug response, pharmacogenomics uses the genomic technologies to identify polymorphisms within genes, which are part of biological pathways involved in disease susceptibility, etiology, and development, or more specifically in drug response pathways responsible for a drug's efficacy, tolerance or toxicity. It can provide tools to refine the design of drug development by decreasing the incidence of adverse events in drug tolerance studies, by better defining patient subpopulations of responders and non-responders in efficacy studies and, by combining the results obtained therefrom, to further allow better enlightened individualized drug usage based on efficacy/tolerance prognosis. Pharmacogenomics can also provide tools to identify new targets for designing drugs and to optimize the use of already existing drugs, in order to either increase their response rate and/or exclude non-responders from corresponding treatment, or decrease their undesirable side effects and/or exclude from corresponding treatment patients with marked susceptibility to undesirable side effects.

Drug-metabolizing enzymes are highly relevant to pharmacogenomics because they are at the core of drug response, drug efficacy and toxicity. Drug-metabolizing enzymes also determine an individual's susceptibility to exogenous chemicals and to a number of diseases associated with exposure to toxic or carcinogenic chemicals.

The complexity of the pathways and enzymes that are involved in detoxification and metabolism of drugs has limited the precise identification of the drug-metabolizing enzymes, which play the causal role in pathologies or in drug response. Therapeutic management and drug development can be markedly improved by the identification of genetic markers derived from drug-metabolizing enzymes that predict patient susceptibility to diseases or patient responses to drugs.

**Genetic Analysis of Complex Traits**

Until recently, the identification of genes linked with detectable traits has relied mainly on a statistical approach called linkage analysis. Linkage analysis is based upon establishing a correlation between the transmission of genetic markers and that of a specific trait throughout generations within a family. Linkage analysis involves the study of families with multiple affected individuals and is useful in the detection of inherited traits, which are caused by a single gene, or possibly a very small number of genes. Linkage analysis has been successfully applied to map simple genetic traits that show clear Mendelian inheritance patterns and which have a high penetrance (the probability that a person with a given genotype will exhibit a trait). About 100 pathological trait-causing genes have been discovered using linkage analysis over the last 10 years.

But, most traits of medical relevance do not follow simple Mendelian monogenic inheritance and linkage studies have proven difficult when applied to complex genetic traits. Many complex traits such as height, blood pressure or cancer susceptibility have been known to run in families and are at least partially determined by genetic factors. However, the genes or combination of genes that underlie these observable characteristics or traits remain unknown in most cases. Such complex traits are often due to the combined action of multiple genes as well as environmental factors. Because of their low penetrance, such complex traits do not segregate in a clear-cut Mendelian manner as they are passed from one generation to the next. Drug efficacy, response and tolerance/toxicity can also be considered as multifactoral traits involving a genetic component in the same way as complex diseases. Linkage analysis is impractical when the trait under study is drug response due to the lack of availability of familial cases. In fact, the likelihood of having more than one individual in a family being exposed to the same drug at the same time is very low. Linkage analysis cannot be applied to the study of such traits for which no large informative families are available. Attempts to map complex traits have been plagued by inconclusive results, demonstrating the need for more sophisticated genetic tools.

Knowledge of genetic variation in drug-metabolizing enzymes is important for understanding why some people are more susceptible to toxicity, pathology or respond differently to drugs. Ways to identify genetic polymorphism and to analyze how they impact and predict disease susceptibility and response to treatment are needed.

Whereas a number of polymorphisms and rare mutations have been identified in drug-metabolizing enzymes (see Weber W.W., Pharmacogenctics, Oxford University Press, New York, 1997), genetic markers for use in determining which genes contribute to multigenic or quantitative traits and suitable methods for exploiting those markers have not been found and brought to bare on the genes coding for drug-metabolizing enzymes.

## SUMMARY OF THE INVENTION

The present invention is based on the discovery of a set of novel MGSTII-related biallelic markers. See the table in claim 1. These markers are located in the non-coding regions adjacent to MGSTII gene which is involved in the metabolic conversion of drugs and other xenobiotics. The position of these markers and knowledge of the surrounding sequence has been used to design polynucleotide compositions which are useful in determining the identity of nucleotides at the marker position, as well as more complex association and haplotyping studies which are useful in determining the genetic basis for variability in drug response and adverse reactions to drugs as well as the genetic basis for disease states involving the metabolic conversion of xenobiotics such as drugs. In addition, the compositions and methods of the invention find use in the identification of the targets for the development of pharmaceutical agents and diagnostic methods, as well as the characterization of the differential efficacious responses to and side effects from pharmaceutical agents.

Described herein is the isolation and characterization of the genomic sequence of the MGST-II gene including its regulatory regions and of the complete cDNA sequence encoding the MGST-II enzyme. Oligonucleotide probes and primers hybridizing specifically with a genomic sequence of MGST-II are also described herein. Also described are recombinant vectors comprising any of the nucleic acid sequences described herein, and in particular of recombinant vectors comprising the promoter region of MOST-II or a sequence encoding the MGST-II enzyme, as well as cell hosts comprising said nucleic acid sequences or recombinant vectors. Also described are methods of screening of molecules which, modulate or inhibit the expression of the MGST-II gene. Also described are biallelic markers that are located within the MGST-II genomic sequence, these biallelic markers representing useful tools in order to identify a statistically significant association between specific alleles of MGST-II gene and one or several disorders related to asthma and/or hepatotoxicity.

In one aspect, the invention provides an isolated, purified, or recombinant polynucleotide consisting of a contiguous span of 18-35 nucleotides of a sequence selected from the sequences of SEQ ID Nos. 3, 5, 9, 12-15 and 25 and the complements thereof, wherein said span includes a MGST-II-related biallelic marker selected from the MGST-II-related biallelic markers shown in the table in claim 1 in said sequence, and said biallelic marker is within 4 nucleotides of the centre of said polynucleotide.

The invention further provides an isolated, purified or recombinant polynucleotide consisting of a contiguous span of at least 12 nucleotides of a sequence selected from the group consisting of SEQ ID NOs: 3, 5, 9, 12-15 and 25 and the complements thereof, wherein said span includes a MGST-II-related biallelic marker of the table set forth in claim 1 in said sequence, wherein:

(i) the 3' end of said contiguous span is located at the 3' end of said polynucleotide; and
(ii) said biallelic marker is present at the 3' end of said polynucleotide.

The invention also provides an isolated, purified or recombinant polynucleotide consisting of a contiguous span of 8-50 nucleotides of a sequence selected from the group consisting of SEQ ID NOs: 3, 5, 9, 12-15 and 25 and the complements thereof, in said sequence, wherein:

(i) the 3' end of said contiguous span is located at the 3' end of said polynucleotide; and
(ii) the 3' end of said polynucleotide is located I nucleotide upstream of a MGST-II-related biallelic marker of the table set forth in claim 1.

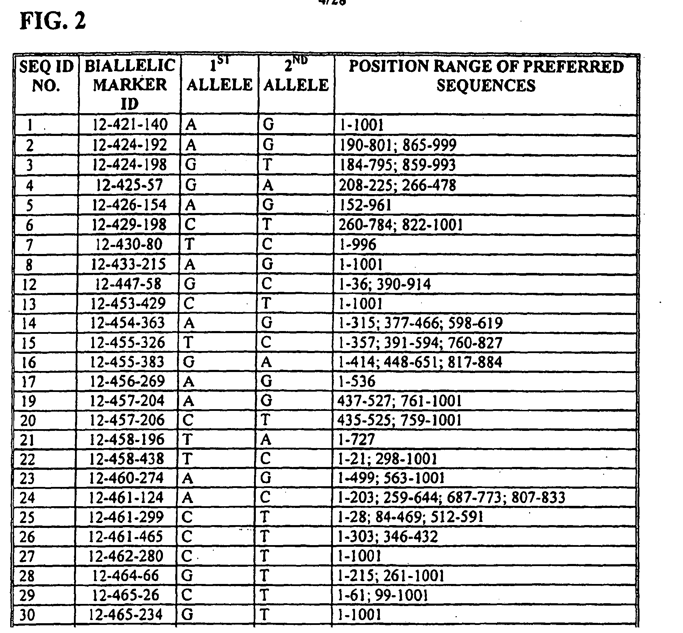

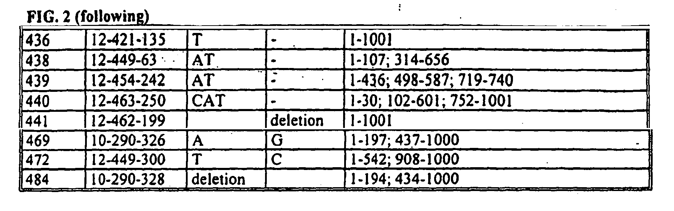

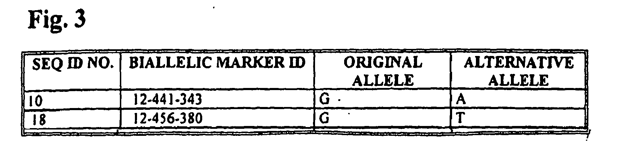

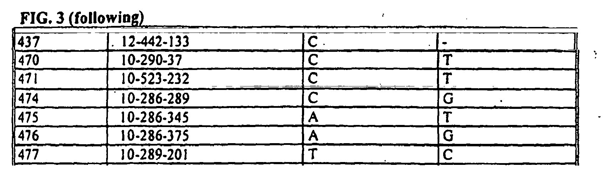

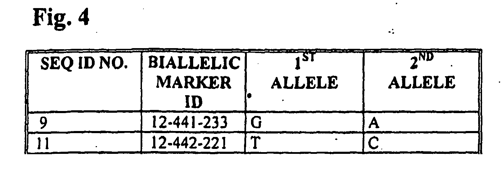

Described herein are polynucleotides consisting of, consisting essentially of, or comprising a contiguous span of nucleotides of a sequence selected as an individual or in any combination from the group consisting of SEQ ID No. 1-30, 436-441, 469-472, 474-477, 484-487, 490-493, the complements thereof, the sequences described in any one or more of Figures 2, 3, 4, 5, 6, 7, and 8, and the complements thereof, wherein said contiguous span is at least 6, 8, 10, -12, 15, 20, 25, 30, 35, 40, 50, 75, 100, 200, 500, or 1000 nucleotides in length, to the extent that such a length is consistent with the lengths of the particular Sequence ID. Also described are polynucleotides hybridizing under stringent or intermediate conditions to a sequence selected from the group consisting of SEQ ID No. 1-30, 436-441, 469-472, 474-477, 484-487, 490-493; and the complements thereof. In addition, the described polynucleotides encompass polynucleotides with any further limitation described in this disclosure, or those following, specified alone or in any combination: Said contiguous span may optionally include the DME-related biallelic marker in said sequence; Optionally either the original or the alternative allele of Figure 3 may be specified as being present at said DME-related biallelic marker; Optionally either the first or the second allele of Figure 2 or 4 may be specified as being present at said DME-related biallelic marker; Optionally, said polynucleotide may comprise, consists of, or consist essentially of a contiguous span which ranges in length from 8, 10, 12, 15, 18 or 20 to 25, 35, 40, 50, 60, 70, or 80 nucleotides, or be specified as being 12, 15, 18, 20, 25, 35, 40 or 50 nucleotides in length and including a DME-related biallelic marker of said sequence, and optionally the original allele of Figure 3 is present at said biallelic marker; Optionally, said biallelic marker may be within 6, 5, 4, 3, 2, or 1 nucleotides of the center of said polynucleotide or at the center of said polynucleotide; Optionally, the 3' end of said contiguous span may be present at the 3' end of said polynucleotide; Optionally, biallelic marker may be present at the 3' end of said polynucleotide; Optionally, the 3' end of said polynucleotide may be located within or at least 2, 4, 6, 8, 10, 12, 15, 18, 20, 25, 50, 100, 250, 500, or 1000 nucleotides upstream of a DME-related biallelic marker in said sequence, to the extent that such distance is consistent with the lengths of the particular Sequence ID; Optionally, the 3' end of said polynucleotide may be located I nucleotide upstream of a DME-related biallelic marker in said sequence; and Optionally, said polynucleotide may further comprise a label.

A second embodiment of the invention encompasses any polynucleotide of the invention attached to a solid support. In addition, the polynucleotides of the invention which are attached to a solid support encompass polynucleotides with any further limitation described in this disclosure, or those following, specified alone or in any combination: Optionally, said polynucleotides may be specified as attached individually or in groups of at least 2, 5, 8, 10, 12, 15, 20, or 25 distinct polynucleotides of the inventions to a single solid support; Optionally, polynucleotides other than those of the invention may attached to the same solid support as polynucleotides of the invention; Optionally, when multiple polynucleotides are attached to a solid support they may be attached at random locations, or in an ordered array; Optionally, said ordered array may be addressable.

The invention also encompasses the use of a polynucleotide for determining the identity of one or more nucleotides at an MGSTII-related biallelic marker listed in claim 1. Polynucleotides for use in determining the identity of one or more nucleotides at a MGSTII-related biallelic marker listed in claim 1 are described in this disclosure. Optionally, said polynucleotide may comprise a sequence disclosed in the present specification; Optionally, said polynucleotide may consist of, or consist essentially of any polynucleotide described in the present specification; Optionally, said determining may be performed in a hybridization assay, sequencing assay, microsequencing assay, or an enzyme-based mismatch detection assay; Optionally, said polynucleotide may be attached to a solid support, array, or addressable array; Optionally, said polynucleotide may be labeled.

The invention also encompasses the use of a polynucleotide for, amplifying a segment of nucleotides comprising a MGSTII-related biallelic marker listed in claim 1. Polynucleotides for use in amplifying a segment of nucleotides comprising a MGSTII-related biallelic marker listed in claim 1 are described in this disclosure; Optionally, said polynucleotide may comprise a sequence disclosed in the present specification; Optionally, said polynucleotide may consist of, or consist essentially of any polynucleotide described in the present specification; Optionally, said amplifying may be performed by a PCR or LCR. Optionally, said polynucleotide may be attached to a solid support, array, or addressable array. Optionally, said polynucleotide may be labeled.

The invention also encompasses a method of genotyping a biological sample comprising determining the identity of a nucleotide at a MGSTII-related biallelic marker wherein said biallelic marker is selected from the markers listed in claim 1. Genotyping methods for determining the identity of a nucleotide at a DME-related biallelic marker are described herein. Optionally, said DME-related biallelic marker may be selected individually or in any combination from the biallelic markers described in claim 1; Optionally, said method further comprises determining the identity of a second nucleotide at said biallelic marker, wherein said first nucleotide and second nucleotide are not base paired (by Watson & Crick base pairing) to one another; Optionally, said biological sample is derived from a single individual or subject; Optionally, said method is performed in vitro; Optionally, said biallelic marker is determined for both copies of said biallelic marker present in said individual's genome; Optionally, said biological sample is derived from multiple subjects or individuals; Optionally, said method further comprises amplifying a portion of said sequence comprising the biallelic marker prior to said determining step; Optionally, wherein said amplifying is performed by PCR, LCR, or replication of a recombinant vector comprising an origin of replication and said portion in a host cell; Optionally, wherein said determining is performed by a hybridization assay, sequencing assay, microsequencing assay, or an enzyme-based mismatch detection assay.

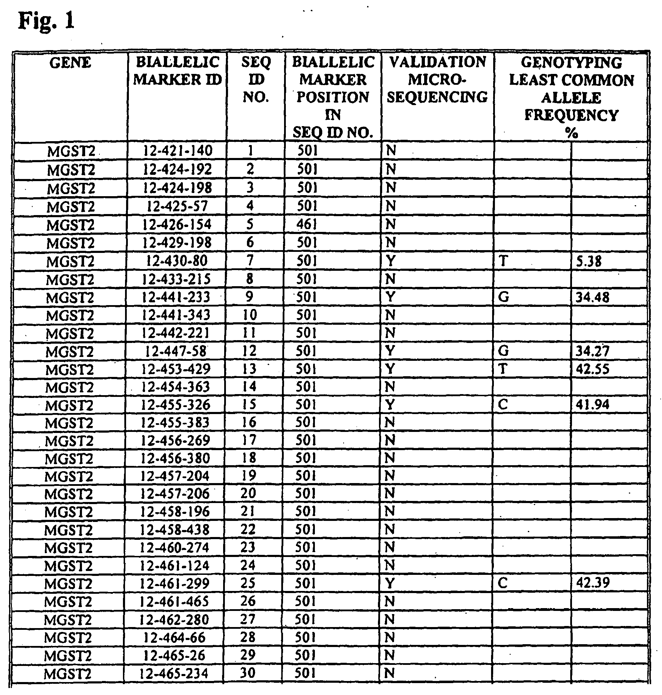

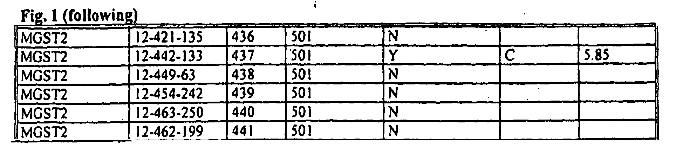

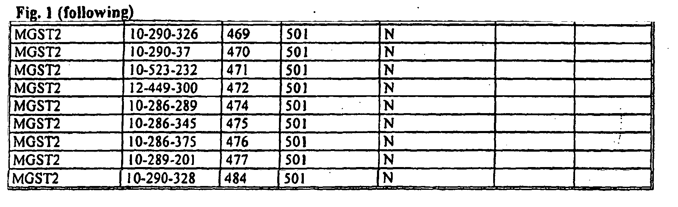

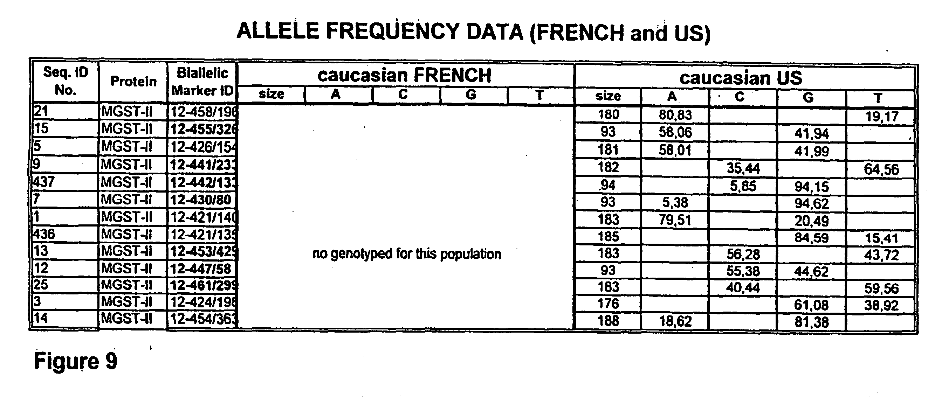

Also described herein are methods of estimating the frequency of an allele in a population comprising genotyping individuals from said population for a DME-related biallelic marker and determining the proportional representation of said biallelic marker in said population. In addition, the methods of estimating the frequency of an allele in a population of the invention encompass methods with any further limitation described in this disclosure, or those following, specified alone or in any combination: Optionally, said DME-related biallelic marker may be in a sequence selected individually or in any combination from the group consisting of SEQ No. 1-30, 436-441, 469-472, 474-477, 484-487, 490-493; and the complements thereof; Optionally, said DME-related biallelic marker may be selected from the biallelic markers described in Figure 1; Optionally, said DME-related biallelic marker may be selected individually or in any combination from the biallelic markers described in Figure 1; Optionally, said DME-related biallelic marker may be selected from the biallelic markers found in Figure 9; Optionally, said DME-related biallelic marker may be selected from the following biallelic markers: 12-455-326, 12-453-429, 12-454-363, 12-441-233, 12-461-299, 12-426-154 and 12-424-198; Optionally, determining the frequency of a biallelic marker allele in a population may be accomplished by determining the identity of the nucleotides for both copies of said biallelic marker present in the genome of each individual in said population and calculating the proportional representation of said nucleotide at said DME-related biallelic marker for the population; Optionally, determining the frequency of a biallelic marker allele in a population may be accomplished by performing a genotyping method on a pooled biological sample derived from a representative number of individuals, or each individual, in said population, and calculating the proportional amount of said nucleotide compared with the total.

Also described are methods of detecting an association between an allele and a phenotype, comprising the steps of a) determining the frequency of at least one DME-related biallelic marker allele in a case (trait positive) population, b) determining the frequency of said DME-related biallelic marker allele in a control population and; c) determining whether a statistically significant association exists between said genotype and said phenotype. In addition, the methods of detecting an association between an allele and a phenotype encompass methods with any further limitation described in this disclosure, or those following, specified alone or in any combination: Optionally, said DME-related biallelic marker may be in a sequence selected individually or in any combination from the group consisting of SEQ ID No.1-30, 436-441, 469-472, 474-477, 484-487, 490-493, and the complements thereof; Optionally, said DME-related biallelic marker may be selected from the biallelic markers described in Figure 1; Optionally, said control population may be a trait negative population, or a random population; Optionally, said phenotype is a response to a drug, or a side effects to a drug, or a disease involving the metabolic conversion of xenobiotics; Optionally, the identity of the nucleotides at the biallelic markers in everyone of the following sequences: SEQ ID No. 1-30, 436-444, 469-472, 474-477, 484-487; 490-493 is determined in steps a) and b).

Also described are methods of estimating the frequency of a haplotype for a set of biallelic markers in a population, comprising the steps of: a) genotyping each individual in said population for at least one DME-related biallelic marker, b) genotyping each individual in said population for a second biallelic marker by determining the identity of the nucleotides at said second biallelic marker for both copies of said second biallelic marker present in the genome; and c) applying a haplotype determination method to the identities of the nucleotides determined in steps a) and b) to obtain an estimate of said frequency. In addition, the methods of estimating the frequency of a haplotype encompass methods with any further limitation described in this disclosure, or those following, specified alone or in any combination: Optionally said haplotype determination method is selected from the group consisting of asymmetric PCR amplification, double PCR amplification of specific alleles, the Clark method, or an expectation maximization algorithm; Optionally, said second biallelic marker is a DME-related biallelic marker in a sequence selected from the group consisting of the biallelic markers of SEQ ID No. 1-30, 436-441, 469-472, 474-477, 484-487, 490-493, and the complements thereof; Optionally, said DME-related biallelic markers may be selected individually or in any combination from the biallelic markers described in Figure 1; Optionally, said DME-related biallelic marker may be selected individually or in any combination from the biallelic markers described in Figure 1; Optionally, said DME-related biallelic marker may be selected from the biallelic markers found in Figure 9;; Optionally, said DME-related biallelic marker may be selected from the following biallelic markers: 12-455-326, 12-453-429, 12-454-363, 12-441-233, 12-461-299, 12-426-154 and 12-424-198; Optionally, the identity of the nucleotides at the biallelic markers in everyone of the sequences of SEQ ID No. 1-30, 436-441, 469-472, 474-477, 484-487, 490-493 is determined in steps a) and b).

Also described are methods of detecting an association between a haplotype and a phenotype, comprising the steps of: a) estimating the frequency of at least one haplotype in a trait positive population according to a method of estimating the frequency of a haplotype of the invention; b) estimating the frequency of said haplotype in a control population according to the method of estimating the frequency of a haplotype of the invention; and c) determining whether a statistically significant association exists between said haplotype and said phenotype. In addition, the methods of detecting an association between a haplotype and a phenotype encompass methods with any further limitation described in this disclosure, or those following, specified alone or in any combination: Optionally, said DME-related biallelic marker may be in a sequence selected individually or in any combination from the group consisting of SEQ ID No. 1-30, 436-441, 469-472, 474-477, 484-487, 490-493, and the complements thereof; Optionally, said DME-related biallelic markers may be selected individually or in any combination from the biallelic markers described in Figure 1; Optionally, said DME-related biallelic marker may be selected individually or in any combination from the biallelic markers described in Figure 1; Optionally, said DME-related biallelic marker may be selected from the biallelic markers found in Figure 9; Optionally, said DME-related biallelic marker may be selected from the following biallelic markers: 12-455-326, 12-453-429, 12-454-363, 12-441-233, 12-461-299, 12-426-154 and 12-424-198; Optionally, said control population may be a trait negative population, or a random population; Optionally, said phenotype is a response to a drug, or a side effects to a drug, or a disease involving the metabolic conversion of xenobiotics; Optionally, said drug is zileuton and said side effect to said drug is hepatoxicity; Optionally, the identity of the nucleotides at the biallelic markers in everyone of the following sequences: SEQ ID No. 1-30, 436-441, 469-472, 474-477, 484-487, 490-493 is included in the estimating steps a) and b).

In one aspect, the invention provides a method of determining whether an individual is at risk of developing asthma, or whether said individual suffers from asthma, comprising the steps of:

a) genotyping said individual for at least one MGST-II-related biallelic marker by a method according to the invention; and
b) correlating the result of step a) with a risk of developing asthma.

In a further aspect the invention provides a method of determining whether an individual is at risk of developing hepatoxicity upon treatment with zileuton, comprising:

a) genotyping said individual for at least one MGST-II-related biallelic marker by a method according to the invention; and
b) correlating the result of step a) with a risk of developing hepatoxicity upon treatment with zileuton.

Also described herein is a method of administering a drug or a treatment comprising the steps of: a) obtaining a nucleic acid sample from an individual; b) determining the identity of the polymorphic base of at least one DME-related biallelic marker which is associated with a positive response to the treatment or the drug; or at least one biallelic DME-related biallelic marker which is associated with a negative response to the treatment or the drug; and c) administering the treatment or the drug to the individual if the nucleic acid sample contains said biallelic marker associated with a positive response to the treatment or the drug or if the nucleic acid sample lacks said biallelic marker associated with a negative response to the treatment or the drug. In addition, the methods for administering a drug or a treatment encompass methods with any further limitation described in this disclosure, or those following, specified alone or in any combination: optionally, said DME-related biallelic marker may be in a sequence selected individually or in any combination from the group consisting of SEQ ID Nos. 1-30, 436-441, 469-472, 474-477, 484-487, 490-493; the complements thereof; or preferably SEQ ID Nos. 3, 5, 9, 13-15, 25; and the complements thereof or optionally, the administering step comprises administering the drug or the treatment to the individual if the nucleic acid sample contains said biallelic marker associated with a positive response to the treatment or the drug and the nucleic acid sample lacks said biallelic marker associated with a negative response to the treatment or the drug.

Also described is a method of selecting an individual for inclusion in a clinical trial of a treatment or drug comprising the steps of: a) obtaining a nucleic acid sample from an individual; b) determining the identity of the polymorphic base of at least one DME-related biallelic marker which is associated with a positive response to the treatment or the drug, or at least one DME-related biallelic marker which is associated with a negative response to the treatment or the drug in the nucleic acid sample, and c) including the individual in the clinical trial if the nucleic acid sample contains said DME-related biallelic marker associated with a positive response to the treatment or the drug or if the nucleic acid sample lacks said biallelic marker associated with a negative response to the treatment or the drug. In addition, the methods for selecting an individual for inclusion in a clinical trial of a treatment or drug encompass methods with any further limitation described in this disclosure, or those following, specified alone or in any combination: Optionally, said DME-related biallelic marker may be in a sequence selected individually or in any combination from the group consisting of SEQ ID Nos. 1-30, 436-441, 469-472, 474-477, 484-487, 490-493; the complements thereof; or preferably SEQ ID Nos. 3, 5, 9, 13-15, 25; and the complements thereof, optionally, the including step comprises administering the drug or the treatment to the individual if the nucleic acid sample contains said biallelic marker associated with a positive response to the treatment or the drug and the nucleic acid sample lacks said biallelic marker associated with a negative response to the treatment or the drug.

Additional embodiments are set forth in the Detailed Description of the Invention and in the Examples.

## BRIEF DESCRIPTION OF THE DRAWINGS

Figure 1 is a chart containing a list of all of the DME-related biallelic markers for the MGSTII gene with an indication of whether the markers have been validated by microsequencing (with a Y indicating that the markers have been validated by microsequencing and an N indicating that it has not), and an indication of the identity and frequency of the least common allele determined by genotyping (with a blank left to indicate that the frequency has not yet been reported for some markers).

Figure 2, 3, and 4 are charts containing lists of the MGSTII-related biallelic markers. Each marker is described by indicating its SEQ ID, the biallelic marker ID, and the two most common alleles. Figure 2 is a chart containing a list of biallelic markers surrounded by preferred sequences. In the column labeled, "POSITION RANGE OF PREFERRED SEQUENCE" of Figure 2, regions of particularly preferred sequences are listed for each SEQ ID, which contain a DME-related biallelic marker, as well as particularly preferred regions of sequences that may not contain a DME-related biallelic marker but, which are in sufficiently close proximity to a DME-related biallelic marker to be useful as amplification or sequencing primers.

Figure 5 is a chart listing particular preferred sequences that are useful for designing primers and probes. Each sequence is described by indicating its Sequence ID and the positions of the first and last nucleotides (position range) of the particular sequence in the Sequence ID.

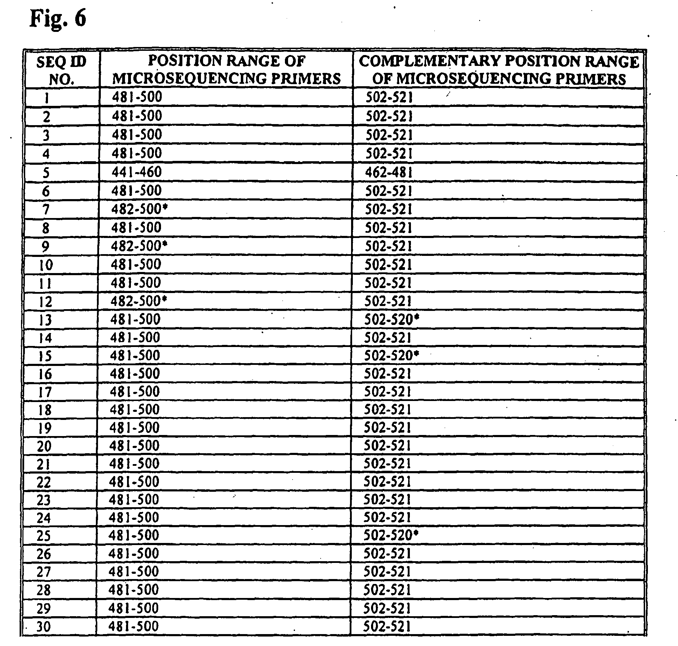

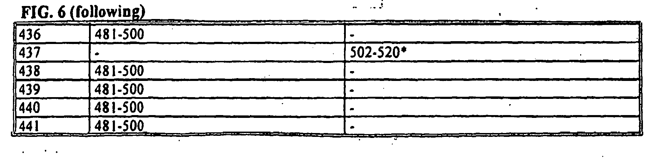

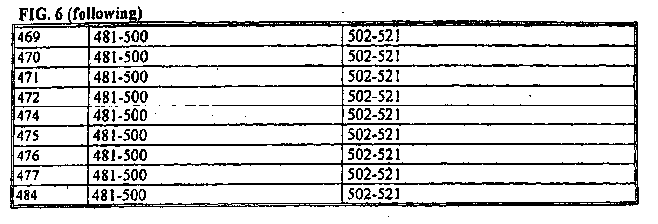

Figure 6 is a chart listing microsequencing primers which have been used to genotype DME-related biallelic markers (indicated by an *) and other preferred microsequencing primers for use in genotyping DME-related biallelic markers. Each of the primers which falls within the strand of nucleotides included in the Sequence Listing are described by indicating their Sequence ID number and the positions of the first and last nucleotides (position range) of the primers in the Sequence ID. Since the sequences in the Sequence Listing are single stranded and half the possible microsequencing primers are composed of nucleotide sequences from the complementary strand, the primers that are composed of nucleotides in the complementary strand are described by indicating their SEQ ID numbers and the positions of the first and last nucleotides to which they are complementary (complementary position range) in the Sequence ID.

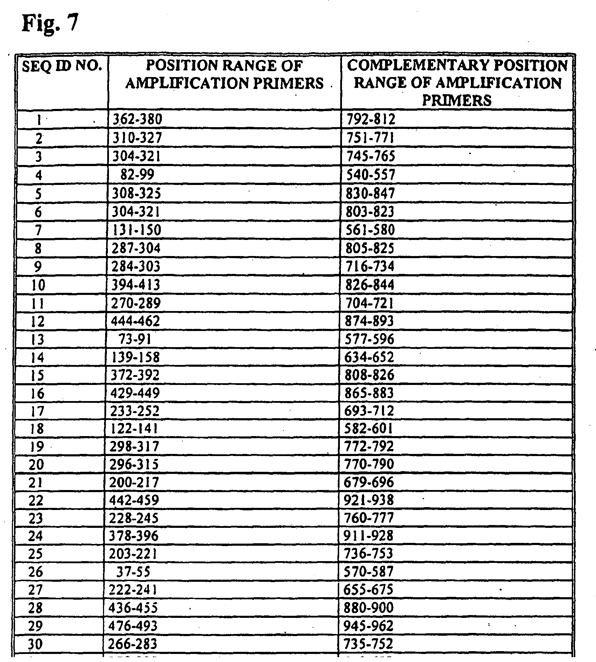

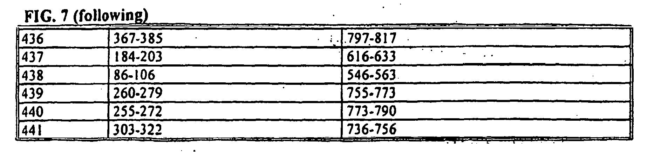

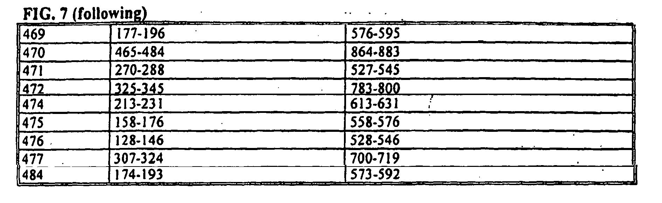

Figure 7 is a chart listing amplification primers which have been used to amplify polynucleotides containing one or more DME-related biallelic markers. Each of the primers which falls within the strand of nucleotides included in the Sequence Listing are described by indicating their Sequence ID number and the positions of the first and last nucleotides (position range) of the primers in the Sequence ID. Since the sequences in the Sequence Listing are single stranded and half the possible amplification primers are composed of nucleotide sequences from the complementary strand, the primers that are composed of nucleotides in the complementary strand are defined by the SEQ ID numbers and the positions of the first and last nucleotides to which they are complementary (complementary position range) in the Sequence ID.

Figure 8 is a chart listing preferred probes useful in genotyping DME-related biallelic markers by hybridization assays. The probes are generally 25-mers with a DME-related biallelic marker in the center position, and described by indicating their Sequence ID number and the positions of the first and last nucleotides (position range) of the probes in the Sequence ID. The probes complementary to the sequences in each position range in each Sequence ID are also understood to be a part of this preferred list even though they are not specified separately.

Figure 9 is a chart containing a list of preferred MGST-II-related biallelic markers with an indication of the frequency of both alleles determined by genotyping. Frequencies were determined in a random US Caucasian population.

Figure 13 is a chart showing the results of a haplotype analysis study demonstrating an association between asthma and MGST-II-related biallelic marker haplotypes.

Figure 14 is a chart showing the results of a permutation test which evaluates the statistical significance of the results obtained for the haplotype analysis.

Figure 15 is a chart showing the results of a haplotype analysis study demonstrating an association between side effects upon treatment with the anti-asthmatic drug Zyflo™ (zileuton) and MGST-II related biallelic marker haplotypes.

Figure 16 is a table showing the results of a phenotypic permutation test which evaluates the statistical significance of the results obtained for the haplotype analysis in Figure 15.

Figure 17 is a block diagram of an exemplary computer system.

Figure 18 is a flow diagram illustrating one embodiment of a process 200 for comparing a new nucleotide or protein sequence with a database of sequences in order to determine the homology levels between the new sequence and the sequences in the database.

Figure 19 is a flow diagram illustrating one embodiment of a process 250 in a computer for determining whether two sequences are homologous.

Figure 20 is a flow diagram illustrating one embodiment of an identifier process 300 for detecting the presence of a feature in a sequence.

## BRIEF DESCRIPTION OF THE SEQUENCES PROVIDED IN THE SEQUENCE LISTING

SEQ ID Nos. 1-30, 436-441, 469-472, 474-477, and 484 contain the nucleotide sequence of genomic DNA fragments located in the vicinity of the MGSTII gene.

SEO ID No. 485 contains the genomic sequence of the MGST-II gene comprising the 5' regulatory region (upstream untranscribed region), the exons and introns, and the 3' regulatory region (downstream untranscribed region).

SEQ ID No. 486 contains a cDNA sequence of MGST-II.

SEQ ID No. 487 contains a cDNA sequence of MGST-II corresponding to an alternative messenger RNA which is due to alternative splicing joining exon 1 to exon 3 and resulting in the absence of exon 2.

SEQ ID No. 488 contains the amino acid sequence encoded by the cDNA of SEQ ID No. 486.

SEQ ID No. 489 contains the amino acid sequence encoded by the cDNA of SEQ ID No. 487.

SEQ ID No. 490 contains a partial cDNA sequence of MGST-II sequence corresponding to a cloned partial messenger RNA.

SEQ ID No. 491 contains a partial cDNA sequence of MGST-II sequence corresponding to a cloned partial messenger RNA.

SEQ ID No. 492 contains a primer containing the additional PU 5' sequence described further in Example 1.

SEQ ID No. 493 contains a primer containing the additional RP 5' sequence described further in Example 1.

## DETAILED DESCRIPTION OF THE INVENTION

### Advantages of the biallelic markers of the present invention

The MGSTII-related biallelic markers of the present invention offer a number of important advantages over other genetic markers such as RFLP (Restriction fragment length polymorphism) and VNTR (Variable Number of Tandem Repeats) markers.

The first generation of markers, were RFLPs, which are variations that modify the length of a restriction fragment. But methods used to identify and to type RFLPs are relatively wasteful of materials, effort, and time. The second generation of genetic markers were VNTRs, which can be categorized as either minisatellites or microsatellites. Minisatellites are tandemly repeated DNA sequences present in units of 5-50 repeats which are distributed along regions of the human chromosomes ranging from 0.1 to 20 kilobases in length. Since they present many possible alleles, their informative content is very high. Minisatellites are scored by performing Southern blots to identify the number of tandem repeats present in a nucleic acid sample from the individual being tested. However, there are only $10^4$ potential VNTRs that can be typed by Southern blotting. Moreover, both RFLP and VNTR markers are costly and time-consuming to develop and assay in large numbers.

Single nucleotide polymorphism or biallelic markers can be used in the same manner as RFLPs and VNTRs but offer several advantages. Single nucleotide polymorphisms are densely spaced in the human genome and represent the most frequent type of variation. An estimated number of more than $10^7$ sites are scattered along the $3x10^9$ base pairs of the human genome. Therefore, single nucleotide polymorphism occur at a greater frequency and with greater uniformity than RFLP or VNTR markers which means that there is a greater probability that such a marker will be found in close proximity to a genetic locus of interest. Single nucleotide polymorphisms are less variable than VNTR markers but are mutationally more stable.

Also, the different forms of a characterized single nucleotide polymorphism, such as the biallelic markers of the present invention, are often easier to distinguish and can therefore be typed easily on a routine basis. Biallelic markers have single nucleotide based alleles and they have only two common alleles, which allows highly parallel detection and automated scoring. The biallelic markers of the present invention offer the possibility of rapid, high-throughput genotyping of a large number of individuals.

Biallelic markers are densely spaced in the genome, sufficiently informative and can be assayed in large numbers. The combined effects of these advantages make biallelic markers extremely valuable in genetic studies. Biallelic markers can be used in linkage studies in families, in allele sharing methods, in linkage disequilibrium studies in populations, in association studies of case-control populations. An important aspect of the present invention is that biallelic markers allow association studies to be performed to identify genes involved in complex traits. Association studies examine the frequency of marker alleles in unrelated case- and control-populations and are generally employed in the detection of polygenic or sporadic traits. Association studies may be conducted within the general population and are not limited to studies performed on related individuals in affected families (linkage studies). Biallelic markers in different genes can be screened in parallel for direct association with disease or response to a treatment. This multiple gene approach is a powerful tool for a variety of human genetic studies as it provides the necessary statistical power to examine the synergistic effect of multiple genetic factors on a particular phenotype, drug response, sporadic trait, or disease state with a complex genetic etiology.

**Candidate genes of the present invention**

Different approaches can be employed to perform association studies: genome-wide association studies, candidate region association studies and candidate gene association studies. Genome-wide association studies rely on the screening of genetic markers evenly spaced and covering the entire genome. Candidate region association studies rely on the screening of genetic markers evenly spaced covering a region identified as linked to the trait of interest. The candidate gene approach is based on the study of genetic markers specifically derived from genes potentially involved in a biological pathway related to the trait of interest. In the present invention, genes involved in drug metabolism have been chosen as candidate genes. As mentioned above, these genes are highly relevant to pharmacogenetics because they are at the core of drug response, drug efficacy and toxicity, moreover, drug-metabolizing enzymes also determine an individuals susceptibility to exogenous chemicals and to a number of diseases associated with exposure to toxic or carcinogenic chemicals. The candidate gene analysis clearly provides a short-cut approach to the identification of genes and gene polymorphisms related to a particular trait when some information concerning the biology of the trait is available. However, it should be noted that all of the biallelic markers disclosed in the instant application can be employed as part of genome-wide association studies or as part of candidate region association studies. All of the markers are known to be in close proximity to the genes with which they are listed in Figure 1. For a portion of the markers, the precise position of the marker with respect to the various coding and non-coding elements of the genes has also been determined.

The following is a table of abbreviations for the candidate genes as they appear throughout the specification and figures:

**Table 1**

| Candidate Gene | Abbreviation |
|---|---|
| Microsomal glutathione S-transferase II | MGST2 or MGST II |
| Malate decarboxylase enzyme | DME or ME1 |
| Cytochrome P450 1A2 | CYP1A2 |

(continued)

| Candidate Gene | Abbreviation |
|---|---|
| Cytochrome P450 2C8 | CYP2C8 |
| Cytochrome P450 2C9 | CYP2C9 |
| Cytochrome P450 2C 18 | CYP2C18 |
| Cytochrome P450 3A4-3A7 | CYP3A4-CYP3A7 |
| Cytochrome P450 3A7 | CYP3A7 |
| Flavin-containing monooxygenases | FMO |
| Glutathione reductase | GSHR |
| Glutathione synthase | GSHS |
| γ-glutamylcysteine synthetase | GLCL |
| γ-glutamyltransferase 5 | GGT5 |
| Dipeptidase | DP |
| Glucose 6-phosphate dehydrogenase | G6PDH |
| Phosphogluconate dehydrogenase | PGDH |
| Uridine diphosphate glucoronosyl transferase 1A7 | UGT I A7 |
| Uridine diphosphate glucoronosyl transferase B4 | UGT2B4 |
| Uridine diphosphate glucoronosyl transferase B7 | UGT2B7 |
| Uridine diphosphate glucoronosyl transferase B 10 | UGT2B10 |
| Uridine diphosphate glucoronosyl transferase B15 | UGT2B15 |

**Definitions**

As used interchangeably herein, the terms "nucleic acids," "oligonucleotides" and "polynucleotides" include RNA, DNA, or RNA/DNA hybrid sequences of more than one nucleotide in either single chain or duplex form. The term "nucleotide" as used herein as an adjective to describe molecules comprising RNA, DNA, or RNA/DNA hybrid sequences of any length in single-stranded or duplex form. The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. Although the term "nucleotide" is also used herein to encompass "modified nucleotides" which comprise at least one modifications (a) an alternative linking group, (b) an analogous form of purine, (c) an analogous form of pyrimidine, or (d) an analogous sugar, for examples of analogous linking groups, purine, pyrimidines, and sugars see for example PCT publication No. WO 95/04064. However, the polynucleotides of the invention are preferably comprised of greater than 50% conventional deoxyribose nucleotides, and most preferably greater than 90% conventional deoxyribose nucleotides. The polynucleotide sequences of the invention may be prepared by any known method, including synthetic, recombinant, *ex vivo* generation, or a combination thereof, as well as utilizing any purification methods known in the art.

Throughout the present specification, the expression "nucleotide sequence" may be employed to designate indifferently a polynucleotide or a nucleic acid. More precisely, the expression "nucleotide sequence" encompasses the nucleic material itself and is thus not restricted to the sequence information (i.e. the succession of letters chosen among the four base letters) that biochemically characterizes a specific DNA or RNA molecule.

The term "polypeptide" refers to a polymer of amino without regard to the length of the polymer; thus, peptides, oligopeptides, and proteins are included within the definition of polypeptide. This term also does not specify or exclude prost-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated, biological system, modified amino acids from mammalian systems etc.), polypeptides with substituted linkages, as well as other modifications known in the art, both naturally occurring and non-naturally occurring.

The term "recombinant polypeptide" is used herein to refer to polypeptides that have been artificially designed and which comprise at least two polypeptide sequences that are not found as contiguous polypeptide sequences in their initial natural environment, or to refer to polypeptides which have been expressed from a recombinant polynucleotide.

The term "isolated" requires that the material be removed from its original environment (e. g., the natural environment if it is naturally occurring). For example, a naturally-occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

The term "purified" is used herein to describe a polynucleotide or polynucleotide vector of the invention which has been separated from other compounds including, but not limited to other nucleic acids, carbohydrates, lipids and proteins (such as the enzymes used in the synthesis of the polynucleotide), or the separation of covalently closed polynucleotides from linear polynucleotides. A polynucleotide is substantially pure when at least about 50 %, preferably 60 to 75% of a sample exhibits a single polynucleotide sequence and conformation (linear versus covalently close). A substantially pure polynucleotide typically comprises about 50 %, preferably 60 to 90% weight/weight of a nucleic acid sample, more usually about 95%, and preferably is over about 99% pure. Polynucleotide purity or homogeneity may be indicated by a number of means well known in the art, such as agarose or polyacrylamide gel electrophoresis of a sample, followed by visualizing a single polynucleotide band upon staining the gel. For certain purposes higher resolution can be provided by using HPLC or other means well known in the art.

The term "purified" is further used herein to describe a polypeptide of the invention which, has been separated from other compounds including, but not limited to nucleic acids, lipids, carbohydrates and other proteins. A polypeptide is substantially pure when at least about 50%, preferably 60 to 75% of a sample exhibits a single polypeptide sequence. A substantially pure polypeptide typically comprises about 50%, preferably 60 to 90% weight/weight of a protein sample, more usually about 95%, and preferably is over about 99% pure. Polypeptide purity or homogeneity is indicated by a number of means well known in the art, such as agarose or polyacrylamide gel electrophoresis of a sample, followed by visualizing a single polypeptide band upon staining the gel. For certain purposes higher resolution can be provided by using HPLC or other means well known in the art.

As used herein, the term "non-human animal" refers to any non-human vertebrate, birds and more usually mammals, preferably primates, farm animals such as swine, goats, sheep, donkeys, and horses, rabbits or rodents, more preferably rats or mice. As used herein, the term "animal" is used to refer to any vertebrate, preferable a mammal. Both the terms "animal" and "mammal" expressly embrace human subjects unless preceded with the term "non-human".

As used herein, the term "antibody" refers to a polypeptide or group of polypeptides which are comprised of at least one binding domain, where an antibody binding domain is formed from the folding of variable domains of an antibody molecule to form three-dimensional binding spaces with an internal surface shape and charge distribution complementary to the features of an antigenic determinant of an antigen., which allows an immunological reaction with the antigen. Antibodies include recombinant proteins comprising the binding domains, as wells as fragments, including Fab, Fab', $F(ab)_2$, and $F(ab')_2$ fragments.

As used herein, an "antigenic determinant" is the portion of an antigen molecule, in this case a MGST-II polypeptide, that determines the specificity of the antigen-antibody reaction. An "epitope" refers to an antigenic determinant of a polypeptide. An epitope can comprise as few as 3 amino acids in a spatial conformation which, is unique to the epitope. Generally an epitope consists of at least 6 such amino acids, and more usually at least 8-10 such amino acids. Methods for determining the amino acids which make up an epitope include x-ray crystallography, 2-dimensional nuclear magnetic resonance, and epitope mapping e.g. the Pepscan method described by H. Mario Geysen et al. 1984. Proc. Natl. Acad. Sci. U.S.A. 81:3998-4002; PCT Publication No. WO 84/03564; and PCT Publication No. WO 84/03506.

The term "primer" denotes a specific oligonucleotide sequence which is complementary to a target nucleotide sequence and used to hybridize to the target nucleotide sequence. A primer serves as an initiation point for nucleotide polymerization catalyzed by either DNA polymerase, RNA polymerase or reverse transcriptase.

The term "probe" denotes a defined nucleic acid segment (or nucleotide analog segment, e.g., polynucleotide as defined herein) which can be used to identify a specific polynucleotide sequence present in samples, said nucleic acid segment comprising a nucleotide sequence complementary of the specific polynucleotide sequence to be identified.

The terms "trait" and "phenotype" are used interchangeably herein and refer to any visible, detectable or otherwise measurable property of an organism such as symptoms of, or susceptibility to a disease for example. Typically the terms "trait" or "phenotype" are used herein to refer to symptoms of, or susceptibility to a disease; or to refer to an individual's response to a drug; or to refer to symptoms of, or susceptibility to side effects to a drug. In addition, the terms "trait" or "phenotype" may be used herein to refer to symptoms of, or susceptibility to a disease involving arachidonic acid metabolism; or to refer to an individual's response to an agent acting on arachidonic acid metabolism; or to refer to symptoms of, or susceptibility to side effects to an agent acting on arachidonic acid metabolism.

The term "allele" is used herein to refer to variants of a nucleotide sequence. A biallelic polymorphism has two forms. Typically the first identified allele is designated as the original allele whereas other alleles are designated as alternative alleles. Diploid organisms may be homozygous or heterozygous for an allelic form.

The term "heterozygosity rate" is used herein to refer to the incidence of individuals in a population, which are heterozygous at a particular allele. In a biallelic system the heterozygosity rate is on average equal to $2P_a(1-P_a)$, where $P_a$ is the frequency of the least common allele. In order to be useful in genetic studies a genetic marker should have an adequate level of heterozygosity to allow a reasonable probability that a randomly selected person will be heterozygous.

The term "genotype" as used herein refers the identity of the alleles present in an individual or a sample. In the context of the present invention a genotype preferably refers to the description of the biallelic marker alleles present in an individual or a sample. The term "genotyping" a sample or an individual for a biallelic marker consists of determining the specific allele or the specific nucleotide carried by an individual at a biallelic marker.

The term "mutation" as used herein refers to a difference in DNA sequence between or among different genomes or individuals which has a frequency below 1%.

The term "haplotype" refers to a combination of alleles present in an individual or a sample. In the context of the present invention a haplotype preferably refers to a combination of biallelic marker alleles found in a given individual and which may be associated with a phenotype.

The term "polymorphism" as used herein refers to the occurrence of two or more alternative genomic sequences or alleles between or among different genomes or individuals. "Polymorphic" refers to the condition in which two or more variants of a specific genomic sequence can be found in a population. A "polymorphic site" is the locus at which the variation occurs. A single nucleotide polymorphism is a single base pair change. Typically a single nucleotide polymorphism is the replacement of one nucleotide by another nucleotide at the polymorphic site. Deletion of a single nucleotide or insertion of a single nucleotide, also give rise to single nucleotide polymorphisms. In the context of the present invention "single nucleotide polymorphism" preferably refers to a single nucleotide substitution. Typically, between different genomes or between different individuals, the polymorphic site may be occupied by two different nucleotides.

The terms "biallelic polymorphism" and "biallelic marker" are used interchangeably herein to refer to a polymorphism having two alleles at a fairly high frequency in the population, preferably a single nucleotide polymorphism. A "biallelic marker allele" refers to the nucleotide variants present at a biallelic marker site. Typically the frequency of the less common allele of the biallelic markers of the present invention has been validated to be greater than 1%, preferably the frequency is greater than 10%, more preferably the frequency is at least 20% (i.e. heterozygosity rate of at least 0.32), even more preferably the frequency is at least 30% (i.e. heterozygosity rate of at least 0.42). A biallelic marker wherein the frequency of the less common allele is 30% or more is termed a "high quality biallelic marker."

The location of nucleotides in a polynucleotide with respect to the center of the polynucleotide are described herein in the following manner. When a polynucleotide has an odd number of nucleotides, the nucleotide at an equal distance from the 3' and 5' ends of the polynucleotide is considered to be "at the center" of the polynucleotide, and any nucleotide immediately adjacent to the nucleotide at the center, or the nucleotide at the center itself is considered to be "within I nucleotide of the center." With an odd number of nucleotides in a polynucleotide any of the five nucleotides positions in the middle of the polynucleotide would be considered to be within 2 nucleotides of the center, and so on. When a polynucleotide has an even number of nucleotides, there would be a bond and not a nucleotide at the center of the polynucleotide. Thus, either of the two central nucleotides would be considered to be "within 1 nucleotide of the center" and any of the four nucleotides in the middle of the polynucleotide would be considered to be "within 2 nucleotides of the center", and so on. For polymorphisms which involve the substitution, insertion or deletion of I or more nucleotides, the polymorphism, allele or biallelic marker is "at the center" of a polynucleotide if the difference between the distance from the substituted, inserted, or deleted polynucleotides of the polymorphism and the 3' end of the polynucleotide, and the distance from the substituted, inserted, or deleted polynucleotides of the polymorphism and the 5' end of the polynucleotide is zero or one nucleotide. If this difference is 0 to 3, then the polymorphism is considered to be "within 1 nucleotide of the center." If the difference is 0 to 5, the polymorphism is considered to be "within 2 nucleotides of the center." If the difference is 0 to 7, the polymorphism is considered to be "within 3 nucleotides of the center," and so on. For polymorphisms which involve the substitution, insertion or deletion of 1 or more nucleotides, the polymorphism, allele or biallelic marker is "at the center" of a polynucleotide if the difference between the distance from the substituted, inserted, or deleted polynucleotides of the polymorphism and the 3' end of the polynucleotide, and the distance from the substituted, inserted, or deleted polynucleotides of the polymorphism and the 5' end of the polynucleotide is zero or one nucleotide. If this difference is 0 to 3, then the polymorphism is considered to be "within 1 nucleotide of the center." If the difference is 0 to 5, the polymorphism is considered to be "within 2 nucleotides of the center." If the difference is 0 to 7, the polymorphism is considered to be "within 3 nucleotides of the center," and so on.

The term "upstream" is used herein to refer to a location which, is toward the 5' end of the polynucleotide from a specific reference point.

The terms "base paired" and "Watson & Crick base paired" are used interchangeably herein to refer to nucleotides which can be hydrogen bonded to one another be virtue of their sequence identities in a manner like that found in double-helical DNA with thymine or uracil residues linked to adenine residues by two hydrogen bonds and cytosine and guanine residues linked by three hydrogen bonds (See Stryer, L., Biochemistry, 4th edition, 1995).

The terms "complementary" or "complement thereof" are used herein to refer to the sequences of polynucleotides which is capable of forming Watson & Crick base pairing with another specified polynucleotide throughout the entirety of the complementary region. This term is applied to pairs of polynucleotides based solely upon their sequences and not any particular set of conditions under which the two polynucleotides would actually bind.

A "promoter" refers to a DNA sequence recognized by the synthetic machinery of the cell required to initiate the specific transcription of a gene.

A sequence which is "operably linked" to a regulatory sequence such as a promoter means that said regulatory element is in the correct location and orientation in relation to the nucleic acid to control RNA polymerase initiation and expression of the nucleic acid of interest.

As used herein, the term "operably linked" refers to a linkage of polynucleotide elements in a functional relationship. For instance, a promoter or enhancer is operably linked to a coding sequence if it affects the transcription of the coding sequence. More precisely, two DNA molecules (such as a polynucleotide containing a promoter region and a polynucleotide encoding a desired polypeptide or polynucleotide) are said to be "operably linked" if the nature of the linkage between the two polynucleotides does not (1) result in the introduction of a frame-shift mutation or (2) interfere with the ability of the polynucleotide containing the promoter to direct the transcription of the coding polynucleotide.

The terms "disease involving the metabolic conversion of xenobiotics" refers to susceptibility to a condition or to a condition linked to any of the genes listed in Table 1. "Disease involving the metabolic conversion ofxenobiotics" further refers to a condition involving the biotransformation of drugs and other xenobiotics such as environmental chemicals, food toxins, plant metabolites, carcinogens and industrial chemicals. Such conditions include susceptibility to the toxic or carcinogenic effect of exogenous compounds. "Disease involving the metabolic conversion of xenobiotics" also refers to disorders in the metabolism of some endogenous compounds such as the metabolism of steroids, vitamins, fatty acids and eicosanoids such as leukotrienes involving any of the drug-metabolizing enzymes shown in Table 1. "Disease involving the metabolic conversion of xenobiotics" includes, but is not limited to, disorders involving the cytochrome P450 enzyme family, the flavin containing monooxygenases, glucoronidation, the metabolism of glutathione, the pentose pathway and the generation of NADPH.

The term "disease involving arachidonic acid metabolism" refers to a condition linked to disturbances in expression, production or cellular response to eicosanoids such as prostaglandins, thromboxanes, prostacyclins, leukotrienes or hydroperoxyeicosaetrenoic acids. A disease involving arachidonic acid metabolism further refers to a condition involving one or several enzymes of the distinct enzyme systems contributing to arachidonate metabolism including particularly the 5-lipoxygenase pathway. "Diseases involving arachidonic acid metabolism" also include chronic inflammatory diseases, acute allergic inflammation and inflammatory conditions such as pain, fever, hypersensitivity, asthma, psoriasis and arthritis. "Diseases involving arachidonic acid metabolism" also include disorders in platelet function, blood pressure, thrombosis, renal function, host defense mechanism, hemostasis, smooth muscle tone, male infertility, primary dysmenorrhea, disorders in parturition, and disorders in tissue injury repair, as well as disorders in cellular function and development. "Diseases involving arachidonic acid metabolism" also include diseases such as gastrointestinal ulceration, coronary and cerebrovascular syndromes, glomerular immune injury and cancer. Preferably the terms "disease involving arachidonic acid metabolism" refer to a disease including diseases such as cancer, prostate cancer, breast cancer, psoriasis and inflammatory diseases. Preferably the terms "disease involving arachidonic acid metabolism" refer to a disease involving the 5-lipoxygenase pathway and the biosynthesis of the leukotrienes. More preferably the terms "disease involving arachidonic acid metabolism" refer to a disease involving the synthesis of leukotriene C4 (LTC4) and refers to disturbances in expression, activity or function of the human MGST-II enzyme.

As used herein the term "DME-related biallelic marker" relates to a set of biallelic markers located in or in the vicinity of the genes disclosed in Table 1 and further relates to biallelic markers in linkage disequilibrium with the biallelic markers disclosed in Figure 1. The term DME-related biallelic marker encompasses all of the biallelic markers disclosed in Figure 1.

The invention also concerns MGST-II-related biallelic markers. The term "MGSTII-related biallelic marker" is used interchangeably herein to relate to all biallelic markers in linkage disequilibrium with the biallelic markers of the MGST-II gene. The term MGST-U-related biallelic marker includes both the genic and non-genic biallelic markers described in Table 2.

The term "non-genic" is used herein to describe MGST-II-related biallelic markers, as well as polynucleotides and primers which occur outside the nucleotide positions shown in the human MGST-II genomic sequence of SEQ ID No. 485. The term "genie" is used herein to describe MGST-II-related biallelic markers as well as polynucleotides and primers which do occur in the nucleotide positions shown in the human MGST-II genomic sequence of SEQ ID No. 485.

The terms "agent acting on arachidonic acid metabolism" refers to a drug or a compound modulating the activity or concentration of one or several enzymes of the distinct enzyme systems contributing to arachidonate metabolism including particularly the 5-lipoxygenase pathway. "Agent acting on arachidonic acid metabolism" also refers to compounds modulating the formation and action of the eicosanoids including particularly the leukotrienes.

The terms "response to a drug " refer to drug efficacy, including but not limited to ability to metabolize a therapeutic compound, to the ability to convert a pro-drug to an active drug, and to the pharmacokinetics (absorption, distribution, elimination) and the pharmacodynamics (receptor-related) of a drug in an individual.

The terms "response to an agent acting on arachidonic acid metabolism" refer to drug efficacy, including but not limited to ability to metabolize a compound, to the ability to convert a pro-drug to an active drug, and to the pharmacokinetics (absorption, distribution, elimination) and the pharmacodynamics (receptor-related) of a drug in an individual.

The terms "side effects to a drug" refer to adverse effects of therapy resulting from extensions of the principal pharmacological action of the drug or to idiosyncratic adverse reactions resulting from an interaction of the drug with unique host factors. "Side effects to a drug" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

The terms "side effects to an agent acting on arachidonic acid metabolism" refer to adverse effects of therapy resulting from extensions of the principal pharmacological action of the drug or to idiosyncratic adverse reactions resulting from an interaction of the drug with unique host factors. The terms "side effects to an agent acting on arachidonic acid metabolism" include, but are not limited to, adverse reactions such as dermatologic, hematologic or hepatologic toxicities.

The term "sequence described in Figure 2" is used herein to refer to the entire collection of nucleotide sequences or any individual sequence defined in Figure 2. The SEQ ID that contains each "sequence described in Figure 2" is provided in the column labeled, "SEQ ID NO." The range of nucleotide positions within the Sequence ID of which each sequence consists is provided in the same row as the Sequence ID in a column labeled, "POSITION RANGE OF PREFERRED SEQUENCE". It should be noted that some of the Sequence ID numbers have multiple sequence ranges listed, because they contain multiple "sequences described in Figure 2." Unless otherwise noted the term "sequence described in Figure 2" is to be construed as encompassing sequences that contain either of the two alleles listed in the columns labeled, "$1^{ST}$ ALLELE" and "$2^{ND}$ ALLELE" at the position identified in field <222> of the allele feature in the appended Sequence Listing for each Sequence ID number referenced in Figure 2.

The term "sequence described in Figure 3" is used herein to refer to the entire collection of nucleotide sequences or any individual sequence defined in Figure 3. Unless otherwise noted, the "sequences described in Figure 3" consist of the entire sequence of each Sequence ID provided in the column labeled, "SEQ ID NO." Also unless otherwise noted the term "sequence described in Figure 3" is to be construed as encompassing sequences that contain either of the two alleles listed in the columns labeled, "ORIGINAL ALLELE" and "ALTERNATIVE ALLELE" at the position identified in field <222> of the allele feature in the appended Sequence Listing for each Sequence ID number referenced in Figure 3.

The term "sequence described in Figure 4" is used herein to refer to the entire collection of nucleotide sequences or any individual sequence defined in Figure 4. Unless otherwise noted, the "sequences described in Figure 4" consist of the entire sequence of each Sequence ID provided in the column labeled, "SEQ ID NO." Also unless otherwise noted the term "sequence described in Figure 4" is to be construed as encompassing sequences that contain either of the two alleles listed in the columns labeled, "$1^{ST}$ ALLELE" and "$2^{ND}$ ALLELE" at the position identified in field <222> of the allele feature in the appended Sequence Listing for each Sequence ID number referenced in Figure 4.

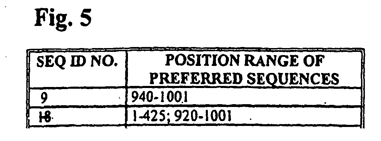

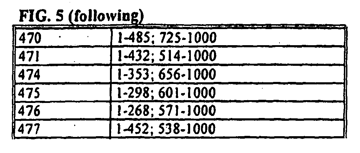

The term "sequence described in Figure 5" is used herein to refer to the entire collection of nucleotide sequences or any individual sequence defined in Figure 5. The SEQ ID that contains each "sequence described in Figure 5 " is provided in the column labeled, "SEQ ID NO." The range of nucleotide positions within the Sequence ID of which each sequence consists is provided in the same row as the Sequence ID in a column labeled, "POSITION RANGE OF PREFERRED SEQUENCE". It should be noted that some of the Sequence ID numbers have multiple sequence ranges listed, because they contain multiple "sequences described in Figure 5."

The term "sequence described in Figure 6" is used herein to refer to the entire collection of nucleotide sequences or any individual sequence defined in Figure 6. The SEQ ID that contains each "sequence described in Figure 6" is provided in the column labeled, "SEQ ID" The range of nucleotide positions within the Sequence ID of which half of the sequences consists is provided in the same row as the Sequence ID in a column labeled, "POSITION RANGE OF MICROSEQUENCING PRIMERS". The remaining half of the sequences described in Figure 6 are complementary to the range of nucleotide positions within the Sequence ID provided in the same row as the Sequence ID in a column labeled, "COMPLEMENTARY POSITION RANGE OF MICROSEQUENCING PRIMERS".

The term "sequence described in Figure 7" is used herein to refer to the entire collection of nucleotide sequences or any individual sequence defined in Figure 7. The SEQ ID that contains each "sequence described in Figure 7 " is provided in the column labeled, "SEQ ID" The range of nucleotide positions within the Sequence ID of which half of the sequences consists is provided in the same row as the Sequence ID in a column labeled, "POSITION RANGE OF AMPLIFICATION PRIMERS". The remaining half of the sequences described in Figure 7 are complementary to the range of nucleotide positions within the Sequence ID provided in the same row as the Sequence ID in a column labeled, "COMPLEMENTARY POSITION RANGE OF AMPLIFICATION PRIMERS".

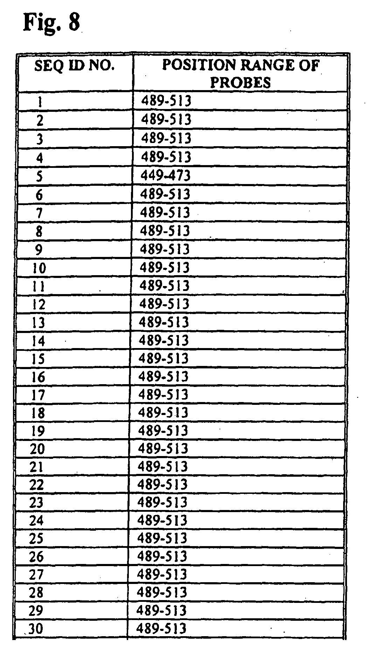

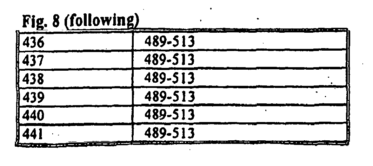

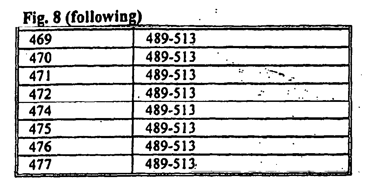

The term "sequence described in Figure 8" is used herein to refer to the entire collection of nucleotide sequences or any individual sequence defined in Figure 8. The SEQ ID that contains each "sequence described in Figure 8 " is provided in the column labeled, "SEQ ID". The range of nucleotide positions within the Sequence ID of which each sequence consists is provided in the same row as the Sequence ID in a column labeled, "POSITION RANGE OF PROBES". The sequences which are complementary to the ranges listed in the column labeled, "POSITION RANGE OF PROBES" are also encompassed by the term, "sequence described in Figure 8." Unless otherwise noted the term "sequence described in Figure 8 " is to be construed as encompassing sequences that contain either of the two alleles listed in the allele feature in the sequence listing.

The terms "biallelic marker described in Figure" and "allele described in Figure" are used herein to refer to any or all alleles which are listed in the allele feature in the appended Sequence Listing for each Sequence ID number referenced in the particular Figure being mentioned.

**Variants and Fragments**

The invention also relates to variants and fragments of the polynucleotides described herein, particularly of a MGST-II gene containing one or more biallelic markers according to the invention.

Variants of polynucleotides, as the term is used herein, are polynucleotides that differ from a reference polynucleotide. A variant of a polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms. Generally, differences are limited so that the nucleotide sequences of the reference and the variant are closely similar overall and, in many regions, identical. Variants of polynucleotides include, without being limited to, nucleotide sequences which are at least 95% identical , preferably at least 99% identical, more particularly at least 99.5% identical, and most preferably at least 99.8% identical to a polynucleotide selected from the group consisting of the polynucleotides of a sequence from any sequence in the Sequence Listing as well as sequences which are complementary thereto or to any polynucleotide fragment of at least 8 consecutive nucleotides of a sequence from any sequence in the Sequence Listing. Nucleotide changes present in a variant polynucleotide may be silent, which means that they do not alter the amino acids encoded by the polynucleotide. However, nucleotide changes may also result in amino acid substitutions, additions, deletions, fusions and truncations in the polypeptide encoded by the reference sequence. The substitutions, deletions or additions may involve one or more nucleotides. The variants may be altered in coding or non-coding regions or both. Alterations in the coding regions may produce conservative or non-conservative amino acid substitutions, deletions or additions variant polynucleotides may include those in which the polynucleotides encode polypeptides which retain substantially the same biological function or activity as the mature MGST-II protein, or those in which the polynucleotides encode polypeptides which maintain or increase a particular biological activity, while reducing a second biological activity. A polynucleotide fragment is a polynucleotide having a sequence that is entirely the same as part but not all of a given nucleotide sequence, preferably the nucleotide sequence of a MGST-II gene, and variants thereof. The fragment can be a portion of an exon or of an intron of a MGST-II gene. It can also be a portion of the regulatory regions of MGST-II, preferably of the promoter sequence of the MGST-II gene. Such fragments may be "free-standing", i.e. not part of or fused to other polynucleotides, or they may be comprised within a single larger polynucleotide of which they form a part or region. Indeed, several of these fragments may be present within a single larger polynucleotide.

**Identity Between Nucleic Acids or Polypeptides**

The terms "percentage of sequence identity" and "percentage homology" are used interchangeably herein to refer to comparisons among polynucleotides and polypeptides, and are determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Homology is evaluated using any of the variety of sequence comparison algorithms and programs known in the art. Such algorithms and programs include, but are by no means limited to, TBLASTN, BLASTP, FASTA, TFASTA, and CLUSTALW (Pearson and Lipman, Proc. Natl. Acad. Sci. 85(8):2444-2448, 1988; Altschul et al., J. Mol. Biol. 215(3): 403-410,1990; Thompson et al., Nucleic Acids Res. 22(2):4673-4680, 1994; Higgins et al., Methods Enzymol. 266: 383-402, 1996; Altschul et al., Nature Genetics 3:266-272, 1993). In a particularly preferred embodiment, protein and nucleic acid sequence homologies are evaluated using the Basic Local Alignment Search Tool ("BLAST") which is well known in the art (See, e.g., Karlin and Altschul,. Proc. Natl. Acad. Sci. USA 87:2267-2268, 1990; Altschul et al., J. Mol. Biol. 215(3):403-410, 1990; Altschul et al., Nature Genetics 3:266-272, 1993; Altschul et al., Nuc. Acids Res. 25: 3389-3402, 1997). In particular, five specific BLAST programs are used to perform the following task:

(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which is preferably obtained from a protein or nucleic acid sequence database. High-scoring segment pairs are preferably identified (i.e., aligned) by means of a scoring matrix, many of which are known in the art. Preferably, the scoring matrix used is the BLOSUM62 matrix (Gonnet et al., Science 256:1443-1445, 1992; Henikoffand Henikoff, Proteins 17:49-61, 1993). Less preferably, the PAM or PAM250 matrices may also be used (See, e.g., Schwartz and Dayhoff, eds., Matrices for Detecting Distance Relationships: Atlas of Protein Sequence and Structure, Washington:National Biomedical Research Foundation, 1978 The BLAST programs evaluate the statistical significance of all high-scoring segment pairs identified, and preferably selects those segments which satisfy a user-specified threshold of significance, such as a user-specified percent homology. Preferably, the statistical significance of a high-scoring segment pair is evaluated using the statistical significance formula of Karlin (see, e.g., Karlin and Altschul, 1990).

**Stringent Hybridization Conditions**

By way of example and not limitation, procedures using conditions of high stringency are as follows: Prehybridization of filters containing DNA is carried out for 8 h to overnight at 65°C in buffer composed of 6X SSC, 50 mM Tris-HCl (pH 7.5), 1 mM EDTA, 0.02% PVP, 0.02% Ficoll, 0.02% BSA, and 500 μg/ml denatured salmon sperm DNA. Filters are hybridized for 48 h at 65°C, the preferred hybridization temperature, in prehybridization mixture containing 100 μg/ml denatured salmon sperm DNA and 5-20 X $10^6$ cpm of $^{32}$P-labeled probe. Alternatively, the hybridization step can be performed at 65°C in the presence of SSC buffer, 1 x SSC corresponding to 0.15M NaCl and 0.05 M Na citrate. Subsequently, filter washes can be done at 37°C for 1 h in a solution containing 2 x SSC, 0.01% PVP, 0.01% Ficoll, and 0.01% BSA, followed by a wash in 0.1 X SSC at 50°C for 45 min. Alternatively, filter washes can be performed in a solution containing 2 x SSC and 0.1% SDS, or 0.5 x SSC and 0.1% SDS, or 0.1 x SSC and 0.1% SDS at 68°C for 15 minute intervals. Following the wash steps, the hybridized probes are detectable by autoradiography. Other conditions of high stringency which may be used are well known in the art and as cited in Sambrook et al., 1989; and Ausubel et al., 1989. These hybridization conditions are suitable for a nucleic acid molecule of about 20 nucleotides in length. There is no need to say that the hybridization conditions described above are to be adapted according to the length of the desired nucleic acid, following techniques well known to the one skilled in the art. The suitable hybridization conditions may for example be adapted according to the teachings disclosed in the book of Hames and Higgins (NucleicAcid Hybridization: A Practical Approach, IRL Press, Oxford, 1985) or in Sambrook et al. (Molecular Cloning: A Laboratory Manual, 2nd edition, Cold Spring Harbor Laboratory, Cold Spring Harbor, New York, 1989).

**I. Biallelic Markers and Polynucleotides Comprising Biallelic Markers**

**I.A. Polynucleotides of the Present Invention**

Described herein are polynucleotides for use as primers and probes in the methods of the invention. These polynucleotides may consist of, consist essentially of, or comprise a contiguous span of nucleotides of a sequence selected from SEQ ID NOS 3, 5, 9, 12, 13, 14, 15 or 25 as well as sequences which are complementary thereto ("complements thereof"). The "contiguous span" may be at least 8, 10, 12, 15, 18, 20, 25, 35, 40, 50, 70, 80, 100, 250, 500 or 1000 nucleotides in length, to the extent that a contiguous span of these lengths is consistent with the lengths of the particular Sequence ID. It should be noted that the polynucleotides are not limited to having the exact flanking sequences surrounding the polymorphic bases which, are enumerated in the Sequence Listing. Rather, it will be appreciated that the flanking sequences surrounding the biallelic markers, or any of the primers of probes which, are more distant from the markers, may be lengthened or shortened to any extent compatible with their intended use. It will be appreciated that the polynucleotides referred to in the Sequence Listing may be of any length compatible with their intended use. Also the flanking regions outside of the contiguous span need not be homologous to native flanking sequences which actually occur in human subjects. The addition of any nucleotide sequence, which is compatible with the nucleotides intended use is specifically contemplated. The contiguous span may optionally include the DME-related biallelic marker in said sequence. Biallelic markers generally consist of a polymorphism at one single base position. Each biallelic marker therefore corresponds to two forms of a polynucleotide sequence which, when compared with one another, present a nucleotide modification at one position. Usually, the nucleotide modification involves the substitution of one nucleotide for another. Optionally either the original or the alternative allele of the biallelic markers disclosed in Figure 3, or the first or second allele disclosed in Figure 2 and 3 may be specified as being present at the DME-related biallelic marker.

Polynucleotides may include the sequence ranges included in any one the sequence ranges of Figures 2, and 5 to 8 individually or in groups consisting of all the possible combinations of the ranges of included in Figures 2, and 5 to 8. The polynucleotides may also include fragments of at least 8, 10, 12, 15, 18, 20, 25, 35, 40, 50, 70, 80, 100, 250, 500 or 1000 consecutive nucleotides of the sequence ranges included in any one of the sequence ranges of Figures 2, and 5 to 8 to the extent that fragments of these lengths are consistent with the lengths of the particular sequence range. The polynucleotides may also include fragments of at least 8, 10, 12, 15, 18, 20, 25, 35, 40, 50, 70, 80, 100, 250, 500 or 1000 consecutive nucleotides of the sequence complementary to the sequence ranges included in any one of the sequence ranges of Figures 2, and 5 to 8 to the extent that fragments of these lengths are consistent with the lengths of the particular sequence range.

Polynucleotides may include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of a sequence selected from the group consisting of the sequences of SEQ ID Nos. 3, 5, 9, 12, 13, 14, 15, 25 and the complements thereof.

Polynucleotides described herein include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 485, wherein said contiguous span comprises at least 1, 2, 3, 4, 5 or 10 of the following nucleotide positions of SEQ ID No. 485: 1 to 7667, 7726 to 20264, 20365 to 36918, 36991 to 45180, 45263 to 45741, and 45980 to 49327, and the complements thereof. Other polynucleotides include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 486, wherein said contiguous span comprises at least 1, 2, 3, 4, 5 or 10 of nucleotide positions 1 to 198 of SEQ ID No. 486 and the complements thereof. Other polynucleotides include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 487, wherein said contiguous span comprises at least 1, 2, 3, 4, 5 or 10 of nucleotide positions 1 to 198 of SEQ ID No. 487 and the complements thereof. Other polynucleotides of include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 490, wherein said contiguous span comprises at least 1, 2, 3, 4, 5 or 10 of nucleotide positions I to 198 of SEQ ID No. 490 and the complements thereof. Other polynucleotides include polynucleotides comprising, consisting of, or consisting essentially of a nucleotide sequence of SEQ ID No. 491.

Polynucleotides described herein include purified, isolated or recombinant polynucleotides comprising a contiguous span of at least 12, 15, 18, 20, 25, 30, 35, 40. 50, 60, 70, 80, 90, 100, 150, 200, 500, or 1000 nucleotides of a sequence selected from the group consisting of SEQ ID SEQ ID Nos. 3, 5, 9, 13-15, 25, or the complements thereof, wherein said span includes a MGST-II-related biallelic marker. Optionally either allele of the biallelic markers described above in the definition of MGST-II-related biallelic marker is specified as being present at the MGST-II-related biallelic marker.

Additional polynucleotides include isolated, purified or recombinant polynucleotide comprising a contiguous span of at least 12 nucleotides of SEQ ID No. 485 or the complementary sequence thereof, wherein said contiguous span comprises a nucleotide selected from the group consisting of a T at position 36971, a C at position 45214 or a T at position 45741 of SEQ ID No. 485. Additional polynucleotides include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides from a sequence of SEQ ID No. 486, Wherein said contiguous span comprises a T at position 426, a C at position 478 or a T at position 526 of SEQ ID No. 486; or the complement thereof. Additional polynucleotides include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides from a sequence of SEQ ID No. 487, wherein said contiguous span comprises a T at position 325, a C at position 378 or a T at position 426 of SEQ ID No. 487; or the complements thereof.

Table 2 contains a list of preferred MGST-II-related biallelic markers. Each marker is described by indicating its Marker ID, the position of the marker in the SEQ ID and the two most common alleles.

**Table 2**

| BIALLELIC MARKER ID | ALLELES | POSITION OF BIALLELIC MARKER IN SEQ ID |
|---|---|---|
| **Non-genic biallelic markers** | | |
| 12-424-192 | A/G | SEQ ID No. 2, position 501 |
| 12-424-198 | G/T | SEQ ID No. 3, position 501 |
| 12-426-154 | G/A | SEQ ID No. 5, position 461 |
| 12-429-198 | C/T | SEQ ID No. 6, position 501 |
| 12-430-80 | C/T | SEQ ID No. 7, position 501 |
| 12-433-215 | A/G | SEQ ID No. 8, position 501 |
| 12-441-233 | A/G | SEQ ID No. 9, position 501 |
| 12-441-343 | G/A | SEQ ID No. 10, position 501 |
| 12-442-221 | T/C | SEQ ID No. 11, position 501 |
| 12-447-58 | G/C | SEQ ID No. 12, position 501 |
| 12-449-300 | T/C | SEQ ID No. 472, position 501 |
| 12-453-429 | C/T | SEQ ID No. 13, position 501 |
| 12-454-363 | A/G | SEQ ID No. 14, position 501 |
| 12-455-326 | T/C | SEQ ID No. 15, position 501 |
| 12-455-383 | G/A | SEQ ID No. 16, position 501 |
| 12-456-269 | A/G | SEQ ID No. 17, position 501 |
| 12-456-380 | G/T | SEQ ID No. 18, position 501 |
| 12-457-204 | A/G | SEQ ID No. 19, position 501 |
| 12-457-206 | C/T | SEQ ID No. 20, position 501 |
| 12-458-196 | A/T | SEQ ID No. 21, position 501 |
| 12-458-438 | T/C | SEQ ID No. 22, position 501 |
| 12-460-274 | A/G | SEQ ID No. 23, position 501 |
| 12-461-124 | A/C | SEQ ID No. 24, position 501 |
| 12-461-299 | C/T | SEQ ID No. 25, position 501 |
| 12-461-465 | C/T | SEQ ID No. 26, position 501 |
| 12-462-280 | C/T | SEQ ID No. 27, position 501 |
| 12-464-66 | G/T | SEQ ID No. 28, position 501 |
| 12-465-234 | G/T | SEQ ID No. 30, position 501 |
| 12-465-26 | C/T | SEQ ID No. 29, position 501 |
| 12-442-133 | Deletion G | SEQ ID No. 437, position 501 |
| 12-449-63 | Insertion AT | SEQ ID No. 438, position 501 |
| 10-454-242 | Deletion AT | SEQ ID No. 439, position 501 |
| 12-463-230 | Deletion CAT | SEQ ID No. 440, position 501 |
| 12-426-199 | Deletion | SEQ ID No. 441, position 501 |
| **Genic Biallelic markers** | | |
| **Biallelic Markers in Genomic sequence (SEQ ID No. 485)** | | |

(continued)

| Genic Biallelic markers | | |
|---|---|---|
| 10-286-289 | G/C | 7564 |
| 10-286-345 | A/T | 7619 |
| 10-286-375 | A/G | 7649 |
| 12-425-57 | G/A | 17258 |
| 12-421-135 | insertion of a T | 21590 |
| 12-421-140 | A/G | 21595 |
| 10-523-232 | C/T | 36971 |
| 10-289-201 | C/T | 45214 |
| 10-290-37 | C/T | 45741 |
| 10-290-326 | A/G | 46029 |
| 10-290-328 | G/T | 46032 |
| **Biallelic Markers in MGST-II cDNA (SEQ ID No. 486)** | | |
| 10-286-289 | G/C | 98 |
| 10-286-345 | A/T | 153 |
| 10-286-375 | A/G | 183 |
| 10-289-201 | C/T | 478 |
| 10-290-37 | C/T | 526 |
| **Biallelic Markers in MGST-II cDNA (SEQ ID No. 487)** | | |
| 10-286-289 | G/C | 98 |
| 10-286-345 | A/T | 153 |
| 10-286-375 | A/G | 183 |
| 10-289-201 | C/T | 378 |
| 10-290-37 | C/T | 426 |

Also described herein are polynucleotides that hybridize, under conditions of high or intermediate stringency, to a polynucleotide of a sequence from any sequence in the Sequence Listing as well as sequences, which are complementary thereto. Preferably such polynucleotides are at least 20, 25, 35, 40, 50, 70, 80, 100, 250, 500 or 1000 nucleotides in length, to the extent that a polynucleotide of these lengths is consistent with the lengths of the particular Sequence ID. Preferred polynucleotides comprise a DME-related biallelic marker. Optionally either the original or the alternative allele of the biallelic markers disclosed in Figure 3 may be specified as being present at the DME-related biallelic marker. Conditions of high and intermediate stringency are further described in III.C.4 "Methods of Genotyping DNA Samples for Biallelic Markers-Hybridization assay methods."

Further described are isolated, purified, and recombinant polynucleotides which encode MGST-II polypeptides comprising a contiguous span of at least 6 amino acids, preferably at least 8 to 10 amino acids, more preferably at least 12, 15, 20, 25, 30, 40, 50, or 100 amino acids of SEQ ID No. 488. Further described are isolated, purified, and recombinant polynucleotides which encode the variant MGST-II polypeptides of SEQ ID Nos. 488 and 489. Further described are isolated, purified, and recombinant polynucleotides which encode a variant MGST-II polypeptide comprising a contiguous span of at least 6 amino acids, preferably at least 8 to 10 amino acids, more preferably at least 12, 15, 20, 25, 30, 40, 50, or 100 amino acids of SEQ ID No. 489. Further described are isolated, purified, and recombinant polynucleotides which encode polypeptides comprising a contiguous span of at least 6 amino acids, preferably at least 8 to 10 amino acids, more preferably at least 12, 15, 20, 25, 30, 40, 50, or 100 amino acids of SEQ ID No. 488 wherein said contiguous span comprises a His residue at amino acid position 93. Further described are isolated, purified, and recombinant polynucleotides which encode polypeptides comprising a contiguous span of at least 6 amino acids, preferably at least 8 to 10 amino acids, more preferably at least 12, 15, 20, 25, 30, 40, 50, or 100 amino acids of amino acid positions 1-108 of SEQ ID No. 488.

In one aspect the invention provides an isolated, purified, or recombinant polynucleotide consisting of a contiguous span of 18-35 nucleotides of a sequence selected from the sequences of SEQ ID Nos. 3, 5, 9, 12-15 and 25 and the complements thereof, wherein said span includes a MGST-II-related biallelic marker selected from the MGST-II-related biallelic markers shown in the table in claim 1 in said sequence,

and said biallelic marker is within 4 nucleotides of the centre of said polynucleotide. The polynucleotide may consist of the contiguous span where the contiguous span is 25 nucleotides in length and the biallelic marker is at the centre of the polynucleotide. In another aspect the invention provides an isolated, purified or recombinant polynucleotide consisting of a contiguous span of at least 12 nucleotides of a sequence selected from the group consisting of SEQ ID NOs: 3, 5, 9, 12-15 and 25 and the complements thereof, wherein said span includes a MGST-II-related biallelic marker of the table set forth in claim 1 in said sequence, wherein:

(i) the 3' end of said contiguous span is located at the 3' end of said polynucleotide; and

(ii) said biallelic marker is present at the 3' end of said polynucleotide.

In a further aspect the invention provides an isolated, purified or recombinant polynucleotide consisting of a contiguous span of 8-50 nucleotides of a sequence selected from the group consisting of SEQ ID NOs: 3, 5, 9, 12-15 and 25 and the complements thereof, in said sequence, wherein:

(i) the 3' end of said contiguous span is located at the 3' end of said polynucleotide; and

(ii) the 3' end of said polynucleotide is located 1 nucleotide upstream of a MGST-II-related biallelic marker of the table set forth in claim 1.

In any of these polynucleotides of the invention, the contiguous span may comprise at least 15, 20 or 25 contiguous nucleotides in the said sequence.

The primers described herein may be designed from the disclosed sequences for any method known in the art. A set of primers may be fashioned such that the 3' end of the contiguous span of identity with the sequences of the Sequence Listing is present at the 3' end of the primer. Such a configuration allows the 3' end of the primer to hybridize to a selected nucleic acid sequence and dramatically increases the efficiency of the primer for amplification or sequencing reactions. In a set of primers the contiguous span may be found in one of the sequences described in Figure 5. Allele specific primers may be designed such that a biallelic marker is at the 3' end of the contiguous span and the contiguous span is present at the 3' end of the primer. Such allele specific primers tend to selectively prime an amplification or sequencing reaction so long as they are used with a nucleic acid sample that contains one of the two alleles present at a biallelic marker. The 3' end of primer of the invention may be located within or at least 2, 4, 6, 8, 10, 12, 15, 18, 20, 25, 50, 100, 250, 500, or 1000, to the extent that this distance is consistent with the particular Sequence ID, nucleotides upstream of a DME-related biallelic marker in said sequence or at any other location which is appropriate for their intended use in sequencing, amplification or the location of novel sequences or markers. A list of amplification primers is disclosed in Figure 7. Primers with their 3' ends located 1 nucleotide upstream of a DME-related biallelic marker have a special utility as microsequencing assays. Microsequencing primers are described in Figure 6.

The probes described herein may be designed from the disclosed sequences for any method known in the art, particularly methods which allow for testing if a particular sequence or marker disclosed herein is present. A set of probes may be designed for use in the hybridization assays of the invention in any manner known in the art such that they selectively bind to one allele of a biallelic marker, but not the other under any particular set of assay conditions. Hybridization probes may consists of, consist essentially of, or comprise a contiguous span which ranges in length from 8, 10, 12, 15, 18 or 20 to 25, 35, 40, 50, 60, 70, or 80 nucleotides, or be specified as being 12, 15, 18, 20, 25, 35, 40, or 50 nucleotides in length and including a DME-related biallelic marker of said sequence. Optionally the original allele or alternative allele disclosed in Figure 3 and 4 may be specified as being present at the biallelic marker site. Optionally, said biallelic marker may be within 6, 5, 4, 3, 2, or 1 nucleotides of the center of the hybridization probe or at the center of said probe. A set of hybridization probes is disclosed in Figure 8 or a sequence complementary thereto.

Any of the polynucleotides of the present invention can be labeled, if desired, by incorporating a label detectable by spectroscopic, photochemical, biochemical, immunochemical, or chemical means. For example, useful labels include radioactive substances, fluorescent dyes or biotin. Preferably, polynucleotides are labeled at their 3' and 5' ends. A label can also be used to capture the primer, so as to facilitate the immobilization of either the primer or a primer extension product, such as amplified DNA, on a solid support. A capture label is attached to the primers or probes and can be a specific binding member which forms a binding pair with the solid's phase reagent's specific binding member (e.g. biotin and streptavidin). Therefore depending upon the type of label carried by a polynucleotide or a probe, it may be employed to capture or to detect the target DNA. Further, it will be understood that the polynucleotides, primers or probes provided herein, may, themselves, serve as the capture label. For example, in the case where a solid phase reagent's binding member is a nucleic acid sequence, it may be selected such that it binds a complementary portion of a primer or probe to thereby immobilize the primer or probe to the solid phase. In cases where a polynucleotide probe itself serves as the binding member, those skilled in the art will recognize that the probe will contain a sequence or "tail" that is not complementary to the target. In the case where a polynucleotide primer itself serves as the capture label, at least a portion of the primer will be free to hybridize with a nucleic acid on a solid phase. DNA Labeling techniques are well known to the skilled technician.

Any of the polynucleotides, primers and probes of the present invention can be conveniently immobilized on a solid support. Solid supports are known to those skilled in the art and include the walls of wells of a reaction tray, test tubes, polystyrene beads, magnetic beads, nitrocellulose strips, membranes, microparticles such as latex particles, sheep (or other animal) red blood cells, duracytes® and others. The solid support is not critical and can be selected by one skilled in the art. Thus, latex particles, microparticles, magnetic or non-magnetic beads, membranes, plastic tubes, walls of microtiter wells, glass or silicon chips, sheep (or other suitable animal's) red blood cells and duracytes are all suitable examples. Suitable methods for immobilizing nucleic acids on solid phases include ionic, hydrophobic, covalent interactions and the like. A solid support, as used herein, refers to any material which is insoluble, or can be made insoluble by a subsequent reaction. The solid support can be chosen for its intrinsic ability to attract and immobilize the capture reagent. Alternatively, the solid phase can retain an additional receptor which has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes® and other configurations known to those of ordinary skill in the art. The polynucleotides of the invention can be attached to or immobilized on a solid support individually or in groups of at least 2, 5, 8, 10, 12, 15, 20, or 25 distinct polynucleotides of the inventions to a single solid support. In addition, polynucleotides other than those of the invention may attached to the same solid support as one or more polynucleotides of the invention.

Any polynucleotide provided herein may be attached in overlapping areas or at random locations on the solid support. Alternatively the polynucleotides of the invention may be attached in an ordered array wherein each polynucleotide is attached to a distinct region of the solid support which does not overlap with the attachment site of any other polynucleotide. Preferably, such an ordered array of polynucleotides is designed to be "addressable" where the distinct locations are recorded and can be accessed as part of an assay procedure. Addressable polynucleotide arrays typically comprise a plurality of different oligonucleotide probes that are coupled to a surface of a substrate in different known locations. The knowledge of the precise location of each polynucleotides location makes these "addressable" arrays particularly useful in hybridization assays. Any addressable array technology known in the art can be employed with the polynucleotides of the invention. One particular embodiment of these polynucleotide arrays is known as the Genechips™, and has been generally described in US Patent 5,143,854; PCT publications WO 90/15070 and 92/10092. These arrays may generally be produced using mechanical synthesis methods or light directed synthesis methods, which incorporate a combination of photolithographic methods and solid phase oligonucleotide synthesis (Fodor et al., Science, 251: 767-777, 1991). The immobilization of arrays of oligonucleotides on solid supports has been rendered possible by the development of a technology generally identified as "Very Large Scale Immobilized Polymer Synthesis" (VLSIPS™) in which, typically, probes are immobilized in a high density array on a solid surface of a chip. Examples of VLSIPS™ technologies are provided in US Patents 5,143,854 and 5,412,087 and in PCT Publications WO 90/15070, WO 92/10092 and WO 95/11995, which describe methods for forming oligonucleotide arrays through techniques such as light-directed synthesis techniques. In designing strategies aimed at providing arrays of nucleotides immobilized on solid supports, further presentation strategies were developed to order and display the oligonucleotide arrays on the chips in an attempt to maximize hybridization patterns and sequence information. Examples of such presentation strategies are disclosed in PCT Publications WO 94/12305, WO 94/11530, WO 97/29212 and WO 97/31256.

Oligonucleotide arrays may comprise at least one of the sequences selected from the group consisting of SEQ ID No. 3, 5, 9, 12, 13, 14, 15 and 25; and the sequences complementary thereto or a fragment thereof of at least 8, 10, 12, 15, 18, 20, 25, 35, 40, 50, 70, 80, 100, 250, 500 or 1000 consecutive nucleotides, to the extent that fragments of these lengths is consistent with the lengths of the particular Sequence ID, for determining whether a sample contains one or more alleles of the biallelic markers of the present invention. Oligonucleotide arrays may also comprise at least one of the sequences selected from the group consisting of SEQ ID No. 3, 5, 9, 12, 13, 14, 15 and 25; and the sequences complementary thereto or a fragment thereof of at least 8, 10, 12, 15, 18, 20, 25, 35, 40, 50, 70, 80, 100, 250, 500 or 1000 consecutive nucleotides, to the extent that fragments of these lengths is consistent with the lengths of the particular Sequence ID, for amplifying one or more alleles of the biallelic markers of the invention. In other embodiments, arrays may also comprise at least one of the sequences selected from the group consisting of SEQ ID No. 3, 5, 9, 12, 13, 14, 15, and 25; and the sequences complementary thereto or a fragment thereof of at least 8, 10, 12, 15, 18, 20, 25, 35, 40, 50, 70, 90, 100, 250, 500 or 1000 consecutive nucleotides, to the extent that fragments of these lengths is consistent with the lengths of the particular Sequence ID, for conducting microsequencing analyses to determine whether a sample contains one or more alleles of the biallelic markers of the invention. In still further embodiments, the oligonucleotide array may comprise at least one of the sequences selecting from the group consisting of SEQ ID No. 3, 5, 9, 12, 13, 14, 15 and 25; and the sequences complementary thereto or a fragment thereof of at least 8, 10, 12, 15, 18, 20, 25, 35, 40, 50, 70, 80, 100, 250, 500 or 1000 nucleotides in length, to the extent that fragments of these lengths is consistent with the lengths of the particular Sequence ID, for determining whether a sample contains one or more alleles of the biallelic markers of the present invention.

The present invention also encompasses diagnostic kits comprising one or more polynucleotides of the invention, optionally with a portion or all of the necessary reagents and instructions for genotyping a test subject by determining the identity of a nucleotide at a DME-related biallelic marker. The polynucleotides of a kit may optionally be attached to a solid support, or be part of an array or addressable array of polynucleotides. The kit may provide for the determination of the identity of the nucleotide at a marker position by any method known in the art including, but not limited to, a sequencing assay method, a microsequencing assay method, a hybridization assay method, or an allele specific amplification method. Optionally such a kit may include instructions for scoring the results of the determination with respect to the test subjects' risk of contracting a diseases involving the metabolic conversion of xenobiotics, or likely response to a drug, or chances of suffering from side effects to a drug, including hepatoxicity.

### I.B. Genomic Sequences of the MGST-II Gene and Biallelic Markers

Described herein is the genomic sequence of the MGST-II gene of SEQ ID No. 485. The MGST-II genomic sequences comprise exons and introns. Genomic sequences of the MGST-II gene may include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 485, wherein said contiguous span comprises at least 1, 2, 3, 4, 5 or 10 of the following nucleotide positions of SEQ ID No. 485: 1 to 7667, 7726 to 20264, 20365 to 36918, 36991 to 45180, 45263 to 45741, and 45980 to 49327, and the complements thereof. The nucleic acids defining the MGST-II intronic polynucleotides may be used as oligonucleotide primers or probes in order to detect the presence of a copy of the MGST-II gene in a test sample, or alternatively in order to amplify a target nucleotide sequence within the MGST-II sequences.

Also described are MGST-II intron and exon polynucleotide sequences including biallelic markers. For example, polynucleotides include purified, isolated or recombinant polynucleotides comprising a contiguous span of at least 12, 15, 18, 20, 25, 30, 35, 40, 50, 60, 70, 80, 90, 100, 150, 200, 500, or 1000 nucleotides of a sequence of SEQ ID No. 485; or the complements thereof; wherein said span includes a MGST-II-related biallelic marker. Polynucleotides may comprise at least one biallelic marker selected from the group consisting of biallelic markers 10-286-289, 10-287-116, 10-286-375, 12-425-57, 12-421-135, 12-421-140, 10-523-232, 10-289-201, 10-290-37, 10-290-326 and 10-290-328. Also described are polynucleotides which, may be used as primers and probes in order to amplify fragments carrying biallelic markers or in order to detect biallelic marker alleles.

### Regulatory sequences

The genomic sequence of the MGST-II gene contains regulatory sequences both in the non-coding 5'- flanking region and in the non-coding 3'- flanking region that border the MGST-II transcribed region containing the 5 exons of this gene. 5'-regulatory sequences of the MGST-II gene comprise the polynucleotide sequences located between the nucleotide in position 1 and the nucleotide in position 7466 of the nucleotide sequence of SEQ ID No. 485. 3'-regulatory sequences of the MOST-II gene comprise the polynucleotide sequences located between the nucleotide in position 45980 and the nucleotide in position 49327 of the nucleotide sequence of SEQ ID No. 485. Regulatory polynucleotides include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 485, wherein said contiguous span comprises at least 1, 2, 3, 4, 5 or 10 of the following nucleotide positions of SEQ ID No. 485: 1 to 7466 and 45966 to 49312; and the complements thereof.

The promoter activity of the regulatory regions contained in the MGST-II genomic sequence of polynucleotide sequence of SEQ ID No. 485 can be assessed by any method known in the art. Methods for identifying the polynucleotide fragments of SEQ ID No. 485 involved in the regulation of the expression of the MGST-II gene are well-known to those skilled in the art (see Sambrook et al., Molecular Cloning A Laboratory Manual, Cold Spring Harbor Laboratory, Cold Spring Harbor, NY, 1989). An example of a typical method, that can be used, involves a recombinant vector carrying a reporter gene and genomic sequences from the MGST-II genomic sequence of SEQ ID No. 485. Briefly, the expression of the reporter gene (for example beta galactosidase or chloramphenicol acetyl transferase) is detected when placed under the control of a biologically active polynucleotide fragment. Genomic sequences located upstream of the first exon of the MGST-II gene may be cloned into any suitable promoter reporter vector, such as the pSEAP-Basic, pSEAP-Enhancer, pβgal-Basic, pβgal-Enhancer, or pEGFP-1 Promoter Reporter vectors available from Clontech, or pGL2-basic or pGL3-basic promoterless luciferase reporter gene vector from Promega. Each of these promoter reporter vectors include multiple cloning sites positioned upstream of a reporter gene encoding a readily assayable protein such as secreted alkaline phosphatase, luciferase, beta galactosidase, or green fluorescent protein. The sequences upstream the first MOST-II exon are inserted into the cloning sites upstream of the reporter gene in both orientations and introduced into an appropriate host cell. The level of reporter protein is assayed and compared to the level obtained with a vector lacking an insert in the cloning site. The presence of an elevated expression level in the vector containing the insert with respect to the control vector indicates the presence of a promoter in the insert.

Promoter sequences within the 5' non-coding regions of the MGST-II gene may be further defined by constructing nested 5' and/or 3' deletions using conventional techniques such as Exonuclease III or appropriate restriction endonuclease digestion. The resulting deletion fragments can be inserted into the promoter reporter vector to determine whether the deletion has reduced or obliterated promoter activity, such as described, for example, by Coles et al. (Hum. Mol. Genet., 7:791-800, 1998). In this way, the boundaries of the promoters may be defined. If desired, potential individual regulatory sites within the promoter may be identified using site directed mutagenesis or linker scanning to obliterate potential transcription factor binding sites within the promoter individually or in combination. The effects of these mutations on transcription levels may be determined by inserting the mutations into cloning sites in promoter reporter vectors. This type of assays are well known to those skilled in the art and are further described in WO 97/17359, US 5 374 544, EP 582 796, US 5 698 389, US 5 643 746, US 5 502 176, and US 5 266 488.

The activity and the specificity of the promoter of the MGST-II gene can further be assessed by monitoring the expression level of a detectable polynucleotide operably linked to the MGST-II promoter in different types of cells and tissues. The detectable polynucleotide may be either a polynucleotide that specifically hybridizes with a predefined oligonucleotide probe, or a polynucleotide encoding a detectable protein, including a MGST-II polypeptide or a fragment or a variant thereof. This type of assay is well known to those skilled in the art and is described in US 5 502 176 and US 5 266 488 for example.

Polynucleotides carrying the regulatory elements located both at the 5' end and at the 3' end of the MGST-II coding region may be advantageously used to control the transcriptional and translational activity of an heterologous polynucleotide of interest, said polynucleotide being heterologous as regards to the MGST-II regulatory region.

Thus, described herein is a purified, isolated, and recombinant nucleic acid comprising a polynucleotide which, is selected from the group consisting of, the polynucleotide sequences located between the nucleotide in position 1 and the nucleotide in position 7466 of the nucleotide sequence of SEQ ID No. 485; or a sequence complementary thereto or a biologically active fragment thereof.

By a "biologically active" fragment of SEQ ID No. 485 is intended a polynucleotide comprising or alternatively consisting of a fragment of said polynucleotide which is functional as a regulatory region for expressing a recombinant polypeptide or a recombinant polynucleotide in a recombinant cell host.

As described herein, a nucleic acid or polynucleotide is "functional" as a regulatory region for expressing a recombinant polypeptide or a recombinant polynucleotide if said regulatory polynucleotide contains nucleotide sequences which contain transcriptional and translational regulatory information, and such sequences are "operably linked" to nucleotide sequences which encode the desired polypeptide or the desired polynucleotide.

The regulatory polynucleotides described herein may be advantageously part of a recombinant expression vector that may be used to express a coding sequence in a desired host cell or host organism.

### I.C. MGST-II cDNA Comprising Biallelic Markers and Variant MGST-II cDNA

Provided herein is a MGST-II cDNA of SEQ ID No. 486. The Open Reading Frame encoding the MGST-II protein spans from the nucleotide in position 202 to the nucleotide in position 642 of the polynucleotide sequence of SEQ ID No. 486. The cDNA of SEQ ID No. 486 also includes a 5'-UTR region and a 3'-UTR region.

cDNA polynucleotides described herein include purified, isolated or recoinbinant polynucleotides comprising a contiguous span of at least 12, 15, 18, 20, 25, 30, 35, 40, 50, 60, 70, 80, 90, 100, 150, 200, 500, or 1000 nucleotides of a sequence of SEQ ID No. 486, or the complements thereof, wherein said span includes a MGST-II-related biallelic marker. For example, cDNA fragments may comprise a biallelic marker selected from the group consisting of 10-286-289 (position 98), 10-286-345 (position 153), 10-286-375 (position 183), 10-523-232 (position 426), 10-289-201 (position 478) and 10-290-37 (position 526). Additional described polynucleotides include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides from a sequence of SEQ ID No. 486, wherein said contiguous span comprises a T at position 426, a C at position 478 or a T at position 526 of SEQ ID No. 486; or the complement thereof. Most biallelic polymorphism represent silent nucleotide substitutions but biallelic marker 10-289-201 is associated with an amino acid change in the corresponding MGST-II polypeptide (TYR replaced by ARG in position 93 of the polypeptide). Moreover, one biallelic marker allele of marker 10-290-37 is associated with a stop codon and the corresponding variant cDNA encodes a truncated MGST-II polypeptide including amino acids 1 to 108.

Also described to a variant MGST-II cDNA of SEQ ID No. 487. corresponding to an alternative splicing form which results in the deletion of exon 2. This alternative splicing of MGST-II yields the variant MGST-II polypeptide of SEQ ID No. 489. MGST-II polypeptides are further described below. cDNAs may include isolated, purified or recombinant polynucleotides comprising a contiguous span of at least 12, 15, 18, 20, 25, 30, 35, 40. 50, 60, 70, 80, 90, 100, 150, 200, 500, or 1000 nucleotides from a sequence of SEQ ID No. 487; or the complements thereof. Additional polynucleotides may include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides from a sequence of SEQ ID No. 487, wherein said contiguous span comprises a T at position 325, a C at position 378 or a T at position 426 of SEQ ID No. 487; or the complements thereof. The new exon 1/exon 3 junction sequence of the splice variant MGST-II cDNA, more particularly the nucleotide sequence comprised between the nucleotide in position 106 and the nucleotide in position 374 of the nucleic acid of SEQ ID No. 486 corresponds to the nucleotide sequence of an EST that has been obtained from a human cDNA library. Polynucleotides comprising this EST of a sequence from SEQ ID No. 490 are also described.

The above disclosed polynucleotides that contain the coding sequence of the MGST-II gene and of MGST-II variants may be expressed in a desired host cell or a desired host organism, when this polynucleotide is placed under the control of suitable expression signals. The expression signals may be either the expression signals contained in the regulatory regions in the MGST-II gene or in contrast the signals may be exogenous regulatory nucleic sequences. Such a polynucleotide, when placed under the suitable expression signals, may also be inserted in a vector for its expression and/or amplification.

Another cDNA fragment comprises the 5'-UTR region (regulatory) beginning at position 1 and ending at position 201 of SEQ ID Nos. 486 and 487. Preferably said 5'-UTR region comprises a biallelic marker selected from the group consisting of biallelic markers 10-286-289, 10-286-345 and 10-286-375. For example 5'-UTR polynucleotides may include isolated, purified or recombinant polynucleotides comprising a contiguous span of at least 12, 15, 18, 20, 25, 30, 35, 40, 50, 60, 70, 80, 90, 100, 150, 200, 500, or 1000 nucleotides of SEQ ID No. 486, wherein said contiguous span comprises at least 1, 2, 3, 4, 5 or 10 of nucleotide positions I to 198 of SEQ ID No. 486; and the complements thereof. The 5'-end sequence of the MGST-II cDNA, more particularly the nucleotide sequence comprised between the nucleotide in position I and the nucleotide in position 357 of the nucleic acid of SEQ ID No. 486 corresponds to the nucleotide sequence of a 5'-EST that has been obtained from a human cDNA library. Polynucleotides comprising this 5'-EST of a sequence from SEQ ID No. 490 are also described.

The polynucleotide disclosed above that contains the coding sequence of the MGST-II gene may be expressed in a desired host cell or a desired host organism, when this polynucleotide is placed under the control of suitable expression signals. The expression signals may be either the expression signals contained in the regulatory regions in the MGST-II gene of the invention or may be exogenous regulatory nucleic sequences. Such a polynucleotide, when placed under the suitable expression signals, may also be inserted in a vector for its expression.

Also described herein is an isolated polynucleotide comprising:

a) a nucleic acid comprising a regulatory nucleotide sequence from a sequence of SEQ ID No. 485;
b) a polynucleotide encoding a desired polypeptide or a nucleic acid of interest, operably linked to the nucleic acid defined in (a) above;
c) Optionally, a nucleic acid comprising a 5'- UTR regulatory polynucleotide, preferably a 5'-UTR regulatory polynucleotide sequence of a sequence of SEQ ID No. 486.

The polypeptide encoded by the nucleic acid described above may be of various nature or origin, encompassing proteins of prokaryotic or eukaryotic origin. Among the polypeptides expressed under the control of a MGST-II regulatory region, there may be cited bacterial, fungal or viral antigens. Also encompassed are eukaryotic proteins such as intracellular proteins, for example "house keeping" proteins, membrane-bound proteins, for example receptors, and secreted proteins, for example cytokines. In a specific example, the desired polypeptide may be the MGST-II protein, especially the proteins of the amino acid sequence of SEQ ID Nos. 488 and 489.

The desired nucleic acids encoded by the above described polynucleotide, usually a RNA molecule, may be complementary to a desired coding polynucleotide, for example to the MGST-II coding sequence, and thus useful as an antisense polynucleotide.

Such a polynucleotide may be included in a recombinant expression vector in order to express the desired polypeptide or the desired nucleic acid in host cell or in a host organism.

### I.D. Polynucleotide Constructs, Recombinant Vectors, Host Cells and Transgenic Animals

#### Polynucleotide Constructs

The terms "polynucleotide construct" and "recombinant polynucleotide" are used interchangeably herein to refer to linear or circular, purified or isolated polynucleotides that have been artificially designed and which comprise at least two nucleotide sequences that are not found as contiguous nucleotide sequences in their initial natural environment.

#### DNA constructs for expressing the MGST-II gene in recombinant host cells and in transgenic animals

In order to study the physiological and phenotype consequences of a lack of synthesis of the MGST-II protein, both at the cellular level and at the multicellular organism level, in particular as regards to disorders related to abnormal cell proliferation, notably cancers, also described are DNA constructs and recombinant vectors enabling a conditional expression of a specific allele of the MGST-II genomic sequence or cDNA

A first preferred DNA construct is based on the tetracycline resistance operon *tet* from *E. coli* transposon Tn110 for controlling the MGST-II gene expression, such as described by Gossen et al. (Science, 268:1766-1769,1995). Such a DNA construct contains seven *tet* operator sequences from Tn10 (*tet*op) that are fused to either a minimal promoter or a 5'-regulatory sequence of the MGST-II gene, said minimal promoter or said MGST-II regulatory sequence being operably linked to a polynucleotide of interest that codes either for a sense or an antisense oligonucleotide or for a polypeptide, including a MGST-II polypeptide or a peptide fragment thereof. This DNA construct is functional as a conditional expression system for the nucleotide sequence of interest when the same cell also comprises a nucleotide sequence coding for either the wild type (tTA) or the mutant (rTA) repressor fused to the activating domain of viral protein VP16 of herpes simplex virus, placed under the control of a promoter, such as the HCMVIE1 enhancer/promoter or the MMTV-LTR. Indeed, a preferred DNA construct will comprise both the polynucleotide containing the *tet* operator sequences and the polynucleotide containing a sequence coding for the tTA or the rTA repressor. In the specific example wherein the conditional expression DNA construct contains the sequence encoding the mutant tetracycline repressor rTA, the expression of the polynucleotide of interest is silent in the absence of tetracycline and induced in its presence.

#### DNA constructs allowing homologous recombination: replacement vectors

A second DNA construct will comprise, from 5'-end to 3'-end : (a) a first nucleotide sequence that is comprised in the MGST-II genomic sequence; (b) a nucleotide sequence comprising a positive selection marker, such as the marker for neomycine resistance (*neo*); and (c) a second nucleotide sequence that is comprised in the MGST-II genomic sequence, and is located on the genome downstream the first MGST-II nucleotide sequence (a).

In one example, this DNA construct also comprises a negative selection marker located upstream the nucleotide sequence (a) or downstream the nucleotide sequence (c). Preferably, the negative selection marker consists of the thymidine kinase (*tk*) gene (Thomas et al., Cell, 44:419-428, 1986), the hygromycine beta gene (Te Riele et al., Nature, 348:649-651, 1990), the *hprt* gene (Van der Lugt et al., Gene, 105:263-267, 1991; Reid et al., Proc. Natl. Acad Sci. USA, 87:4299-4303, 1990) or the Diphteria toxin A fragment (*Dt-A*) gene (Nada et al., Cell, 73:1125-1135, 1993; Yagi et al., Proc. Natl; Acad. Sci. USA, 87:9918-9922, 1990). The positive selection marker may be located within a MGST-II exon sequence so as to interrupt the sequence encoding a MGST-II protein. These replacement vectors are further described by Mansour et al. (Nature, 336:348-352, 1988) and Koller et al. (Ann. Rev. Immunol., 10:705-730, 1992). The first and second nucleotide sequences (a) and (c) may be indifferently located within a MGST-II regulatory sequence, an intronic sequence, an exon sequence or a sequence containing both regulatory and/or intronic and/or exon sequences. The size of the nucleotide sequences (a) and (c) is ranging from 1 to 50 kb, preferably from 1 to 10 kb, more preferably from 2 to 6 kb and most preferably from 2 to 4 kb.

#### DNA constructs allowing homologous recombination: Cre-loxP system

These new DNA constructs make use of the site specific recombination system of the P1 phage. The P1 phage possesses a recombinase called Cre which, interacts specifically with a 34 base pairs *lox*P site. The *lox*P site is composed of two palindromic sequences of 13 bp separated by a 8 bp conserved sequence (Hoess et al., Nucleic Acids Res., 14: 2287-2300, 1986). The recombination by the Cre enzyme between two *lox*P sites having an identical orientation leads to the deletion of the DNA fragment.

The *Cre-loxP* system used in combination with a homologous recombination technique has been first described by Gu et al. (Cell, 73:1155-1164, 1993). Briefly, a nucleotide sequence of interest to be inserted in a targeted location of the genome harbors at least two *lox*P sites in the same orientation and located at the respective ends of a nucleotide sequence to be excised from the recombinant genome. The excision event requires the presence of the recombinase (Cre) enzyme within the nucleus of the recombinant cell host. The recombinase enzyme may be brought at the desired time either by (a) incubating the recombinant cell hosts in a culture medium containing this enzyme, by injecting the Cre enzyme directly into the desired cell, such as described by Araki et al. (Proc. Natl; Acad. Sci. USA, 92: 160-164, 1995), or by lipofection of the enzyme into the cells, such as described by Baubonis et al. (Nucleic Acids Res., 21:2025-2029, 1993); (b) transfecting the cell host with a vector comprising the *Cre* coding sequence operably linked to a promoter functional in the recombinant cell host, which promoter being optionally inducible, said vector being introduced in the recombinant cell host, such as described by Gu et al. (Cell, 73:1155-1164, 1993) and Sauer et al. (Proc. Natl; Acad. Sci. USA, 85:5166-5170, 1988); (c) introducing in the genome of the cell host a polynucleotide comprising the *Cre* coding sequence operably linked to a promoter functional in the recombinant cell host, which promoter is optionally inducible, and said polynucleotide being inserted in the genome of the cell host either by a random insertion event or an homologous recombination event, such as described by Gu et al. (Science, 265:103-106, 1994).

Where the vector containing the sequence to be inserted in the MGST-II gene by homologous recombination is constructed in such a way that selectable markers are flanked by *lox*P sites of the same orientation, it is possible, by treatment by the Cre enzyme, to eliminate the selectable markers while leaving the MGST-II sequences of interest that have been inserted by an homologous recombination event. Again, two selectable markers are needed: a positive selection marker to select for the recombination event and a negative selection marker to select for the homologous recombination event. Vectors and methods using the Cre-*lox*P system are further described by Zou et al. (Curr. Biol., 4:1099-1103, 1994).

Thus, a third DNA construct comprises, from 5'-end to 3'-end: (a) a first nucleotide sequence that is comprised in the MGST-II genomic sequence; (b) a nucleotide sequence comprising a polynucleotide encoding a positive selection marker, said nucleotide sequence comprising additionally two sequences defining a site recognized by a recombinase, such as a *lox*P site, the two sites being placed in the same orientation; and (c) a second nucleotide sequence that is comprised in the MGST-II genomic sequence, and is located on the genome downstream of the first MGST-II nucleotide sequence (a).

The sequences defining a site recognized by a recombinase, such as a *lox*P site, are preferably located within the nucleotide sequence (b) at suitable locations bordering the nucleotide sequence for which the conditional excision is sought. In one specific example, two *lox*P sites are located at each side of the positive selection marker sequence, in order to allow its excision at a desired time after the occurrence of the homologous recombination event.

In one example of a method using the third DNA construct described above, the excision of the polynucleotide fragment bordered by the two sites recognized by a recombinase, preferably two *lox*P sites, is performed at a desired time, due to the presence within the genome of the recombinant cell host of a sequence encoding the Cre enzyme operably linked to a promoter sequence, preferably an inducible promoter, more preferably a tissue-specific promoter sequence and most preferably a promoter sequence which is both inducible and tissue-specific, such as described by Gu et al. (Science, 265:103-106, 1994).

The presence of the Cre enzyme within the genome of the recombinant cell host may result of the breeding of two transgenic animals, the first transgenic animal bearing the MGST-II-derived sequence of interest containing the *lox*P sites as described above and the second transgenic animal bearing the *Cre* coding sequence operably linked to a suitable promoter sequence, such as described by Gu et al. (Science, 265:103-106, 1994).

Spatio-temporal control of the Cre enzyme expression may also be achieved with an adenovirus based vector that contains the Cre gene thus allowing infection of cells, or *in vivo* infection of organs, for delivery of the Cre enzyme, such as described by Anton et al. (J. Virol., 69:4600-4606, 1995) and Kanegae et al. (Nucleic Acids Res., 23:3816-3821, 1995).

The DNA constructs described above may be used to introduce a desired nucleotide sequence, preferably a MGST-II genomic sequence or a MGST-II cDNA sequence, and most preferably an altered copy of a MGST-II genomic or cDNA sequence, within a predetermined location of the targeted genome, leading either to the generation of an altered copy of a targeted gene (knock-out homologous recombination) or to the replacement of a copy of the targeted gene by another copy sufficiently homologous to allow an homologous recombination event to occur (knock-in homologous recombination).

## Recombinant Vectors

The term "vector" is used herein to designate either a circular or a linear DNA or RNA molecule, which is either double-stranded or single-stranded, and which comprise at least one polynucleotide of interest that is sought to be transferred in a cell host or in a unicellular or multicellular host organism.

Described herein is a family of recombinant vectors that comprise a regulatory polynucleotide derived from the MGST-II genomic sequence, or a coding polynucleotide from the MGST-11 genomic sequence. Consequently, also described is a recombinant vector comprising either a regulatory polynucleotide comprised in the nucleic acid of SEQ ID Nos. 485 and 486 or a polynucleotide comprising the MGST-II coding sequence or both.

In a first example, a recombinant vector is used to amplify the inserted polynucleotide derived from a MGST-II genomic sequence selected from the group consisting of the nucleic acids of SEQ ID No. 485 or a MGST-II cDNA, for example the cDNA of SEQ ID Nos. 486 and 487 in a suitable host cell, this polynucleotide being amplified each time the recombinant vector replicates. Generally, a recombinant vector of the invention may comprise any of the polynucleotides described herein, including regulatory sequences and coding sequences, as well as any MGST-II primer or probe as defined above.

A second example of the recombinant vectors described herein consists of expression vectors comprising either a regulatory polynucleotide or a coding nucleic acid described herein, or both. Within certain examples, expression vectors are employed to express the MGST-II polypeptide which can be then purified and, for example be used in ligand screening assays or as an immunogen in order to raise specific antibodies directed against the MGST-II protein. In other examples, the expression vectors are used for constructing transgenic animals and also for gene therapy. Expression requires that appropriate signals are provided in the vectors, said signals including various regulatory elements, such as enhancers/promoters from both viral and mammalian sources that drive expression of the genes of interest in host cells. Dominant drug selection markers for establishing permanent, stable cell clones expressing the products are generally included in the expression vectors of the invention, as they are elements that link expression of the drug selection markers to expression of the polypeptide.

More particularly, described herein are expression vectors which include nucleic acids encoding a MGST-II protein, preferably the MGST-II protein of the amino acid sequence of SEQ ID Nos. 488 and 489, under the control of a regulatory sequence selected among theMGST-II regulatory polynucleotides of SEQ ID Nos. 485 and 486, or alternatively under the control of an exogenous regulatory sequence. Consequently, expression vectors may be selected from the group consisting of : (a) the MGST-II regulatory sequence comprised therein drives the expression of a coding polynucleotide operably linked thereto; (b) the MGST-II coding sequence is operably linked to regulation sequences allowing its expression in a suitable cell host and/or host organism. Additionally, the recombinant expression vector described above may also comprise a nucleic acid comprising a 5'-regulatory polynucleotide or a 3'-regulatory polynucleotide, preferably a 5'-regulatory polynucleotide or a 3'-regulatory polynucleotide of the MGST-II gene. The MGST-II 5'-regulatory polynucleotide may also comprise the 5'-UTR sequence contained in the nucleotide sequence of SEQ ID Nos. 486 and 487; or a biologically active fragment or variant thereof. Also described is a recombinant expression vector useful for the expression of the MGST-II coding sequence, wherein said vector comprises any of the MGST-II cDNAs or cDNA variants described above; or fragments thereof.

Some of the elements which, can be found in the present vectors are described in further detail in the following sections.

1. General features of the expression vectors

A recombinant vector may comprise, but is not limited to, a YAC (Yeast Artificial Chromosome), a BAC (Bacterial Artificial Chromosome), a phage, a phagemid, a cosmid, a plasmid or even a linear DNA molecule which may consist of a chromosomal, non-chromosomal, semi-synthetic and synthetic DNA. Such a recombinant vector can comprise a transcriptional unit comprising an assembly of :

(1) a genetic element or elements having a regulatory role in gene expression, for example promoters or enhancers. Enhancers are cis-acting elements of DNA, usually from about 10 to 300 bp in length that act on the promoter to increase the transcription.

(2) a structural or coding sequence which is transcribed into mRNA and eventually translated into a polypeptide, said structural or coding sequence being operably linked to the regulatory elements described in (1); and

(3) appropriate transcription initiation and termination sequences. Structural units intended for use in yeast or eukaryotic expression systems preferably include a leader sequence enabling extracellular secretion of translated protein by a host cell. Alternatively, when a recombinant protein is expressed without a leader or transport sequence, it may include a N-terminal residue. This residue may or may not be subsequently cleaved from the expressed recombinant protein to provide a final product.

Generally, recombinant expression vectors will include origins of replication, selectable markers permitting transformation of the host cell, and a promoter derived from a highly expressed gene to direct transcription of a downstream structural sequence. The heterologous structural sequence is assembled in appropriate phase with translation initiation and termination sequences, and preferably a leader sequence capable of directing secretion of the translated protein into the periplasmic space or the extracellular medium. In a specific embodiment wherein the vector is adapted for transfecting and expressing desired sequences in mammalian host cells, preferred vectors will comprise an origin of replication in the desired host, a suitable promoter and enhancer, and also any necessary ribosome binding sites, polyadenylation site, splice donor and acceptor sites, transcriptional termination sequences, and 5'-flanking non-transcribed sequences. DNA sequences derived from the SV40 viral genome, for example SV40 origin, early promoter, enhancer, splice and polyadenylation sites may be used to provide the required non-transcribed genetic elements.

The *in vivo* expression of a MGST-II polypeptide of SEQ ID Nos. 488 and 489 may be useful in order to correct a genetic defect related to the expression of the native gene in a host organism or to the production of a biologically inactive MGST-II protein.

Consequently, also described are recombinant expression vectors mainly designed for the *in vivo* production of the MGST-II polypeptide of SEQ ID Nos. 488 and 489 or fragments or variants thereof by the introduction of the appropriate genetic material in the organism of the patient to be treated. This genetic material may be introduced *in vitro* in a cell that has been previously extracted from the organism, the modified cell being subsequently reintroduced in the said organism, directly *in vivo* into the appropriate tissue.

<u>2. Regulatory elements</u>

Promoters:

The suitable promoter regions used in the expression vectors are chosen taking into account the cell host in which the heterologous gene has to be expressed. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell, such as, for example, a human or a viral promoter.

A suitable promoter may be heterologous with respect to the nucleic acid for which it controls the expression or alternatively can be endogenous to the native polynucleotide containing the coding sequence to be expressed. Additionally, the promoter is generally heterologous with respect to the recombinant vector sequences within which the construct promoter/coding sequence has been inserted.

Promoter regions can be selected from any desired gene using, for example, CAT (chloramphenicol transferase) vectors and more preferably pKK232-8 and pCM7 vectors.

Preferred bacterial promoters are the LacI, LacZ, the T3 or T7 bacteriophage RNA polymerase promoters, the gpt, lambda PR, PL and trp promoters (EP 0036776), the polyhedrin promoter, or the p10 protein promoter from baculovirus (Kit Novagen) (Smith et al., 1983; O'Reilly et al., 1992), the lambda PR promoter or also the trc promoter.

Eukaryotic promoters include CMV immediate early, HSV thymidine kinase, early and late SV40, LTRs from retrovirus, and mouse metallothionein-L. Selection of a convenient vector and promoter is well within the level of ordinary skill in the art.

The choice of a promoter is well within the ability of a person skilled in the field of genetic egineering. For example, one may refer to the book of Sambrook et al. (Molecular Cloning: A Laboratory Manual, 2nd edition, Cold Spring Harbor Laboratory, Cold Spring Harbor, New York, 1989).

Other regulatory elements:

Where a cDNA insert is employed, one will typically desire to include a polyadenylation signal to effect proper polyadenylation of the gene transcript. The nature of the polyadenylation signal is not believed to be crucial to the successful practice of the invention, and any such sequence may be employed such as human growth hormone and SV40 polyadenylation signals. Also contemplated as an element of the expression cassette is a terminator. These elements can serve to enhance message levels and to minimize read through from the cassette into other sequences.

The vector containing the appropriate DNA sequence as described above, more preferably MGST-II gene regulatory polynucleotide, a polynucleotide encoding the MGST-II polypeptides of SEQ ID Nos. 488 and 489 or both of them, can be utilized to transform an appropriate host to allow the expression of the desired polypeptide or polynucleotide.

<u>3. Selectable markers</u>

Such markers would confer an identifiable change to the cell permitting easy identification of cells containing the expression construct. The selectable marker genes for selection of transformed host cells are preferably dihydrofolate reductase or neomycin resistance for eukaryotic cell culture, TRP1 for *S. cerevisiae* or tetracycline, rifampicin or ampicillin resistance in *E. coli*, or levan saccharase for mycobacteria, this latter marker being a negative selection marker.

4. Preferred vectors

Bacterial vectors:

As a representative but non-limiting example, useful expression vectors for bacterial use can comprise a selectable marker and a bacterial origin of replication derived from commercially available plasmids comprising genetic elements of pBR322 (ATCC 37017). Such commercial vectors include, for example, pKK223-3 (Pharmacia, Uppsala. Sweden), and GEM I (Promega Biotec. Madison, WI. USA). Large numbers of other suitable vectors are known to those of skill in the art, and commercially available, such as the following bacterial vectors : pQE70, pQE60, pQE-9 (Qiagen), pbs, pD10, phagescript, psiX174, pbluescript SK, pbsks, pNH8A, pNH16A, pNH18A, pNH46A (Stratagene); ptrc99a, pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIAexpress). Bacteriophage vectors

The P I bacteriophage vector may contain large inserts ranging from about 80 to about 100 kb. The construction of P1 bacteriophage vectors such as p158 or p158/neo8 have been described by Sternberg (Mamm. Genome, 5:397-404, 1994). Recombinant P1 clones comprising MGST-II nucleotide sequences may be designed for inserting large polynucleotides of more than 40 kb (Linton et al., J. Clin. Invest., 92:3029-3037, 1993). To generate P1 DNA for transgenic experiments, a preferred protocol is the protocol described by McCormick et al. (Genet. Anal. Tech. Appl., 11:158-164, 1994). Briefly, *E. coli* (preferably strain NS3529) harboring the P1 plasmid are grown overnight in a suitable broth medium containing 25 μg/ml of kanamycin. The P1 DNA is prepared from the *E. coli* by alkaline lysis using the Qiagen Plasmid Maxi kit (Qiagen, Chatsworth, CA, USA), according to the manufacturer's instructions. The P1 DNA is purified from the bacterial lysate on two Qiagen-tip 500 columns, using the washing and elution buffers contained in the kit. A phenol/chloroform extraction is then performed before precipitating the DNA with 70% ethanol. After solubilizing the DNA in TE (10 mM Tris-HCl, pH 7.4, 1 mM EDTA), the concentration of the DNA is assessed by spectrophotometry.

When the goal is to express a P1 clone comprising MGST-II nucleotide sequences in a transgenic animal, typically in transgenic mice, it is desirable to remove vector sequences from the P1 DNA fragment, for example by cleaving the P1 DNA at rare-cutting sites within the P1 polylinker (*Sfi*I*, Not*I or *Sal*I). The P 1 insert is then purified from vector sequences on a pulsed-field agarose gel, using methods similar using methods similar to those originally reported for the isolation of DNA from YACs (Schedl et al., 1993a; Peterson et al., 1993). At this stage, the resulting purified insert DNA can be concentrated, if necessary, on a Millipore Ultrafree-MC Filter Unit (Millipore, Bedford, MA, USA - 30,000 molecular weight limit) and then dialyzed against microinjection buffer (10 mM Tris-HCl, pH 7.4; 250 μM EDTA) containing 100 mM NaCl, 30 μM spermine, 70 μM spermidine on a microdyalisis membrane (type VS, 0.025 μM from Millipore). The intactness of the purified P1 DNA insert is assessed by electrophoresis on 1% agarose (Sea Kem GTG; FMC Bioproducts) pulse-field gel and staining with ethidium bromide.

Baculovirus vectors:

A suitable vector for the expression of the MGST-II polypeptides of SEQ ID Nos. 488 and 489 is a baculovirus vector that can be propagated in insect cells and in insect cell lines. A specific suitable host vector system is the pVL1392/1393 baculovirus transfer vector (Pharmingen) that is used to transfect the SF9 cell line (ATCC N°CRL 1711) which is derived from *Spodoptera frugiperda.*

Other suitable vectors for the expression of the MGST-II polypeptides of SEQ ID Nos. 488 and 489 in a baculovirus expression system include those described by Chai et al. (Biotech. Appl. Blochem., 18:259-273, 1993), Vlasak et al. (Eur. J. Biochem., 135: 123-126, 1983) and Lenhard et al. (Gene, 169: 187-190, 1996).

Viral vectors

Retrovirus vectors and adeno-associated virus vectors are generally understood to be the recombinant gene delivery systems of choice for the transfer of exogenous polynucleotides *in vivo,* particularly to mammals, including humans. These vectors provide efficient delivery of genes into cells, and the transferred nucleic acids are stably integrated into the chromosomal DNA of the host.

Particularly preferred retroviruses for the preparation or construction of retroviral in *vitro* or *in vitro* gene delivery vehicles of the present invention include retroviruses selected from the group consisting of Mink-Cell Focus Inducing Virus, Murine Sarcoma Virus, Reticuloendotheliosis virus and Rous Sarcoma virus. Particularly preferred Murine Leukemia Viruses include the 4070A and the 1504A viruses, Abelson (ATCC No VR-999), Friend (ATCC No VR-245), Gross (ATCC No. VR-590), Rauscher (ATCC No VR-998) and Moloney Murine Leukemia Virus (ATCC No VR-190; PCT Application No WO 94/24298). Particularly preferred Rous Sarcoma Viruses include Bryan high titer (ATCC Nos. VR-334, VR-657, VR-726, VR-659 and VR-728). Other preferred retroviral vectors are those described in Roth et al. (Nature Medicine, 2:985-991, 1996), PCT Application No. WO 93/25234 and PCT Application No. WO 94/ 06920.

Yet another viral vector system that is contemplated consists in the adeno-associated virus (AAV). The adeno-associated virus is a naturally occurring defective virus that requires another virus, such as an adenovirus or a herpes virus, as a helper virus for efficient replication and a productive life cycle (Muryczka et al., Current Topics in Microbiol. Immunol., 158:97-129, 1992). It is also one of the few viruses that may integrate its DNA into non-dividing cells, and exhibits a high frequency of stable integration (McLaughlin et al., Am. J. Hum. Genet., 59: 561-569, 1989). One advantageous feature of AAV derives from its reduced efficacy for transducing primary cells relative to transformed cells.

BAC vectors:

The bacterial artificial chromosome (BAC) cloning system (Shizuya et al., 1992) has been developed to stably maintain large fragments of genomic DNA (100-300 kb) in *E. coli.* A preferred BAC vector consists of pBeloBAC11 vector that has been described by Kim et al. (Genomics, 34:213-218,1996). BAC libraries are prepared with this vector using size-selected genomic DNA that has been partially digested using enzymes that permit ligation into either the *Bam* HI or *Hind*III sites in the vector. Flanking these cloning sites are T7 and SP6 RNA polymerase transcription initiation sites that can be used to generate end probes by either RNA transcription or PCR methods. After the construction of a BAC library in *E. coli,* BAC DNA is purified from the host cell as a supercoiled circle. Converting these circular molecules into a linear form precedes both size determination and introduction of the BACs into recipient cells. The cloning site is flanked by two *Not* I sites, permitting cloned segments to be excised from the vector by *Not* I digestion. Alternatively, the DNA insert contained in the pBeloBAC11 vector may be linearized by treatment of the BAC vector with the commercially available enzyme lambda terminase that leads to the cleavage at the unique *cos*N site, but this cleavage method results in a full length BAC clone containing both the insert DNA and the BAC sequences.

5. Delivery of the recombinant vectors

In order to effect expression of the present polynucleotides and polynucleotide constructs, these constructs must be delivered into a cell. This delivery may be accomplished *in vitro,* as in laboratory procedures for transforming cell lines, or *in vivo* or *ex vivo*, as in the treatment of certain diseases states. One mechanism is viral infection where the expression construct is encapsidated in an infectious viral particle. Several non-viral methods for the transfer of polynucleotides into cultured mammalian cells are also contemplated by the present invention, and include, without being limited to, calcium phosphate precipitation (Chen et al., Proc. Natl. Acad Sci. USA, 94:10756-10761, 1987), DEAE-dextran (Gopal, Mol. Cell. Biol., 5:1188-1190, 1985), electroporation (Tur-Kaspa et al., Mol. Cell. Biol., 6:716-7181986), direct microinjection (Harland et al., 1985), DNA. loaded liposomes (Nicolau et al.,1982; Fraley et al., 1979), and receptor-mediate transfection (Wu and Wu, 1987; 1988). Some of these techniques may be successfully adapted for *in vivo* or *ex vivo* use.

Once the expression polynucleotide has been delivered into the cell, it may be stably integrated into the genome of the recipient cell. This integration may be in the cognate location and orientation via homologous recombination (gene replacement) or it may be integrated in a random, non-specific location (gene augmentation). In yet further examples, the nucleic acid may be stably maintained in the cell as a separate, episomal segment of DNA. Such nucleic acid segments or "episomes" encode sequences sufficient to permit maintenance and replication independent of or in synchronization with the host cell cycle.

One specific example of a method for delivering a protein or peptide to the interior of a cell of a vertebrate *in vivo* comprises the step of introducing a preparation comprising a physiologically acceptable carrier and a naked polynucleotide operatively coding for the polypeptide of interest into the interstitial space of a tissue comprising the cell, whereby the naked polynucleotide is taken up into the interior of the cell and has a physiological effect. This is particularly applicable for transfer *in vitro* but it may be applied to *in vivo* as well.

Compositions for use *in vitro* and *in vivo* comprising a "naked" polynucleotide are described in PCT application No. WO 90/11092 (Vical Inc.) and also in PCT application No. WO 95/11307.

In still another example, the transfer of a naked polynucleotide described herein, including a polynucleotide construct as described herein, into cells may be proceeded with a particle bombardment (biolistic), said particles being DNA-coated microprojectiles accelerated to a high velocity allowing them to pierce cell membranes and enter cells without killing them, such as described by Klein et al. (Nature 327:70-73, 1987).

In a further example, the polynucleotide may be entrapped in a liposome (Ghosh and Bacchawat, Targeting of liposomes to hepatocytes. In: Liver Diseases, Targeted diagnosis and therapy using specific rceptors and ligands, Marcel Dekeker, New York, 87-104, 1991; Wong et al., Gene 10:87-94, 1980; Nicolau et al., Biochim. Biophys. Acta. 721:185-190, 1982).

Also described is a composition for the *in vivo* production of the MGST-II protein or polypeptide described herein. It comprises a naked polynucleotide operatively coding for this polypeptide, in solution in a physiologically acceptable carrier, and suitable for introduction into a tissue to cause cells of the tissue to express the said protein or polypeptide.

The amount of vector to be injected to the desired host organism varies according to the site of injection. As an indicative dose, it will be injected between 0.1 and 100 μg of the vector in an animal body, preferably a mammal body, for example a mouse body.

In another example, the vector may be introduced *in vitro* in a host cell, preferably in a host cell previously harvested from the animal to be treated and more preferably a somatic cell such as a muscle cell. In a subsequent step, the cell that has been transformed with the vector coding for the desired MGST-II polypeptide or the desired fragment thereof is reintroduced into the animal body in order to deliver the recombinant protein within the body either locally or systemically.

## Host Cells

Described herein is a host cell that has been transformed or transfected with one of the polynucleotides described therein, and more precisely a polynucleotide either comprising a MGST-II regulatory polynucleotide or the coding sequence of the MGST-II polypeptide having the amino acid sequence of SEQ ID Nos. 488 and 489. This includes host cells that are transformed (prokaryotic cells) or that are transfected (eukaryotic cells) with a recombinant vector such as one of those described above. Generally, a recombinant host cell of the invention comprises any one of the polynucleotides or the recombinant vectors described therein.

A preferred recombinant host cell comprises a polynucleotide selected from the following group of polynucleotides :

a) a purified or isolated nucleic acid encoding a MGST-II polypeptide, or a polypeptide fragment or variant thereof.
b) a purified or isolated nucleic comprising at least 8, preferably at least 15, more preferably at least 25, consecutive nucleotides of the nucleotide sequence SEQ ID No. 485, a nucleotide sequence complementary thereto, or a variant thereof.
c) a purified or isolated nucleic acid comprising at least 8 consecutive nucleotides, preferably at least 15, more preferably at least 25 of the nucleotide sequence SEQ ID Nos. 486 and 487, a nucleotide sequence complementary thereto or a variant thereof.
d) a purified or isolated nucleic acid comprising an exon of the MGST-II gene, a sequence complementary thereto or a fragment or a variant thereof.
e) a purified or isolated nucleic acid comprising a combination of at least two exons of the MGST-II gene, or the sequences complementary thereto wherein the polynucleotides are arranged within the nucleic acid, from the 5' end to the 3'end of said nucleic acid, in the same order than in SEQ ID No. 485.
f) a purified or isolated nucleic acid comprising the nucleotide sequence SEQ ID No. 485 or the sequences complementary thereto or a biologically active fragment thereof.
g) a purified or isolated nucleic acid comprising the nucleotide sequence SEQ ID No. 486, or the sequence complementary thereto or a biologically active fragment thereof.
h) a polynucleotide consisting of:

(1) a nucleic acid comprising a regulatory polynucleotide of SEQ ID No. 485 or the sequences complementary thereto or a biologically active fragment thereof
(2) a polynucleotide encoding a desired polypeptide or nucleic acid.
(3) Optionally, a nucleic acid comprising a regulatory polynucleotide of SEQ ID No. 485, or the sequence complementary thereto or a biologically active fragment thereof.

i) a DNA construct as described previously in the present specification.

Another recombinant cell host is characterized in that its genome or genetic background (including chromosome, plasmids) is modified by the nucleic acid coding for the MGST-II polypeptide of SEQ ID Nos. 488 and 489 or fragments or variants thereof.

Preferred host cells used as recipients for the expression vectors of the invention are the following:

a) Prokaryotic host cells: *Escherichia coli* strains (I.E. DH5-α strain), *Bacillus subtilis, Salmonella typhimurium*, and strains from species like *Pseudomonas, Streptomyces* and *Staphylococcus*..
b) Eukaryotic host cells: HeLa cells (ATCC N°CCL2; N°CCL2.1; N°CCL2.2), Cv 1 cells (ATCC N°CCL70), COS cells (ATCC N°CRL1650; N°CRL1651), Sf-9 cells (ATCC N°CRL1711), C127 cells (ATCC N° CRL-1804), 3T3 (ATCC N° CRL-6361), CHO (ATCC N° CCL-61), human kidney 293 (ATCC N° 45504; N° CRL-1573) and BHK (ECACC N° 84100501; N° 84111301)

c) Other mammalian host cells:

The MGST-II gene expression in mammalian, and typically human, cells may be rendered defective, or alternatively it may be proceeded with the insertion of a MGST-II genomic or cDNA sequence with the replacement of the MGST-II gene counterpart in the genome of an animal cell by a MGST-II polynucleotide described herein. These genetic alterations may be generated by homologous recombination events using specific DNA constructs that have been previously described.

One kind of cell hosts that may be used are mammal zygotes, such as murine zygotes. For example, murine zygotes may undergo microinjection with a purified DNA molecule of interest, for example a purified DNA molecule that has previously been adjusted to a concentration range from 1 ng/ml (for BAC inserts) 3 ng/μl (for P1 bacteriophage inserts) in 10 mM Tris-HCl, pH 7.4, 250 μM EDTA containing 100 mM NaCl, 30 μM spermine, and70 μM spermidine. When the DNA to be microinjected has a large size, polyamines and high salt concentrations can be used in order to avoid mechanical breakage of this DNA, as described by Schedl et al (Nucleic Acids Res. 21:4783-4787, 1993).

Anyone of the polynucleotides described herein including the DNA constructs described herein, may be introduced in an embryonic stem (ES) cell line, preferably a mouse ES cell line. ES cell lines are derived from pluripotent, uncommitted cells of the inner cell mass of pre-implantation blastocysts. Preferred ES cell lines are the following: ES-E14TG2a (ATCC n° CRL-1821), ES-D3 (ATCC n° CRL1934 and n° CRL-11632), YS001 (ATCC n° CRL-11776), 36.5 (ATCC n° CRL-11116). To maintain ES cells in an uncommitted state, they are cultured in the presence of growth inhibited feeder cells which, provide the appropriate signals to preserve this embryonic phenotype and serve as a matrix for ES cell adherence. Preferred feeder cells consist of primary embryonic fibroblasts that are established from tissue of day 13- day 14 embryos of virtually any mouse strain, that are maintained in culture, such as described by Abbondanzo et al. (Methods in Enzymology, Academic Press, NewYork, 803-823, 1993) and are inhibited in growth by irradiation, such as described by Robertson ("Embryo-Derived StemCell Lines," E.J. Robertson Ed., Teratocarcinomas and Embrionic Stem Cells: A Practical Approach. IRL Press, Oxford, 71, 1987), or by the presence of an inhibitory concentration of LIF, such as described by Pease and Williams (Exp. Cell. Res. 190:09-211, 1990).

The constructs in the host cells can be used in a conventional manner to produce the gene product encoded by the recombinant sequence.

Following transformation of a suitable host and growth of the host to an appropriate cell density, the selected promoter is induced by appropriate means, such as temperature shift or chemical induction, and cells are cultivated for an additional period.

Cells are typically harvested by centrifugation, disrupted by physical or chemical means, and the resulting crude extract retained for further purification.

Microbial cells employed in the expression of proteins can be disrupted by any convenient method, including freeze-thaw cycling, sonication, mechanical disruption, or use of cell lysing agents. Such methods are well known by one skilled in the art.

## Transgenic animals

The terms "transgenic animals" or "host animals" are used herein designate animals that have their genome genetically and artificially manipulated so as to include one of the nucleic acids described herein. Preferred animals are non-human mammals and include those belonging to a genus selected from *Mus* (e.g. mice), *Rattus* (e.g. rats) and *Oryctogalus* (e.g. rabbits) which have their genome artificially and genetically altered by the insertion of a nucleic acid as described herein.

The transgenic animals of the invention all include within a plurality of their cells a cloned recombinant or synthetic DNA sequence, more specifically one of the purified or isolated nucleic acids comprising a MGST-II coding sequence, a MGST-II regulatory polynucleotide or a DNA sequence encoding an antisense polynucleotide such as described in the present specification.

Preferred transgenic animals contains in their somatic cells and/or in their germ line cells a polynucleotide selected from the following group of polynucleotides :

a) a purified or isolated nucleic acid encoding a MGST-II polypeptide, or a polypeptide fragment or variant thereof.
b) a purified or isolated nucleic comprising at least 8, preferably at least 15, more preferably at least 25, consecutive nucleotides of the nucleotide sequence SEQ ID No. 485, a nucleotide sequence complementary thereto.
c) a purified or isolated nucleic acid comprising at least 8 consecutive nucleotides, preferably at least 15, more preferably at least 25 of the nucleotide sequence SEQ ID Nos. 486 and 487, a nucleotide sequence complementary thereto.

d) a purified or isolated nucleic acid comprising an exon of the MGST-II gene, a sequence complementary thereto or a fragment or a variant thereof.
e) a purified or isolated nucleic acid comprising a combination of at least two exons of the MGST-II gene, or the sequences complementary thereto wherein the polynucleotides are arranged within the nucleic acid, from the 5' end to the 3'end of said nucleic acid, in the same order than in SEQ ID No. 485.
f) a purified or isolated nucleic acid comprising the nucleotide sequence SEQ ID No. 485 or the sequences complementary thereto or a biologically active fragment thereof.
g) a purified or isolated nucleic acid comprising the nucleotide sequence SEQ ID No. 486, or the sequence complementary thereto or a biologically active fragment thereof.
h) a polynucleotide consisting of :

(1) a nucleic acid comprising a regulatory polynucleotide of SEQ ID No. 485 or the sequences complementary thereto or a biologically active fragment thereof
(2) a polynucleotide encoding a desired polypeptide or nucleic acid.
(3) Optionally, a nucleic acid comprising a regulatory polynucleotide of SEQ ID No. 486, or the sequence complementary thereto or a biologically active fragment thereof.

i) a DNA construct as described previously in the present specification.

The transgenic animals thus contain specific sequences of exogenous genetic material such as the nucleotide sequences described above in detail.
In a first example, these transgenic animals may be good experimental models in order to study the diverse pathologies related to cell differentiation, in particular concerning the transgenic animals within the genome of which has been inserted one or several copies of a polynucleotide encoding a native MGST-II protein, or alternatively a mutant MGST-II protein.
In a second example, these transgenic animals may express a desired polypeptide of interest under the control of the regulatory polynucleotides of the MGST-II gene, leading to good yields in the synthesis of this protein of interest, and eventually a tissue specific expression of this protein of interest.
The design of the transgenic animals may be made according to the conventional techniques well known from the one skilled in the art. For more details regarding the production of transgenic animals, and specifically transgenic mice, it may be referred to US Patents Nos. 4,873,191, issued October10, 1989, 5,464,764 issued November 7,1995 and 5,789,215, issued August 4, 1998, these documents being herein incorporated by reference to disclose methods producing transgenic mice.
Transgenic animals of the present invention are produced by the application of procedures which result in an animal with a genome that has incorporated exogenous genetic material. The procedure involves obtaining the genetic material, or a portion thereof, which encodes either a MGST-II coding sequence, a MGST-II regulatory polynucleotide or a DNA sequence encoding a MGST-II antisense polynucleotide such as described in the present specification.
A recombinant polynucleotide as described herein is inserted into an embryonic or ES stem cell line. The insertion is preferably made using electroporation, such as described by Thomas et al. (Cell 51:503-512, 1997). The cells subjected to electroporation are screened (e.g. by selection via selectable markers, by PCR or by Southern blot analysis) to find positive cells which have integrated the exogenous recombinant polynucleotide into their genome, preferably via an homologous recombination event. An illustrative positive-negative selection procedure that may be used according to the invention is described by Mansour et al. (Nature 336:348-352, 1988).
Then, the positive cells are isolated, cloned and injected into 3.5 days old blastocysts from mice, such as described by Bradley ("Production and Analysis of Chimaeric Mice," E.J. Robertson (Ed.). Teratocarcinomas and embryonic stem cells: A practical approach IRL Press, Oxford, 113, 1987). The blastocysts are then inserted into a female host animal and allowed to grow to term.
Alternatively, the positive ES cells are brought into contact with embryos at the 2.5 days old 8-16 cell stage (morulae) such as described by Wood et al. (Proc. Natl. Acad Sci. U.S.A. 90:4582-4585, 1993) or by Nagy et al. (Proc. Natl. Acad. Sci. USA. 90: 8424-8428, 1993), the ES cells being internalized to colonize extensively the blastocyst including the cells which will give rise to the germ line.
The offspring of the female host are tested to determine which animals are transgenic e.g. include the inserted exogenous DNA sequence and which are wild-type.
Thus, the present invention also concerns a transgenic animal containing a nucleic acid, a recombinant expression vector or a recombinant host cell described hierein.
Also described are recombinant host cells obtained from a transgenic animal described herein.
Recombinant cell lines may be established *in vitro* from cells obtained from any tissue of a transgenic animal according to the invention, for example by transfection of primary cell cultures with vectors expressing one-genes such as SV40 large T antigen, as described by Chou (Mol. Endocrinol. 3:1511-1514, 1989) and Shay et al. (Biochem. Biophys. Acta. 1072:1-7, 1991).

### I.E. MGST-II Polypeptides

The term "MGST-II polypeptides" is used herein to embrace all of the proteins and polypeptides described herein. Also described are polypeptides encoded by the present polynucleotides, as well as fusion polypeptides comprising such polypeptides. Described are MGST-II proteins from humans, including isolated or purified MGST-II proteins consisting, consisting essentially, or comprising the sequence of SEQ ID Nos. 488 and 489. It should be noted the MGST-II proteins are based on the naturally-occurring variants of the amino acid sequence of human MGST-II.

In a first example, described is a variant MGST-II protein; wherein the Tyr residue of amino acid position 93 has been replaced with a His residue. Variant proteins and the fragments thereof which contain amino acid position 93 are collectively referred to herein as "93-His variants." More particularly, described are isolated, purified, and recombinant polypeptides comprising a contiguous span of at least 6 amino acids, preferably at least 8 to 10 amino acids, more preferably at least 12, 15, 20, 25, 30, 40, 50, or 100 amino acids of SEQ ID No. 488, wherein said contiguous span comprises a His residue at amino acid position 93. In this amino acid substitution the original residue (Tyr) is replaced by a non-equivalent amino acid (His) presenting different chemical properties.

Also described is another naturally-occurring variant of the MGST-II protein that consists or consists essentially of amino acids 1-109 of SEQ ID No. 488. This variant MGST-II polypeptide corresponds to one allele of biallelic marker 10-290-37.

Another naturally-occurring variant of the MGST-II protein is encoded by a cDNA obtained by alternative splicing. MGST-II cDNAs and cDNA variants are further described above. This variant polypeptide of a sequence from SEQ ID No. 489 is identical to the MGST-II protein of SEQ ID No. 488 from amino acid position 1 to amino acid position 19 but comprises 11 additional amino acids. Described are isolated, purified, and recombinant polypeptides comprising, consisting of or consisting essentially of an amino acid sequence from SEQ ID No. 489. Moreover, described are isolated, purified, and recombinant polypeptides comprising a contiguous span of at least 6 amino acids, preferably at least 8 to 10 amino acids, more preferably at least 12, 15, 20, 25, or 30 amino acids of SEQ ID No. 489, wherein said contiguous span comprises a least one of amino acid positions 20 to 30 of SEQ ID No. 489.

All the variant MGST-II polypeptides described above most probably show alterations in the activity, specificity and function of the MGST-II enzyme. In some examples the polypeptides comprise the site of a mutation or functional mutation, including a deletion, substitution or truncation in the amino acid sequence in the MGST-II protein.

MGST-II proteins are preferably isolated from human or mammalian tissue samples or expressed from human or mammalian genes. The MGST-II polypeptides can be made using routine expression methods known in the art. The polynucleotide encoding the desired polypeptide, is ligated into an expression vector suitable for any convenient host. Both eukaryotic and prokaryotic host systems are used in forming recombinant polypeptides. The polypeptide is then isolated from lysed cells or from the culture medium and purified to the extent needed for its intended use. Purification is by any technique known in the art, for example, differential extraction, salt fractionation, chromatography, centrifugation, and the like. See, for example, Methods in Enzymology for a variety of methods for purifying proteins.

In addition, shorter protein fragments are produced by chemical synthesis. Alternatively the proteins are extracted from cells or tissues of humans or non-human animals. Methods for purifying proteins are known in the art, and include the use of detergents or chaotropic agents to disrupt particles followed by differential extraction and separation of the polypeptides by ion exchange chromatography, affinity chromatography, sedimentation according to density, and gel electrophoresis.

Any MGST-II cDNA described herein is used to express MGST-II proteins and polypeptides. The nucleic acid encoding the MGST-II protein or polypeptide to be expressed is operably linked to a promoter in an expression vector using conventional cloning technology. The MGST-II insert in the expression vector may comprise the full coding sequence for the MGST-II protein or a portion thereof. For example, the MGST-II derived insert may encode a polypeptide comprising a contiguous span of at least 6 amino acids, preferably at least 8 to 10 amino acids, more preferably at least 12, 15, 20, 25, 30, 40, 50, or 100 amino acids of SEQ ID No. 488, wherein said contiguous span comprises a His residue at amino acid position 93. The MGST-II derived insert may further encode a polypeptide comprising, consisting of or consisting essentially of an amino acid sequence from amino acid positions 1-108 of SEQ ID No. 488. The MGST-II derived insert may further encode a polypeptide comprising, consisting of or consisting essentially of an amino acid sequence from SEQ ID No. 489. The MGST-II derived insert may also encode a polypeptide comprising a contiguous span of at least 6 amino acids, preferably at least 8 to 10 amino acids, more preferably at least 12, 15, 20, 25,or 30 amino acids of SEQ ID No. 489, wherein said contiguous span comprises a least one of amino acid positions 20 to 30 of SEQ ID No. 489.

The expression vector is any of the mammalian, yeast, insect or bacterial expression systems known in the art. Commercially available vectors and expression systems are available from a variety of suppliers including Genetics Institute (Cambridge, MA), Stratagene (La Jolla, Califomia), Promega (Madison, Wisconsin), and Invitrogen (San Diego, California). If desired, to enhance expression and facilitate proper protein folding, the codon context and codon pairing of the sequence is optimized for the particular expression organism in which the expression vector is introduced, as explained by Hatfield, et al., U.S. Patent No. 5,082,767.

In one example, the entire coding sequence of the MGST-II cDNA through the poly A signal of the cDNA is operably linked to a promoter in the expression vector. Alternatively, if the nucleic acid encoding a portion of the MGST-II protein lacks a methionine to serve as the initiation site, an initiating methionine can be introduced next to the first codon of the nucleic acid using conventional techniques. Similarly, if the insert from the MGST-II cDNA lacks a poly A signal, this sequence can be added to the construct by, for example, splicing out the Poly A signal from pSG5 (Stratagene) using BglI and SalI restriction endonuclease enzymes and incorporating it into the mammalian expression vector pXT1 (Stratagene). pXT1 contains the LTRs and a portion of the gag gene from Moloney Murine Leukemia Virus. The position of the LTRs in the construct allow efficient stable transfection. The vector includes the Herpes Simplex Thymidine Kinase promoter and the selectable neomycin gene. The nucleic acid encoding the MGST-II protein or a portion thereof is obtained by PCR from a bacterial vector containing a MGST-II cDNA described herein using oligonucleotide primers complementary to the MGST-II cDNA or portion thereof and containing restriction endonuclease sequences for Pst I incorporated into the 5'primer and BglII at the 5' end of the corresponding cDNA 3' primer, taking care to ensure that the sequence encoding the MGST-II protein or a portion thereof is positioned properly with respect to the poly A signal. The purified fragment obtained from the resulting PCR reaction is digested with PstI, blunt ended with an exonuclease, digested with BglII, purified and ligated to pXT1, now containing a poly A signal and digested with BglII.

The ligated product is transfected into mouse NIH 3T3 cells using Lipofectin (Life Technologies, Inc., Grand Island, New York) under conditions outlined in the product specification. Positive transfectants are selected after growing the transfected cells in 600ug/ml G418 (Sigma, St. Louis, Missouri).

Alternatively, the nucleic acids encoding the MGST-II protein or a portion thereof is cloned into pED6dpc2 (Genetics Institute, Cambridge, MA). The resulting pED6dpc2 constructs is transfected into a suitable host cell, such as COS 1 cells. Methotrexate resistant cells are selected and expanded.

The above procedures may also be used to express a mutant MGST-II protein responsible for a detectable phenotype or a portion thereof.

The expressed proteins are purified using conventional purification techniques such as ammonium sulfate precipitation or chromatographic separation based on size or charge. The protein encoded by the nucleic acid insert may also be purified using standard immunochromatography techniques. In such procedures, a solution containing the expressed MGST-II protein or portion thereof, such as a cell extract, is applied to a column having antibodies against the MGST-II protein or portion thereof is attached to the chromatography matrix. The expressed protein is allowed to bind the immunochromatography column. Thereafter, the column is washed to remove non-specifically bound proteins. The specifically bound expressed protein is then released from the column and recovered using standard techniques.

To confirm expression of the MGST-II protein or a portion thereof, the proteins expressed from host cells containing an expression vector containing an insert encoding the MGST-II protein or a portion thereof can be compared to the proteins expressed in host cells containing the expression vector without an insert. The presence of a band in samples from cells containing the expression vector with an insert which is absent in samples from cells containing the expression vector without an insert indicates that the MGST-II protein or a portion thereof is being expressed. Generally, the band will have the mobility expected for the MGST-II protein or portion thereof. However, the band may have a mobility different than that expected as a result of modifications such as glycosylation, ubiquitination, or enzymatic cleavage.

Antibodies capable of specifically recognizing the expressed MGST-II protein or a portion thereof, are described below.

If antibody production is not possible, the nucleic acids encoding the MGST-II protein or a portion thereof is incorporated into expression vectors designed for use in purification schemes employing chimeric polypeptides. In such strategies the nucleic acid encoding the MGST-II protein or a portion thereof is inserted in frame with the gene encoding the other half of the chimera. The other half of the chimera is β-globin or a nickel binding polypeptide encoding sequence. A chromatography matrix having antibody to β-globin or nickel attached thereto is then used to purify the chimeric protein. Protease cleavage sites is engineered between the β-globin gene or the nickel binding polypeptide and the MGST-II protein or portion thereof. Thus, the two polypeptides of the chimera are separated from one another by protease digestion.

One useful expression vector for generating β-globin chimerics is pSG5 (Stratagene), which encodes rabbit β-globin. Intron II of the rabbit β-globin gene facilitates splicing of the expressed transcript, and the polyadenylation signal incorporated into the construct increases the level of expression. These techniques are well known to those skilled in the art of molecular biology. Standard methods are published in methods texts such as Davis et al., (Basic Methods in Molecular Biology, L.G. Davis, M.D. Dibner, and J.F. Battey, ed., Elsevier Press, NY, 1986) and many of the methods are available from Stratagene, Life Technologies, Inc., or Promega. Polypeptide may additionally be produced from the construct using in vitro translation systems such as the In vitro Express™ Translation Kit (Stratagene).

### I.F. Production of Antibodies Against MGST-II Polypeptides

Any MGST-II polypeptide or whole protein may be used to generate antibodies capable of specifically binding to expressed MGST-II protein or fragments thereof or variants thereof. Preferably the antibody compositions are capable of specifically binding to the 93-His variant of the MGST-II protein. Alternatively the antibody compositions are capable of specifically binding the variant MGST-II polypeptide of SEQ ID No. 489. Described herein are isolated or purified antibody compositions capable of selectively binding, or which are capable of binding to an epitope-containing fragment of a polypeptide of the invention, wherein said epitope comprises at least one amino acid position selected from the group consisting of His residue at amino acid position 93 of SEQ ID No. 488 and of amino acid positions 20-30 of SEQ ID No. 489. For an antibody composition to specifically bind to these MGST-II variants it must demonstrate at least a 5%, 10%, 15%, 20%, 25%, 50%, or 100% greater binding affinity for full length MGST-II variants in an ELISA, RIA, or other antibody-based binding assay than to full length MGST-II protein described in SEQ ID No. 488. Affinity of the antibody composition for the epitope can further be determined by preparing competitive binding curves, as described, for example, by Fisher, D. (Chap. 42 in: Manual of Clinical Immunology, 2d Ed. (Rose and Friedman,Eds.) Amer. Soc. For Microbiol., Washington, D.C., 1980).

Also contemplated is the use of variant MGST-II polypeptides in the manufacture of antibodies. In one example such polypeptides are useful in the manufacture of antibodies to detect the presence and absence of the 93-His variant and of the MGST-II variant of SEQ ID No. 489.

Non-human animals or mammals, whether wild-type or transgenic, which express a different species of MGST-II than the one to which antibody binding is desired, and animals which do not express MGST-II (i.e. an MGST-II knock out animal as described in herein) are particularly useful for preparing antibodies. MGST-II knock out animals will recognize all or most of the exposed regions of MGST-II as foreign antigens, and therefore produce antibodies with a wider array of MGST-II epitopes. Moreover, smaller polypeptides with only 10 to 30 amino acids may be useful in obtaining specific binding to the 93-His variant and to the MGST-II variant of SEQ ID No. 489. In addition, the humoral immune system of animals which produce a species of MGST-II that resembles the antigenic sequence will preferentially recognize the differences between the animal's native MGST-II species and the antigen sequence, and produce antibodies to these unique sites in the antigen sequence. Such a technique will be particularly useful in obtaining antibodies that specifically bind to the 93-His variant and to the MGST-II variant of SEQ ID No. 489. The preparation of antibody compositions is further described in Example 6.

Antibody preparations prepared as described herein are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body. The antibodies of the invention may be labeled, either by a radioactive, a fluorescent or an enzymatic label. Consequently, also described is a method for detecting specifically the presence of a variant MGST-II polypeptide in a biological sample, said method comprising the following steps : a) bringing into contact the biological sample with a polyclonal or monoclonal antibody that specifically binds a variant MGST-II polypeptide or to a peptide fragment or variant thereof; and b) detecting the antigen-antibody complex formed. Also described is a diagnostic kit for detecting *in vitro* the presence of a variant MGST-II polypeptide in a biological sample, wherein said kit comprises: a) polyclonal or monoclonal antibody that specifically binds a variant MGST-II polypeptide or to a peptide fragment or variant thereof, optionally labeled; b) a reagent allowing the detection of the antigen-antibody complexes formed, said reagent carrying optionally a label, or being able to be recognized itself by a labeled reagent, more particularly in the case when the above-mentioned monoclonal or polyclonal antibody is not labeled by itself.

## II. Methods for *De Novo* Identification of Biallelic Markers

Large fragments of human DNA, carrying genes of interest involved in the biotransformation ofxenobiotics such as therapeutic drugs; were cloned, sequenced and screened for biallelic markers. Biallelic markers within the candidate genes themselves as well as markers located on the same genomic fragment were identified. It will be clear to one of skill in the art that large fragments of human genomic DNA may be obtained from any appropriate source and may be cloned into a number of suitable vectors.

In one example, BAC (Bacterial Artificial Chromosomes) vectors were used to construct DNA libraries covering the entire human genome. Specific amplification primers were designed for each candidate gene and the BAC library was screened by PCR until there was at least one positive BAC clone per candidate gene. Genomic sequence, screened for biallelic markers, was generated by sequencing ends of BAC subclones. Details of a preferred embodiment are provided in Example 1. As a preferred alternative to sequencing the ends of an adequate number of BAC subclones, high throughput deletion-based sequencing vectors, which allow the generation of a high quality sequence information covering fragments of about 6kb, may be used. Having sequence fragments longer than 2.5 or 3kb enhances the chances of identifying biallelic markers therein. Methods of constructing and sequencing a nested set of deletions are disclosed in the related U.S. Patent Application entitled "High Throughput DNA Sequencing Vector" (Serial No. 09/058,746).

In another example, genomic sequences of candidate genes were available in public databases allowing direct screening for biallelic markers.

Any of a variety of methods can be used to screen a genomic fragment for single nucleotide polymorphisms such as differential hybridization with oligonucleotide probes, detection of changes in the mobility measured by gel electrophoresis or direct sequencing of the amplified nucleic acid. A preferred method for identifying biallelic markers involves comparative sequencing of genomic DNA fragments from an appropriate number of unrelated individuals.

In a first example, DNA samples from unrelated individuals are pooled together, following which the genomic DNA of interest is amplified and sequenced. The nucleotide sequences thus obtained are then analyzed to identify significant polymorphisms. One of the major advantages of this method resides in the fact that the pooling of the DNA samples substantially reduces the number of DNA amplification reactions and sequencing reactions, which must be carried out. Moreover, this method is sufficiently sensitive so that a biallelic marker obtained thereby usually demonstrates a sufficient frequency of its less common allele to be useful in conducting association studies. Usually, the frequency of the least common allele of a biallelic marker identified by this method is at least 10%.

In a second example, the DNA samples are not pooled and are therefore amplified and sequenced individually. This method is usually preferred when biallelic markers need to be identified in order to perform association studies within candidate genes. Preferably, highly relevant gene regions such as promoter regions or exon regions may be screened for biallelic markers. A biallelic marker obtained using this method may show a lower degree of informativeness for conducting association studies, e.g. if the frequency of its less frequent allele may be less than about 10%. Such a biallelic marker will however be sufficiently informative to conduct association studies and it will further be appreciated that including less informative biallelic markers in the genetic analysis studies of the present invention, may allow in some cases the direct identification of causal mutations, which may, depending on their penetrance, be rare mutations.

The following is a description of the various parameters of a preferred method used by the inventors for the identification of the biallelic markers of the present invention.

### II.A. Genomic DNA Samples

The genomic DNA samples from which the biallelic markers of the present invention are generated are preferably obtained from unrelated individuals corresponding to a heterogeneous population of known ethnic background. The number of individuals from whom DNA samples are obtained can vary substantially, preferably from about 10 to about 1000, more preferably from about 50 to about 200 individuals. Usually, DNA samples are collected from at least about 100 individuals in order to have sufficient polymorphic diversity in a given population to identify as many markers as possible and to generate statistically significant results.

As for the source of the genomic DNA to be subjected to analysis, any test sample can be foreseen without any particular limitation. These test samples include biological samples, which can be tested by the methods of the present invention described herein, and include human and animal body fluids such as whole blood, serum, plasma, cerebrospinal fluid, urine, lymph fluids, and various external secretions of the respiratory, intestinal and genitourinary tracts, tears, saliva, milk, white blood cells, myelomas and the like; biological fluids such as cell culture supernatants; fixed tissue specimens including tumor and non-tumor tissue and lymph node tissues; bone marrow aspirates and fixed cell specimens. The preferred source of genomic DNA used in the present invention is from peripheral venous blood of each donor. Techniques to prepare genomic DNA from biological samples are well known to the skilled technician. Details of a preferred embodiment are provided in Example 1. The person skilled in the art can choose to amplify pooled or unpooled DNA samples.

### II.B. DNA Amplification

The identification of biallelic markers in a sample of genomic DNA may be facilitated through the use of DNA amplification methods. DNA samples can be pooled or unpooled for the amplification step. DNA amplification techniques are well known to those skilled in the art. Various methods to amplify DNA fragments carrying biallelic markers are further described hereinafter in III.B. The PCR technology is the preferred amplification technique used to identify new biallelic markers.

Biallelic markers may be identified using genomic sequence information generated by the inventors. Genomic DNA fragments, such as the inserts of the BAC clones described above, are sequenced and used to design primers for the amplification of 500 bp fragments. These 500 bp fragments are amplified from genomic DNA and are scanned for biallelic markers. Primers may be designed using the OSP software (Hillier L. and Green P., 1991). All primers may contain, upstream of the specific target bases, a common oligonucleotide tail that serves as a sequencing primer. Those skilled in the art are familiar with primer extensions, which can be used for these purposes.

In another example, genomic sequences of candidate genes available in public databases allowing direct screening for biallelic markers. Preferred primers, useful for the amplification of genomic sequences encoding the candidate genes, focus on promoters, exons and splice sites of the genes. A biallelic marker present in these functional regions of the gene have a higher probability to be a causal mutation.
Preferred primers include those disclosed in Figure 7.

### II.C. Sequencing of Amplified Genomic DNA and Identification of Single Nucleotide Polymorphisms

The amplification products generated as described above, are then sequenced using any method known and available to the skilled technician. Methods for sequencing DNA using either the dideoxy-mediated method (Sanger method) or the Maxam-Gilbert method are widely known to those of ordinary skill in the art. Such methods are for example disclosed in Maniatis et al. (Molecular Cloning, A Laboratory Manual, Cold Spring Harbor press, Second Edition, 1989). Alternative approaches include hybridization to high-density DNA probe arrays as described in Chee et al. (Science 274, 610, 1996).
Preferably, the amplified DNA is subjected to automated dideoxy terminator sequencing reactions using a dye-primer cycle sequencing protocol. The products of the sequencing reactions are run on sequencing gels and the sequences are determined using gel - image analysis. The polymorphism search is based on the presence of superimposed peaks in the electrophoresis pattern resulting from different bases occurring at the same position. Because each dideoxy terminator is labeled with a different fluorescent molecule, the two peaks corresponding to a biallelic site present distinct colors corresponding to two different nucleotides at the same position on the sequence. However, the presence of two peaks can be an artifact due to background noise. To exclude such an artifact, the two DNA strands are sequenced and a comparison between the peaks is carried out. In order to be registered as a polymorphic sequence, the polymorphism has to be detected on both strands.
The above procedure permits those amplification products, which contain biallelic markers to be identified. The detection limit for the frequency of biallelic polymorphisms detected by sequencing pools of 100 individuals is approximately 0.1 for the minor allele, as verified by sequencing pools of known allelic frequencies. However, more than 90% of the biallelic polymorphisms detected by the pooling method have a frequency for the minor allele higher than 0.25. Therefore, the biallelic markers selected by this method have a frequency of at least 0.1 for the minor allele and less than 0.9 for the major allele. Preferably at least 0.2 for the minor allele and less than 0.8 for the major allele, more preferably at least 0.3 for the minor allele and less than 0.7 for the major allele, thus a heterozygosity rate higher than 0.18, preferably higher than 0.32, more preferably higher than 0.42.
In another embodiment, biallelic markers are detected by sequencing individual DNA samples, the frequency of the minor allele of such a biallelic marker may be less than 0.1.
The markers carried by the same fragment of genomic DNA, such as the insert in a BAC clone, need not necessarily be ordered with respect to one another within the genomic fragment to conduct association studies. However, the order of biallelic markers carried by the same fragment of genomic DNA may be determined.

### II.D. Validation of the Biallelic Markers

The polymorphisms are evaluated for their usefulness as genetic markers by validating that both alleles are present in a population. Validation of the biallelic markers is accomplished by genotyping a group of individuals by a method of the invention and demonstrating that both alleles are present. Microsequencing is a preferred method of genotyping alleles. The validation by genotyping step may be performed on individual samples derived from each individual in the group or by genotyping a pooled sample derived from more than one individual. The group can be as small as one individual if that individual is heterozygous for the allele in question. Preferably the group contains at least three individuals, more preferably the group contains five or six individuals, so that a single validation test will be more likely to result in the validation of more of the biallelic markers that are being tested. It should be noted, however, that when the validation test is performed on a small group it may result in a false negative result if as a result of sampling error none of the individuals tested carries one of the two alleles. Thus, the validation process is less useful in demonstrating that a particular initial result is an artifact, than it is at demonstrating that there is a *bona fide* biallelic marker at a particular position in a sequence. For an indication of whether a particular biallelic marker has been validated see Figure 1. All of the genotyping, haplotyping, association, and interaction study methods of the invention may optionally be performed solely with validated biallelic markers.

### II.E. Evaluation of the Frequency of the Biallelic Markers

The validated biallelic markers are further evaluated for their usefulness as genetic markers by determining the frequency of the least common allele at the biallelic marker site. The determination of the least common allele is accomplished by genotyping a group of individuals by a method of the invention and demonstrating that both alleles are present. This determination of frequency by genotyping step may be performed on individual samples derived from each individual in the group or by genotyping a pooled sample derived from more than one individual. The group must be large enough to be representative of the population as a whole. Preferably the group contains at least 20 individuals, more preferably the group contains at least 50 individuals, most preferably the group contains at least 100 individuals. Of course the larger the group the greater the accuracy of the frequency determination because of reduced sampling error. For an indication of the frequency for the less common allele of a particular biallelic marker of the invention see Figure 1. A biallelic marker wherein the frequency of the less common allele is 30% or more is termed a "high quality biallelic marker." All of the genotyping, haplotyping, association, and interaction study methods of the invention may optionally be performed solely with high quality biallelic markers.

## III. Methods of Genotyping an Individual for Biallelic Markers

Methods are provided to genotype a biological sample for one or more biallelic markers of the present invention, all of which may be performed *in vitro.* Such methods of genotyping comprise determining the identity of a nucleotide at a DME-related biallelic marker by any method known in the art. These methods find use in genotyping case-control populations in association studies as well as individuals in the context of detection of alleles of biallelic markers which, are known to be associated with a given trait, in which case both copies of the biallelic marker present in individual's genome are determined so that an individual may be classified as homozygous or heterozygous for a particular allele.

These genotyping methods can be performed nucleic acid samples derived from a single individual or pooled DNA samples.

Genotyping can be performed using similar methods as those described above for the identification of the biallelic markers, or using other genotyping methods such as those further described below. In some examples, the comparison of sequences of amplified genomic fragments from different individuals is used to identify new biallelic markers whereas microsequencing is used for genotyping known biallelic markers in diagnostic and association study applications.

### III.A. Source of DNA for Genotyping

Any source of nucleic acids, in purified or non-purified form, can be utilized as the starting nucleic acid, provided it contains or is suspected of containing the specific nucleic acid sequence desired. DNA or RNA may be extracted from cells, tissues, body fluids and the like as described above in II.A. "Genomic DNA Samples." While nucleic acids for use in the genotyping methods of the invention can be derived from any mammalian source, the test subjects and individuals from which nucleic acid samples are taken are generally understood to be human.

### III.B. Amplification of DNA Fragments Comprising Biallelic Markers

Methods and polynucleotides are provided to amplify a segment of nucleotides comprising one or more biallelic marker of the present invention. It will be appreciated that amplification of DNA fragments comprising biallelic markers may be used in various methods and for various purposes and is not restricted to genotyping. Nevertheless, many genotyping methods, although not all, require the previous amplification of the DNA region carrying the biallelic marker of interest. Such methods specifically increase the concentration or total number of sequences that span the biallelic marker or include that site and sequences located either distal or proximal to it. Diagnostic assays may also rely on amplification of DNA segments carrying a biallelic marker of the present invention.

Amplification of DNA may be achieved by any method known in the art. The established PCR (polymerase chain reaction) method or by developments thereof or alternatives. Amplification methods which can be utilized herein include but are not limited to Ligase Chain Reaction (LCR) as described in EP A 320 308 and EP A 439 182, Gap LCR (Wolcott, M.J., Clin. Mcrobiol. Rev. 5:370-386), the so-called "NASBA" or "3SR" technique described in Guatelli J.C. et al. (Proc. Natl. Acad Sci. USA 87:1874-1878, 1990) and in Compton J. (Nature 350:91-92, 1991), Q-beta amplification as described in European Patent Application no 4544610, strand displacement amplification as described in Walker et al. (Clin. Chem. 42:9-13, 1996) and EP A 684 315 and, target mediated amplification as described in PCT Publication WO 9322461.

LCR and Gap LCR are exponential amplification techniques, both depend on DNA ligase to join adjacent primers annealed to a DNA molecule. In Ligase Chain Reaction (LCR), probe pairs are used which include two primary (first and second) and two secondary (third and fourth) probes, all of which are employed in molar excess to target. The first probe hybridizes to a first segment of the target strand and the second probe hybridizes to a second segment of the target strand, the first and second segments being contiguous so that the primary probes abut one another in 5' phosphate-3'hydroxyl relationship, and so that a ligase can covalently fuse or ligate the two probes into a fused product.

In addition, a third (secondary) probe can hybridize to a portion of the first probe and a fourth (secondary) probe can hybridize to a portion of the second probe in a similar abutting fashion. Of course, if the target is initially double stranded, the secondary probes also will hybridize to the target complement in the first instance. Once the ligated strand of primary probes is separated from the target strand, it will hybridize with the third and fourth probes which can be ligated to form a complementary, secondary ligated product. It is important to realize that the ligated products are functionally equivalent to either the target or its complement. By repeated cycles of hybridization and ligation, amplification of the target sequence is achieved. A method for multiplex LCR has also been described (WO 9320227). Gap LCR (GLCR) is a version of LCR where the probes are not adjacent but are separated by 2 to 3 bases.

For amplification of mRNAs, it is within the scope of the present invention to reverse transcribe mRNA into cDNA followed by polymerase chain reaction (RT-PCR); or, to use a single enzyme for both steps as described in U.S. Patent No. 5,322,770 or, to use Asymmetric Gap LCR (RT-AGLCR) as described by Marshall R.L. et al. (PCR Methods and Applications 4:80-84, 1994). AGLCR is a modification of GLCR that allows the amplification of RNA.

Some of these amplification methods are particularly suited for the detection of single nucleotide polymorphisms and allow the simultaneous amplification of a target sequence and the identification of the polymorphic nucleotide as it is further described in IIIC.

The PCR technology is the preferred amplification technique used in the present invention. A variety of PCR techniques are familiar to those skilled in the art. For a review of PCR technology, see Molecular Cloning to Genetic Engineering White. B.A. Ed. in Methods in Molecular Biology 67: Humana Press, Totowa (1997) and the publication entitled "PCR Methods and Applications" (1991, Cold Spring Harbor Laboratory Press). In each of these PCR procedures, PCR primers on either side of the nucleic acid sequences to be amplified are added to a suitably prepared nucleic acid sample along with dNTPs and a thermostable polymerase such as Taq polymerase, Pfu polymerase, or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites. PCR has further been described in several patents including US Patents 4,683,195, 4,683,202 and 4,965,188.

The identification of biallelic markers as described above allows the design of appropriate oligonucleotides, which can be used as primers to amplify DNA fragments comprising the biallelic markers of the present invention. Amplification can be performed using the primers initially used to discover new biallelic markers which are described herein or any set of primers allowing the amplification of a DNA fragment comprising a biallelic marker of the present invention. Primers can be prepared by any suitable method. As for example, direct chemical synthesis by a method such as the phosphodiester method of Narang S.A. et al. (Methods Enzymol. 68:90-98, 1979), the phosphodiester method of Brown E.L. et al. (Methods Enzymol. 68:109-151, 1979), the diethylphosphoramidite method of Beaucage et al. (Tetrahedron Lett. 22:1859-1862, 1981) and the solid support method described in EP 0 707 592.

In some embodiments the present invention provides primers for amplifying a DNA fragment containing one or more biallelic markers of the present invention. Preferred amplification primers are listed in Figure 7. It will be appreciated that the primers listed are merely exemplary and that any other set of primers which produce amplification products containing one or more biallelic markers of the present invention.

The primers are selected to be substantially complementary to the different strands of each specific sequence to be amplified. The length of the primers of the present invention can range from 8 to 100 nucleotides, preferably from 8 to 50, 8 to 30 or more preferably 8 to 25 nucleotides. Shorter primers tend to lack specificity for a target nucleic acid sequence and generally require cooler temperatures to form sufficiently stable hybrid complexes with the template. Longer primers are expensive to produce and can sometimes self-hybridize to form hairpin structures. The formation of stable hybrids depends on the melting temperature (Tm) of the DNA. The Tm depends on the length of the primer, the ionic strength of the solution and the G+C content. The higher the G+C content of the primer, the higher is the melting temperature because G:C pairs are held by three H bonds whereas A:T pairs have only two. The G+C content of the amplification primers of the present invention preferably ranges between 10 and 75 %, more preferably between 35 and 60 %, and most preferably between 40 and 55 %. The appropriate length for primers under a particular set of assay conditions may be empirically determined by one of skill in the art.

The spacing of the primers determines the length of the segment to be amplified. In the context of the present invention amplified segments carrying biallelic markers can range in size from at least about 25 bp to 35 kbp. Amplification fragments from 25-3000 bp are typical, fragments from 50-1000 bp are preferred and fragments from 100-600 bp are highly preferred. It will be appreciated that amplification primers for the biallelic markers may be any sequence which allow the specific amplification of any DNA fragment carrying the markers. Amplification primers may be labeled or immobilized on a solid support as described in I.

### III.C. Methods of Genotyping DNA samples for Biallelic Markers

Any method known in the art can be used to identify the nucleotide present at a biallelic marker site. Since the biallelic marker allele to be detected has been identified and specified in the present invention, detection will prove simple for one of ordinary skill in the art by employing any of a number of techniques. Many genotyping methods require the previous amplification of the DNA region carrying the biallelic marker of interest. While the amplification of target or signal is often preferred at present, ultrasensitive detection methods which do not require amplification are also encompassed by the present genotyping methods. Methods well-known to those skilled in the art that can be used to detect biallelic polymorphisms include methods such as, conventional dot blot analyzes, single strand conformational polymorphism analysis (SSCP) described by Orita et al. (Proc. Natl. Acad. Sci. U.S.A 86:27776-2770, 1989), denaturing gradient gel electrophoresis (DGGE), heteroduplex analysis, mismatch cleavage detection, and other conventional techniques as described in Sheffield, V.C. et al. (Proc. Natl. Acad Sci. USA 49:699-706, 1991), White et al. (Genomics 12:301-306. 1992), Grompe, M. et al. (Proc. Natl. Acad Sci. USA 86:5855-5892, 1989) and Grompe, M. (Nature Genetics 5:111-117, 1993). Another method for determining the identity of the nucleotide present at a particular polymorphic site employs a specialized exonuclease-resistant nucleotide derivative as described in US patent 4,656,127.

Preferred methods involve directly determining the identity of the nucleotide present at a biallelic marker site by sequencing assay, enzyme-based mismatch detection assay, or hybridization assay. The following is a description of some preferred methods. A highly preferred method is the microsequencing technique. The term "sequencing assay" is used herein to refer to polymerase extension of duplex primer/template complexes and includes both traditional sequencing and microsequencing.

#### 1) Sequencing assays

The nucleotide present at a polymorphic site can be determined by sequencing methods. In a preferred embodiment, DNA samples are subjected to PCR amplification before sequencing as described above. DNA sequencing methods are described in IIC.

Preferably, the amplified DNA is subjected to automated dideoxy terminator sequencing reactions using a dye-primer cycle sequencing protocol. Sequence analysis allows the identification of the base present at the biallelic marker site.

#### 2) Microsequencing assays

In microsequencing methods, a nucleotide at the polymorphic site that is unique to one of the alleles in a target DNA is detected by a single nucleotide primer extension reaction. This method involves appropriate microsequencing primers which, hybridize just upstream of a polymorphic base of interest in the target nucleic acid. A polymerase is used to specifically extend the 3' end of the primer with one single ddNTP (chain terminator) complementary to the selected nucleotide at the polymorphic site. Next the identity of the incorporated nucleotide is determined in any suitable way.

Typically, microsequencing reactions are carried out using fluorescent ddNTPs and the extended microsequencing primers are analyzed by electrophoresis on ABI 377 sequencing machines to determine the identity of the incorporated nucleotide as described in EP 412 883. Alternatively capillary electrophoresis can be used in order to process a higher number of assays simultaneously. An example of a typical microsequencing procedure that can be used in the context of the present invention is provided in Example 2.

Different approaches can be used to detect the nucleotide added to the microsequencing primer. A homogeneous phase detection method based on fluorescence resonance energy transfer has been described by Chen and Kwok (Nucleic Acids Research 25:347-353 1997) and Chen et al. (Proc. Natl. Acad. Sci. USA 94/20 10756-10761,1997). In this method amplified genomic DNA fragments containing polymorphic sites are incubated with a 5'-fluorescein-labeled primer in the presence of allelic dye-labeled dideoxyribonucleoside triphosphates and a modified Taq polymerase. The dye-labeled primer is extended one base by the dye-terminator specific for the allele present on the template. At the end of the genotyping reaction, the fluorescence intensities of the two dyes in the reaction mixture are analyzed directly without separation or purification. All these steps can be performed in the same tube and the fluorescence changes can be monitored in real time. Alternatively, the extended primer may be analyzed by MALDI-TOF Mass Spectrometry. The base at the polymorphic site is identified by the mass added onto the microsequencing primer (see Haff L.A. and Smirnov I.P., Genome Research, 7:378-388, 1997).

Microsequencing may be achieved by the established microsequencing method or by developments or derivatives thereof. Alternative methods include several solid-phase microsequencing techniques. The basic microsequencing protocol is the same as described previously, except that the method is conducted as a heterogenous phase assay, in which the primer or the target molecule is immobilized or captured onto a solid support. To simplify the primer separation and the terminal nucleotide addition analysis, oligonucleotides are attached to solid supports or are modified in such ways that permit affinity separation as well as polymerase extension. The 5' ends and internal nucleotides of synthetic oligonucleotides can be modified in a number of different ways to permit different affinity separation approaches, e.g., biotinylation. If a single affinity group is used on the oligonucleotides, the oligonucleotides can be separated from the incorporated terminator regent. This eliminates the need of physical or size separation. More than one oligonucleotide can be separated from the terminator reagent and analyzed simultaneously if more than one affinity group is used. This permits the analysis of several nucleic acid species or more nucleic acid sequence information per extension reaction. The affinity group need not be on the priming oligonucleotide but could alternatively be present on the template. For example, immobilization can be carried out via an interaction between biotinylated DNA and streptavidin-coated microtitration wells or avidin-coated polystyrene particles. In the same manner oligonucleotides or templates may be attached to a solid support in a high-density format. In such solid phase microsequencing reactions, incorporated ddNTPs can be radiolabeled (Syvänen, Clinica Chimica Acta 226:225-236, 1994) or linked to fluorescein (Livak and Hainer, Human Mutation 3:379-385,1994). The detection of radiolabeled ddNTPs can be achieved through scintillation-based techniques. The detection of fluorescein-linked ddNTPs can be based on the binding of antifluorescein antibody conjugated with alkaline phosphatase, followed by incubation with a chromogenic substrate (such as *p*-nitrophenyl phosphate). Other possible reporter-detection pairs include: ddNTP linked to dinitrophenyl (DNP) and anti-DNP alkaline phosphatase conjugate (Harju et al., Clin. Chem. 39/112282-2287, 1993) or biotinylated ddNTP and horseradish peroxidase-conjugated streptavidin with *o*-phenylenediamine as a substrate (WO 92/15712). As yet another alternative solid-phase microsequencing procedure, Nyren et al. (Analytical Biochemistry 208:171-175, 1993) described a method relying on the detection of DNA polymerase activity by an enzymatic luminometric inorganic pyrophosphate detection assay (ELIDE).

Pastinen et al. (Genome research 7:606-614, 1997), describe a method for multiplex detection of single nucleotide polymorphism in which the solid phase minisequencing principle is applied to an oligonucleotide array format. High-density arrays of DNA probes attached to a solid support (DNA chips) are further described in III.C.5.

In one aspect the present invention provides polynucleotides and methods to genotype one or more biallelic markers of the present invention by performing a microsequencing assay. Preferred microsequencing primers include those being featured Figure 6. It will be appreciated that the microsequencing primers listed in Figure 6 are merely exemplary and that, any primer having a 3' end immediately adjacent to a polymorphic nucleotide may be used. Similarly, it will be appreciated that microsequencing analysis may be performed for any biallelic marker or any combination of biallelic markers of the present invention. Described herein is a solid support which includes one or more microsequencing primers listed in Figure 6, or fragments comprising at least 8, at least 12, at least 15, or at least 20 consecutive nucleotides thereof and having a 3' terminus immediately upstream of the corresponding biallelic marker, for determining the identity of a nucleotide at biallelic marker site.

### 3) Mismatch detection assays based on polymerases and ligases

In one aspect the present invention provides polynucleotides and methods to determine the allele of one or more biallelic markers of the present invention in a biological sample, by mismatch detection assays based on polymerases and/or ligases. These assays are based on the specificity of polymerases and ligases. Polymerization reactions places particularly stringent requirements on correct base pairing of the 3' end of the amplification primer and the joining of two oligonucleotides hybridized to a target DNA sequence is quite sensitive to mismatches close to the ligation site, especially at the 3' end. The terms "enzyme based mismatch detection assay" are used herein to refer to any method of determining the allele of a biallelic marker based on the specificity of ligases and polymerases. Preferred methods are described below. Methods, primers and various parameters to amplify DNA fragments comprising biallelic markers of the present invention are further described above in III.B.

### Allele specific amplification

Discrimination between the two alleles of a biallelic marker can also be achieved by allele specific amplification, a selective strategy, whereby one of the alleles is amplified without amplification of the other allele. This is accomplished by placing a polymorphic base at the 3' end of one of the amplification primers. Because the extension forms from the 3'end of the primer, a mismatch at or near this position has an inhibitory effect on amplification. Therefore, under appropriate amplification conditions, these primers only direct amplification on their complementary allele. Designing the appropriate allele-specific primer and the corresponding assay conditions are well with the ordinary skill in the art.

### Ligation/amplification based methods

The "Oligonucleotide Ligation Assay" (OLA) uses two oligonucleotides which are designed to be capable of hybridizing to abutting sequences of a single strand of a target molecules. One of the oligonucleotides is biotinylated, and the other is detectably labeled. If the precise complementary sequence is found in a target molecule, the oligonucleotides will hybridize such that their termini abut, and create a ligation substrate that can be captured and detected. OLA is capable of detecting biallelic markers and may be advantageously combined with PCR as described by Nickerson D.A. et al. (Proc. Natl. Acad. Sci. U.S.A. 87:8923-8927, 1990). In this method, PCR is used to achieve the exponential amplification of target DNA, which is then detected using OLA.

Other methods which are particularly suited for the detection of biallelic markers include LCR (ligase chain reaction), Gap LCR (GLCR) which are described above in III.B. As mentioned above LCR uses two pairs of probes to exponentially amplify a specific target. The sequences of each pair of oligonucleotides, is selected to permit the pair to hybridize to abutting sequences of the same strand of the target. Such hybridization forms a substrate for a template-dependant ligase. In accordance with the present invention, LCR can be performed with oligonucleotides having the proximal and distal sequences of the same strand of a biallelic marker site. In one embodiment, either oligonucleotide will be designed to include the biallelic marker site. In such an embodiment, the reaction conditions are selected such that the oligonucleotides can be ligated together only if the target molecule either contains or lacks the specific nucleotide (s) that is complementary to the biallelic marker on the oligonucleotide. In an alternative embodiment, the oligonucleotides will not include the biallelic marker, such that when they hybridize to the target molecule, a "gap" is created as described in WO 90/01069. This gap is then "filled" with complementary dNTPs (as mediated by DNA polymerase), or by an additional pair of oligonucleotides. Thus at the end of each cycle, each single strand has a complement capable of serving as a target during the next cycle and exponential allele-specific amplification of the desired sequence is obtained.

Ligase/Polymerase-mediated Genetic Bit Analysis™ is another method for determining the identity of a nucleotide at a preselected site in a nucleic acid molecule (WO 95/21271). This method involves the incorporation of a nucleoside triphosphate that is complementary to the nucleotide present at the preselected site onto the terminus of a primer molecule, and their subsequent ligation to a second oligonucleotide. The reaction is monitored by detecting a specific label attached to the reaction's solid phase or by detection in solution.

**4) Hybridization assay methods**

A preferred method of determining the identity of the nucleotide present at a biallelic marker site involves nucleic acid hybridization. The hybridization probes, which can be conveniently used in such reactions, preferably include the probes defined herein. Any hybridization assay may be used including Southern hybridization, Northern hybridization, dot blot hybridization and solid-phase hybridization (see Sambrook et al., Molecular Cloning - A Laboratory Manual, Second Edition, Cold Spring Harbor Press, N.Y., 1989).

Hybridization refers to the formation of a duplex structure by two single stranded nucleic acids due to complementary base pairing. Hybridization can occur between exactly complementary nucleic acid strands or between nucleic acid strands that contain minor regions of mismatch. Specific probes can be designed that hybridize to one form of a biallelic marker and not to the other and therefore are able to discriminate between different allelic forms. Allele-specific probes are often used in pairs, one member of a pair showing perfect match to a target sequence containing the original allele and the other showing a perfect match to the target sequence containing the alternative allele. Hybridization conditions should be sufficiently stringent that there is a significant difference in hybridization intensity between alleles, and preferably an essentially binary response, whereby a probe hybridizes to only one of the alleles. Stringent, sequence specific hybridization conditions, under which a probe will hybridize only to the exactly complementary target sequence are well known in the art (Sambrook et al., Molecular Cloning - A Laboratory Manual, Second Edition. Cold Spring Harbor Press, N.Y., 1989). Stringent conditions are sequence dependent and will be different in different circumstances. Generally, stringent conditions are selected to be about 5°C lower than the thermal melting point (Tm) for the specific sequence at a defined ionic strength and pH. By wy of example and not limitation, procedures using conditions of high stringency are as follows: Prehybridization of filters containing DNA is carried out for 8 h to overnight at 65°C in buffer composed of 6X SSC, 50 mM Tris-HCl (pH 7.5), 1 mM EDTA, 0.02% PVP, 0.02% Ficoll, 0.02% BSA, and 500 $\mu$g/ml denatured salmon sperm DNA. Filters are hybridized for 48 h at 65°C, the preferred hybridization temperature, in prehybridization mixture containing 100 $\mu$g/ml denatured salmon sperm DNA and 5-20 X $10^6$ cpm of $^{32}$P-labeled probe. Alternatively, the hybridization step can be performed at 65°C in the presence of SSC buffer, 1 x SSC corresponding to 0.15M NaCl and 0.05 M Na citrate. Subsequently, filter washes can be done at 37°C for 1 h in a solution containing 2X SSC, 0.01% PVP, 0.01% Ficoll, and 0.01% BSA, followed by a wash in 0.1X SSC at 50°C for 45 min. Alternatively, filter washes can be performed in a solution containing 2 x SSC and 0.1% SDS, or 0.5 x SSC and 0.1% SDS, or 0.1 x SSC and 0.1% SDS at 68°C for 15 minute intervals. Following the wash steps, the hybridized probes are detectable by autoradiography. By way of example and not limitation, procedures using conditions of intermediate stringency are as follows: Filters containing DNA are prehybridized, and then hybridized at a temperature of 60°C in the presence of a 5 x SSC buffer and labeled probe. Subsequently, filters washes are performed in a solution containing 2x SSC at 50°C and the hybridized probes are detectable by autoradiography. Other conditions of high and intermediate stringency which may be used are well known in the art and as cited in Sambrook et al. (Molecular Cloning - A Laboratory Manual, Second Edition, Cold Spring Harbor Press, N.Y., 1989) and Ausubel et al. (Current Protocols in Molecular Biology, Green Publishing Associates and Wiley Interscience, N.Y., 1989).

Although such hybridizations can be performed in solution, it is preferred to employ a solid-phase hybridization assay. The target DNA comprising a biallelic marker of the present invention may be amplified prior to the hybridization reaction. The presence of a specific allele in the sample is determined by detecting the presence or the absence of stable hybrid duplexes formed between the probe and the target DNA. The detection of hybrid duplexes can be carried out by a number of methods. Various detection assay formats are well known which utilize detectable labels bound to either the target or the probe to enable detection of the hybrid duplexes. Typically, hybridization duplexes are separated from unhybridized nucleic acids and the labels bound to the duplexes are then detected. Those skilled in the art will recognize that wash steps may be employed to wash away excess target DNA or probe. Standard heterogeneous assay formats are suitable for detecting the hybrids using the labels present on the primers and probes.

Two recently developed assays allow hybridization-based allele discrimination with no need for separations or washes (see Landegren U. et al., Genome Research, 8:769-776,199). The TaqMan assay takes advantage of the 5' nuclease activity ofTaq DNA polymerase to digest a DNA probe annealed specifically to the accumulating amplification product. TaqMan probes are labeled with a donor-acceptor dye pair that interacts via fluorescence energy transfer. Cleavage of the TaqMan probe by the advancing polymerase during amplification dissociates the donor dye from the quenching acceptor dye, greatly increasing the donor fluorescence. All reagents necessary to detect two allelic variants can be assembled at the beginning of the reaction and the results are monitored in real time (*see* Livak et al., Nature Genetics, 9:341-342, 1995). In an alternative homogeneous hybridization-based procedure, molecular beacons are used for allele discriminations. Molecular beacons are hairpin-shaped oligonucleotide probes that report the presence of specific nucleic acids in homogeneous solutions. When they bind to their targets they undergo a conformational reorganization that restores the fluorescence of an internally quenched fluorophore (Tyagi et al., Nature Biotechnology, 16:49-53, 1998).

The polynucleotides provided herein can be used in hybridization assays for the detection of biallelic marker alleles in biological samples. These probes are **characterized in that** they preferably comprise between 8 and 50 nucleotides, and in that they are sufficiently complementary to a sequence comprising a biallelic marker of the present invention to hybridize thereto and preferably sufficiently specific to be able to discriminate the targeted sequence for only one nucleotide variation. The GC content in the probes of the invention usually ranges between 10 and 75 %, preferably between 35 and 60 %, and more preferably between 40 and 55 %. The length of these probes can range from 10, 15, 20, or 30 to at least 100 nucleotides, preferably from 10 to 50, more preferably from 18 to 35 nucleotides. A particularly preferred probe is 25 nucleotides in length. Preferably the biallelic marker is within 4 nucleotides of the center of the polynucleotide probe. In particularly preferred probes the biallelic marker is at the center of said polynucleotide. Shorter probes may lack specificity for a target nucleic acid sequence and generally require cooler temperatures to form sufficiently stable hybrid complexes with the template. Longer probes are expensive to produce and can sometimes self-hybridize to form hairpin structures. Methods for the synthesis of oligonucleotide probes have been described above and can be applied to the present probes.

Preferably the probes are labeled or immobilized on a solid support. Labels and solid supports are further described in I. Detection probes are generally nucleic acid sequences or uncharged nucleic acid analogs such as, for example peptide nucleic acids which are disclosed in International Patent Application WO 9220702, d, morpholino analogs which are described in U.S. Patents Numbered 5,185,444; 5,034,506 and 5,142,047. The probe may have to be rendered "non-extendable" in that additional dNTPs cannot be added to the probe. In and of themselves analogs usually are non-extendable and nucleic acid probes can be rendered non-extendable by modifying the 3' end of the probe such that the hydroxyl group is no longer capable of participating in elongation. For example, the 3' end of the probe can be functionalized with the capture or detection label to thereby consume or otherwise block the hydroxyl group. Alternatively, the 3' hydroxyl group simply can be cleaved, replaced or modified, U.S. Patent Application Serial No. 07/049,061 filed April 19, 1993 describes modifications, which can be used to render a probe non-extendable.

The probes are useful for a number of purposes. They can be used in Southern hybridization to genomic DNA or Northern hybridization to mRNA. The probes can also be used to detect PCR amplification products. By assaying the hybridization to an allele specific probe, one can detect the presence or absence of a biallelic marker allele in a given sample.

High-Throughput parallel hybridizations in array format are specifically encompassed within "hybridization assays" and are described below.

**Hybridization to addressable arrays of oligonucleotides**

Hybridization assays based on oligonucleotide arrays rely on the differences in hybridization stability of short oligonucleotides to perfectly matched and mismatched target sequence variants. Efficient access to polymorphism information is obtained through a basic structure comprising high-density arrays of oligonucleotide probes attached to a solid support (the chip) at selected positions. Each DNA chip can contain thousands to millions of individual synthetic DNA probes arranged in a grid-like pattern and miniaturized to the size of a dime.

The chip technology has already been applied with success in numerous cases. For example, the screening of mutations has been undertaken in the BRCA1 gene, in *S. cerevisiae* mutant strains, and in the protease gene of HIV-1 virus (Hacia et al., Nature Genetics, 14(4):441-447, 1996; Shoemaker et al., Nature Genetics, 14(4):450-456, 1996 ; Kozal et al., Nature Medicine, 2:753-759, 1996). Chips of various formats for use in detecting biallelic polymorphisms can be produced on a customized basis by Affymetrix (Genechip™), Hyseq (HyChip and HyGnostics), and Protogene Laboratories.

In general, these methods employ arrays of oligonucleotide probes that are complementary to target nucleic acid sequence segments from an individual which, target sequences include a polymorphic marker. EP785280, describes a tiling strategy for the detection of single nucleotide polymorphisms. Briefly, arrays may generally be "tiled" for a large number of specific polymorphisms. By "tiling" is generally meant the synthesis of a defined set of oligonucleotide probes which is made up of a sequence complementary to the target sequence of interest, as well as preselected variations of that sequence, e.g., substitution of one or more given positions with one or more members of the basis set of monomers, i.e. nucleotides. Tiling strategies are further described in PCT application No. WO 95/11995. In a particular aspect, arrays are tiled for a number of specific, identified biallelic marker sequences. In particular the array is tiled to include a number of detection blocks, each detection block being specific for a specific biallelic marker or a set of biallelic markers. For example, a detection block may be tiled to include a number of probes, which span the sequence segment that includes a specific polymorphism. To ensure probes that are complementary to each allele, the probes are synthesized in pairs differing at the biallelic marker. In addition to the probes differing at the polymorphic base, monosubstituted probes are also generally tiled within the detection block. These monosubstituted probes have bases at and up to a certain number of bases in either direction from the polymorphism, substituted with the remaining nucleotides (selected from A, T, G, C and U). Typically the probes in a tiled detection block will include substitutions of the sequence positions up to and including those that are 5 bases away from the biallelic marker. The monosubstituted probes provide internal controls for the tiled array, to distinguish actual hybridization from artefactual cross-hybridization. Upon completion of hybridization with the target sequence and washing of the array, the array is scanned to determine the position on the array to which the target sequence hybridizes. The hybridization data from the scanned array is then analyzed to identify which allele or alleles of the biallelic marker are present in the sample. Hybridization and scanning may be carried out as described in PCT application No. WO 92/10092 and WO 95/11995 and US patent No. 5,424,186.

Thus, in some examples, the chips may comprise an array of nucleic acid sequences of fragments of about 15 nucleotides in length. In further examples, the chip may comprise an array including at least one of the sequences selected from the group consisting of SEQ ID No. 1-30, 436-441, 469-472, 474-477, 484-487, 490-493 and the sequences complementary thereto, or a fragment thereof at least about 8 consecutive nucleotides, preferably 10, 15, 20, more preferably 25, 30, 40, 47, or 50 consecutive nucleotides. In some examples, the chip may comprise an array of at least 2, 3, 4, 5, 6, 7, 8 or more of these polynucleotides. Solid supports and polynucleotides attached to solid supports are further described in I. "Biallelic Markers and Polynucleotides Comprising Biallelic Markers."

**5) Integrated Systems**

Another technique, which may be used to analyze polymorphisms, includes multicomponent integrated systems, which miniaturize and compartmentalize processes such as PCR and capillary electrophoresis reactions in a single functional device. An example of such technique is disclosed in US patent 5,589,136, which describes the integration of PCR amplification and capillary electrophoresis in chips.

Integrated systems can be envisaged mainly when microfluidic systems are used. These systems comprise a pattern of microchannels designed onto a glass, silicon, quartz, or plastic wafer included on a microchip. The movements of the samples are controlled by electric, electroosmotic or hydrostatic forces applied across different areas of the microchip. For genotyping biallelic markers, the microfluidic system may integrate nucleic acid amplification, microsequencing, capillary electrophoresis and a detection method such as laser-induced fluorescence detection.

## IV. Methods of Genetic Analysis Using the Biallelic Markers

Different methods are available for the genetic analysis of complex traits (see Lander and Schork, Science, 265, 2037-2048, 1994). The search for disease-susceptibility genes is conducted using two main methods: the linkage approach in which evidence is sought for cosegregation between a locus and a putative trait locus using family studies, and the association approach in which evidence is sought for a statistically significant association between an allele and a trait or a trait causing allele (Khoury J. et al., Fundamentals of Genetic Epidemiology, Oxford University Press, NY, 1993). In general, the biallelic markers of the present invention find use in any method known in the art to demonstrate a statistically significant correlation between a genotype and a phenotype. The biallelic markers may be used in parametric and non-parametric linkage analysis methods. Preferably, the biallelic markers of the present invention are used to identify genes associated with detectable traits using association studies, an approach which does not require the use of affected families and which permits the identification of genes associated with complex and sporadic traits.

The genetic analysis using the biallelic markers of the present invention may be conducted on any scale. The whole set of biallelic markers of the present invention or any subset of biallelic markers of the present invention may be used. Further, any set of genetic markers including a biallelic marker of the present invention may be used. A set of biallelic polymorphisms that, could be used as genetic markers in combination with the biallelic markers of the present invention, has been described in WO 98/20165. As mentioned above, it should be noted that the biallelic markers of the present invention may be included in any complete or partial genetic map of the human genome.

### IV.A. Linkage Analysis

Linkage analysis is based upon establishing a correlation between the transmission of genetic markers and that of a specific trait throughout generations within a family. Thus, the aim of linkage analysis is to detect marker loci that show cosegregation with a trait of interest in pedigrees.

#### Parametric methods

When data are available from successive generations there is the opportunity to study the degree of linkage between pairs of loci. Estimates of the recombination faction enable loci to be ordered and placed onto a genetic map. With loci that are genetic markers, a genetic map can be established, and then the strength of linkage between markers and traits can be calculated and used to indicate the relative positions of markers and genes affecting those traits (Weir, B.S., Genetic data Analysis II: Methods for Discrete population genetic Data, Sinauer Assoc., Inc., Sunderland, MA, USA, 1996). The classical method for linkage analysis is the logarithm of odds (lod) score method (see Morton N.E., Am.J. Hum. Genet., 7:277-318, 1955; Ott J., Analysis of Human Genetic Linkage, John Hopkins University Press, Baltimore, 1991). Calculation of lod scores requires specification of the mode of inheritance for the disease (parametric method). Generally, the length of the candidate region identified using linkage analysis is between 2 and 20Mb. Once a candidate region is identified as described above, analysis of recombinant individuals using additional markers allows further delineation of the candidate region. Linkage analysis studies have generally relied on the use of a maximum of 5,000 microsatellite markers, thus limiting the maximum theoretical attainable resolution of linkage analysis to about 600 kb on average.

Linkage analysis has been successfully applied to map simple genetic traits that show clear Mendelian inheritance patterns and have a high penetrance (i.e., the ratio between the number of trait positive carriers of allele *a* and the total number of *a* carriers in the population). However, parametric linkage analysis suffers from a variety of drawbacks. First, it is limited by its reliance on the choice of a genetic model suitable for each studied trait. Furthermore, as already mentioned, the resolution attainable using linkage analysis is limited, and complementary studies are required to refine the analysis of the typical 2Mb to 20Mb regions initially identified through linkage analysis. In addition, parametric linkage analysis approaches have proven difficult when applied to complex genetic traits, such as those due to the combined action of multiple genes and/or environmental factors. It is very difficult to model these factors adequately in a lod score analysis. In such cases, too large an effort and cost are needed to recruit the adequate number of affected families required for applying linkage analysis to these situations, as recently discussed by Rich, N. and Merikangas, K. (Science, 273:1516-1517, 1996).

#### Non-parametric methods

The advantage of the so-called non-parametric methods for linkage analysis is that they do not require specification of the mode of inheritance for the disease, they tend to be more useful for the analysis of complex traits. In non-parametric methods, one tries to prove that the inheritance pattern of a chromosomal region is not consistent with random Mendelian segregation by showing that affected relatives inherit identical copies of the region more often than expected by chance. Affected relatives should show excess "allele sharing" even in the presence of incomplete penetrance and polygenic inheritance. In non-parametric linkage analysis the degree of agreement at a marker locus in two individuals can be measured either by the number of alleles identical by state (IBS) or by the number of allele identical by descent (IBD). Affected sib pair analysis is a well-known special case and is the simplest form of these methods.

The biallelic markers of the present invention may be used in both parametric and non-parametric linkage analysis. Preferably biallelic markers may be used in non-parametric methods which allow the mapping of genes involved in complex traits. The biallelic markers of the present invention may be used in both IBD- and IBS- methods to map genes affecting a complex trait. In such studies, taking advantage of the high density of biallelic markers, several adjacent biallelic marker loci may be pooled to achieve the efficiency attained by multi-allelic markers (Zhao et al., Am. J. Hum.

Genet., 63:225-240, 1998).

However, both parametric and non-parametric linkage analysis methods analyse affected relatives, they tend to be of limited value in the genetic analysis of drug responses or in the analysis of side effects to treatments. This type of analysis is impractical in such cases due to the lack of availability of familial cases. In fact, the likelihood of having more than one individual in a family being exposed to the same drug at the same time is extremely low.

### IV.B. Population Association Studies

Described herein are methods for identifying one or several genes among a set of candidate genes that are associated with a detectable trait using the biallelic markers of the present invention. In one example, described are methods to detect an association between a biallelic marker allele or a biallelic marker haplotype and a trait. Further, described are methods to identify a trait causing allele in linkage disequilibrium with any biallelic marker allele of the present invention.

As described above, alternative approaches can be employed to perform association studies: genome-wide association studies, candidate region association studies and candidate gene association studies. In a preferred embodiment, the biallelic markers of the present invention are used to perform candidate gene association studies. The candidate gene analysis clearly provides a short-cut approach to the identification of genes and gene polymorphisms related to a particular trait when some information concerning the biology of the trait is available. Further, the biallelic markers of the present invention may be incorporated in any map of genetic markers of the human genome in order to perform genome-wide association studies. Methods to generate a high-density map of biallelic markers has been described in US Provisional Patent application serial number 60/082,614. The biallelic markers of the present invention may further be incorporated in any map of a specific candidate region of the genome (a specific chromosome or a specific chromosomal segment for example).

As mentioned above, association studies may be conducted within the general population and are not limited to studies performed on related individuals in affected families. Association studies are extremely valuable as they permit the analysis of sporadic or multifactor traits. Moreover, association studies represent a powerful method for fine-scale mapping enabling much finer mapping of trait causing alleles than linkage studies. Studies based on pedigrees often only narrow the location of the trait causing allele. Association studies using the biallelic markers of the present invention can therefore be used to refine the location of a trait causing allele in a candidate region identified by Linkage Analysis methods. Moreover, once a chromosome segment of interest has been identified, the presence of a candidate gene such as a candidate gene of the present invention, in the region of interest can provide a shortcut to the identification of the trait causing allele. Biallelic markers of the present invention can be used to demonstrate that a candidate gene is associated with a trait.

#### 1) Determining the frequency of a biallelic marker allele or of a biallelic marker haplotype in a population

Association studies explore the relationships among frequencies for sets of alleles between loci.

#### Determining the frequency of an allele in a population

Allelic frequencies of the biallelic markers in a population can be determined using one of the methods described above under the heading "Methods for genotyping an individual for biallelic markers", or any genotyping procedure suitable for this intended purpose. Genotyping pooled samples or individual samples can determine the frequency of a biallelic marker allele in a population. One way to reduce the number of genotypings required is to use pooled samples. A major obstacle in using pooled samples is in terms of accuracy and reproducibility for determining accurate DNA concentrations in setting up the pools. Genotyping individual samples provides higher sensitivity, reproducibility and accuracy and; is the preferred method used in the present invention. Preferably, each individual is genotyped separately and simple gene counting is applied to determine the frequency of an allele of a biallelic marker or of a genotype in a given population.

#### Determining the frequency of a haplotype in a population

The gametic phase of haplotypes is unknown when diploid individuals are heterozygous at more than one locus. Using genealogical information in families gametic phase can sometimes be inferred (Perlin et al., Am. J. Hum. Genet., 55:777-787, 1994). When no genealogical information is available different strategies may be used. One possibility is that the multiple-site heterozygous diploids can be eliminated from the analysis, keeping only the homozygotes and the single-site heterozygote individuals, but this approach might lead to a possible bias in the sample composition and the underestimation of low-frequency haplotypes. Another possibility is that single chromosomes can be studied independently, for example, by asymmetric PCR amplification (see Newton et al., Nucleic Acids Res., 17:2503-2516, 1989; Wu et al., Proc. Natl. Acad Sci. USA, 86:2757, 1989), or by isolation of single chromosome by limit dilution followed by PCR amplification (see Ruano et al., Proc. Natl. Acad Sci. USA, 87:6296-6300, 1990). Further, a sample may be haplotyped for sufficiently close biallelic markers by double PCR amplification of specific alleles (Sarkar, G. and Sommer S.S., Biotechniques, 1991). These approaches are not entirely satisfying either because of their technical complexity, the additional cost they entail, their lack of generalisation at a large scale, or the possible biases they introduce. To overcome these difficulties, an algorithm to infer the phase of PCR-amplified DNA genotypes introduced by Clark A.G. (Mol. Biol. Evol., 7:111-122, 1990), may be used. Briefly, the principle is to start filling a preliminary list of haplotypes present in the sample by examining unambiguous individuals, that is, the complete homozygotes and the single-site heterozygotes. Then other individuals in the same sample are screened for the possible occurrence of previously recognised haplotypes. For each positive identification, the complementary haplotype is added to the list of recognised haplotypes, until the phase information for all individuals is either resolved or identified as unresolved. This method assigns a single haplotype to each multiheterozygous individual, whereas several haplotypes are possible when there are more than one heterozygous site. Alternatively, one can use methods estimating haplotype frequencies in a population without assigning haplotypes to each individual. Preferably, a method based on an expectation-maximization (EM) algorithm (Dempster et al., J. R. Stat. Soc., 39B: 1-38, 1977), leading to maximum-likelihood estimates of haplotype frequencies under the assumption of Hardy-Weinberg proportions (random mating) is used (see Excoffier L. and Slatkin M., Mol. Biol. Evol., 12(5): 921-927, 1995). The EM algorithm is a generalised iterative maximum-likelihood approach to estimation that is useful when data are ambiguous and/or incomplete. The EM algorithm is used to resolve heterozygotes into haplotypes. Haplotype estimations are further described below under the heading "Statistical methods." Any other method known in the art to determine or to estimate the frequency of a haplotype in a population may also be used.

**2) Linkage Disequilibrium analysis**

Linkage disequilibrium is the non-random association of alleles at two or more loci and represents a powerful tool for mapping genes involved in disease traits (see Ajioka R.S. et al., Am. J. Hum. Genet., 60:1439-1447, 1997). Biallelic markers, because they are densely spaced in the human genome and can be genotyped in more numerous numbers than other types of genetic markers (such as RFLP or VNTR markers), are particularly useful in genetic analysis based on linkage disequilibrium. The biallelic markers of the present invention may be used in any linkage disequilibrium analysis method known in the art.

Briefly, when a disease mutation is first introduced into a population (by a new mutation or the immigration of a mutation carrier), it necessarily resides on a single chromosome and thus on a single "background" or "ancestral" haplotype of linked markers. Consequently, there is complete disequilibrium between these markers and the disease mutation: one finds the disease mutation only in the presence of a specific set of marker alleles. Through subsequent generations recombinations occur between the disease mutation and these marker polymorphisms, and the disequilibrium gradually dissipates. The pace of this dissipation is a function of the recombination frequency, so the markers closest to the disease gene will manifest higher levels of disequilibrium than those that are further away. When not broken up by recombination, "ancestral" haplotypes and linkage disequilibrium between marker alleles at different loci can be tracked not only through pedigrees but also through populations. Linkage disequilibrium is usually seen as an association between one specific allele at one locus and another specific allele at a second locus.

The pattern or curve of disequilibrium between disease and marker loci is expected to exhibit a maximum that occurs at the disease locus. Consequently, the amount of linkage disequilibrium between a disease allele and closely linked genetic markers may yield valuable information regarding the location of the disease gene. For fine-scale mapping of a disease locus, it is useful to have some knowledge of the patterns of linkage disequilibrium that exist between markers in the studied region. As mentioned above the mapping resolution achieved through the analysis of linkage disequilibrium is much higher than that of linkage studies. The high density of biallelic markers combined with linkage disequilibrium analysis provides powerful tools for fine-scale mapping. Different methods to calculate linkage disequilibrium are described below under the heading "Statistical Methods."

**3) Population-based case-control studies of trait-marker associations**

As mentioned above, the occurrence of pairs of specific alleles at different loci on the same chromosome is not random and the deviation from random is called linkage disequilibrium. Association studies focus on population frequencies and rely on the phenomenon of linkage disequilibrium. If a specific allele in a given gene is directly involved in causing a particular trait, its frequency will be statistically increased in an affected (trait positive) population, when compared to the frequency in a trait negative population or in a random control population. As a consequence of the existence of linkage disequilibrium, the frequency of all other alleles present in the haplotype carrying the trait-causing allele will also be increased in trait positive individuals compared to trait negative individuals or random controls. Therefore, association between the trait and any allele (specifically a biallelic marker allele) in linkage disequilibrium with the trait-causing allele will suffice to suggest the presence of a trait-related gene in that particular region. Case-control populations can be genotyped for biallelic markers to identify associations that narrowly locate a trait causing allele. As any marker in linkage disequilibrium with one given marker associated with a trait will be associated with the trait. Linkage disequilibrium allows the relative frequencies in case-control populations of a limited number of genetic polymorphisms (specifically biallelic markers) to be analysed as an alternative to screening all possible functional polymorphisms in order to find trait-causing alleles. Association studies compare the frequency of marker alleles in unrelated case-control populations, and represent powerful tools for the dissection of complex traits.

**Case-control populations (inclusion criteria)**

Population-based association studies do not concern familial inheritance but compare the prevalence of a particular genetic marker, or a set of markers, in case-control populations. They are case-control studies based on comparison of unrelated case (affected or trait positive) individuals and unrelated control (unaffected or trait negative or random) individuals. Preferably the control group is composed of unaffected or trait negative individuals. Further; the control group is ethnically matched to the case population. Moreover, the control group is preferably matched to the case-population for the main known confusion factor for the trait under study (for example age-matched for an age-dependent trait). Ideally, individuals in the two samples are paired in such a way that they are expected to differ only in their disease status. In the following "trait positive population", "case population" and "affected population" are used interchangeably.

An important step in the dissection of complex traits using association studies is the choice of case-control populations (see Lander and Schork, Science, 265, 2037-2048, 1994). A major step in the choice of case-control populations is the clinical definition of a given trait or phenotype. Any genetic trait may be analysed by the association method proposed here by carefully selecting the individuals to be included in the trait positive and trait negative phenotypic groups. Four criteria are often useful: clinical phenotype, age at onset, family history and severity. The selection procedure for continuous or quantitative traits (such as blood pressure for example) involves selecting individuals at opposite ends of the phenotype distribution of the trait under study, so as to include in these trait positive and trait negative populations individuals with non-overlapping phenotypes. Preferably, case-control populations consist of phenotypically homogeneous populations. Trait positive and trait negative populations consist of phenotypically uniform populations of individuals representing each between 1 and 98%, preferably between 1 and 80%, more preferably between 1 and 50%, and more preferably between 1 and 30%, most preferably between 1 and 20% of the total population under study, and selected among individuals exhibiting non-overlapping phenotypes. The clearer the difference between the two trait phenotypes, the greater the probability of detecting an association with biallelic markers. The selection of those drastically different but relatively uniform phenotypes enables efficient comparisons in association studies and the possible detection of marked differences at the genetic level, provided that the sample sizes of the populations under study are significant enough.

In some examples, a first group of between 50 and 300 trait positive individuals, preferably about 100 individuals, are recruited according to their phenotypes. A similar number of trait negative individuals are included in such studies.

Typical examples of inclusion criteria include a disease involving the metabolic conversion of xenobiotics or the evaluation of the response to a drug or side effects to treatment with drugs.

Suitable examples of association studies using biallelic markers including the biallelic markers of the present invention, are studies involving the following populations:

a case population suffering from a disease involving the metabolic conversion of xenobiotics and a healthy unaffected control population, or

a case population treated with therapeutic agents suffering from side-effects resulting from the treatment and a control population treated with the same agents showing no side-effects, or

a case population treated with therapeutic agents showing a beneficial response and a control population treated with same agents showing no beneficial response.

In a preferred embodiment, the trait considered was a side-effect upon drug treatment, the study involved two populations derived from a clinical study of the anti-asthmatic drug zileuton. The case population was composed of asthmatic individuals treated with zileuton showing zileuton-associated hepatotoxicity monitored by the serum level of alanine aminotransferase (ALT) and the control population was composed of asthmatic individuals treated with zileuton and having no increased serum level of ALT. Inclusion criteria and association between the biallelic markers of the present invention and zileuton-associated hepatotoxicity are further described below and in Example 4.

## Association analysis

The general strategy to perform association studies using biallelic markers derived from a region carrying a candidate gene is to scan two groups of individuals (case-control populations) in order to measure and statistically compare the allele frequencies of the biallelic markers of the present invention in both groups.

If a statistically significant association with a trait is identified for at least one or more of the analysed biallelic markers, one can assume that: either the associated allele is directly responsible for causing the trait (the associated allele is the trait causing allele), or more likely the associated allele is in linkage disequilibrium with the trait causing allele. The specific characteristics of the associated allele with respect to the candidate gene function usually gives further insight into the relationship between the associated allele and the trait (causal or in linkage disequilibrium). If the evidence indicates that the associated allele within the candidate gene is most probably not the trait causing allele but is in linkage disequilibrium with the real trait causing allele, then the trait causing allele can be found by sequencing the vicinity of the associated marker.

Association studies are usually run in two successive steps. In a first phase, the frequencies of a reduced number of biallelic markers from one or several candidate genes are determined in the trait positive and trait negative populations. In a second phase of the analysis, the identity of the candidate gene and the position of the genetic loci responsible for the given trait is further refined using a higher density of markers from the relevant region. However, if the candidate gene under study is relatively small in length, a single phase may be sufficient to establish significant associations.

## Haplotype analysis

As described above, when a chromosome carrying a disease allele first appears in a population as a result of either mutation or migration, the mutant allele necessarily resides on a chromosome having a set of linked markers: the ancestral haplotype. This haplotype can be tracked through populations and its statistical association with a given trait can be analysed. Complementing single point (allelic) association studies with multi-point association studies also called haplotype studies increases the statistical power of association studies. Thus, a haplotype association study allows one to define the frequency and the type of the ancestral carrier haplotype: A haplotype analysis is important in that it increases the statistical power of an analysis involving individual markers.

In a first stage of a haplotype frequency analysis, the frequency of the possible haplotypes based on various combinations of the identified biallelic markers of the invention is determined. The haplotype frequency is then compared for distinct populations of trait positive and control individuals. The number of trait positive individuals, which should be, subjected to this analysis to obtain statistically significant results usually ranges between 30 and 300, with a preferred number of individuals ranging between 50 and 150. The same considerations apply to the number of unaffected individuals (or random control) used in the study. The results of this first analysis provide haplotype frequencies in case-control populations, for each evaluated haplotype frequency a p-value and an odd ratio are calculated. If a statistically significant association is found, the relative risk for an individual carrying the given haplotype of being affected with the trait under study can be approximated.

The preferred 2, 3 and 4 marker haplotypes described herein are listed in Table 3 below:

**Table 3**

| GENE | MARKER 1 | MARKER 2 | MARKER 3 | MARKER 4 |
|---|---|---|---|---|
| MOST-II | 12-455-326 | 12-453-429 | 12-424-198 | |
| MGST-II | 12-455-326 | 12-453-429 | 12-424-198 | 12-454-363 |
| MGST-II | 12-447-58 | 12-455-326 | 12-461-299 | 12-453-429 |
| MGST-II | 12-441-233 | 12-461-299 | 12-453-429 | |
| MGST-II | 12-441-233 | 12-461-299 | 12-453-429 | 12-426-154 |
| MOST-II | 12-426-154 | 12-424-198 | | |
| MGST-II | 12-426-154 | 12-461-299 | 12-424-198 | |

The most preferred 2, 3 and 4 marker haplotypes described herein are listed in Table 4 below:

Table 4

| GENE | MARKER 1 | MARKER 2 | MARKER 3 | MARKER 4 |
|---|---|---|---|---|
| MGST-II | 12-455-326 | 12-453-429 | 12-424-198 | |
| MGST-II | 12-455-326 | 12-453-429 | 12-424-198 | 12-454-363 |
| MGST-II | 12-447-58 | 12-455-326 | 12-461-299 | 12-453-429 |
| MGST-II | 12-426-154 | 12-424-198 | | |

**Interaction Analysis**

The biallelic markers of the present invention may also be used to identify patterns of biallelic markers associated with detectable traits resulting from polygenic interactions. The analysis of genetic interaction between alleles at unlinked loci requires individual genotyping using the techniques described herein. The analysis of allelic interaction among a selected set of biallelic markers with appropriate level of statistical significance can be considered as a haplotype analysis. Interaction analysis consists in stratifying the case-control populations with respect to a given haplotype for the first loci and performing a haplotype analysis with the second loci with each subpopulation.

Statistical methods used in association studies are further described below in IV.C. "Statistical Methods."

**4) Testing for linkage in the presence of association**

The biallelic markers of the present invention may further be used in TDT (transmission/disequilibrium test). TDT tests for both linkage and association and is not affected by population stratification. TDT requires data for affected individuals and their parents or data from unaffected sibs instead of from parents (see Spielmann S. et al.; Am. J. Hum. Genet., 52:506-516,1993; Schaid D.J. et al., Genet. Epidemiol.,13:423-450, 1996, Spielmann S. and Ewens W.J., Am. J. Hum. Genet., 62:450-458, 1998). Such combined tests generally reduce the false-positive errors produced by separate analyses. **IV.G Statistical Methods**

In general, any method known in the art to test whether a trait and a genotype show a statistically significant correlation may be used.

**1) Methods in linkage analysis**

Statistical methods and computer programs useful for linkage analysis are well-known to those skilled in the art (see Terwilliger J.D. and Ott J., Handbook of Human Genetic Linkage, John Hopkins University Press, London, 1994; Ott J., Analysis of Human Genetic Linkage, John Hopkins University Press, Baltimore, 1991).

**2) Methods to estimate haplotype frequencies in a population**

As described above, when genotypes are scored, it is often not possible to distinguish heterozygotes so that haplotype frequencies cannot be easily inferred. When the gametic phase is not known, haplotype frequencies can be estimated from the multilocus genotypic data. Any method known to person skilled in the art can be used to estimate haplotype frequencies (see Lange K., Mathematical and Statistical Methods for Genetic Analysis, Springer, New York, 1997; Weir, B.S., Genetic data Analysis II: Methods for Discrete population genetic Data, Sinauer Assoc., Inc., Sunderland, MA, USA, 1996*)*. Preferably, maximum-likelihood haplotype frequencies are computed using an Expectation-Maximization (EM) algorithm (see Dempster et al., J. R. Stat. Soc., 39B:1-38, 1977; Excoffier L. and Slatkin M., Mol. Biol. Evol., 12(5): 921-927, 1995). This procedure is an iterative process aiming at obtaining maximum-likelihood estimates of haplotype frequencies from multi-locus genotype data when the gametic phase is unknown. Haplotype estimations are usually performed by applying the EM algorithm using for example the EM-HAPLO program (Hawley M.E. et al., Am. J. Phys. Anthropol., 18:104, 1994) or the Arlequin program (Schneider et al., Arlequin: a software for population genetics data analysis, University of Geneva, 1997). The EM algorithm is a generalised iterative maximum likelihood approach to estimation and is briefly described below.

In the following part of this text, phenotypes will refer to multi-locus genotypes with unknown phase. Genotypes will refer to known-phase multi-locus genotypes.

Suppose a sample of N unrelated individuals typed for K markers. The data observed are the unknown-phase K-locus phenotypes that can categorised in F different phenotypes. Suppose that we have H underlying possible haplotypes (in case of K biallelic markers, $H=2^k$).

For phenotype j, suppose that $c_j$ genotypes are possible. We thus have the following equation

$$P_j = \sum_{i=1}^{c_j} pr(genotype_i) = \sum_{i=1}^{c_j} pr(h_k, h_l) \qquad \underline{\text{Equation 1}}$$

where $Pj$ is the probability of the phenotype $j$, $h_k$ and $h_l$ are the two haplotypes constituent the genotype i. Under the Hardy-Weinberg equilibrium, $pr(h_k,h_l)$ becomes :

$$pr(h_k, h_l) = pr(h_k)^2 \text{ if } h_k = h_l,\ pr(h_k, h_l) = 2pr(h_k).pr(h_l) \text{ if } h_k \neq h_l. \quad \underline{\text{Equation 2}}$$

The successive steps of the E-M algorithm can be described as follows:

Starting with initial values of the of haplotypes frequencies, noted $p_1^{(0)}, p_2^{(0)}, .....p_H^{(0)}$, these initial values serve to estimate the genotype frequencies (Expectation step) and then estimate another set of haplotype frequencies (Maximisation step), noted $p_1^{(1)}, p_2^{(1)}, .....p_H^{(1)}$, these two steps are iterated until changes in the sets of haplotypes frequency are very small.

A stop criterion can be that the maximum difference between haplotype frequencies between two iterations is less than $10^{-7}$. These values can be adjusted according to the desired precision of estimations.

In details, at a given iteration s, the Expectation step consists in calculating the genotypes frequencies by the following equation:

$$\begin{aligned} pr(genotype_i)^{(s)} &= pr(phenotype_j).pr(genotype_i | phenotype_j)^{(s)} \\ &= \frac{n_j}{N}.\frac{pr(h_k, h_l)^{(s)}}{P_j^{(s)}} \end{aligned} \qquad \underline{\text{Equation 3}}$$

where genotype i occurs in phenotype $j$, and where $h_k$ and $h_l$ constitute genotype $i$. Each probability is derived according to eq.1, and eq.2 described above.

Then the Maximisation step simply estimates another set of haplotype frequencies given the genotypes frequencies. This approach is also known as gene-counting method (Smith, Ann. Hum. Genet., 21:254-276, 1957).

$$p_t^{(s+1)} = \frac{1}{2} \sum_{j=1}^{F} \sum_{i=1}^{c_j} \delta_{it}.pr(genotype_i)^{(s)} \qquad \underline{\text{Equation 4}}$$

Where $\delta_{it}$ is an indicator variable which count the number of time haplotype $t$ in genotype i. It takes the values of 0, 1 or 2.

To ensure that the estimation finally obtained is the maximum-likelihood estimation several values of departures are required. The estimations obtained are compared and if they are different the estimations leading to the best likelihood are kept.

**3) Methods to calculate linkage disequilibrium between markers**

A number of methods can be used to calculate linkage disequilibrium between any two genetic positions, in practice linkage disequilibrium is measured by applying a statistical association test to haplotype data taken from a population.

Linkage disequilibrium between any pair of biallelic markers comprising at least one of the biallelic markers of the present invention ($M_i$, $M_j$) having alleles ($a_i/b_i$) at marker $M_i$ and alleles ($a_j/b_j$) at marker $M_j$ can be calculated for every allele combination ($a_i,a_j$; $a_i,b_j$; $b_i,a_j$ and $b_i,b_j$), according to the Piazza formula :

$$\Delta_{aiaj} = \sqrt{\theta 4} - \sqrt{(\theta 4 + \theta 3)(\theta 4 + \theta 2)},$$

where :

$\theta 4$= - - = frequency of genotypes not having allele $a_i$ at $M_i$ and not having allele $a_j$ at $M_j$
$\theta 3$= - + = frequency of genotypes not having allele $a_i$ at $M_i$ and having allele $a_j$ at $M_j$
$\theta 2$= + - = frequency of genotypes having allele $a_i$ at $M_i$ and not having allele $a_j$ at $M_j$

Linkage disequilibrium (LD) between pairs of biallelic markers ($M_i$, $M_j$) can also be calculated for every allele combination (ai,aj; ai,bj; $b_i$,$a_j$ and $b_i$,$b_j$), according to the maximum-likelihood estimate (MLE) for delta (the composite genotypic disequilibrium coefficient), as described by Weir (Weir B.S., Genetic Data Analysis, Sinauer Ass. Eds, 1996). The MLE for the composite linkage disequilibrium is:

$$D_{aiaj} = (2n_1 + n_2 + n_3 + n_4/2)/N - 2(pr(a_i).pr(a_j))$$

Where $n_1 = \Sigma$ phenotype ($a_i/a_i$, $a_j/a_j$), $n_2 = \Sigma$ phenotype ($a_i/a_i$, $a_j/b_j$), $n_3 = \Sigma$ phenotype ($a_i/b_i$, $a_j/a_j$), n4= $\Sigma$ phenotype ($a_i/b_i$, $a_j/b_j$) and N is the number of individuals in the sample.
This formula allows linkage disequilibrium between alleles to be estimated when only genotype, and not haplotype, data are available.
Another means of calculating the linkage disequilibrium between markers is as follows. For a couple of biallelic markers, $M_i$ ($a_i/b_i$) and $M_j(a_j/b_j)$, fitting the Hardy-Weinberg equilibrium, one can estimate the four possible haplotype frequencies in a given population according to the approach described above.
The estimation of gametic disequilibrium between *ai* and *aj* is simply:

$$D_{aiaj} = pr(haplotype(a_i, a_j)) - pr(a_i).pr(a_j).$$

Where $pr(a_i)$ is the probability of allele $a_i$ and $pr(a_j)$ is the probability of allele $a_j$ and where $pr(haplotype\ (a_i, a_j))$ is estimated as in Equation 3 above.
For a couple of biallelic marker only one measure of disequilibrium is necessary to describe the association between $M_i$ and $M_j$.
Then a normalised value of the above is calculated as follows:

$$D'_{aiaj} = D_{aiaj} / \max(-pr(a_i).pr(a_j), -pr(b_i).pr(b_j)) \quad \text{with } D_{aiaj}<0$$

$$D'_{aiaj} = D_{aiaj} / \max(pr(b_i).pr(a_j), pr(a_i).pr(b_j)) \quad \text{with } D_{aiaj}>0$$

The skilled person will readily appreciate that other LD calculation methods can be used without undue experimentation.
Linkage disequilibrium among a set of biallelic markers having an adequate heterozygosity rate can be determined by genotyping between 50 and 1000 unrelated individuals, preferably between 75 and 200, more preferably around 100.

**4) Testing for association**

Methods for determining the statistical significance of a correlation between a phenotype and a genotype, in this case an allele at a biallelic marker or a haplotype made up of such alleles, may be determined by any statistical test known in the art and with any accepted threshold of statistical significance being required. The application of particular methods and thresholds of significance are well with in the skill of the ordinary practitioner of the art.

Testing for association is performed by determining the frequency of a biallelic marker allele in case and control populations and comparing these frequencies with a statistical test to determine if their is a statistically significant difference in frequency which would indicate a correlation between the trait and the biallelic marker allele under study. Similarly, a haplotype analysis is performed by estimating the frequencies of all possible haplotypes for a given set of biallelic markers in case and control populations, and comparing these frequencies with a statistical test to determine if their is a statistically significant correlation between the haplotype and the phenotype (trait) under study. Any statistical tool useful to test for a statistically significant association between a genotype and a phenotype may be used. Preferably the statistical test employed is a chi-square test with one degree of freedom. A P-value is calculated (the P-value is the probability that a statistic as large or larger than the observed one would occur by chance).

**Statistical significance**

In preferred embodiments, significance for diagnosis purposes, either as a positive basis for further diagnostic tests or as a preliminary starting point for early preventive therapy, the p value related to a biallelic marker association is preferably about 1 x 10-2 or less, more preferably about 1 x 10-4 or less, for a single biallelic marker analysis and about 1 x 10-3 or less, still more preferably 1 x 10-6 or less and most preferably of about 1 x 10-8 or less, for a haplotype analysis involving several markers. These values are believed to be applicable to any association studies involving single or multiple marker combinations.

The skilled person can use the range of values set forth above as a starting point in order to carry out association studies with biallelic markers of the present invention. In doing so, significant associations between the biallelic markers of the present invention and responses to drugs or side effects upon treatment with drugs or diseases involving the metabolic conversion of xenobiotics can be revealed and used for diagnosis and drug screening purposes.

**Phenotypic permutation**

In order to confirm the statistical significance of the first stage haplotype analysis described above, it might be suitable to perform further analyses in which genotyping data from case-control individuals are pooled and randomised with respect to the trait phenotype. Each individual genotyping data is randomly allocated to two groups, which contain the same number of individuals as the case-control populations used to compile the data obtained in the first stage. A second stage haplotype analysis is preferably run on these artificial groups, preferably for the markers included in the haplotype of the first stage analysis showing the highest relative risk coefficient. This experiment is reiterated preferably at least between 100 and 10000 times. The repeated iterations allow the determination of the percentage of obtained haplotypes with a significant p-value level.

**Assessment of statistical association**

To address the problem of false positives similar analysis may be performed with the same case-control populations in random genomic regions. Results in random regions and the candidate region are compared as described in US Provisional Patent Application entitled "Methods, software and apparati for identifying genomic regions harbouring a gene associated with a detectable trait".

**5) Evaluation of risk factors**

The association between a risk factor (in genetic epidemiology the risk factor is the presence or the absence of a certain allele or haplotype at marker loci) and a disease is measured by the odds ratio (OR) and by the relative risk (RR). If $P(R^+)$ is the probability of developing the disease for individuals with R and $P(R^-)$ is the probability for individuals without the risk factor, then the relative risk is simply the ratio of the two probabilities, that is:

$$RR = P(R^+)/P(R^-)$$

In case-control studies, direct measures of the relative risk cannot be obtained because of the sampling design. However, the odds ratio allows a good approximation of the relative risk for low-incidence diseases and can be calculated:

$$OR = \left[\frac{F^+}{1-F^+}\right] \Big/ \left[\frac{F^-}{(1-F^-)}\right]$$

$F^+$ is the frequency of the exposure to the risk factor in cases and F is the frequency of the exposure to the risk factor in controls. $F^+$ and $F^-$ are calculated using the allelic or haplotype frequencies of the study and further depend on the underlying genetic model (dominant, recessive, additive...).

One can further estimate the attributable risk (AR) which describes the proportion of individuals in a population exhibiting a trait due to a given risk factor. This measure is important in quantitating the role of a specific factor in disease etiology and in terms of the public health impact of a risk factor. The public health relevance of this measure lies in estimating the proportion of cases of disease in the population that could be prevented if the exposure of interest were absent. AR is determined as follows:

$$AR = P_E (RR-1) / (P_E (RR-1)+1)$$

AR is the risk attributable to a biallelic marker allele or a biallelic marker haplotype. $P_E$ is the frequency of exposure to an allele or a haplotype within the population at large; and RR is the relative risk which, is approximated with the odds ratio when the trait under study has a relatively low incidence in the general population.

**IV.D. Identifcation of Biallelic Markers in Linkage Disequilibrium with the Biallelic Markers of the Invention**

Once a first biallelic marker has been identified in a genomic region of interest, the practitioner of ordinary skill in the art, using the teachings of the present invention, can easily identify additional biallelic markers in linkage disequilibrium with this first marker. As mentioned before any marker in linkage disequilibrium with a first marker associated with a trait will be associated with the trait. Therefore, once an association has been demonstrated between a given biallelic marker and a trait, the discovery of additional biallelic markers associated with this trait is of great interest in order to increase the density of biallelic markers in this particular region. The causal gene or mutation will be found in the vicinity of the marker or set of markers showing the highest correlation with the trait.

Identification of additional markers in linkage disequilibrium with a given marker involves: (a) amplifying a genomic fragment comprising a first biallelic marker from a plurality of individuals; (b) identifying of second biallelic markers in the genomic region harboring said first biallelic marker; (c) conducting a linkage disequilibrium analysis between said first biallelic marker and second biallelic markers; and (d) selecting said second biallelic markers as being in linkage disequilibrium with said first marker. Subcombinations comprising steps (b) and (c) are also contemplated.

Methods to identify biallelic markers and to conduct linkage disequilibrium analysis are described herein and can be carried out by the skilled person without undue experimentation. Described herein are biallelic markers which are in linkage disequilibrium with the specific biallelic markers shown in Figure 1 and which are expected to present similar characteristics in terms of their respective association with a given trait.

**IV.E. Identification of Functional Mutations**

Once a positive association is confirmed with a biallelic marker of the present invention, the associated candidate gene can be scanned for mutations by comparing the sequences of a selected number of trait positive and trait negative or control individuals. In a one example, functional regions such as exons and splice sites, promoters and other regulatory regions of the candidate gene are scanned for mutations. Preferably, trait positive individuals carry the haplotype shown to be associated with the trait and trait negative individuals do not carry the haplotype or allele associated with the trait. The mutation detection procedure is essentially similar to that used for biallelic site identification.

The method used to detect such mutations generally comprises the following steps: (a) amplification of a region of the candidate gene comprising a biallelic marker or a group of biallelic markers associated with the trait from DNA samples of trait positive patients and trait negative controls; (b) sequencing of the amplified region; (c) comparison of DNA sequences from trait-positive patients and trait-negative controls; and (d) determination of mutations specific to trait-positive patients. Subcombinations which comprise steps (b) and (c) are specifically contemplated.

It is preferred that candidate polymorphisms be then verified by screening a larger population of cases and controls by means of any genotyping procedure such as those described herein, preferably using a microsequencing technique in an individual test format. Polymorphisms are considered as candidate mutations when present in cases and controls at frequencies compatible with the expected association results.

Identification of mutations and low frequency polymorphisms in exons 3-5, in the 5'UTR region and in the 3' flanking region of the MGST-II gene is further described in Example 5. Eight polymorphisms were identified in the region of the MGST-II gene that was scanned. Three mutations were identified in the 3'UTR region. One mutation in exon 4 causes an amino acid substitution (Tyr→His) at the polypeptide level. A mutation in exon 5 introduces a stop codon into the ORF leading to the expression of a truncated MGST-II polypeptide. These mutations modify the specificity, activity and function of the MGST-II enzyme and therefore represent functional mutations of the MGST-II gene. Candidate polymorphisms and mutations suspected of being responsible for the detectable phenotype, such as hepatoxicity to zileuton or asthma, can be confirmed by screening a larger population of affected and unaffected individuals using any of the genotyping procedures described herein. Preferably the microsequencing technique is used. In a preferred embodiement trait positive and trait negative populations are genotyped for the candidate polymorphisms identified in Example 5 (10.286-289, 10-286-345, 10-286-375, 10-523-232, 10-289-201, 10-290-37, 10-290-326 and 10-290-328) most preferably for the mutations in exons 4 and 5 (10-289-201 and 10-290-37). Polymorphisms are considered as candidate "trait-causing" mutations when they exhibit a statistically significant correlation with the detectable phenotype.

## V. Biallelic Markers of the Invention in Methods of Genetic Diagnostics

The biallelic markers of the present invention can also be used to develop diagnostics tests capable of identifying individuals who express a detectable trait as the result of a specific genotype or individuals whose genotype places them at risk of developing a detectable trait at a subsequent time. The trait analyzed using the present diagnostics may be any detectable trait, including a response to a drug or side effects to a drug upon treatment or a disease involving the metabolic conversion of xenobiotics.

The diagnostic techniques of the present invention may employ a variety of methodologies to determine whether a test subject has a biallelic marker pattern associated with an increased risk of developing a detectable trait or whether the individual suffers from a detectable trait as a result of a particular mutation, including methods which enable the analysis of individual chromosomes for haplotyping, such as family studies, single sperm DNA analysis or somatic hybrids.

The present invention provides diagnostic methods to determine whether an individual is at risk of developing a disease or suffers from a disease resulting from a mutation or a polymorphism in a candidate gene of the present invention. The present invention also provides methods to determine whether an individual is likely to respond positively to a therapeutic agent or whether an individual is at risk of developing an adverse side effect to a therapeutic agent.

These methods involve obtaining a nucleic acid sample from the individual and, determining, whether the nucleic acid sample contains at least one allele or at least one biallelic marker haplotype, indicative of a risk of developing the trait or indicative that the individual expresses the trait as a result of possessing a particular candidate gene polymorphism or mutation (trait-causing allele).

Preferably, in such diagnostic methods, a nucleic acid sample is obtained from the individual and this sample is genotyped using methods described above in III. "Methods of Genotyping an Individual for Biallelic Markers." The diagnostics may be based on a single biallelic marker or a on group of biallelic markers.

In each of these methods, a nucleic acid sample is obtained from the test subject and the biallelic marker pattern of one or more of the biallelic markers listed in claim 1 is determined.

In one embodiment, a PCR amplification is conducted on the nucleic acid sample to amplify regions in which polymorphisms associated with a detectable phenotype have been identified. The amplification products are sequenced to determine whether the individual possesses one or more polymorphisms associated with a detectable phenotype. The primers used to generate amplification products may comprise the primers listed in Figure 7. Alternatively, the nucleic acid sample is subjected to microsequencing reactions as described above to determine whether the individual possesses one or more polymorphisms associated with a detectable phenotype resulting from a mutation or a polymorphism in a candidate gene. The primers used in the microsequencing reactions may include the primers listed in Figure 6. In another embodiment, the nucleic acid sample is contacted with one or more allele specific oligonucleotide probes which, specifically hybridize to one or more candidate gene alleles associated with a detectable phenotype. The probes used in the hybridization assay may include the probes listed in Figure 8. Diagnostic kits comprising polynucleotides of the present invention are further described in section I.

These diagnostic methods are extremely valuable as they can, in certain circumstances, be used to initiate preventive treatments or to allow an individual carrying a significant haplotype to foresee warning signs such as minor symptoms. For diseases in which attacks may be extremely violent and sometimes fatal if not treated on time, the knowledge of a potential predisposition, even if this predisposition is not absolute, might contribute in a very significant manner to treatment efficacy. Similarly, a diagnosed predisposition to a potential side effect could immediately direct the physician toward a treatment for which such side effects have not been observed during clinical trials.

Diagnostics, which analyze and predict response to a drug or side effects to a drug, may be used to determine whether an individual should be treated with a particular drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the markers of the present invention. One or more markers indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

In a preferred embodiment the identity of the nucleotide present at, at least one, biallelic marker selected from the group consisting of the markers listed in the table in claim 1 is determined, and wherein the detectable trait is asthma, or the detectable trait is hepatoxicity to the and-asthmatic drug zileuton. Diagnostic kits comprising polynucleotides of the present invention are further described in section I. "Biallelic Markers and Polynucleotides Comprising Biallelic Markers."

## VI. Association of Biallelic Markers of the Invention With Asthma

In the context of the present invention, an association between the MGST-II, gene and asthma was established.

Asthma affects over 5% of the population in industrialized countries. It is increasing in prevalence and severity and has a rising mortality (Rang H.P., Ritter J.M. and Dale M.M.; Pharmacology; Churchill Livingstone, NY, 1995). Bronchial asthma is a multifactorial syndrome rather than a single disease, defined as airway obstruction characterized by inflammatory changes in the airways and bronchial hyper-responsiveness. In addition to the evidenced impact of environmental factors on the development of asthma, patterns of clustering and segregation in asthmatic families have suggested a genetic component to asthma. However the lack of a defined and specific asthma phenotype and of suitable markers for genetic analysis is proving to be a major hurdle for reliably identifying genes associated with asthma. The identification of genes implicated in asthma would represent a major step towards the identification of new molecular targets for the development of anti-asthma drugs. Moreover there is no straightforward physiological or biological blood test for the asthmatic state. As a result, adequate asthma treatment is often delayed, thereby allowing the inflammation process to better establish itself. Thus, there is a need for the identification of asthma susceptibility genes in order to develop an efficient and reliable asthma diagnostic test.

As mentioned above, products of arachidonic acid metabolism are important inflammatory mediators and have been involved in a number of inflammatory diseases, including asthma. More specifically, prostaglandins and leukotrienes are thought to play a major role in the inflammatory process observed in asthma patients.

In order to investigate and identify a genetic origin to asthma, a candidate gene scan was conducted. This approach comprised:

- selecting candidate genes potentially involved in the pathological pathway of interest, in this case arachidonic acid metabolism, and
- identifying biallelic markers in those genes, and finally
- conducting association studies to identify biallelic marker alleles or haplotypes associated with asthma.

Further details concerning this association study are provided in Example 3, results are briefly summarized below.

Two groups of independent individuals were used in this association study in accordance with the invention: the case-control populations. The two groups corresponded to 297 asthmatic individuals and 178 control individuals. The trait positive asthma population was mostly composed of individuals from Caucasian ethnic background (>90 %). The control population was composed of individuals from a random US Caucasian population.

In the association study described in Example 3, several biallelic marker haplotypes were shown to be significantly associated with asthma. A preferred haplotype consisting of three biallelic markers (12-455-326, 12-453-429 and 12-424-198 ) presented a p-value of $3.2\ 10^{-5}$. Another preferred haplotype consisting of four biallelic markers (12-455-326, 12-453-429, 12-424-198 and 12-454-363) had a p-value of $1.2\ 10^{-6}$. Phenotypic permutation tests confirmed the statistical significance of these results. These haplotypes can therefore be considered to be significantly associated with asthma.

This information is extremely valuable. The knowledge of a potential genetic predisposition to asthma, even if this predisposition is not absolute, might contribute in a very significant manner to treatment efficacy of asthma patients and to the development of new therapeutic and diagnostic tools.

## VII. Association of Biallelic Markers of the Invention with Hepatotoxicity to Anti-Asthma Drug Zileuton (Zyflo™)

In the context of the present invention, an association between the MGST-II gene and side effects related to treatment with the anti-asthmatic drug zileuton was discovered.

As mentioned above, bronchial asthma is a multifactorial syndrome rather than a single disease, defined as airway obstruction characterized by inflammatory changes in the airways and bronchial hyper-responsiveness. Although initially reversible with bronchiodilators, airway obstruction becomes increasingly irreversible if treated poorly. Asthma management therefore relies on early and regular use of drugs that control the disease. As a consequence, there is a strong need for efficient and safe therapeutic opportunities for patients with asthma. There are two main categories of anti-asthmatic drugs- bronchodilators and anti-inflammatory agents. There is now general agreement on the need to implement early anti-inflammatory treatment rather than relying on symptomatic treatment with bronchiodilators alone. The leukotrienes, a family of proinflammatory mediators arising via arachidonic acid metabolism, have been implicated in the inflammatory cascade that occurs in asthmatic airways. Of great relevance to the pathogenesis of asthma are the 5-lipoxygenase and the 5-lipoxygenase activating protein (FLAP), which catalyze the initial steps in the biosynthesis of leukotrienes from arachidonic acid. Given the significant role of the inflammatory process in asthma, pharmacological agents, such as leukotriene antagonists, FLAP inhibitors and 5-lipoxygenase inhibitors have been developed.

Zileuton (Zyflo™) is an active inhibitor of 5-lipoxygenase, the enzyme that catalyzes the formation of leukotrienes from arachidonic acid, indicated for prophylaxis and chronic treatment of asthma. A minority of zileuton-treated patients develop liver function abnormalities as close monitoring revealed that elevations of liver function tests may occur during treatment with zileuton. In the present invention the ALT test (serum level of alanine aminotransferase) was used, which is considered the most sensitive indicator of liver injury.

In order to investigate and identify a genetic origin to zileuton-associated hepatotoxicity, a candidate gene scan was conducted. This approach comprised:

- selecting candidate genes potentially involved in the pathological pathway of interest or in the metabolism of zileuton, and
- identifying biallelic markers in those genes, and finally
- conducting association studies to identify biallelic marker alleles or haplotypes associated with elevations of liver function tests upon treatment with zileuton.

Further details concerning this association study are provided in Example 4, results are briefly summarized below.

Two groups of unrelated individuals were used in this association study in accordance with the invention: the case-control populations. The case population was composed of 89 asthmatic individuals treated with zileuton showing zileuton-associated hepatotoxicity monitored by the serum level of alanine aminotransferase (ALT) and the control population was composed of 208 asthmatic individuals treated with zileuton and having no increased serum ALT level.

The association study conducted with the biallelic markers derived from the MGST-II locus showed that several haplotypes were significantly associated with zileuton-associated hepatotoxicity. A preferred haplotype consisting of three biallelic markers (12-441-233, 12-461-299 and 12-453-429) presented a p-value of $1.5\ 10^{-5}$ and an odd ratio of 3.63. A second preferred haplotype consisting of four biallelic markers (12-441-233, 12-461-299, 12-453-429 and 12-426-154) had a p-value of $5.2\ 10^{-7}$ and an odd ratio of 5.75.

This information is extremely valuable. The knowledge of a potential genetic predisposition to hepatoxicity upon treatment with zileuton, even if this predisposition is not absolute, might contribute in a very significant manner to the safety of asthma treatment and to the development of diagnostic tools.

Similar association studies, with different case-control populations, can be routinely carried out by the skilled technician using the biallelic markers of the present invention in order to identify other association between traits and MGST-II-related biallelic marker alleles or haplotypes.

## VI. Computer-Related Embodiments

As used herein the term "nucleic acid codes" encompass the nucleotide sequences comprising, consisting essentially of, or consisting of any one of the following: a) a contiguous span of at least 12, 15, 18, 20, 25, 30, 35, 40, 50, 60, 70, 80, 90, 100, 150, 200, 500 or 1000 nucleotides, to the extent that a polynucleotide of these lengths is consistent with the lengths of the particular Sequence ID, of a sequence selected from the group consisting of the sequences described in Figure 2, and the complements thereof; b) a contiguous span of at least 12, 15, 18, 20, 25, 30, 35, 40, 50, 60, 70, 80, 90, 100, 150, 200, 500 or 1000 nucleotides, to the extent that a polynucleotide of these lengths is consistent with the lengths of the particular Sequence ID, of a sequence selected from the group consisting of the sequences described in Figure 3, and the complements thereof; c) a contiguous span of at least 12, 15, 18, 20, 25, 30, 35, 40, 50, 60, 70, 80, 90, 100, 150, 200, or 500 nucleotides, to the extent that a polynucleotide of these lengths is consistent with the lengths of the particular Sequence ID, of a sequence selected from the group consisting of the sequences described in Figure 6, more preferably a set of markers or sequences consisting of those markers or sequences found in SEQ ID Nos. 3, 5, 9, 13-15, 25, and the complements thereof, wherein said span includes an MGSTII-related biallelic marker, preferably an MGSTII-related biallelic marker described in Figure 1, preferably the biallelic markers found in Figure 9; or more preferably the biallelic markers found in SEQ ID Nos. 3, 5, 9, 13-15, 25, in said sequence with the alternative allele present at said biallelic marker.

The "nucleic acid codes" further encompass nucleotide sequences homologous to a contiguous span of at least 30, 35, 40, 50, 60, 70, 80, 90, 100, 150, 200, 500 or 1000 nucleotides, to the extent that a contiguous span of these lengths is consistent with the lengths of the particular Sequence ID, of a sequence selected from the group consisting of the sequences described in Figure 2, Figure 3 and Figure 6 and the complements thereof. Homologous sequences refer to a sequence having at least 99%, 98%, 97%, 96%, 95%, 90%, 85%, 80%, or 75% homology to these contiguous spans. Homology may be determined using any method described herein, including BLAST2N with the default parameters or with any modified parameters. Homologous sequences also may include RNA sequences in which uridines replace the thymines in the nucleic acid codes of the invention. It will be appreciated that the nucleic acid codes of the invention can be represented in the traditional single character format (See the inside back cover of Stryer, Lubert. Biochemistry, 3rd edition. W. H Freeman & Co., New York.) or in any other format or code which records the identity of the nucleotides in a sequence.

It will be appreciated by those skilled in the art that the nucleic acid codes, one or more of the polypeptide codes of SEQ ID Nos. 488 and 489 can be stored, recorded, and manipulated on any medium which can be read and accessed by a computer. As used herein, the words "recorded" and "stored" refer to a process for storing information on a computer medium. A skilled artisan can readily adopt any of the presently known methods for recording information on a computer readable medium to generate manufactures comprising one or more of the nucleic acid codes and one or more of the polypeptide codes of SEQ ID Nos. 488 and 489. Described herein is a computer readable medium having recorded thereon at least 2, 5, 10,15, 20, 25, 30, or 50 nucleic acid codes and the complements thereof. Also described is a computer readable medium having recorded thereon at least 2, 5, 10, 15, 20, 25, 30, or 50 polypeptide codes of SEQ ID Nos. 488 and 489.

Computer readable media include magnetically readable media, optically readable media, electronically readable media and magnetic/optical media. For example, the computer readable media may be a hard disk, a floppy disk, a magnetic tape, CD-ROM, Digital Versatile Disk (DVD), Random Access Memory (RAM), or Read Only Memory (ROM) as well as other types of other media known to those skilled in the art.

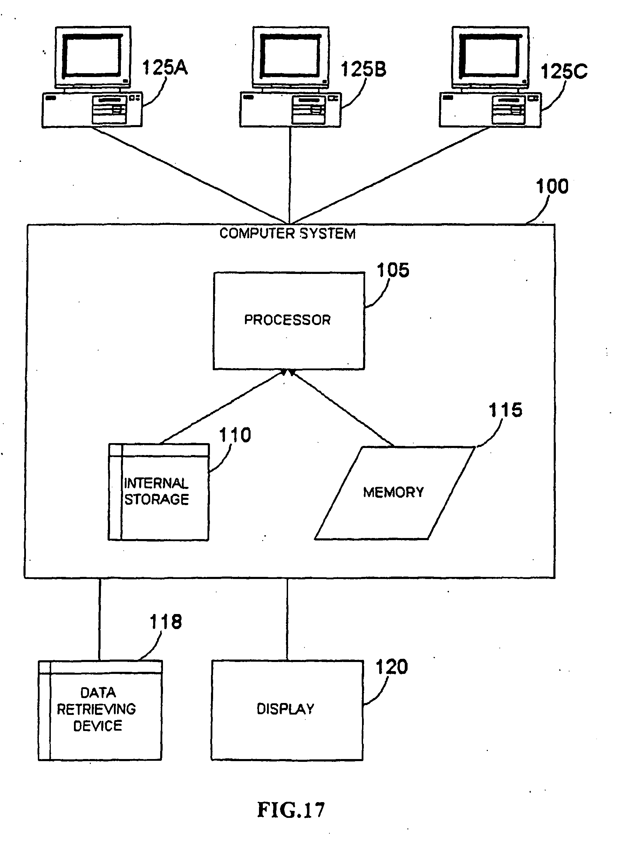

Also described are systems, particularly computer systems which store and manipulate the sequence information described herein. One example of a computer system 100 is illustrated in block diagram form in Figure 17. As used herein, "a computer system" refers to the hardware components, software components, and data storage components used to analyze the nucleotide sequences of the nucleic acid codes, or the amino acid sequences of the polypeptide codes of SEQ ID Nos. 488 and 489. In one embodiment, the computer system 100 is a Sun Enterprise 1000 server (Sun Microsystems, Palo Alto, CA). The computer system 100 may include a processor for processing, accessing and manipulating the sequence data. The processor 105 can be any well-known type of central processing unit, such as the Pentium III from Intel Corporation, or similar processor from Sun, Motorola, Compaq or International Business Machines. The computer system 100 may be a general purpose system that comprises the processor 105 and one or more internal data storage components 110 for storing data, and one or more data retrieving devices for retrieving the data stored on the data storage components. A skilled artisan can readily appreciate that any one of the currently available computer systems are suitable. In one particular example, the computer system 100 includes a processor 105 connected to a bus which is connected to a main memory 115 (preferably implemented as RAM) and one or more internal data storage devices 110, such as a hard drive and/or other computer readable media having data recorded thereon. In some examples, the computer system 100 further includes one or more data retrieving device 118 for reading the data stored on the internal data storage devices 110. The data retrieving device 118 may represent, for example, a floppy disk drive, a compact disk drive, a magnetic tape drive, etc. In some examples, the internal data storage device 110 is a removable computer readable medium such as a floppy disk, a compact disk, a magnetic tape, etc. containing control logic and/or data recorded thereon. The computer system 100 may include or be programmed by appropriate software for reading the control logic and/or the data from the data storage component once inserted in the data retrieving device. The computer system 100 includes a display 120 which is used to display output to a computer user. It should also be noted that the computer system 100 can be linked to other computer systems 125a-c in a network or wide area network to provide centralized access to the computer system 100. Software for accessing and processing the nucleotide sequences of the nucleic acid codes of the invention, or the amino acid sequences of the polypeptide codes of SEQ ID Nos. 488 and 489 (such as search tools, compare tools, and modeling tools etc.) may reside in main memory 115 during execution. In some examples the computer system 100 may further comprise a sequence comparer for comparing the above-described nucleic acid codes of the invention or polypeptide codes of SEQ ID Nos. 488 and 489 stored on a computer readable medium to reference nucleotide or polypeptide sequences stored on a computer readable medium. A "sequence comparer" refers to one or more programs which are implemented on the computer system 100 to compare a nucleotide or polypeptide sequence with other nucleotide or polypeptide sequences and/or compounds including but not limited to peptides, peptidomimetics, and chemicals stored within the data storage means. For example, the sequence comparer may compare the nucleotide sequences of the nucleic acid codes of the invention, or the amino acid sequences of the polypeptide codes of SEQ ID Nos. 488 and 489 stored on a computer readable medium to reference sequences stored on a computer readable medium to identify homologies, motifs implicated in biological function, or structural motifs. The various sequence comparer programs identified elsewhere in this patent specification are particularly contemplated.

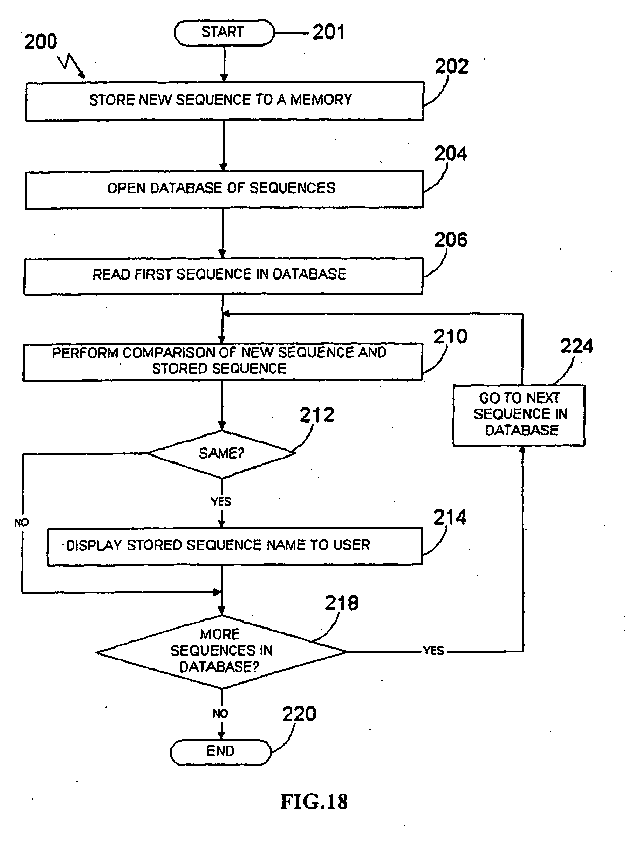

Figure 18 is a flow diagram illustrating one embodiment of a process 200 for comparing a new nucleotide or protein sequence with a database of sequences in order to determine the homology levels between the new sequence and the sequences in the database. The database of sequences can be a private database stored within the computer system 100, or a public database such as GENBANK, PIR OR SWISSPROT that is available through the Internet.

The process 200 begins at a start state 201 and then moves to a state 202 wherein the new sequence to be compared is stored to a memory in a computer system 100. As discussed above, the memory could be any type of memory, including RAM or an internal storage device. The process 200 then moves to a state 204 wherein a database of sequences is opened for analysis and comparison. The process 200 then moves to a state 206 wherein the first sequence stored in the database is read into a memory on the computer. A comparison is then performed at a state 210 to determine if the first sequence is the same as the second sequence. It is important to note that this step is not limited to performing an exact comparison between the new sequence and the first sequence in the database. Well-known methods are known to those of skill in the art for comparing two nucleotide or protein sequences, even if they are not identical. For example, gaps can be introduced into one sequence in order to raise the homology level between the two tested sequences. The parameters that control whether gaps or other features are introduced into a sequence during comparison are normally entered by the user of the computer system. Once a comparison of the two sequences has been performed at the state 210, a determination is made at a decision state 210 whether the two sequences are the same. Of course, the term "same" is not limited to sequences that are absolutely identical. Sequences that are within the homology parameters entered by the user will be marked as "same" in the process 200. If a determination is made that the two sequences are the same, the process 200 moves to a state 214 wherein the name of the sequence from the database is displayed to the user. This state notifies the user that the sequence with the displayed name fulfills the homology constraints that were entered. Once the name of the stored sequence is displayed to the user, the process 200 moves to a decision state 218 wherein a determination is made whether more sequences exist in the database. If no more sequences exist in the databases, then the process 200 terminates at an end state 220. However, if more sequences do exist in the database, then the process 200 moves to a state 224 wherein a pointer is moved to the next sequence in the database so that it can be compared to the new sequence. In this manner, the new sequence is aligned and compared with every sequence in the database. It should be noted that if a determination had been made at the decision state 212 that the sequences were not homologous, then the process 200 would move immediately to the decision state 218 in order to determine if any other sequences were available in the database for comparison. Accordingly, described herein is a computer system comprising a processor, a data storage device having stored thereon a nucleic acid code or a polypeptide code of SEQ ID Nos. 488 and 489, a data storage device having retrievably stored thereon reference nucleotide sequences or polypeptide sequences to be compared to the nucleic acid code or polypeptide code of SEQ ID Nos. 488 and 489 and a sequence comparer for conducting the comparison. The sequence comparer may indicate a homology level between the sequences compared or identify structural motifs in the above described nucleic acid code and polypeptide codes of SEQ ID Nos. 488 and 489 or it may identify structural motifs in sequences which are compared to these nucleic acid codes and polypeptide codes. In some examples, the data storage device may have stored thereon the sequences of at least 2, 5, 10, 15, 20, 25, 30, or 50 of the nucleic acid codes or polypeptide codes of SEQ ID Nos. 488 and 489.

Also described herein is a method for determining the level of homology between a nucleic acid code and a reference nucleotide sequence, comprising the steps of reading the nucleic acid code and the reference nucleotide sequence through the use of a computer program which determines homology levels and determining homology between the nucleic acid code and the reference nucleotide sequence with the computer program. The computer program may be any of a number of computer programs for determining homology levels, including those specifically enumerated herein, including BLAST2N with the default parameters or with any modified parameters. The method may be implemented using the computer systems described above. The method may also be performed by reading 2, 5, 10, 15, 20, 25, 30, or 50 of the above described nucleic acid codes through use of the computer program and determining homology between the nucleic acid codes and reference nucleotide sequences .

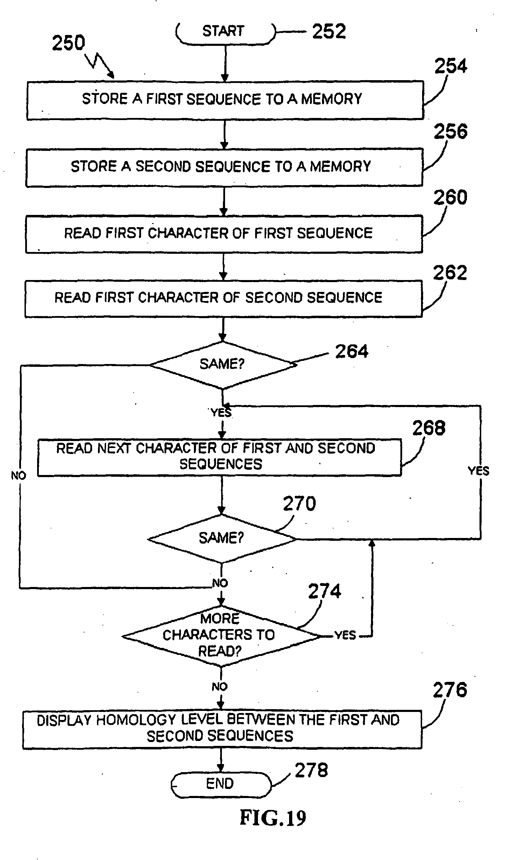

Figure 19 is a flow diagram illustrating one example of a process 250 in a computer for determining whether two sequences are homologous. The process 250 begins at a start state 252 and then moves to a state 254 wherein a first sequence to be compared is stored to a memory. The second sequence to be compared is then stored to a memory at a state 256. The process 250 then moves to a state 260 wherein the first character in the first sequence is read and then to a state 262 wherein the first character of the second sequence is read. It should be understood that if the sequence is a nucleotide sequence, then the character would normally be either A, T, C, G or U. If the sequence is a protein sequence, then it should be in the single letter amino acid code so that the first and sequence sequences can be easily compared.

A determination is then made at a decision state 264 whether the two characters are the same. If they are the same, then the process 250 moves to a state 268 wherein the next characters in the first and second sequences are read. A determination is then made whether the next characters are the same. If they are, then the process 250 continues this loop until two characters are not the same. If a determination is made that the next two characters are not the same, the process 250 moves to a decision state 274 to determine whether there are any more characters either sequence to read. If there aren't any more characters to read, then the process 250 moves to a state 276 wherein the level of homology between the first and second sequences is displayed to the user. The level of homology is determined by calculating the proportion of characters between the sequences that were the same out of the total number of sequences in the first sequence. Thus, if every character in a first 100 nucleotide sequence aligned with a every character in a second sequence, the homology level would be 100%. Alternatively, the computer program may be a computer program which compares the nucleotide sequences of the nucleic acid codes, to reference nucleotide sequences in order to determine whether the nucleic acid code differs from a reference nucleic acid sequence at one or more positions. Optionally such a program records the length and identity of inserted, deleted or substituted nucleotides with respect to the sequence of either the reference polynucleotide or the nucleic acid code. In one example, the computer program may be a program which determines whether the nucleotide sequences of the nucleic acid codes contain a biallelic marker or single nucleotide polymorphism (SNP) with respect to a reference nucleotide sequence. This single nucleotide polymorphism may comprise a single base substitution, insertion, or deletion, while this biallelic marker may comprise about one to ten consecutive bases substituted, inserted or deleted.

Also described is a method for determining the level of homology between a polypeptide code of SEQ ID Nos. 488 and 489 and a reference polypeptide sequence, comprising the steps of reading the polypeptide code of SEQ ID Nos. 488 and 489 and the reference polypeptide sequence through use of a computer program which determines homology levels and determining homology between the polypeptide code and the reference polypeptide sequence using the computer program.

Accordingly, also described herein is a method for determining whether a nucleic acid code differs at one or more nucleotides from a reference nucleotide sequence comprising the steps of reading the nucleic acid code and the reference nucleotide sequence through use of a computer program which identifies differences between nucleic acid sequences and identifying differences between the nucleic acid code and the reference nucleotide sequence with the computer program. In some examples, the computer program is a program which identifies single nucleotide polymorphisms. The method may be implemented by the computer systems described above and the method illustrated in Figure 19. The method may also be performed by reading at least 2, 5, 10, 15, 20, 25, 30, or 50 of the nucleic acid codes of the invention and the reference nucleotide sequences through the use of the computer program and identifying differences between the nucleic acid codes and the reference nucleotide sequences with the computer program. In other examples the computer based system may further comprise an identifier for identifying features within the nucleotide sequences of the nucleic acid codes or the amino acid sequences of the polypeptide codes of SEQ ID Nos. 488 and 489. An "identifier" refers to one or more programs which identifies certain features within the above-described nucleotide sequences of the nucleic acid codes or the amino acid sequences of the polypeptide codes of SEQ ID Nos. 488 and 489. In one example, the identifier may comprise a program which identifies an open reading frame in the cDNAs codes of SEQ ID Nos. 486 and 487.

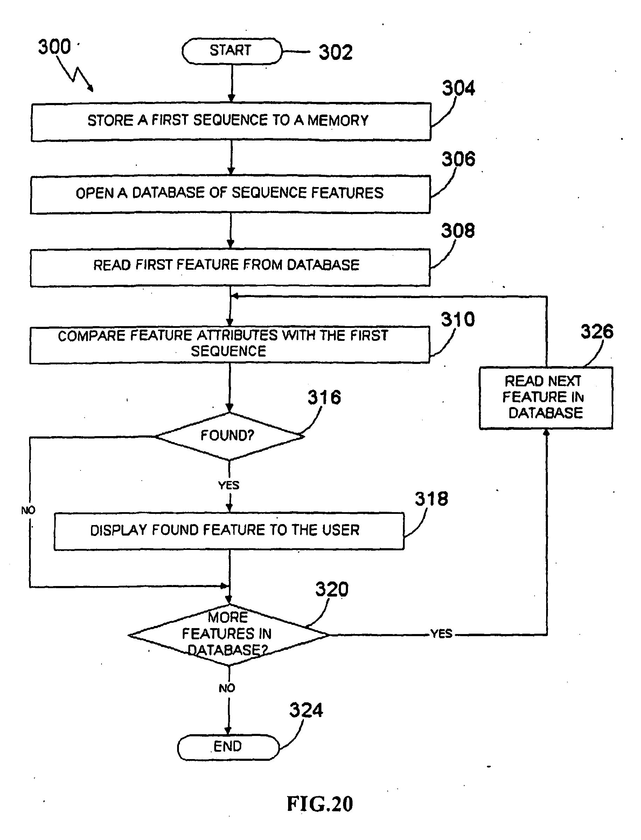

Figure 20 is a flow diagram illustrating one example of an identifier process 300 for detecting the presence of a feature in a sequence. The process 300 begins at a start state 302 and then moves to a state 304 wherein a first sequence that is to be checked for features is stored to a memory 115 in the computer system 100. The process 300 then moves to a state 306 wherein a database of sequence features is opened. Such a database would include a list of each feature's attributes along with the name of the feature. For example, a feature name could be "Initiation Codon" and the attribute would be "ATG." Another example would be the feature name "TAATAA Box" and the feature attribute would be "TAATAA". An example of such a database is produced by the University of Wisconsin Genetics Computer Group (www.gcg.com). Once the database of features is opened at the state 306, the process 300 moves to a state 308 wherein the first feature is read from the database. A comparison of the attribute of the first feature with the first sequence is then made at a state 310. A determination is then made at a decision state 316 whether the attribute of the feature was found in the first sequence. If the attribute was found, then the process 300 moves to a state 318 wherein the name of the found feature is displayed to the user. The process 300 then moves to a decision state 320 wherein a determination is made whether move features exist in the database. If no more features do exist, then the process 300 terminates at an end state 324. However, if more features do exist in the database, then the process 300 reads the next sequence feature at a state 326 and loops back to the state 310 wherein the attribute of the next feature is compared against the first sequence. It should be noted, that if the feature attribute is not found in the first sequence at the decision state 316, the process 300 moves directly to the decision state 320 in order to determine if any more features exist in the database. In another example, the identifier may comprise molecular modeling program which determines the 3-dimensional structure of the polypeptides codes of SEQ ID Nos. 488 and 489. In some examples the molecular modeling program identifies target sequences that are most compatible with profiles representing the structural environments of the residues in known three-dimensional protein structures. (See, e.g., Eisenberg et al., U.S. Patent No. 5,436,850 issued July 25, 1995). In another technique, the known three-dimensional structures of proteins in a given family are superimposed to define the structurally conserved regions in that family. This protein modeling technique also uses the known three-dimensional structure of a homologous protein to approximate the structure of the polypeptide codes of SEQ ID Nos. 488 and 489. (See e.g., Srinivasan, et al., U.S. Patent No. 5,557,535 issued September 17, 1996). Conventional homology modeling techniques have been used routinely to build models of proteases and antibodies. (Sowdhamini et al., Protein Engineering 10:207, 215 (1997)). Comparative approaches can also be used to develop three-dimensional protein models when the protein of interest has poor sequence identity to template proteins. In some cases, proteins fold into similar three-dimensional structures despite having very weak sequence identities. For example, the three-dimensional structures of a number of helical cytokines fold in similar three-dimensional topology in spite of weak sequence homology. The recent development of threading methods now enables the identification of likely folding patterns in a number of situations where the structural relatedness between target and template(s) is not detectable at the sequence level. Hybrid methods, in which fold recognition is performed using Multiple Sequence Threading (MST), structural equivalencies are deduced from the threading output using a distance geometry program DRAGON to construct a low resolution model, and a full-atom representation is constructed using a molecular modeling package such as QUANTA.

According to this 3-step approach, candidate templates are first identified by using the novel fold recognition algorithm MST, which is capable of performing simultaneous threading of multiple aligned sequences onto one or more 3-D structures. In a second step, the structural equivalencies obtained from the MST output are converted into interresidue distance restraints and fed into the distance geometry program DRAGON, together with auxiliary information obtained from secondary structure predictions. The program combines the restraints in an unbiased manner and rapidly generates a large number of low resolution model confirmations. In a third step, these low resolution model confirmations are converted into full-atom models and subjected to energy minimization using the molecular modeling package QUANTA. (See e.g., Aszódi et al., Proteins: Structure, Function, and Genetics; Supplement 1:38-42 (1997)).

The results of the molecular modeling analysis may then be used in rational drug design techniques to identify agents which modulate the activity of the polypeptide codes of SEQ ID Nos. 488 and 489. Accordingly, described herein is a method of identifying a feature within the nucleic acid codes or the polypeptide codes of SEQ ID Nos. 488 and 489 comprising reading the nucleic acid code(s) or the polypeptide code(s) through the use of a computer program which identifies features therein and identifying features within the nucleic acid code(s) or polypeptide code(s) with the computer program. In one example, computer program comprises a computer program which identifies open reading frames. In a further example, the computer program identifies structural motifs in a polypeptide sequence. In another example, the computer program comprises a molecular modeling program. The method may be performed by reading a single sequence or at least 2, 5,10, 15, 20, 25, 30, or 50 of the nucleic acid codes or the polypeptide codes of SEQ ID Nos. 488 and 489 through the use of the computer program and identifying features within the nucleic acid codes or polypeptide codes with the computer program. The nucleic acid codes or the polypeptide codes of SEQ ID Nos. 488 and 489 may be stored and manipulated in a variety of data processor programs in a variety of formats. For example, the nucleic acid codes or the polypeptide codes of SEQ ID Nos. 488 and 489 may be stored as text in a word processing file, such as MicrosoftWORD or WORDPERFECT or as an ASCII file in a variety of database programs familiar to those of skill in the art, such as DB2, SYBASE, or ORACLE. In addition, many computer programs and databases may be used as sequence comparers, identifiers, or sources of reference nucleotide or polypeptide sequences to be compared to the nucleic acid codes or the polypeptide codes of SEQ ID Nos. 488 and 489. The following list is intended to provide guidance to programs and databases which are useful with the nucleic acid codes or the polypeptide codes of SEQ ID No. 488 and 489. The programs and databases which may be used include, but are not limited to: MacPattern (EMBL), DiscoveryBase (Molecular Applications Group), GeneMine (Molecular Applications Group), Look (Molecular Applications Group), MacLook (Molecular Applications Group), BLAST and BLAST2 (NCBI), BLASTN and BLASTX (Altschul et al, J. Mol. Biol. 215: 403 (1990)), FASTA (Pearson and Lipman, Proc. Natl. Acad. Sci. USA, 85: 2444 (1988)), FASTDB (Brutlag et al. Comp. App. Biosci. 6:237-245. 1990), Catalyst (Molecular Simulations Inc.), Catalyst/SHAPE (Molecular Simulations Inc.), Cerius$^2$.DBAccess (Molecular Simulations Inc.), HypoGen (Molecular Simulations Inc.), Insight II, (Molecular Simulations Inc.), Discover (Molecular Simulations Inc.), CHARMm (Molecular Simulations Inc.), Felix (Molecular Simulations Inc.), DelPhi, (Molecular Simulations Inc.), QuanteMM, (Molecular Simulations Inc.), Homology (Molecular Simulations Inc.), Modeler (Molecular Simulations Inc.), ISIS (Molecular Simulations Inc.), Quanta/Protein Design (Molecular Simulations Inc.), WebLab (Molecular Simulations Inc.), WebLab Diversity Explorer (Molecular Simulations Inc.), Gene Explorer (Molecular Simulations Inc.), SeqFold (Molecular Simulations Inc.), the EMBL/Swissprotein database, the MDL Available Chemicals Directory database, the MDL Drug Data Report data base, the Comprehensive Medicinal Chemistry database, Derwents's World Drug Index database, the BioByteMasterFile database, the Genbank database, and the Genseqn database. Many other programs and data bases would be apparent to one of skill in the art given the present disclosure. Motifs which may be detected using the above programs include sequences encoding leucine zippers, helix-turn-helix motifs, glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites.

It should be noted that the nucleic acid codes further encompass all of the polynucleotides disclosed, described or claimed herein. Also, it should be noted that the polypeptide codes of SEQ ID Nos. 488 and 489 further encompass all of the polypeptides disclosed or described herein.

## EXAMPLES

Several of the methods of the present invention are described in the following examples, which are offered by way of illustration and not by way of limitation.

### Example 1

### *De Novo* Identification Of Biallelic Markers

The biallelic markers set forth in this application were isolated from human genomic sequences. To identify biallelic markers, genomic fragments were amplified, sequenced and compared in a plurality of individuals.

#### DNA samples

Donors were unrelated and healthy. They represented a sufficient diversity for being representative of a French heterogeneous population. The DNA from 100 individuals was extracted and tested for the *de novo* identification of biallelic markers.

DNA samples were prepared from peripheral venous blood as follows. Thirty ml of peripheral venous blood were taken from each donor in the presence of EDTA. Cells (pellet) were collected after centrifugation for 10 minutes at 2000 rpm. Red cells were lysed in a lysis solution (50 ml final volume: 10 mM Tris pH7.6; 5 mM $MgCl_2$; 10 mM NaCl). The solution was centrifuged (10 minutes, 2000 rpm) as many times as necessary to eliminate the residual red cells present in the supernatant, after resuspension of the pellet in the lysis solution. The pellet of white cells was lysed overnight at 42°C with 3.7 ml of lysis solution composed of: (a) 3 ml TE 10-2 (Tris-HCl 10 mM, EDTA 2 mM) / NaCl 0.4 M; (b) 200 µl SDS 10%; and (c) 500 µl proteinase K (2 mg proteinase K in TE 10-2 / NaCl 0.4 M).

For the extraction of proteins, 1 ml saturated NaCl (6M) (1/3.5 v/v) was added. After vigorous agitation, the solution was centrifuged for 20 minutes at 10000 rpm. For the precipitation of DNA, 2 to 3 volumes of 100% ethanol were added to the previous supernatant, and the solution was centrifuged for 30 minutes at 2000 rpm. The DNA solution was rinsed three times with 70% ethanol to eliminate salts, and centrifuged for 20 minutes at 2000 rpm. The pellet was dried at 37°C, and resuspended in 1 ml TE 10-1 or 1 ml water. The DNA concentration was evaluated by measuring the OD at 260 nm (1 unit OD = 50 µg/ml DNA). To determine the presence of proteins in the DNA solution, the OD 260 / OD 280 ratio was determined. Only DNA preparations having a OD 260 / OD 280 ratio between 1.8 and 2 were used in the subsequent examples described below. DNA pools were constituted by mixing equivalent quantities of DNA from each individual.

#### Amplification of genomic DNA by PCR

Amplification of specific genomic sequences was carried out on pooled DNA samples obtained as described above.

Amplification primers

The primers used for the amplification of human genomic DNA fragments were defined with the OSP software (Hillier & Green, 1991). Preferably, primers included, upstream of the specific bases targeted for amplification, a common oligonucleotide tail useful for sequencing. Primers PU contain the following additional PU 5' sequence : TGTAAAACGACGGCCAGT; primers RP contain the following RP 5' sequence : CAGGAAACAGCTATGACC. Primers are listed in Figure 7.

Amplification

PCR assays were performed using the following protocol:

| | |
|---|---|
| Final volume | 25 µl |
| DNA | 2 ng/µl |
| $MgCl_2$ | 2 mM |
| dNTP (each) | 200 µM |
| primer (each) | 2.9 ng/µl |
| Ampli Taq Gold DNA polymerase | 0.05 unit/µl |
| PCR buffer (10x = 0.1 M TrisHCl pH8.3 0.5M KCl) | 1x |

DNA amplification was performed on a Genius II thermocycler. After heating at 94°C for 10 min, 40 cycles were performed. Cycling times and temperatures were: 30 sec at 94°C, 55°C for 1 min and 30 sec at 72°C. Holding for 7 min at 72°C allowed final elongation. The quantities of the amplification products obtained were determined on 96-well microtiter plates, using a fluorometer and Picogreen as intercalant agent (Molecular Probes).

**Sequencing of amplified genomic DNA and identification of biallelic polymorphisms**

Sequencing of the amplified DNA was carried out on ABI 377 sequencers. The sequences of the amplification products were determined using automated dideoxy terminator sequencing reactions with a dye terminator cycle sequencing protocol. The products of the sequencing reactions were run on sequencing gels and the sequences were determined using gel image analysis (ABI Prism DNA Sequencing Analysis software 2.1.2 version).

The sequence data were further evaluated to detect the presence of biallelic markers within the amplified fragments. The polymorphism search was based on the presence of superimposed peaks in the electrophoresis pattern resulting from different bases occurring at the same position. However, the presence of two peaks can be an artifact due to background noise. To exclude such an artifact, the two DNA strands were sequenced and a comparison between the two strands was carried out. In order to be registered as a polymorphic sequence, the polymorphism had to be detected on both strands. Further, some biallelic single nucleotide polymorphisms were confirmed by microsequencing as described below.

Biallelic markers were identified in the analyzed fragments and are shown in Figure 1 and Table 2.

**Example 2**

**Genotyping Of Biallelic Markers**

The biallelic markers identified as described above were further confirmed and their respective frequencies were determined through microsequencing. Microsequencing was carried out on individual DNA samples obtained as described herein.

**Microsequencing primers**

Amplification of genomic DNA fragments from individual DNA samples was performed as described in Example 1 using the same set of PCR primers (Figure 7). Microsequencing was carried out on the amplified fragments using specific primers. See Figure 6. The preferred primers used in microsequencing had about 19 nucleotides in length and hybridized just upstream of the considered polymorphic base.

The microsequencing reactions were performed as follows: 5 µl of PCR products were added to 5 µl purification mix (2U SAP (Shrimp alkaline phosphate) (Amersham E70092X)); 2U Exonuclease I (Amersham E70073Z); and 1 µl SAP buffer (200 mM Tris-HCl pH8, 100 mM $MgCl_2$) in a microtiter plate. The reaction mixture was incubated 30 minutes at 37°C, and denatured 10 minutes at 94°C afterwards. To each well was then added 20 µl of microsequencing reaction mixture containing: 10 pmol microsequencing oligonucleotide (19mers, GENSET, crude synthesis. 5 OD), 1 U Thermosequenase (Amersham E79000G), 1.25 µl Thermosequenase buffer (260 mM Tris HCl pH 9.5, 65 mM $MgCl_2$), and the two appropriate fluorescent ddNTPs complementary to the nucleotides at the polymorphic site corresponding to both polymorphic bases (11.25 nM TAMRA-ddTTP ; 16.25 nM ROX-ddCTP ; 1.675 nM REG-ddATP ; 1.25 nM RHO-ddGTP ; Perkin Elmer, Dye Terminator Set 401095). After 4 minutes at 94°C, 20 PCR cycles of 15 sec at 55°C, 5 sec at 72°C, and 10 sec at 94°C were carried out in a Tetrad PTC-225 thermocycler (MJ Research). The microtiter plate was centrifuged 10 sec at 1500 rpm. The unincorporated dye terminators were removed by precipitation with 19 μl $MgCl_2$ 2mM and 55 μl 100 % ethanol. After 15 minute incubation at room temperature, the microtiter plate was centrifuged at 3300 rpm 15 minutes at 4°C. After discarding the supernatants, the microplate was evaporated to dryness under reduced pressure (Speed Vac). Samples were resuspended in 2.5 μl formamide EDTA loading buffer and heated for 2 min at 95°C. 0.8 μl microsequencing reaction were loaded on a 10 % (19:1) polyacrylamide sequencing gel. The data were collected by an ABI PRISM 377 DNA sequencer and processed using the GENESCAN software (Perkin Elmer).

**Frequency of biallelic markers**

Frequencies for the MGSTII related markers are reported for the less common allele only and are shown in Figure 1. The frequencies for both alleles are shown for the MGST-II, gene in Figure 9.

**Example 3**

**Association Between Asthma and the Biallelic Markers of the MGST-II Gene**

**Collection of DNA samples from trait positive and control individuals**

The disease trait followed in this association study was asthma, a disease involving the leukotriene pathway. The asthmatic population corresponded to 298 individuals that took part in a clinical study for the evaluation of the anti-asthmatic drug zileuton. More than 90 % of these 297 asthmatic individuals had a Caucasian ethnic background. The control population corresponded to 178 individuals from a random US Caucasian population.

**Genotyping of affected and control individuals**

The general strategy to perform the association studies was to individually scan the DNA samples from all individuals in each of the populations described above in order to establish the allele frequencies of the above described biallelic markers in each of these populations.

Allelic frequencies of the above-described biallelic marker alleles in each population were determined by performing microsequencing reactions on amplified fragments obtained by genomic PCR performed on the DNA samples from each individual. Genomic PCR and microsequencing were performed as detailed above in Examples I and 2 using the described PCR and microsequencing primers.

**Haplotype frequency analysis**

None of the single marker alleles showed a significant association with asthma however, significant results were obtained in haplotype studies. Allelic frequencies were useful to check that the markers used in the haplotype studies meet the Hardy-Weinberg proportions (random mating).

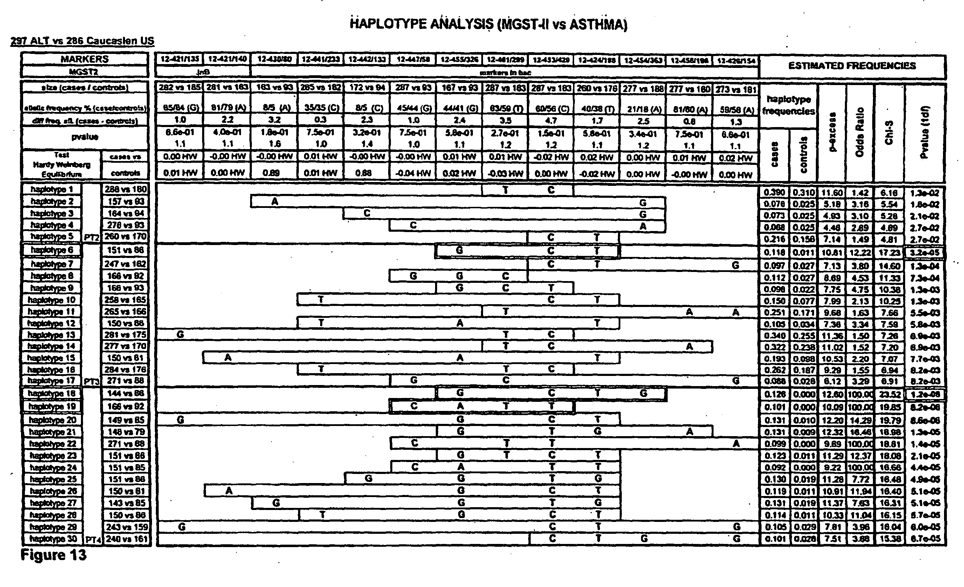

The results of the haplotype analysis using 13 biallelic markers (12-421-135, 12-421-140, 12-430-80, 12-441-233, 12-442-133, 12-447-58, 12-455-326, 12-461-299, 12-453-429, 12-424-198, 12-454-363, 12-458-196 and 12-426-154) are shown in Figure 13. Haplotype analysis for association of MGST-II biallelic markers and asthma was performed by estimating the frequencies of all possible 2, 3 and 4 marker haplotypes in the asthmatic and control populations described above. Haplotype estimations were performed by applying the Expectation-Maximization (EM) algorithm (Excoffier and Slatkin, Mol. Biol. Evol., 12:921-927, 1995), using the EM-HAPLO program (Hawley et al., Am. J. Phys. Anthropol., 18:104, 1994) as described above. Estimated haplotype frequencies in the asthmatic and control population were compared by means of a chi-square statistical test (one degree of freedom).

Figure 13 shows the most significant haplotypes obtained. Haplotype No. 6 consisting of three biallelic markers (12-455-326, 12-453-429 and 12-424-198) had a p-value of $3.2e10^{-5}$ and an odds ratio of 12.22. Estimated haplotype frequencies were 11.8 % in the cases and 1.1 % in the controls. Haplotype No. 18 consisting of four biallelic markers (12-455-326, 12-453-429,12-424-198 and 12-454-363) had a p-value of $1.2e10^{-6}$ and an odds ratio of 100.00. Both haplotypes are related as three out of four biallelic marker alleles (G at 12-455-326, C at 12-453-429 and T and 12-454-363) are common to both haplotypes. Haplotype No. 19 consisting of four biallelic markers (12-447-58, 12-455-326, 12-461-299 and 12-453-429) had a p-value of $8.2e\ 10^{-6}$ and an odds ratio of 100.00. Markers 12-455-326 and 12-453-429 are common in all three significant haplotypes; therefore, they represent preferred markers for the diagnosis of asthma. Haplotypes Nos. 6, 18 and 19 are strongly associated with asthma. Haplotypes Nos. 7-17 and 20-30 also showed very significant association (see Figure 13).

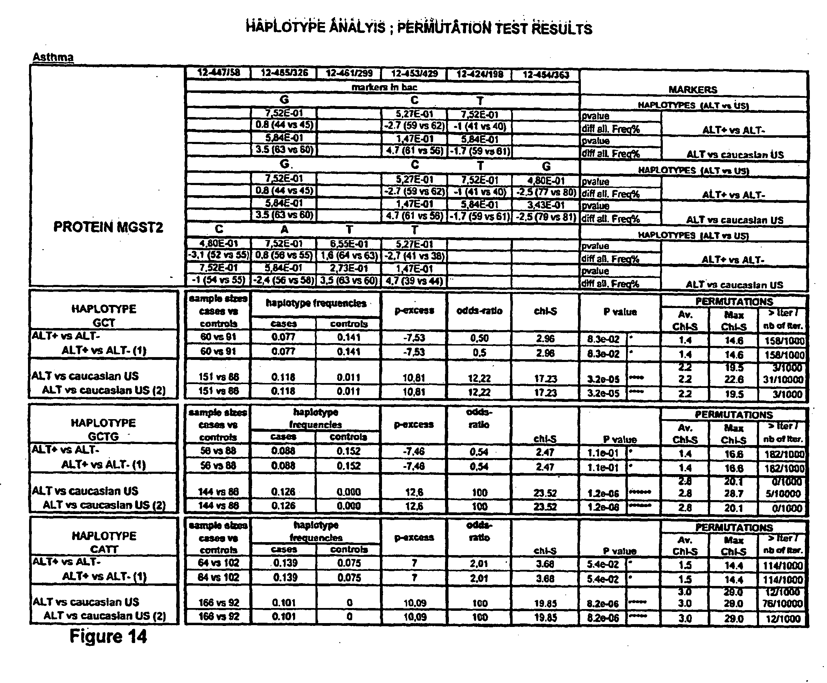

The statistical significance of the results obtained for the haplotype analysis was evaluated by a phenotypic permutation test reiterated 1000 or 10,000. For this computer simulation, data from the asthmatic and control individuals were pooled and randomly allocated to two groups which contained the same number of individuals as the case-control populations used to produce the data summarized in Figure 13. A haplotype analysis was then run on these artificial groups for the 3 markers included in haplotype No. 6 (haplotype GCT), the 4 markers included in haplotype No. 18 (haplotype GCTG) and the 4 markers included in haplotype No. 19 (haplotype CATT), all of which showed a strong association with asthma. This experiment was reiterated 1000 and 10.000 times and the results are shown in Figure 14. These results demonstrate that among 1000 iterations only 3 and among 10.000 iterations only 31 of the obtained haplotypes had a p-value comparable to the one obtained for haplotype No. 6 (haplotype GCT). These results demonstrate that among 1000 iterations 0 and among 10.000 iterations only 5 of the obtained haplotypes had a p-value comparable to the one obtained for haplotype No. 18 (haplotype GCTG). These results further demonstrate that among 1000 iterations only 12 and among 10,000 iterations only 76 of the obtained haplotypes had a p-value comparable to the one obtained for haplotype No. 19 (haplotype CATT). These results clearly validate the statistical significance of the association between the haplotypes shown in Figure 13 and asthma.

## Example 4

## Association Between Side Effects Upon Treatment With the Anti-Asthmatic Drug Zileuton (Zvflo™) and the Biallelic Markers of the MGST-II Gene

### Collection of DNA samples from trait positive and control individuals

The side effect examined in this study was the hepatotoxicity experienced by asthmatic individuals as a result of their treatment with Zileuton as part of a clinical study. Asthmatic individuals were unrelated and more than 90% of the individuals had a Caucasian ethnic background. Hepatotoxicity was monitored by measuring the serum levels of alanine aminotransferase (ALT), which is a sensitive indicator of liver cell damage.

More than 90% of the asthmatic individuals participating in this study did not experience Zileuton-associated ALT increase compared to their ALT levels prior to zileuton intake. As mentioned above, an association study is more informative if the populations considered present extreme phenotypes. Therefore, the asthmatic individuals, which were selected for the side effect positive trait (ALT+), corresponded to 89 individuals that presented at least 3 times the upper limit of normal (ULN) level of ALT. On the other side, the asthmatic individuals that were selected for the side effect negative trait (ALT-) corresponded to 208 individuals that presented less than 1xULN of ALT. ALT+ and ALT-populations corresponded to 4% and 35% respectively of the total asthmatic individuals that participated in this study.

### Genotyping of affected and control individuals

The general strategy to perform the Association studies was to individually scan the DNA samples from all individuals in each of the populations described above in order to establish the allele frequencies of the above described biallelic markers in each of these populations.

Allelic frequencies of the above-described biallelic marker alleles in each population were determined by performing microsequencing reactions on amplified fragments obtained by genomic PCR performed on the DNA samples from each individual. Genomic PCR and microsequencing were performed as detailed above in Examples 1 and 2 using the described PCR and microsequencing primers.

### Haplotype frequency analysis

None of the single marker alleles showed a significant association with hepatotoxicity to zileuton however, significant results were obtained in haplotype studies.

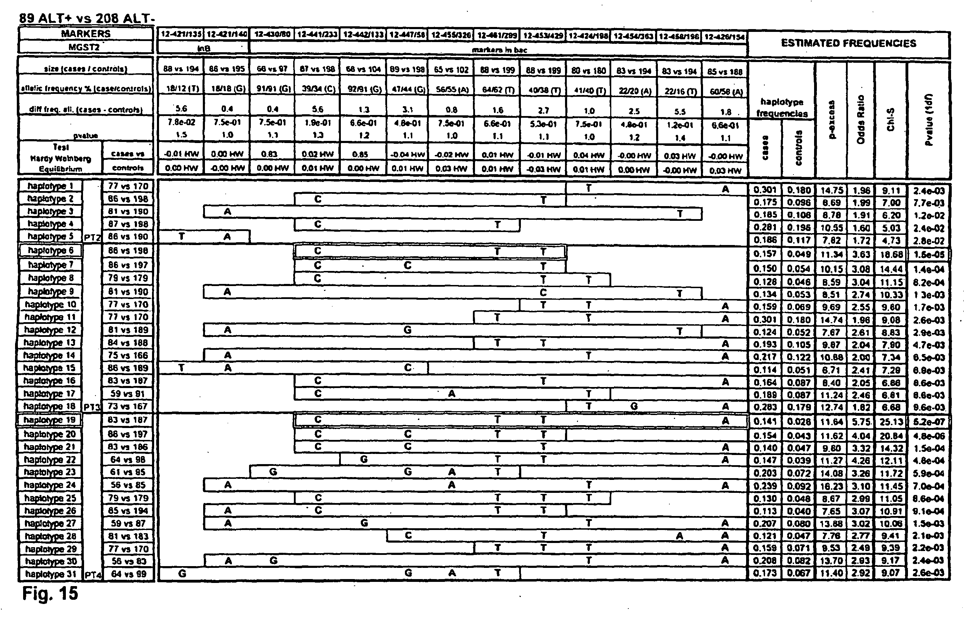

The results of the haplotype analysis using 13 biallelic markers (12-421-135, 12-421-140, 12-430-80, 12-441-233, 12-442-133, 12-447-58, 12-455-326, 12-461-299, 12-453-429, 12-424-198, 12-454-363, 12-458-196 and 12-426-154) are shown in Figure 15. Haplotype analysis for association of MGST-II biallelic markers and asthma was performed by estimating the frequencies of all possible 2, 3 and 4 marker haplotypes in the ALT+ and ALT- populations described above. Haplotype estimations were performed by applying the Expectation-Maximization (EM) algorithm (Excoffier and Slatkin, Mol. Biol. Evol., 12:921-927, 1995), using the EM-HAPLO program (Hawley et al., Am. J. Phys. Anthropol., 18:104, 1994) as described above. Estimated haplotype frequencies in the ALT+ and ALT-populations were compared by means of a chi-square statistical test (one degree of freedom).

Figure 15 shows the most significant haplotypes obtained. Haplotype No. 6 consisting of three biallelic markers (12-441-233, 12-461-299 and 12-453-429) presented a p-value of $1.5\ 10^{-5}$ and an odd-ratio of 3.63. Estimated haplotype frequencies were 15.7 % in the cases and 4.9 % in the controls. Haplotype No. 19 consisting of four biallelic markers (12-441-233, 12-461-299, 12-453-429 and 12-426-154) had a p-value of $5.2\ 10^{-7}$ and an odd ratio of 5.75. Estimated haplotype frequencies were 14.1 % in the cases and 2.8 % in the controls. Both haplotypes showed strong association with elevated serum ALT level upon treatment with zileuton. Both haplotypes are related as three out of four biallelic marker alleles (C at 12-441-233, T at 12-461-299 and T at 12-453-429) are common to both haplotypes. Haplotypes Nos. 7-18 and 20-31 of Figure 15 also showed very significant association.

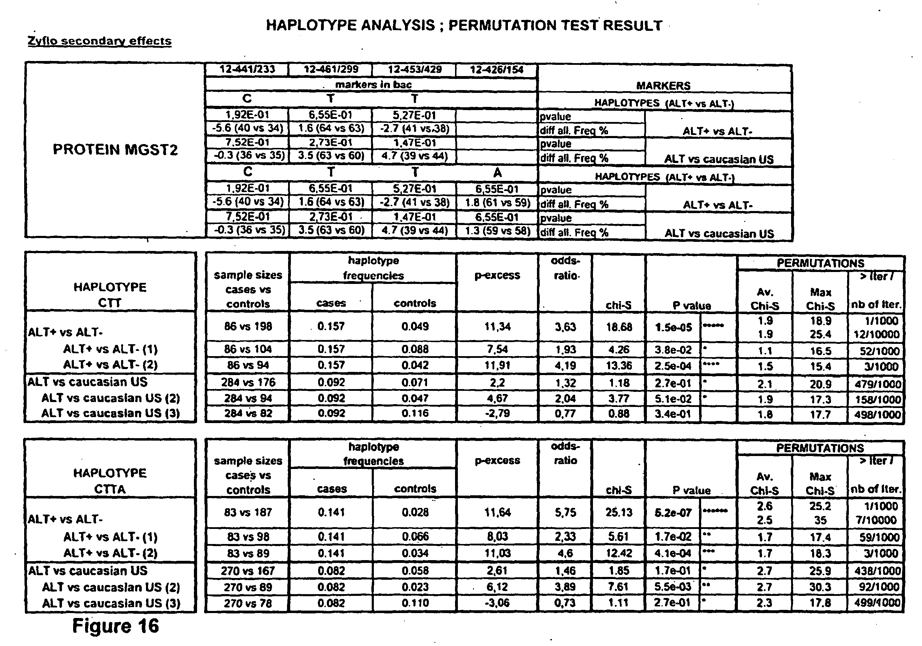

The statistical significance of the results obtained for the haplotype analysis was evaluated by a phenotypic permutation test reiterated 1000 or 10,000 times on a computer. For this computer simulation, data from the ALT+ and ALT- populations were pooled and randomly allocated to two groups which contained the same number of individuals as the ALT+ and ALT- populations used to produce the data summarized in Figure 15. A haplotype analysis was then run on these artificial groups for the 3 markers included in haplotype No. 6 (haplotype CTT) and for the 4 markers included in haplotype No. 19 (haplotype CTTA) which, showed the strongest association with secondary effects to zileuton. This experiment was reiterated 1000 and 10,000 times and the results are shown in Figure 16. These results demonstrate that among 1000 iterations only 1 and among 10,000 iterations only 12 of the obtained haplotypes had a p-value comparable to the one obtained for haplotype No. 6 (haplotype CTT). These results further demonstrate that among 1000 iterations only 1 and among 10,000 iterations only 7 of the obtained haplotypes had a p-value comparable to the one obtained for haplotype No. 19 (haplotype CTTA). These results clearly validate the statistical significance of the association between hepatotoxicity to Zyflo™ and the haplotypes shown in Figure 15.

## Example 5

## Identification of Mutations and of Low Frequency Alleles of the MGST-II Gene

Exons 3-5, the 5'UTR region and the 3'region of the MGST-II gene were screened for mutations by comparing their sequence in individuals exhibiting elevated ALT levels upon treatment with zileuton (ALT+) and in individuals showing normal ALT levels upon treatment with zileuton (ALT-). ALT + and ALT- individuals are further described in Example 4. Intron sequences immediately flanking the exons were also screened.

To identify mutations, fragments of the MGST-II gene were amplified, sequenced and compared in ALT+ and ALT- individuals. DNA samples from each individual were processed separately.

### DNA samples

Individual DNA samples were obtained as described in Example 1.

### Amplification of the MGST-II gene

Amplification primers are described in Table 5 and PCR assays were performed as described in Example 1.

### Sequencing of amplified genomic DNA: identification of mutations and of low frequency polymorphisms

Sequencing of the amplified DNA was carried out on ABI 377 sequencers. The sequences of the amplification products were determined using automated dideoxy terminator sequencing reactions with a dye terminator cycle sequencing protocol. The products of the sequencing reactions were run on sequencing gels and the sequences were determined using gel image analysis (ABI Prism DNA Sequencing Analysis software 2.1.2 version).

The sequence data was further analyzed to detect the presence of mutations and of low frequency alleles. The sequences obtained with 79 ALT+ individuals and 105 ALT-individuals were compared. New polymorphisms/mutations were detected and the genotype of each individual for these markers was determined. Results are shown below:

| Marker ID | Position in MGST-II gene | Least common allele/ mutation | Original allele | Genotype of ALT+ and ALT- individuals |
|---|---|---|---|---|
| 10-286-289 | 5'UTR | G | C | 79 ALT+ C/C<br>104 ALT- C/C<br>1 ALT- GC |

(continued)

| Marker ID | Position in MGST-II gene | Least common allele/ mutation | Original allele | Genotype of ALT+ and ALT- individuals |
|---|---|---|---|---|
| 10-286-345 | 5'UTR | T | A | 65 ALT + A/A<br>12 ALT + A/T<br>2 ALT + T/T<br>82 ALT- A/A<br>19 ALT- A/T<br>3 ALT- T/T |
| 10-286-375 | 5'UTR | G | A | 78 ALT + A/A<br>1 ALT+ A/G<br>104 ALT- A/A |
| 10-523-232 | Exon 3 Exon 3 | T | C | 75 ALT+ C/C<br>100 ALT- C/C<br>1 ALT- C/T |
| 10-289-201 | Exon 4 | C | T | 76 ALT+ T/T<br>2 ALT+ C/T<br>100 ALT- T/T<br>1 ALT- C/T |
| 10-290-37 | Exon 5 | T | C | 78 ALT+ C/C<br>1 ALT+ C/T<br>104 ALT- C/C |
| 10-290-326 | 3' region | A | G | 79 ALT+ G/G<br>103 ALT- G/G<br>1 ALT- A/G |
| 10-290-328 | 3'region | deletion | - | 1 ALT+ deletion<br>78 ALT+ -<br>104 ALT- - |

Eight polymorphisms were identified in the region of the MGST-II gene that was scanned. Three mutations were identified in the 3'UTR region. One mutation in exon 4 causes an amino acid substitution (Tyr→His) at the polypeptide level. A mutation in exon 5 introduces a stop codon into the ORF leading to the expression of a truncated MGST-II polypeptide. These mutations modify the specificity, activity and function of the MGST-II enzyme and therefore represent functional mutations of the MGST-II gene.

**Example 6**

**Preparation of Antibody Compositions to MGST-II Variants**

Preferably antibody compositions, specifically binding the 93-His variant or the SEQ ID No. 489 variant of MGST-II, are prepared.

Substantially pure protein or polypeptide is isolated from transfected or transformed cells containing an expression vector encoding the MGST-II protein or a portion thereof. The concentration of protein in the final preparation is adjusted, for example, by concentration on an Amicon filter device, to the level of a few micrograms per ml. Monoclonal or polyclonal antibodies to the protein can then be prepared as follows:

**Monoclonal Antibody Production by Hybridoma Fusion**

Monoclonal antibody to epitopes in the MGST-II protein or a portion thereof can be prepared from murine hybridomas according to the classical method of Kohler and Milstein (Nature, 256:495. 1975) or derivative methods thereof (see Harlow and Lane, Antibodies A Laboratory Manual, Cold Spring Harbor Laboratory, pp. 53-242, 1988).

Briefly, a mouse is repetitively inoculated with a few micrograms of the MGST-II protein or a portion thereof over a period of a few weeks. The mouse is then sacrificed, and the antibody producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall, E., Meth. Enzymol. 70:419 (1980), and derivative methods thereof. Selected positive clones can be expanded and their monoclonal antibody product harvested for use. Detailed procedures for monoclonal antibody production are described in Davis, L. et al. Basic Methods in Molecular Biology Elsevier, New York. Section 21-2.

**Polyclonal Antibody Production by Immunization**

Polyclonal antiserum containing antibodies to heterogeneous epitopes in the MGST-II protein or a portion thereof can be prepared by immunizing suitable non-human animal with the MGST-II protein or a portion thereof, which can be unmodified or modified to enhance immunogenicity. A suitable non-human animal is preferably a non-human mammal is selected, usually a mouse, rat, rabbit, goat, or horse. Alternatively, a crude preparation which, has been enriched for MGST-II concentration can be used to generate antibodies. Such proteins, fragments or preparations are introduced into the non-human mammal in the presence of an appropriate adjuvant (e.g. aluminum hydroxide, RIBI, etc.) which is known in the art. In addition the protein, fragment or preparation can be pretreated with an agent which will increase antigenicity, such agents are known in the art and include, for example, methylated bovine serum albumin (mBSA), bovine serum albumin (BSA), Hepatitis B surface antigen, and keyhole limpet hemocyanin (KLH). Serum from the immunized animal is collected, treated and tested according to known procedures. If the serum contains polyclonal antibodies to undesired epitopes, the polyclonal antibodies can be purified by immunoaffinity chromatography.

Effective polyclonal antibody production is affected by many factors related both to the antigen and the host species. Also, host animals vary in response to site of inoculations and dose, with both inadequate or excessive doses of antigen resulting in low titer antisera. Small doses (ng level) of antigen administered at multiple intradermal sites appears to be most reliable. Techniques for producing and processing polyclonal antisera are known in the art, see for example, Mayer and Walker (1987). An effective immunization protocol for rabbits can be found in Vaitukaitis, J. et al. J. Clin. Endocrinol. Metab. 33:988-991 (1971).

Booster injections can be given at regular intervals, and antiserum harvested when antibody titer thereof, as determined semi-quantitatively, for example, by double immunodiffusion in agar against known concentrations of the antigen, begins to fall. (See, for example, Ouchterlony, O. et al., Chap. 19 in: Handbook of Experimental Immunology D. Wier (ed) Blackwell,1973). Plateau concentration of antibody is usually in the range of 0.1 to 0.2 mg/ml of serum. Affinity of the antisera for the antigen is determined by preparing competitive binding curves, as described, for example, by Fisher, D., (Chap. 42: Manual of Clinical Immunology, 2d Ed. Rose and Friedman, Eds., Amer. Soc. For Microbiol., Washington, D.C., 1980).

Antibody preparations prepared according to either the monoclonal or the polyclonal protocol are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body.

It should be noted that in the accompanying Sequence Listing, all instances of the symbol "n" in the nucleic acid sequences mean that the nucleotide can be adenine, guanine, cytosine or thymine.

In some instances, the polymorphic bases of the biallelic markers alter the identity of amino acids in the encoded polypeptide. This is indicated in the accompanying Sequence Listing by use of the feature VARIANT, placement of a Xaa at the position of the polymorphic amino acid, and definition of Xaa as the two alternative amino acids. For example, if one allele of a biallelic marker is the codon CAC. which encodes histidine, while the other allele of the biallelic marker is CAA, which encodes glutamine, the Sequence Listing for the encoded polypeptide will contain an Xaa at the location of the polymorphic amino acid. In this instance, Xaa would be defined as being histidine or glutamine.

In other instances, Xaa may indicate an amino acid whose identity is unknown because of nucleotide sequence ambiguity. In this instance, the feature UNSURE is used, Xaa is placed at the position of the unknown amino acid, and Xaa is defined as being any of the 20 amino acids or a limited number of amino acids suggested by the genetic code.

SEQUENCE LISTING FREE TEXT

The following free text appears in the accompanying sequence listing:

biallelic
marker
drug metabolism
Homo Sapiens
allele
polymorphic base
misc_binding
potential
complement
potential complement
primer_bind
upstream amplification primer
upstream amplification primer, complement
downstream amplification primer
potential probe
misc_feature
5' regulatory region
exon
5'UTR
CDS
3'UTR
polyA_signal
PRT
VARIANT
Stop
Artificial Sequence
sequencing oligonucleotide PrimerPU
sequencing oligonucleotide PrimerRP

## Claims

1. A method of genotyping comprising the step of determining the identity of a nucleotide at a MGST-II-releted biallelic marker in a biological sample, wherein said biallelic marker is selected from the MGST-II-related biallelic markers shown in the table below:

| Biallelic Marker | Alternative alleles | Position |
|---|---|---|
| 12-424-198 | G/T | Nucleotide 501 in SEQ ID NO: 3 |
| 12-426-154 | A/G | Nucleotide 461 in SEQ ID NO: 5 |
| 12-441-233 | G/A | Nucleotide 501 in SEQ ID NO: 9 |
| 12-447-58 | G/C | Nucleotide 501 in SEQ ID NO: 12 |
| 12-453-429 | C/T | Nucleotide 501 in SEQ ID NO: 13 |
| 12-454-363 | A/G | Nucleotide 501 in SEQ ID NO: 14 |
| 12-455-326 | T/C | Nucleotide 501 in SEQ ID NO: 15 |
| 12-461-299 | C/T | Nucleotide 501 in SEQ ID NO: 25 |

and wherein the biallelic marker is one which is included in an asthma or zileuton hepatoxicity associated haplotype.

2. A method according to claim 1, wherein said biological sample is derived from a single subject.

3. A method according to claim 2, wherein the identity of the nucleotides at said biallelic marker is determined for both copies of said biallelic marker present in said subject's genome.

4. A method according to claim 1, wherein said biological sample is derived from multiple subjects.

5. A method according to claim 1, further comprising amplifying a portion of said sequence comprising the biallelic marker prior to said determining step.

6. A method according to claim 5, wherein said amplifying is performed by PCR.

7. A method according to claim 1, wherein said determining is performed by a hybridization assay.

8. A method according to claim 1, wherein said determining is performed by a sequencing assay.

9. A method according to claim 1, wherein said determining is performed by a microsequencing assay.

10. A method according to claim 1, wherein said determining is performed by an enzyme-based mismatch detection assay.

11. Use of a polynucleotide in a hybridization assay for determining the identity of a nucleotide at a MGST-II-related biallelic marker selected from the MGST-II-related biallelic markers shown in the table in claim 1.

12. Use of a polynucleotide in a sequencing assay for determining the identity of a nucleotide at a MGST-II-related biallelic marker selected from the MGST-II-related biallelic markers shown in the table in claim 1.

**13.** Use of a polynucleotide in an allele specific amplification assay for determining the identity of a nucleotide at a MGST-II-related biallelic marker selected from the MGST-II-related biallelic markers shown in the table in claim 1.

**14.** Use of a polynucleotide in amplifying a segment of nucleotides comprising a MGST-II-related biallelic marker selected from the MGST-II-related biallelic markers shown in the table in claim 1.

**15.** Use according to any one of claims 11 to 14, wherein said polynucleotide comprises a fragment of at least 12 nucleotides of any one of SEQ ID NOs. 3, 5, 9, 12-15 and 25.

**16.** A method of determining whether an individual is at risk of developing asthma, or whether said individual suffers from asthma, comprising the steps of:

a) genotyping said individual for at least one MGST-II-related biallelic marker by a method according to any one of claims 1 to 10; and
b) correlating the result of step a) with a risk of developing asthma.

**17.** A method according to claim 16, wherein said at least one MGST-II-related biallelic marker is a set of MGST-II-related biallelic markers selected from:

a) 12-455-326. 12-453-429 and 12-424-198;
b) 12-455-326, 12-453-429, 12-424-198 and 12-454-363; and
c) 12-447-58, 12-455-326, 12-461-299 and 12-453-429.

**18.** A method according to claim 17, wherein the presence of a:

a) G at MGST-II-related biallelic marker 12-455-326;
b) C at MGST-II-related biallelic marker 12-453-429; and
c) T at MGST-II-related biallelic marker 12-424-198;

indicates that said individual is at risk of developing asthma, or suffers from asthma.

**19.** A method according to claim 17, wherein the presence of a:

a) G at MGST-II-related biallelic marker 12-455-326;
b) C at MGST-II-related biallelic marker 12-453-429;
c) T at MGST-II-related biallelic marker 12-424-198; and
d) G at MGST-II-related biallelic marker 12-454-363;

indicates that said individual is at risk of developing asthma, or suffers from asthma.

**20.** A method according to claim 17, wherein the presence of a:

a) C at MGST-II-related biallelic marker 12-447-58;
b) A at MGST-II-related biallelic marker 12-455-326;
c) T at MGST-II-related biallelic marker 12-461-299; and
d) T at MGST-II-related biallelic marker 12-453-429; indicates that said individual is at risk of developing asthma, or suffers from asthma.

**21.** A method of determining whether an individual is at risk of developing hepatoxicity upon treatment with zileuton, comprising:

a) genotyping said individual for at least one MGST-II-related biallelic marker by a method according to any one of claims 1 to 10; and
b) correlating the result of step a) with a risk of developing hepatoxicity upon treatment with zileuton.

**22.** A method according to claim 21, wherein said at least one MGST-II-related biallelic marker is a set of MGST-II-related biallelic markers selected from:

a) 12-441-233, 12-461-299 and 12-453-429; and
b) 12-441-233, 12-461-299, 12-453-429 and 12-426-154.

**23.** A method according to claim 22, wherein the presence of a:

a) C at MGST-II-related bialielic marker 12-441-233;
b) T at MGST-II-related biallelic marker 12-461-299; and
c) T at MGST-II-related biallelic marker 12-453-429;

indicates that said individual is at risk of developing hepatoxicity upon treatment with zileuton.

**24.** A method according to claim 22, wherein the presence of a:

a) C at MGST-II-related biallelic marker 12-441-233;
b) T at MGST-II-related biallelic marker 12-461-299;
c) T at MGST-II-related biallelic marker 12-453-429; and
d) A at MGST-II-related biallelic marker 12-426-154;

indicates that said individual is at risk of developing hepatoxicity upon treatment with zileuton.

**25.** An isolated, purified, or recombinant polynucleotide consisting of a contiguous span of 18-35 nucleotides of a sequence selected from the sequences of SEQ ID Nos. 3, 5, 9, 12-15 and 25 and the complements thereof, wherein said span includes a MGST-II-related biallelic marker selected from the MGST-II-related biallelic markers shown in the table in claim 1 in said sequence and wherein said biallelic marker is within 4 nucleotide of the centre of said polynucleotide.

**26.** A polynucleotide according to claim 25, wherein said polynucleotide consists of said contiguous span and said contiguous span is 25 nucleotides in length and said biallelic marker is at the center of said polynucleotide.

**27.** An isolated, purified or recombinant polynucleotide consisting of a contiguous span of at least 12 nucleotides of a sequence selected from the group consisting of SEQ ID NOs: 3, 5, 9, 12-15 and 25 and the complements thereof, wherein said span includes a MGST-II-related biallelic marker of the table set forth in claim 1 in said sequence, wherein:

(i) the 3' end of said contiguous span is located at the 3' end of said polynucleotide; and
(ii) said biallelic marker is present at the 3' end of said polynucleotide.

**28.** An isolated, purified or recombinant polynucleotide consisting of a contiguous span of 8-50 nucleotides of a sequence selected from the group consisting of SEQ ID NOs: 3, 5, 9, 12-15 and 25 and the complements thereof, in said sequence, wherein:

(i) the 3' end of said contiguous span is located at the 3' end of said polynucleotide; and
(ii) the 3' end of said polynucleotide is located 1 nucleotide upstream of a MGST-II-related biallelic marker of the table set forth in claim 1.

**29.** A polynucleotide according to any one of claims 25 to 28, wherein said contiguous span comprises at least 15, 20, or 25 contiguous nucleotides in said sequence.

**30.** A polynucleotide according to any one of claims 25 to 29 attached to a solid support.

**31.** A polynucleotide according to any one of claims 25 to 30 further comprising a label.

**32.** A diagnostic kit comprising one or more polynucleotide(s) according to any one of claims 25-31 together with:

(i) at least one reagent; and
(ii) instructions for genotyping a test subject by determining the identity of a nucleotide at a MGST-II-related biallelic marker selected from the MGST-II-related biallelic markers shown in the table in claim 1.

**Patentansprüche**

1. Verfahren zur Genotypisierung, den Schritt des Bestimmens der Identität eines Nucleotids an einem MGST-II-verwandten biallelen Marker in einer biologischen Probe umfassend, worin der biallele Marker aus den in der nachstehenden Tabelle angeführten MGST-II-verwandten biallelen Markern ausgewählt ist:

| bialleler Marker | alternative Allele | Position |
|---|---|---|
| 12-424-198 | G/T | Nucleotid 501 in Seq.-ID Nr. 3 |
| 12-426-154 | A/G | Nucleotid 461 in Seq.-ID Nr. 5 |
| 12-441-233 | G/A | Nucleotid 501 in Seq.-ID Nr. 9 |
| 12-447-58 | G/C | Nucleotid 501 in Seq.-ID Nr. 12 |
| 12-453-429 | C/T | Nucleotid 501 in Seq.-ID Nr. 13 |
| 12-454-363 | A/G | Nucleotid 501 in Seq.-ID Nr. 14 |
| 12-455-326 | T/C | Nucleotid 501 in Seq.-ID Nr. 15 |
| 12-461-299 | C/T | Nucleotid 501 in Seq.-ID Nr. 25 |

und worin der biallele Marker ein solcher ist, der in einem Asthma- oder Zileuton-Hepatotoxizitäts-assoziierten Haplotyp enthalten ist.

2. Verfahren nach Anspruch 1, worin die biologische Probe von einem einzigen Individuum stammt.

3. Verfahren nach Anspruch 2, worin die Identität der Nucleotide am biallelen Marker für beide Kopien des biallelen Markers bestimmt wird, die im Genom des Individuums vorhanden sind.

4. Verfahren nach Anspruch 1, worin die biologische Probe von mehreren Individuen stammt.

5. Verfahren nach Anspruch 1, weiters das Amplifizieren eines Abschnitts der Sequenz, welche den biallelen Marker umfasst, vor dem Bestimmungsschritt umfassend.

6. Verfahren nach Anspruch 5, worin das Amplifizieren durch PCR erfolgt.

7. Verfahren nach Anspruch 1, worin das Bestimmen durch einen Hybridisierungstest erfolgt.

8. Verfahren nach Anspruch 1, worin das Bestimmen durch einen Sequenzierungstest erfolgt.

9. Verfahren nach Anspruch 1, worin das Bestimmen durch einen Mikrosequenzierungstest erfolgt.

10. Verfahren nach Anspruch 1, worin das Bestimmen durch einen enzymbasierten Fehlpaarungsdetektionstest erfolgt.

11. Verwendung eines Polynucleotids in einem Hybridisierungstest zur Bestimmung der Identität eines Nucleotids an einem MGST-II-verwandten biallelen Marker, der aus den in der Tabelle aus Anspruch 1 angeführten MGST-II-verwandten biallelen Markern ausgewählt ist.

12. Verwendung eines Polynucleotids in einem Sequenzierungstest zur Bestimmung der Identität eines Nucleotids an einem MGST-II-verwandten biallelen Marker, der aus den in der Tabelle aus Anspruch 1 angeführten MGST-II-verwandten biallelen Markern ausgewählt ist.

13. Verwendung eines Polynucleotids in einem allelspezifischen Amplifikationstest zur Bestimmung der Identität eines Nucleotids an einem MGST-II-verwandten biallelen Marker, der aus den in der Tabelle aus Anspruch 1 angeführten MGST-II-verwandten biallelen Markern ausgewählt ist.

**14.** Verwendung eines Polynucleotids bei der Amplifikation eines Segments von Nucleotiden, die einen MGST-II-verwandten biallelen Marker umfassen, der aus den in der Tabelle aus Anspruch 1 angeführten MGST-II-verwandten biallelen Markern ausgewählt ist.

**15.** Verwendung nach einem der Ansprüche 11 bis 14, worin das Polynucleotid ein Fragment aus zumindest 12 Nucleotiden mit einer der Seq.-ID Nr. 3, 5, 9, 12-15 oder 25 umfasst.

**16.** Verfahren zur Bestimmung, ob ein Individuum ein Risiko hat, Asthma zu entwickeln, oder ob das Individuum an Asthma leidet, folgende Schritte umfassend:

a) das Genotypisieren des Individuums für zumindest einen MGST-II-verwandten biallelen Marker durch ein Verfahren nach einem der Ansprüche 1 bis 10; und
b) das Korrelieren der Ergebnisse aus Schritt a) mit dem Risiko, Asthma zu entwickeln.

**17.** Verfahren nach Anspruch 16, worin der zumindest eine MGST-II-verwandte biallele Marker eine Gruppe von MGST-II-verwandten biallelen Markern ist, die ausgewählt sind aus:

a) 12-455-326, 12-453-429 und 12-424-198;
b) 12-455-326, 12-453-429, 12-424-198 und 12-454-363; und
c) 12-447-58, 12-455-326, 12-461-299 und 12-453-429.

**18.** Verfahren nach Anspruch 17, worin die Gegenwart eines:

a) G am MGST-II-verwandten biallelen Marker 12-455-326;
b) C am MGST-II-verwandten biallelen Marker 12-453-429; und
c) T am MGST-II-verwandten biallelen Marker 12-424-198;

anzeigt, dass das Individuum ein Risiko hat, Asthma zu entwickeln, oder an Asthma leidet.

**19.** Verfahren nach Anspruch 17, worin die Gegenwart eines:

a) G am MGST-II-verwandten biallelen Marker 12-455-326;
b) C am MGST-II-verwandten biallelen Marker 12-453-429;
c) T am MGST-II-verwandten biallelen Marker 12-424-198; und
d) G am MGST-II-verwandten biallelen Marker 12-454-363;

anzeigt, dass das Individuum ein Risiko hat, Asthma zu entwickeln, oder an Asthma leidet.

**20.** Verfahren nach Anspruch 17, worin die Gegenwart eines:

a) G am MGST-II-verwandten biallelen Marker 12-447-58;
b) A am MGST-II-verwandten biallelen Marker 12-455-326;
c) T am MGST-II-verwandten biallelen Marker 12-461-299; und
d) T am MGST-II-verwandten biallelen Marker 12-453-429;

anzeigt, dass das Individuum ein Risiko hat, Asthma zu entwickeln, oder an Asthma leidet.

**21.** Verfahren zur Bestimmung, ob ein Individuum ein Risiko hat, Hepatotoxizität bei Behandlung mit Zileuton zu entwickeln, umfassend:

a) das Genotypisieren des Individuums für zumindest einen MGST-II-verwandten biallelen Marker durch ein Verfahren nach einem der Ansprüche 1 bis 10; und
b) das Korrelieren der Ergebnisse aus Schritt a) mit dem Risiko, Hepatotoxizität bei Behandlung mit Zileuton zu entwickeln.

**22.** Verfahren nach Anspruch 21, worin der zumindest eine MGST-II-verwandte biallele Marker eine Gruppe von MGST-II-verwandten biallelen Markern ist, die ausgewählt sind aus:

a) 12-441-233, 12-461-299 und 12-453-429; und
b) 12-441-233, 12-461-299, 12-453-429 und 12-426-154.

**23.** Verfahren nach Anspruch 22, worin die Gegenwart eines

a) C am MGST-II-verwandten biallelen Marker 12-441-233;
b) T am MGST-II-verwandten biallelen Marker 12-461-299; und
c) T am MGST-II-verwandten biallelen Marker 12-453-429;

anzeigt, dass das Individuum ein Risiko hat, Hepatotoxizität bei Behandlung mit Zileuton zu entwickeln.

**24.** Verfahren nach Anspruch 22, worin die Gegenwart eines

a) C am MGST-II-verwandten biallelen Marker 12-441-233;
b) T am MGST-II-verwandten biallelen Marker 12-461-299;
c) T am MGST-II-verwandten biallelen Marker 12-453-429; und
d) A am MGST-II-verwandten biallelen Marker 12-426-154;

anzeigt, dass das Individuum ein Risiko hat, Hepatotoxizität bei Behandlung mit Zileuton zu entwickeln.

**25.** Isoliertes, gereinigtes oder rekombinantes Polynucleotid, das aus einer zusammenhängende Kette aus 18-35 Nucleotiden einer Sequenz besteht, die aus den Sequenzen mit den Seq.-ID Nr. 3, 5, 9, 12-15 und 25 und den Komplementen davon ausgewählt ist, worin die Kette einen MGST-II-verwandten biallelen Marker, der aus den in der Tabelle aus Anspruch 1 angeführten MGST-II-verwandten biallelen Markern ausgewählt ist, in der Sequenz enthält und worin der biallele Marker innerhalb einer Entfernung von 4 Nucleotiden von der Mitte des Polynucleotids liegt.

**26.** Polynucleotid nach Anspruch 25, worin das Polynucleotid aus der zusammenhängenden Kette besteht, wobei die zusammenhängende Kette 25 Nucleotide lang ist und der biallele Marker in der Mitte des Polynucleotids liegt.

**27.** Isoliertes, gereinigtes oder rekombinantes Polynucleotid, das aus einer zusammenhängenden Kette aus zumindest 12 Nucleotiden einer Sequenz besteht, die aus der aus Seq.-ID Nr. 3, 5, 9, 12-15 und 25 und den Komplementen davon bestehenden Gruppe ausgewählt ist, worin die Kette einen in der Tabelle aus Anspruch 1 angeführten MGST-II-verwandten biallelen Marker in der Sequenz enthält, worin:

(i) sich das 3'-Ende der zusammenhängenden Kette am 3'-Ende des Polynucleotids befindet; und
(ii) der biallele Marker am 3'-Ende des Polynucleotids vorhanden ist.

**28.** Isoliertes, gereinigtes oder rekombinantes Polynucleotid, das aus einer zusammenhängenden Kette aus 8-50 Nucleotiden einer Sequenz besteht, die aus der aus Seq.-ID Nr. 3, 5, 9, 12-15 und 25 und den Komplementen davon bestehenden Gruppe ausgewählt ist, worin:

(i) sich das 3'-Ende der zusammenhängenden Kette am 3'-Ende des Polynucleotids befindet; und
(ii) sich das 3'-Ende des Polynucleotids 1 Nucleotid stromauf von einem MGST-II-verwandten biallelen Marker aus der in Anspruch 1 dargestellten Tabelle befindet.

**29.** Polynucleotid nach einem der Ansprüche 25 bis 28, worin die zusammenhängende Kette zumindest 15, 20 oder 25 zusammenhängende Nucleotide in der Sequenz umfasst.

**30.** Polynucleotid nach einem der Ansprüche 25 bis 29, das an einen festen Träger gebunden ist.

**31.** Polynucleotid nach einem der Ansprüche 25 bis 30, weiters eine Markierung umfassend.

**32.** Diagnoseset, umfassend ein oder mehrere Polynucleotide nach einem der Ansprüche 25-31 zusammen mit

(i) zumindest einem Reagens; und
(ii) Anleitungen zur Genotypisierung eines Testindividuums durch Bestimmung der Identität eines Nucleotids an einem MGST-II-verwandten biallelen Marker, der aus den in der Tabelle aus Anspruch 1 angeführten MGST-II-verwandten biallelen Markern ausgewählt ist.

## Revendications

1. Méthode de génotypage comprenant l'étape de déterminer l'identité d'un nucléotide sur un marqueur biallélique en rapport avec MGST-II dans un échantillon biologique, où ledit marqueur bialléliques est sélectionné parmi les marqueurs biallélique en rapport avec MGST-II qui sont montrés au tableau ci-dessous :

| Marqueur biallélique | Autres allèles | Position |
|---|---|---|
| 12-424-198 | G/T | Nucléotide 501 dans SEQ ID NO:3 |
| 12-426-154 | A/G | Nucléotide 461 dans SEQ ID NO:5 |
| 12-441-233 | G/A | Nucléotide 501 dans SEQ ID NO:9 |
| 12-447-58 | G/C | Nucléotide 501 dans SEQ ID NO:12 |
| 12-453-429 | C/T | Nucléotide 501 dans SEQ ID NO:13 |
| 12-454-363 | A/G | Nucléotide 501 dans SEQ ID NO:14 |
| 12-455-326 | T/C | Nucléotide 501 dans SEQ ID NO:15 |
| 12-461-299 | C/T | Nucléotide 501 dans SEQ ID NO:25 |

et où le marqueur biallélique en est un qui est incorporé dans un haplotype associé à l'hépatoxicité du zileuton ou l'asthme.

2. Méthode selon la revendication 1, où ledit échantillon biologique est dérivé d'un seul sujet.

3. Méthode selon la revendication 2, où l'identité des nucléotides audit marqueur biallélique est déterminée à partir des deux copies dudit marqueur biallélique présentes dans le génome dudit sujet.

4. Méthode selon la revendication 1, où ledit échantillon biologique est dérivé de sujets multiples.

5. Méthode selon la revendication 1, comprenant de plus l'amplification d'une portion de ladite séquence comprenant le marqueur biallélique avant ladite étape de détermination.

6. Méthode selon la revendication 5, où ladite amplification est accomplie par PCR.

7. Méthode selon la revendication 1, où ladite détermination est accomplie par un essai d'hybridation.

8. Méthode selon la revendication 1, où ladite détermination est accomplie par un essai de séquençage.

9. Méthode selon la revendication 1, où ladite détermination est accomplie par un essai de microséquençage.

10. Méthode selon la revendication 1, où ladite détermination est accomplie par un essai de détection de défaut d'appariement à base d'enzymes.

11. Utilisation d'un polynucléotide dans un essai d'hybridation pour déterminer l'identité d'un nucléotide sur un marqueur biallélique en rapport avec MGST-II sélectionné parmi les marqueurs bialléliques en rapport avec MGST-II montrés au tableau de la revendication 1.

12. Utilisation d'un polynucléotide dans un essai de séquençage pour déterminer l'identité d'un nucléotide sur un marqueur biallélique en rapport avec MGST-II sélectionné parmi des marqueurs bialléliques en rapport avec MGST-II montrés au tableau de la revendication 1.

13. Utilisation d'un polynucléotide dans un essai d'amplification spécifique de l'allèle pour déterminer l'identité d'un nucléotide sur un marqueur biallélique en rapport avec MGST-II sélectionné parmi les marqueurs bialléliques en rapport avec MGST-II montrés au tableau de la revendication 1.

**14.** Utilisation d'un polynucléotide dans l'amplification d'un segment de nucléotides comprenant un marqueur biallélique en rapport avec MGST-II sélectionné parmi les marqueurs bialléliques en rapport avec MGST-II montrés au tableau de la revendication 1.

**15.** Utilisation selon l'une quelconque des revendications 11 à 14, où ledit polynucléotide comprend un fragment d'au moins 12 nucléotides de l'une quelconque des séquences N° 3, 5, 9, 12-15 et 25.

**16.** Méthode pour déterminer si un individu risque de développer de l'asthme ou bien si ledit individu souffre d'asthme comprenant les étapes de :

a) génotyper ledit individu pour au moins un marqueur biallélique en rapport avec MGST-II par une méthode selon l'une quelconque des revendications 1 à 10 ;
b) mettre en corrélation le résultat de l'étape a) avec un risque de développer de l'asthme.

**17.** Méthode selon la revendication 16, où ledit au moins un marqueur biallélique en rapport avec MGST-II est un groupe de marqueurs bialléliques en rapport avec MGST-II sélectionnés parmi :

a) 12-455-326, 12-453-429 et 12-424-198 ;
b) 12-455-326, 12-453-429, 12-424-198 et 12-454-363 ; et
c) 12-447-58, 12-455-326, 12-461-299 et 12-453-429.

**18.** Méthode selon la revendication 17, où la présence d'un :

a) G au marqueur biallélique en rapport avec MGST-II 12-455-326 ;
b) C au marqueur biallélique en rapport avec MGST-II 12-453-429 ; et
c) T au marqueur biallélique en rapport avec MGST-II 12-424-198

indique que ledit individu risque de développer de l'asthme ou bien souffre d'asthme.

**19.** Méthode selon la revendication 17, où la présence d'un :

a) G au marqueur biallélique en rapport avec MGST-II 12-455-326 ;
b) C au marqueur biallélique en rapport avec MGST-II 12-453-429 ;
c) T au marqueur biallélique en rapport avec MGST-II 12-424-198 ; et
d) G au marqueur biallélique en rapport avec MGST-II 12-454-363 ;

indique que ledit individu risque de développer de l'asthme ou bien souffre d'asthme.

**20.** Méthode selon la revendication 17, où la présence d'un :

a) C au marqueur biallélique en rapport avec MGST-II 12-447-58 ;
b) A au marqueur biallélique en rapport avec MGST-II 12-455-326 ;
c) T au marqueur biallélique en rapport avec MGST-II 12-461-299 ; et
d) T au marqueur bialléligue en rapport avec MGST-II 12-453-429 ;

indique que ledit individu risque de développer de l'asthme ou bien souffre d'asthme.

**21.** Méthode pour déterminer si un individu risque de développer une hépatotoxicité lors d'un traitement avec du zileuton, consistant à :

a) génotyper ledit individu pour au moins un marqueur biallélique en rapport avec MGST-II par une méthode selon l'une quelconque des revendications 1 à 10 ; et
b) mettre en corrélation le résultat d'une étape a) avec un risque de développer une hépatotoxicité lors d'un traitement avec du zileuton.

**22.** Méthode selon la revendication 21, où ledit au moins un marqueur biallélique en rapport avec MGST-II est un groupe de marqueurs bialléliques en rapport avec MGST-II sélectionnés parmi :

a) 12-441-233, 12-461-299 et 12-453-429 ; et
b) 12-441-233, 12-461-299, 12-453-429 et 12-426-154.

**23.** Méthode selon la revendication 22, où la présence d'un :

a) C au marqueur biallélique en rapport avec MGST-II 12-441-233 ;
b) T au marqueur biallélique en rapport avec MGST-II 12-461-299 ; et
c) T au marqueur biallélique en rapport avec MGST-II 12-453-429 ;

indique que ledit individu risque de développer une hépatotoxicité lors d'un traitement avec du zileuton.

**24.** Méthode selon la revendication 22, où la présence d'un :

a) C au marqueur biallélique en rapport avec MGST-II 12-441-233 ;
b) T au marqueur biallélique en rapport avec MGST-II 12-461-299 ;
c) T au marqueur biallélique en rapport avec MGST-II 12-453-429 ; et
d) A au marqueur biallélique en rapport avec MGST-II 12-426-154 ;

indique que ledit individu risque de développer une hépatotoxicité lors d'un traitement avec du zileuton.

**25.** Polynucléotide isolé, purifié ou recombinant consistant en un tronçon contigu de 18-35 nucléotides d'une séquence sélectionnée parmi les séquences de SEQ ID No 3, 5, 9, 12-15 et 25 et leurs compléments, où ledit tronçon contient un marqueur biallélique en rapport avec MGST-II sélectionné parmi des marqueurs bialléliques en rapport avec MGST-II montrés au tableau de la revendciation 1 dans ladite séquence et où ledit marqueur biallélique est à 4 nucléotides du centre dudit polynucléotide.

**26.** Polynucléotide selon la revendication 25, où ledit polynucléotide consiste en ledit tronçon contigu et ledit tronçon contigu a 25 nucléotides de long et ledit marqueur biallélique est au centre dudit polynucléotide.

**27.** Polynucléotide isolé, purifié ou recombinant consistant en un tronçon contigu d'au moins 12 nucléotides d'une séquence sélectionnée dans le groupe consistant en SEQ ID N° 3, 5, 9, 12-15 et 25 et leurs compléments, où ledit tronçon contient un marqueur biallélique en rapport avec MGST-II du tableau indiqué à la revendication 1 dans ladite séquence, où :

(i) l'extrémité 3' dudit tronçon contigu est placée à l'extrémité 3' dudit polynucléotide ; et
(ii) ledit marqueur biallélique est présent à l'extrémité 3' dudit polynucléotide.

**28.** Polynucléotide isolé, purifié ou recombinant consistant en un tronçon contigu de 8-50 nucléotides d'une séquence sélectionnée dans le groupe consistant en SEQ ID NO 3, 5, 9, 12-15 et 25 et leurs compléments dans ladite séquence, où

(i) l'extrémité 3' dudit tronçon contigu est placée à l'extrémité 3' dudit polynucléotide ; et
(ii) l'extrémité 3' dudit polynucléotide est placée à 1 nucléotide en amont d'un marqueur biallélique en rapport avec MGST-II du tableau indiqué à la revendication 1.

**29.** Polynucléotide selon l'une quelconque des revendications 25 à 28, où ledit tronçon contigu comprend au moins 15, 20 ou 25 nucléotides contigus dans ladite séquence.

**30.** Polynucléotide selon l'une quelconque des revendications 25 à 29 attaché à un support solide.

**31.** Polynucléotide selon l'une quelconque des revendications 25 à 30 comprenant de plus un marqueur.

**32.** Kit de diagnostic comprenant un ou plusieurs polynucléotides selon l'une quelconque des revendications 25-31 en même temps que :

(i) au moins un réactif ; et

(ii) des instructions pour le génotypage d'un sujet de test en déterminant l'identité d'un nucléotide sur un marqueur biallélique en rapport avec MGST-II sélectionné parmi les marqueurs bialléliques en rapport avec MGST-II montrés au tableau de la revendication 1.

**Fig. 1**

| GENE | BIALLELIC MARKER ID | SEQ ID NO. | BIALLELIC MARKER POSITION IN SEQ ID NO. | VALIDATION MICRO-SEQUENCING | GENOTYPING LEAST COMMON ALLELE FREQUENCY % | |
|---|---|---|---|---|---|---|
| MGST2 | 12-421-140 | 1 | 501 | N | | |
| MGST2 | 12-424-192 | 2 | 501 | N | | |
| MGST2 | 12-424-198 | 3 | 501 | N | | |
| MGST2 | 12-425-57 | 4 | 501 | N | | |
| MGST2 | 12-426-154 | 5 | 461 | N | | |
| MGST2 | 12-429-198 | 6 | 501 | N | | |
| MGST2 | 12-430-80 | 7 | 501 | Y | T | 5.38 |
| MGST2 | 12-433-215 | 8 | 501 | N | | |
| MGST2 | 12-441-233 | 9 | 501 | Y | G | 34.48 |
| MGST2 | 12-441-343 | 10 | 501 | N | | |
| MGST2 | 12-442-221 | 11 | 501 | N | | |
| MGST2 | 12-447-58 | 12 | 501 | Y | G | 34.27 |
| MGST2 | 12-453-429 | 13 | 501 | Y | T | 42.55 |
| MGST2 | 12-454-363 | 14 | 501 | N | | |
| MGST2 | 12-455-326 | 15 | 501 | Y | C | 41.94 |
| MGST2 | 12-455-383 | 16 | 501 | N | | |
| MGST2 | 12-456-269 | 17 | 501 | N | | |
| MGST2 | 12-456-380 | 18 | 501 | N | | |
| MGST2 | 12-457-204 | 19 | 501 | N | | |
| MGST2 | 12-457-206 | 20 | 501 | N | | |
| MGST2 | 12-458-196 | 21 | 501 | N | | |
| MGST2 | 12-458-438 | 22 | 501 | N | | |
| MGST2 | 12-460-274 | 23 | 501 | N | | |
| MGST2 | 12-461-124 | 24 | 501 | N | | |
| MGST2 | 12-461-299 | 25 | 501 | Y | C | 42.39 |
| MGST2 | 12-461-465 | 26 | 501 | N | | |
| MGST2 | 12-462-280 | 27 | 501 | N | | |
| MGST2 | 12-464-66 | 28 | 501 | N | | |
| MGST2 | 12-465-26 | 29 | 501 | N | | |
| MGST2 | 12-465-234 | 30 | 501 | N | | |

**Fig. 1 (following)**

| | | | | | | |
|---|---|---|---|---|---|---|
| MGST2 | 12-421-135 | 436 | 501 | N | | |
| MGST2 | 12-442-133 | 437 | 501 | Y | C | 5.85 |
| MGST2 | 12-449-63 | 438 | 501 | N | | |
| MGST2 | 12-454-242 | 439 | 501 | N | | |
| MGST2 | 12-463-250 | 440 | 501 | N | | |
| MGST2 | 12-462-199 | 441 | 501 | N | | |

**Fig. 1 (following)**

| | | | | | | |
|---|---|---|---|---|---|---|
| MGST2 | 10-290-326 | 469 | 501 | N | | |
| MGST2 | 10-290-37 | 470 | 501 | N | | |
| MGST2 | 10-523-232 | 471 | 501 | N | | |
| MGST2 | 12-449-300 | 472 | 501 | N | | |
| MGST2 | 10-286-289 | 474 | 501 | N | | |
| MGST2 | 10-286-345 | 475 | 501 | N | | |
| MGST2 | 10-286-375 | 476 | 501 | N | | |
| MGST2 | 10-289-201 | 477 | 501 | N | | |
| MGST2 | 10-290-328 | 484 | 501 | N | | |

-/-0

FIG. 2

| SEQ ID NO. | BIALLELIC MARKER ID | $1^{ST}$ ALLELE | $2^{ND}$ ALLELE | POSITION RANGE OF PREFERRED SEQUENCES |
|---|---|---|---|---|
| 1 | 12-421-140 | A | G | 1-1001 |
| 2 | 12-424-192 | A | G | 190-801; 865-999 |
| 3 | 12-424-198 | G | T | 184-795; 859-993 |
| 4 | 12-425-57 | G | A | 208-225; 266-478 |
| 5 | 12-426-154 | A | G | 152-961 |
| 6 | 12-429-198 | C | T | 260-784; 822-1001 |
| 7 | 12-430-80 | T | C | 1-996 |
| 8 | 12-433-215 | A | G | 1-1001 |
| 12 | 12-447-58 | G | C | 1-36; 390-914 |
| 13 | 12-453-429 | C | T | 1-1001 |
| 14 | 12-454-363 | A | G | 1-315; 377-466; 598-619 |
| 15 | 12-455-326 | T | C | 1-357; 391-594; 760-827 |
| 16 | 12-455-383 | G | A | 1-414; 448-651; 817-884 |
| 17 | 12-456-269 | A | G | 1-536 |
| 19 | 12-457-204 | A | G | 437-527; 761-1001 |
| 20 | 12-457-206 | C | T | 435-525; 759-1001 |
| 21 | 12-458-196 | T | A | 1-727 |
| 22 | 12-458-438 | T | C | 1-21; 298-1001 |
| 23 | 12-460-274 | A | G | 1-499; 563-1001 |
| 24 | 12-461-124 | A | C | 1-203; 259-644; 687-773; 807-833 |
| 25 | 12-461-299 | C | T | 1-28; 84-469; 512-591 |
| 26 | 12-461-465 | C | T | 1-303; 346-432 |
| 27 | 12-462-280 | C | T | 1-1001 |
| 28 | 12-464-66 | G | T | 1-215; 261-1001 |
| 29 | 12-465-26 | C | T | 1-61; 99-1001 |
| 30 | 12-465-234 | G | T | 1-1001 |

**FIG. 2 (following)**

| | | | | |
|---|---|---|---|---|
| 436 | 12-421-135 | T | - | 1-1001 |
| 438 | 12-449-63 | AT | - | 1-107; 314-656 |
| 439 | 12-454-242 | AT | - | 1-436; 498-587; 719-740 |
| 440 | 12-463-250 | CAT | - | 1-30; 102-601; 752-1001 |
| 441 | 12-462-199 | | deletion | 1-1001 |
| 469 | 10-290-326 | A | G | 1-197; 437-1000 |
| 472 | 12-449-300 | T | C | 1-542; 908-1000 |
| 484 | 10-290-328 | deletion | | 1-194; 434-1000 |

Fig. 3

| SEQ ID NO. | BIALLELIC MARKER ID | ORIGINAL ALLELE | ALTERNATIVE ALLELE |
|---|---|---|---|
| 10 | 12-441-343 | G | A |
| 18 | 12-456-380 | G | T |

**FIG. 3 (following)**

| | | | |
|---|---|---|---|
| 437 | 12-442-133 | C | - |
| 470 | 10-290-37 | C | T |
| 471 | 10-523-232 | C | T |
| 474 | 10-286-289 | C | G |
| 475 | 10-286-345 | A | T |
| 476 | 10-286-375 | A | G |
| 477 | 10-289-201 | T | C |

**Fig. 4**

| SEQ ID NO. | BIALLELIC MARKER ID | 1ST ALLELE | 2ND ALLELE |
|---|---|---|---|
| 9 | 12-441-233 | G | A |
| 11 | 12-442-221 | T | C |

**Fig. 5**

| SEQ ID NO. | POSITION RANGE OF PREFERRED SEQUENCES |
|---|---|
| 9 | 940-1001 |
| 18 | 1-425; 920-1001 |

**FIG. 5 (following)**

| | |
|---|---|
| 470 | 1-485; 725-1000 |
| 471 | 1-432; 514-1000 |
| 474 | 1-353; 656-1000 |
| 475 | 1-298; 601-1000 |
| 476 | 1-268; 571-1000 |
| 477 | 1-452; 538-1000 |

**Fig. 6**

| SEQ ID NO. | POSITION RANGE OF MICROSEQUENCING PRIMERS | COMPLEMENTARY POSITION RANGE OF MICROSEQUENCING PRIMERS |
|---|---|---|
| 1 | 481-500 | 502-521 |
| 2 | 481-500 | 502-521 |
| 3 | 481-500 | 502-521 |
| 4 | 481-500 | 502-521 |
| 5 | 441-460 | 462-481 |
| 6 | 481-500 | 502-521 |
| 7 | 482-500* | 502-521 |
| 8 | 481-500 | 502-521 |
| 9 | 482-500* | 502-521 |
| 10 | 481-500 | 502-521 |
| 11 | 481-500 | 502-521 |
| 12 | 482-500* | 502-521 |
| 13 | 481-500 | 502-520* |
| 14 | 481-500 | 502-521 |
| 15 | 481-500 | 502-520* |
| 16 | 481-500 | 502-521 |
| 17 | 481-500 | 502-521 |
| 18 | 481-500 | 502-521 |
| 19 | 481-500 | 502-521 |
| 20 | 481-500 | 502-521 |
| 21 | 481-500 | 502-521 |
| 22 | 481-500 | 502-521 |
| 23 | 481-500 | 502-521 |
| 24 | 481-500 | 502-521 |
| 25 | 481-500 | 502-520* |
| 26 | 481-500 | 502-521 |
| 27 | 481-500 | 502-521 |
| 28 | 481-500 | 502-521 |
| 29 | 481-500 | 502-521 |
| 30 | 481-500 | 502-521 |

**FIG. 6 (following)**

| | | |
|---|---|---|
| 436 | 481-500 | - |
| 437 | - | 502-520* |
| 438 | 481-500 | - |
| 439 | 481-500 | - |
| 440 | 481-500 | - |
| 441 | 481-500 | - |

**FIG. 6 (following)**

| | | |
|---|---|---|
| 469 | 481-500 | 502-521 |
| 470 | 481-500 | 502-521 |
| 471 | 481-500 | 502-521 |
| 472 | 481-500 | 502-521 |
| 474 | 481-500 | 502-521 |
| 475 | 481-500 | 502-521 |
| 476 | 481-500 | 502-521 |
| 477 | 481-500 | 502-521 |
| 484 | 481-500 | 502-521 |

# Fig. 7

| SEQ ID NO. | POSITION RANGE OF AMPLIFICATION PRIMERS | COMPLEMENTARY POSITION RANGE OF AMPLIFICATION PRIMERS |
|---|---|---|
| 1 | 362-380 | 792-812 |
| 2 | 310-327 | 751-771 |
| 3 | 304-321 | 745-765 |
| 4 | 82-99 | 540-557 |
| 5 | 308-325 | 830-847 |
| 6 | 304-321 | 803-823 |
| 7 | 131-150 | 561-580 |
| 8 | 287-304 | 805-825 |
| 9 | 284-303 | 716-734 |
| 10 | 394-413 | 826-844 |
| 11 | 270-289 | 704-721 |
| 12 | 444-462 | 874-893 |
| 13 | 73-91 | 577-596 |
| 14 | 139-158 | 634-652 |
| 15 | 372-392 | 808-826 |
| 16 | 429-449 | 865-883 |
| 17 | 233-252 | 693-712 |
| 18 | 122-141 | 582-601 |
| 19 | 298-317 | 772-792 |
| 20 | 296-315 | 770-790 |
| 21 | 200-217 | 679-696 |
| 22 | 442-459 | 921-938 |
| 23 | 228-245 | 760-777 |
| 24 | 378-396 | 911-928 |
| 25 | 203-221 | 736-753 |
| 26 | 37-55 | 570-587 |
| 27 | 222-241 | 655-675 |
| 28 | 436-455 | 880-900 |
| 29 | 476-493 | 945-962 |
| 30 | 266-283 | 735-752 |

FIG. 7 (following)

| | | |
|---|---|---|
| 436 | 367-385 | 797-817 |
| 437 | 184-203 | 616-633 |
| 438 | 86-106 | 546-563 |
| 439 | 260-279 | 755-773 |
| 440 | 255-272 | 773-790 |
| 441 | 303-322 | 736-756 |

**FIG. 7 (following)**

| | | |
|---|---|---|
| 469 | 177-196 | 576-595 |
| 470 | 465-484 | 864-883 |
| 471 | 270-288 | 527-545 |
| 472 | 325-345 | 783-800 |
| 474 | 213-231 | 613-631 |
| 475 | 158-176 | 558-576 |
| 476 | 128-146 | 528-546 |
| 477 | 307-324 | 700-719 |
| 484 | 174-193 | 573-592 |

# Fig. 8

| SEQ ID NO. | POSITION RANGE OF PROBES |
|---|---|
| 1 | 489-513 |
| 2 | 489-513 |
| 3 | 489-513 |
| 4 | 489-513 |
| 5 | 449-473 |
| 6 | 489-513 |
| 7 | 489-513 |
| 8 | 489-513 |
| 9 | 489-513 |
| 10 | 489-513 |
| 11 | 489-513 |
| 12 | 489-513 |
| 13 | 489-513 |
| 14 | 489-513 |
| 15 | 489-513 |
| 16 | 489-513 |
| 17 | 489-513 |
| 18 | 489-513 |
| 19 | 489-513 |
| 20 | 489-513 |
| 21 | 489-513 |
| 22 | 489-513 |
| 23 | 489-513 |
| 24 | 489-513 |
| 25 | 489-513 |
| 26 | 489-513 |
| 27 | 489-513 |
| 28 | 489-513 |
| 29 | 489-513 |
| 30 | 489-513 |

Fig. 8 (following)

| | |
|---|---|
| 436 | 489-513 |
| 437 | 489-513 |
| 438 | 489-513 |
| 439 | 489-513 |
| 440 | 489-513 |
| 441 | 489-513 |

**Fig. 8 (following)**

| | |
|---|---|
| 469 | 489-513 |
| 470 | 489-513 |
| 471 | 489-513 |
| 472 | 489-513 |
| 474 | 489-513 |
| 475 | 489-513 |
| 476 | 489-513 |
| 477 | 489-513 |

# ALLELE FREQUENCY DATA (FRENCH and US)

| Seq. ID No. | Protein | Biallelic Marker ID | caucasian FRENCH | | | | | caucasian US | | | | |
|---|---|---|---|---|---|---|---|---|---|---|---|---|
| | | | size | A | C | G | T | size | A | C | G | T |
| 21 | MGST-II | 12-458/19 | | | | | | 180 | 80,83 | | | 19,17 |
| 15 | MGST-II | 12-455/32 | | | | | | 93 | 58,06 | | 41,94 | |
| 5 | MGST-II | 12-426/15 | | | | | | 181 | 58,01 | | 41,99 | |
| 9 | MGST-II | 12-441/23 | | | | | | 182 | | 35,44 | | 64,56 |
| 437 | MGST-II | 12-442/13 | | | | | | 94 | | 5,85 | 94,15 | |
| 7 | MGST-II | 12-430/80 | | | | | | 93 | 5,38 | | 94,62 | |
| 1 | MGST-II | 12-421/14 | | | | | | 183 | 79,51 | | 20,49 | |
| 436 | MGST-II | 12-421/13 | | | | | | 185 | | | 84,59 | 15,41 |
| 13 | MGST-II | 12-453/42 | no genotyped for this population | | | | | 183 | | 56,28 | | 43,72 |
| 12 | MGST-II | 12-447/58 | | | | | | 93 | | 55,38 | 44,62 | |
| 25 | MGST-II | 12-461/29 | | | | | | 183 | | 40,44 | | 59,56 |
| 3 | MGST-II | 12-424/19 | | | | | | 176 | | | 61,08 | 38,92 |
| 14 | MGST-II | 12-454/36 | | | | | | 188 | 18,62 | | 81,38 | |

Figure 9

# HAPLOTYPE ANALYSIS (MGST-II vs ASTHMA)

297 ALT vs 286 Caucasian US

| MARKERS | | | 12-421/135 | 12-421/140 | 12-430/80 | 12-441/233 | 12-442/133 | 12-447/58 | 12-455/326 | 12-461/299 | 12-453/429 | 12-424/188 | 12-454/363 | 12-458/186 | 12-426/154 | ESTIMATED FREQUENCIES | | | | | |
|---|---|---|---|---|---|---|---|---|---|---|---|---|---|---|---|---|---|---|---|---|---|
| MGST2 | | | JnB | | markers in bac | | | | | | | | | | | | | | | | |
| size (cases / controls) | | | 282 vs 185 | 281 vs 183 | 183 vs 93 | 285 vs 182 | 172 vs 94 | 287 vs 93 | 167 vs 93 | 287 vs 183 | 287 vs 183 | 260 vs 176 | 277 vs 188 | 277 vs 180 | 273 vs 181 | haplotype frequencies | | p-excess | Odds Ratio | Chi-S | Pvalue (1df) |
| allelic frequency % (case/controls) | | | 85/84 (G) | 81/79 (A) | 8/5 (A) | 35/35 (C) | 8/5 (C) | 45/44 (G) | 44/41 (G) | 63/59 (T) | 60/56 (C) | 40/38 (T) | 21/18 (A) | 81/80 (A) | 59/58 (A) | | | | | | |
| diff freq. all. (cases - controls) | | | 1.0 | 2.2 | 3.2 | 0.3 | 2.3 | 1.0 | 2.4 | 3.5 | 4.7 | 1.7 | 2.5 | 0.8 | 1.3 | | | | | | |
| pvalue | | | 6.6e-01<br>1.1 | 4.0e-01<br>1.1 | 1.8e-01<br>1.6 | 7.5e-01<br>1.0 | 3.2e-01<br>1.4 | 7.5e-01<br>1.0 | 5.8e-01<br>1.1 | 2.7e-01<br>1.2 | 1.5e-01<br>1.2 | 5.8e-01<br>1.1 | 3.4e-01<br>1.2 | 7.5e-01<br>1.1 | 6.6e-01<br>1.1 | cases | controls | | | | |
| Test Hardy Weinberg Equilibrium | | cases vs | 0.00 HW | -0.00 HW | -0.00 HW | 0.01 HW | -0.00 HW | -0.00 HW | 0.01 HW | 0.01 HW | -0.02 HW | 0.02 HW | 0.00 HW | 0.01 HW | 0.02 HW | | | | | | |
| | | controls | 0.01 HW | 0.00 HW | 0.89 | 0.01 HW | 0.88 | -0.04 HW | 0.02 HW | -0.03 HW | 0.00 HW | -0.02 HW | 0.00 HW | -0.00 HW | 0.00 HW | | | | | | |
| haplotype 1 | | 288 vs 180 | | | | | | | | T | C | | | | | 0.390 | 0.310 | 11.60 | 1.42 | 6.16 | 1.3e-02 |
| haplotype 2 | | 157 vs 83 | | | A | | | | | | | | G | | | 0.076 | 0.025 | 5.18 | 3.16 | 5.54 | 1.8e-02 |
| haplotype 3 | | 164 vs 84 | | | | | C | | | | | | G | | | 0.073 | 0.025 | 4.93 | 3.10 | 5.28 | 2.1e-02 |
| haplotype 4 | | 276 vs 83 | | | | | | C | | | | | A | | | 0.068 | 0.025 | 4.46 | 2.89 | 4.89 | 2.7e-02 |
| haplotype 5 | PT2 | 260 vs 170 | | | | | | | | | C | T | | | | 0.216 | 0.156 | 7.14 | 1.49 | 4.81 | 2.7e-02 |
| haplotype 6 | | 151 vs 86 | | | | | | | G | | C | T | | | | 0.118 | 0.011 | 10.81 | 12.22 | 17.23 | 3.2e-05 |
| haplotype 7 | | 247 vs 162 | | | | | | | | | C | T | | | G | 0.097 | 0.027 | 7.13 | 3.80 | 14.60 | 1.3e-04 |
| haplotype 8 | | 168 vs 82 | | | | | | G | G | C | | | | | | 0.112 | 0.027 | 8.69 | 4.53 | 11.33 | 7.3e-04 |
| haplotype 9 | | 168 vs 83 | | | | | | | G | C | T | | | | | 0.098 | 0.022 | 7.75 | 4.75 | 10.38 | 1.3e-03 |
| haplotype 10 | | 258 vs 165 | | | | T | | | | | C | T | | | | 0.150 | 0.077 | 7.99 | 2.13 | 10.25 | 1.3e-03 |
| haplotype 11 | | 265 vs 166 | | | | | | | | T | | | | A | A | 0.251 | 0.171 | 9.68 | 1.63 | 7.66 | 5.5e-03 |
| haplotype 12 | | 150 vs 86 | | | | T | | | A | | | T | | | | 0.105 | 0.034 | 7.36 | 3.34 | 7.58 | 5.8e-03 |
| haplotype 13 | | 281 vs 175 | G | | | | | | | T | C | | | | | 0.340 | 0.255 | 11.36 | 1.50 | 7.26 | 6.9e-03 |
| haplotype 14 | | 277 vs 170 | | | | | | | | T | C | | | A | | 0.322 | 0.238 | 11.02 | 1.52 | 7.20 | 6.9e-03 |
| haplotype 15 | | 150 vs 81 | | A | | | | | A | | | T | | | | 0.193 | 0.098 | 10.53 | 2.20 | 7.07 | 7.7e-03 |
| haplotype 16 | | 284 vs 176 | | | | T | | | | T | C | | | | | 0.262 | 0.187 | 9.29 | 1.55 | 6.94 | 8.2e-03 |
| haplotype 17 | PT3 | 271 vs 88 | | | | | | G | | C | | | | | G | 0.088 | 0.028 | 6.12 | 3.29 | 6.91 | 8.2e-03 |
| haplotype 18 | | 144 vs 88 | | | | | | | G | | C | T | G | | | 0.128 | 0.000 | 12.60 | 100.00 | 23.52 | 1.2e-06 |
| haplotype 19 | | 166 vs 92 | | | | | | C | A | T | T | | | | | 0.101 | 0.000 | 10.09 | 100.00 | 19.85 | 8.2e-06 |
| haplotype 20 | | 149 vs 85 | G | | | | | | G | | C | T | | | | 0.131 | 0.010 | 12.20 | 14.29 | 19.79 | 8.6e-06 |
| haplotype 21 | | 148 vs 79 | | | | | | | G | | T | G | | A | | 0.131 | 0.009 | 12.32 | 16.46 | 18.98 | 1.3e-05 |
| haplotype 22 | | 271 vs 88 | | | | | | C | | T | T | | | | A | 0.099 | 0.000 | 9.89 | 100.00 | 18.81 | 1.4e-05 |
| haplotype 23 | | 151 vs 86 | | | | | | | G | T | C | T | | | | 0.123 | 0.011 | 11.29 | 12.37 | 18.08 | 2.1e-05 |
| haplotype 24 | | 151 vs 85 | | | | | | C | A | | T | T | | | | 0.092 | 0.000 | 9.22 | 100.00 | 16.66 | 4.4e-05 |
| haplotype 25 | | 151 vs 86 | | | | | G | | G | | T | G | | | | 0.130 | 0.019 | 11.28 | 7.72 | 16.48 | 4.9e-05 |
| haplotype 26 | | 150 vs 81 | | A | | | | | G | | C | T | | | | 0.119 | 0.011 | 10.91 | 11.94 | 16.40 | 5.1e-05 |
| haplotype 27 | | 143 vs 85 | | | G | | | | G | | T | G | | | | 0.131 | 0.019 | 11.37 | 7.63 | 16.31 | 5.1e-05 |
| haplotype 28 | | 150 vs 86 | | | | T | | | G | | C | T | | | | 0.114 | 0.011 | 10.33 | 11.04 | 16.15 | 5.7e-05 |
| haplotype 29 | | 243 vs 159 | G | | | | | | | | C | T | | | G | 0.105 | 0.029 | 7.81 | 3.96 | 16.04 | 6.0e-05 |
| haplotype 30 | PT4 | 240 vs 161 | | | | | | | | | C | T | G | | G | 0.101 | 0.028 | 7.51 | 3.88 | 15.38 | 6.7e-05 |

Figure 13

# HAPLOTYPE ANALYIS ; PERMUTATION TEST RESULTS

Asthma

| PROTEIN MGST2 | 12-447/58 | 12-455/326 | 12-461/299 | 12-453/429 | 12-424/198 | 12-454/363 | MARKERS | |
|---|---|---|---|---|---|---|---|---|
| | markers in bac | | | | | | | |
| | | G | | C | T | | HAPLOTYPES (ALT vs US) | |
| | | 7,52E-01 | | 5,27E-01 | 7,52E-01 | | pvalue | ALT+ vs ALT- |
| | | 0.8 (44 vs 45) | | -2.7 (59 vs 62) | -1 (41 vs 40) | | diff all. Freq% | |
| | | 5,84E-01 | | 1,47E-01 | 5,84E-01 | | pvalue | ALT vs caucasian US |
| | | 3.5 (63 vs 60) | | 4.7 (61 vs 56) | -1.7 (59 vs 61) | | diff all. Freq% | |
| | | G | | C | T | G | HAPLOTYPES (ALT vs US) | |
| | | 7,52E-01 | | 5,27E-01 | 7,52E-01 | 4,80E-01 | pvalue | ALT+ vs ALT- |
| | | 0.8 (44 vs 45) | | -2.7 (59 vs 62) | -1 (41 vs 40) | -2,5 (77 vs 80) | diff all. Freq% | |
| | | 5,84E-01 | | 1,47E-01 | 5,84E-01 | 3,43E-01 | pvalue | ALT vs caucasian US |
| | | 3.5 (63 vs 60) | | 4.7 (61 vs 56) | -1.7 (59 vs 61) | -2,5 (79 vs 81) | diff all. Freq% | |
| | C | A | T | T | | | HAPLOTYPES (ALT vs US) | |
| | 4,80E-01 | 7,52E-01 | 6,55E-01 | 5,27E-01 | | | pvalue | ALT+ vs ALT- |
| | -3,1 (52 vs 55) | 0,8 (56 vs 55) | 1,6 (64 vs 63) | -2,7 (41 vs 38) | | | diff all. Freq% | |
| | 7,52E-01 | 5,84E-01 | 2,73E-01 | 1,47E-01 | | | pvalue | ALT vs caucasian US |
| | -1 (54 vs 55) | -2,4 (56 vs 58) | 3,5 (63 vs 60) | 4,7 (39 vs 44) | | | diff all. Freq% | |

| HAPLOTYPE GCT | sample sizes cases vs controls | haplotype frequencies cases | haplotype frequencies controls | p-excess | odds-ratio | chi-S | P value | | PERMUTATIONS Av. Chi-S | Max Chi-S | > iter / nb of iter. |
|---|---|---|---|---|---|---|---|---|---|---|---|
| ALT+ vs ALT- | 60 vs 91 | 0.077 | 0.141 | -7,53 | 0,50 | 2.96 | 8.3e-02 | * | 1.4 | 14.6 | 158/1000 |
| ALT+ vs ALT- (1) | 60 vs 91 | 0.077 | 0.141 | -7,53 | 0,5 | 2.96 | 8.3e-02 | * | 1.4 | 14.6 | 158/1000 |
| ALT vs caucasian US | 151 vs 88 | 0.118 | 0.011 | 10,81 | 12,22 | 17.23 | 3.2e-05 | **** | 2.2<br>2.2 | 19.5<br>22.6 | 3/1000<br>31/10000 |
| ALT vs caucasian US (2) | 151 vs 88 | 0.118 | 0.011 | 10,81 | 12,22 | 17.23 | 3.2e-05 | **** | 2.2 | 19.5 | 3/1000 |

| HAPLOTYPE GCTG | sample sizes cases vs controls | haplotype frequencies cases | haplotype frequencies controls | p-excess | odds-ratio | chi-S | P value | | PERMUTATIONS Av. Chi-S | Max Chi-S | > iter / nb of iter. |
|---|---|---|---|---|---|---|---|---|---|---|---|
| ALT+ vs ALT- | 56 vs 88 | 0.088 | 0.152 | -7,46 | 0,54 | 2.47 | 1.1e-01 | * | 1.4 | 16.6 | 182/1000 |
| ALT+ vs ALT- (1) | 56 vs 88 | 0.088 | 0.152 | -7,46 | 0,54 | 2.47 | 1.1e-01 | * | 1.4 | 16.6 | 182/1000 |
| ALT vs caucasian US | 144 vs 88 | 0.126 | 0.000 | 12,6 | 100 | 23.52 | 1.2e-06 | ****** | 2.8<br>2.8 | 20.1<br>28.7 | 0/1000<br>5/10000 |
| ALT vs caucasian US (2) | 144 vs 88 | 0.126 | 0.000 | 12,6 | 100 | 23.52 | 1.2e-06 | ****** | 2.8 | 20.1 | 0/1000 |

| HAPLOTYPE CATT | sample sizes cases vs controls | haplotype frequencies cases | haplotype frequencies controls | p-excess | odds-ratio | chi-S | P value | | PERMUTATIONS Av. Chi-S | Max Chi-S | > iter / nb of iter. |
|---|---|---|---|---|---|---|---|---|---|---|---|
| ALT+ vs ALT- | 64 vs 102 | 0.139 | 0.075 | 7 | 2,01 | 3.68 | 5.4e-02 | * | 1.5 | 14.4 | 114/1000 |
| ALT+ vs ALT- (1) | 64 vs 102 | 0.139 | 0.075 | 7 | 2,01 | 3.68 | 5.4e-02 | * | 1.5 | 14.4 | 114/1000 |
| ALT vs caucasian US | 166 vs 92 | 0.101 | 0 | 10,09 | 100 | 19.85 | 8.2e-06 | ***** | 3.0<br>3.0 | 29.0<br>29.0 | 12/1000<br>76/10000 |
| ALT vs caucasian US (2) | 166 vs 92 | 0.101 | 0 | 10,09 | 100 | 19.85 | 8.2e-06 | ***** | 3.0 | 29.0 | 12/1000 |

Figure 14

# HAPLOTYPE ANALYSIS ; PERMUTATION TEST RESULT

Zyflo secondary effects

| PROTEIN MGST2 | 12-441/233 | 12-461/299 | 12-453/429 | 12-426/154 | | |
|---|---|---|---|---|---|---|
| | markers in bac | | | | MARKERS | |
| | C | T | T | | HAPLOTYPES (ALT+ vs ALT-) | |
| | 1,92E-01 | 6,55E-01 | 5,27E-01 | | pvalue | ALT+ vs ALT- |
| | -5.6 (40 vs 34) | 1.6 (64 vs 63) | -2.7 (41 vs 38) | | diff all. Freq % | |
| | 7,52E-01 | 2,73E-01 | 1,47E-01 | | pvalue | ALT vs caucasian US |
| | -0.3 (36 vs 35) | 3.5 (63 vs 60) | 4.7 (39 vs 44) | | diff all. Freq % | |
| | C | T | T | A | HAPLOTYPES (ALT+ vs ALT-) | |
| | 1,92E-01 | 6,55E-01 | 5,27E-01 | 6,55E-01 | pvalue | ALT+ vs ALT- |
| | -5.6 (40 vs 34) | 1.6 (64 vs 63) | -2.7 (41 vs 38) | 1.8 (61 vs 59) | diff all. Freq % | |
| | 7,52E-01 | 2,73E-01 | 1,47E-01 | 6,55E-01 | pvalue | ALT vs caucasian US |
| | -0.3 (36 vs 35) | 3.5 (63 vs 60) | 4.7 (39 vs 44) | 1.3 (59 vs 58) | diff all. Freq % | |

| HAPLOTYPE CTT | sample sizes cases vs controls | haplotype frequencies | | p-excess | odds-ratio | chi-S | P value | | PERMUTATIONS | | |
|---|---|---|---|---|---|---|---|---|---|---|---|
| | | cases | controls | | | | | | Av. Chi-S | Max Chi-S | > iter / nb of iter. |
| ALT+ vs ALT- | 86 vs 198 | 0.157 | 0.049 | 11,34 | 3,63 | 18.68 | 1.5e-05 | ***** | 1.9<br>1.9 | 18.9<br>25.4 | 1/1000<br>12/10000 |
| ALT+ vs ALT- (1) | 86 vs 104 | 0.157 | 0.088 | 7,54 | 1,93 | 4.26 | 3.8e-02 | * | 1.1 | 16.5 | 52/1000 |
| ALT+ vs ALT- (2) | 86 vs 94 | 0.157 | 0.042 | 11,91 | 4,19 | 13.36 | 2.5e-04 | **** | 1.5 | 15.4 | 3/1000 |
| ALT vs caucasian US | 284 vs 176 | 0.092 | 0.071 | 2,2 | 1,32 | 1.18 | 2.7e-01 | * | 2.1 | 20.9 | 479/1000 |
| ALT vs caucasian US (2) | 284 vs 94 | 0.092 | 0.047 | 4,67 | 2,04 | 3.77 | 5.1e-02 | * | 1.9 | 17.3 | 158/1000 |
| ALT vs caucasian US (3) | 284 vs 82 | 0.092 | 0.116 | -2,79 | 0,77 | 0.88 | 3.4e-01 | | 1.8 | 17.7 | 498/1000 |

| HAPLOTYPE CTTA | sample sizes cases vs controls | haplotype frequencies | | p-excess | odds-ratio | chi-S | P value | | PERMUTATIONS | | |
|---|---|---|---|---|---|---|---|---|---|---|---|
| | | cases | controls | | | | | | Av. Chi-S | Max Chi-S | > iter / nb of iter. |
| ALT+ vs ALT- | 83 vs 187 | 0.141 | 0.028 | 11,64 | 5,75 | 25.13 | 5.2e-07 | ****** | 2.6<br>2.5 | 25.2<br>35 | 1/1000<br>7/10000 |
| ALT+ vs ALT- (1) | 83 vs 98 | 0.141 | 0.066 | 8,03 | 2,33 | 5.61 | 1.7e-02 | ** | 1.7 | 17.4 | 59/1000 |
| ALT+ vs ALT- (2) | 83 vs 89 | 0.141 | 0.034 | 11,03 | 4,6 | 12.42 | 4.1e-04 | *** | 1.7 | 18.3 | 3/1000 |
| ALT vs caucasian US | 270 vs 167 | 0.082 | 0.058 | 2,61 | 1,46 | 1.85 | 1.7e-01 | * | 2.7 | 25.9 | 438/1000 |
| ALT vs caucasian US (2) | 270 vs 89 | 0.082 | 0.023 | 6,12 | 3,89 | 7.61 | 5.5e-03 | ** | 2.7 | 30.3 | 92/1000 |
| ALT vs caucasian US (3) | 270 vs 78 | 0.082 | 0.110 | -3,06 | 0,73 | 1.11 | 2.7e-01 | * | 2.3 | 17.8 | 499/1000 |

Figure 16

89 ALT+ vs 208 ALT-

| MARKERS | | | 12-421/135 | 12-421/140 | 12-430/80 | 12-441/233 | 12-442/133 | 12-447/58 | 12-455/328 | 12-461/289 | 12-453/429 | 12-424/198 | 12-454/363 | 12-458/196 | 12-420/154 | ESTIMATED FREQUENCIES | | | | | |
|---|---|---|---|---|---|---|---|---|---|---|---|---|---|---|---|---|---|---|---|---|---|
| MGST2 | | | lnB | | markers in bac | | | | | | | | | | | | | | | | |
| size (cases / controls) | | | 88 vs 194 | 86 vs 195 | 66 vs 97 | 87 vs 198 | 68 vs 104 | 89 vs 198 | 65 vs 102 | 88 vs 199 | 88 vs 199 | 80 vs 180 | 83 vs 194 | 83 vs 194 | 85 vs 188 | haplotype frequencies | | p-excess | Odds Ratio | Chi-S | Pvalue (1df) |
| allelic frequency % (case/controls) | | | 18/12 (T) | 18/18 (G) | 91/91 (G) | 39/34 (C) | 92/91 (G) | 47/44 (G) | 56/55 (A) | 64/62 (T) | 40/38 (T) | 41/40 (T) | 22/20 (A) | 22/16 (T) | 60/58 (A) | | | | | | |
| diff freq. all. (cases - controls) | | | 5.6 | 0.4 | 0.4 | 5.6 | 1.3 | 3.1 | 0.8 | 1.6 | 2.7 | 1.0 | 2.5 | 5.5 | 1.8 | | | | | | |
| pvalue | | | 7.8e-02 | 7.5e-01 | 7.5e-01 | 1.9e-01 | 6.6e-01 | 4.8e-01 | 7.5e-01 | 6.6e-01 | 5.3e-01 | 7.5e-01 | 4.8e-01 | 1.2e-01 | 6.6e-01 | | | | | | |
| | | | 1.5 | 1.0 | 1.1 | 1.3 | 1.2 | 1.1 | 1.0 | 1.1 | 1.1 | 1.0 | 1.2 | 1.4 | 1.1 | | | | | | |
| Test Hardy Weinberg Equilibrium | | cases vs | -0.01 HW | 0.00 HW | 0.83 | 0.02 HW | 0.85 | -0.04 HW | -0.02 HW | 0.01 HW | -0.01 HW | 0.04 HW | -0.00 HW | 0.03 HW | -0.00 HW | cases | controls | | | | |
| | | controls | 0.00 HW | -0.00 HW | 0.00 HW | 0.01 HW | 0.00 HW | 0.01 HW | 0.03 HW | 0.01 HW | -0.03 HW | 0.01 HW | 0.00 HW | -0.00 HW | 0.03 HW | | | | | | |
| haplotype 1 | | 77 vs 170 | | | | | | | | | | T | | | A | 0.301 | 0.180 | 14.75 | 1.96 | 9.11 | 2.4e-03 |
| haplotype 2 | | 86 vs 198 | | | | C | | | | | T | | | | | 0.175 | 0.096 | 8.69 | 1.99 | 7.00 | 7.7e-03 |
| haplotype 3 | | 81 vs 190 | | A | | | | | | | | | | T | | 0.185 | 0.106 | 8.78 | 1.91 | 6.20 | 1.2e-02 |
| haplotype 4 | | 87 vs 198 | | | | C | | | | T | | | | | | 0.281 | 0.196 | 10.55 | 1.60 | 5.03 | 2.4e-02 |
| haplotype 5 | PT2 | 86 vs 190 | T | A | | | | | | | | | | | | 0.186 | 0.117 | 7.82 | 1.72 | 4.73 | 2.8e-02 |
| haplotype 6 | | 86 vs 198 | | | | C | | | | T | T | | | | | 0.157 | 0.049 | 11.34 | 3.63 | 18.68 | 1.5e-05 |
| haplotype 7 | | 86 vs 197 | | | | C | | C | | | T | | | | | 0.150 | 0.054 | 10.15 | 3.08 | 14.44 | 1.4e-04 |
| haplotype 8 | | 79 vs 179 | | | | C | | | | | T | T | | | | 0.128 | 0.046 | 8.59 | 3.04 | 11.15 | 8.2e-04 |
| haplotype 9 | | 81 vs 190 | | A | | | | | | | C | | | T | | 0.134 | 0.053 | 8.51 | 2.74 | 10.33 | 1.3e-03 |
| haplotype 10 | | 77 vs 170 | | | | | | | | | T | T | | | A | 0.159 | 0.069 | 9.69 | 2.55 | 9.80 | 1.7e-03 |
| haplotype 11 | | 77 vs 170 | | | | | | | | T | | T | | | A | 0.301 | 0.180 | 14.74 | 1.96 | 9.08 | 2.6e-03 |
| haplotype 12 | | 81 vs 189 | | A | | | | G | | | | | | T | | 0.124 | 0.052 | 7.67 | 2.61 | 8.83 | 2.9e-03 |
| haplotype 13 | | 84 vs 188 | | | | | | | | T | T | | | | A | 0.193 | 0.105 | 9.87 | 2.04 | 7.90 | 4.7e-03 |
| haplotype 14 | | 75 vs 166 | | A | | | | | | | | T | | | A | 0.217 | 0.122 | 10.88 | 2.00 | 7.34 | 6.5e-03 |
| haplotype 15 | | 86 vs 189 | T | A | | | | C | | | | | | | | 0.114 | 0.051 | 6.71 | 2.41 | 7.28 | 6.8e-03 |
| haplotype 16 | | 83 vs 187 | | | | C | | | | | T | | | | A | 0.164 | 0.087 | 8.40 | 2.05 | 6.86 | 8.6e-03 |
| haplotype 17 | | 59 vs 91 | | | | C | | | A | | | T | | | | 0.189 | 0.087 | 11.24 | 2.46 | 6.81 | 8.6e-03 |
| haplotype 18 | PT3 | 73 vs 167 | | | | | | | | | | T | G | | A | 0.283 | 0.179 | 12.74 | 1.82 | 6.68 | 9.6e-03 |
| haplotype 19 | | 83 vs 187 | | | | C | | | | T | T | | | | A | 0.141 | 0.028 | 11.64 | 5.75 | 25.13 | 5.2e-07 |
| haplotype 20 | | 86 vs 197 | | | | C | | C | | T | T | | | | | 0.154 | 0.043 | 11.62 | 4.04 | 20.84 | 4.8e-06 |
| haplotype 21 | | 83 vs 186 | | | | C | | C | | | T | | | | A | 0.140 | 0.047 | 9.80 | 3.32 | 14.32 | 1.5e-04 |
| haplotype 22 | | 64 vs 98 | | | | | G | | | T | T | | | | A | 0.147 | 0.039 | 11.27 | 4.26 | 12.11 | 4.8e-04 |
| haplotype 23 | | 61 vs 85 | | | G | | | G | A | T | | | | | | 0.203 | 0.072 | 14.08 | 3.26 | 11.72 | 5.9e-04 |
| haplotype 24 | | 56 vs 85 | | A | | | | | A | | | T | | | A | 0.239 | 0.092 | 16.23 | 3.10 | 11.45 | 7.0e-04 |
| haplotype 25 | | 79 vs 179 | | | | C | | | | T | T | T | | | | 0.130 | 0.048 | 8.67 | 2.99 | 11.05 | 8.6e-04 |
| haplotype 26 | | 85 vs 194 | | A | | C | | | | T | T | | | | | 0.113 | 0.040 | 7.65 | 3.07 | 10.91 | 9.1e-04 |
| haplotype 27 | | 59 vs 87 | | A | | | G | | | | | T | | | A | 0.207 | 0.080 | 13.88 | 3.02 | 10.06 | 1.5e-03 |
| haplotype 28 | | 81 vs 183 | | | | | | C | | | T | | | A | A | 0.121 | 0.047 | 7.78 | 2.77 | 9.41 | 2.1e-03 |
| haplotype 29 | | 77 vs 170 | | | | | | | | T | T | T | | | A | 0.159 | 0.071 | 9.53 | 2.49 | 9.39 | 2.2e-03 |
| haplotype 30 | | 56 vs 83 | | A | G | | | | | | | T | | | A | 0.208 | 0.082 | 13.70 | 2.93 | 9.17 | 2.4e-03 |
| haplotype 31 | PT4 | 64 vs 99 | G | | | | | G | A | T | | | | | | 0.173 | 0.067 | 11.40 | 2.92 | 9.07 | 2.6e-03 |

Fig. 15

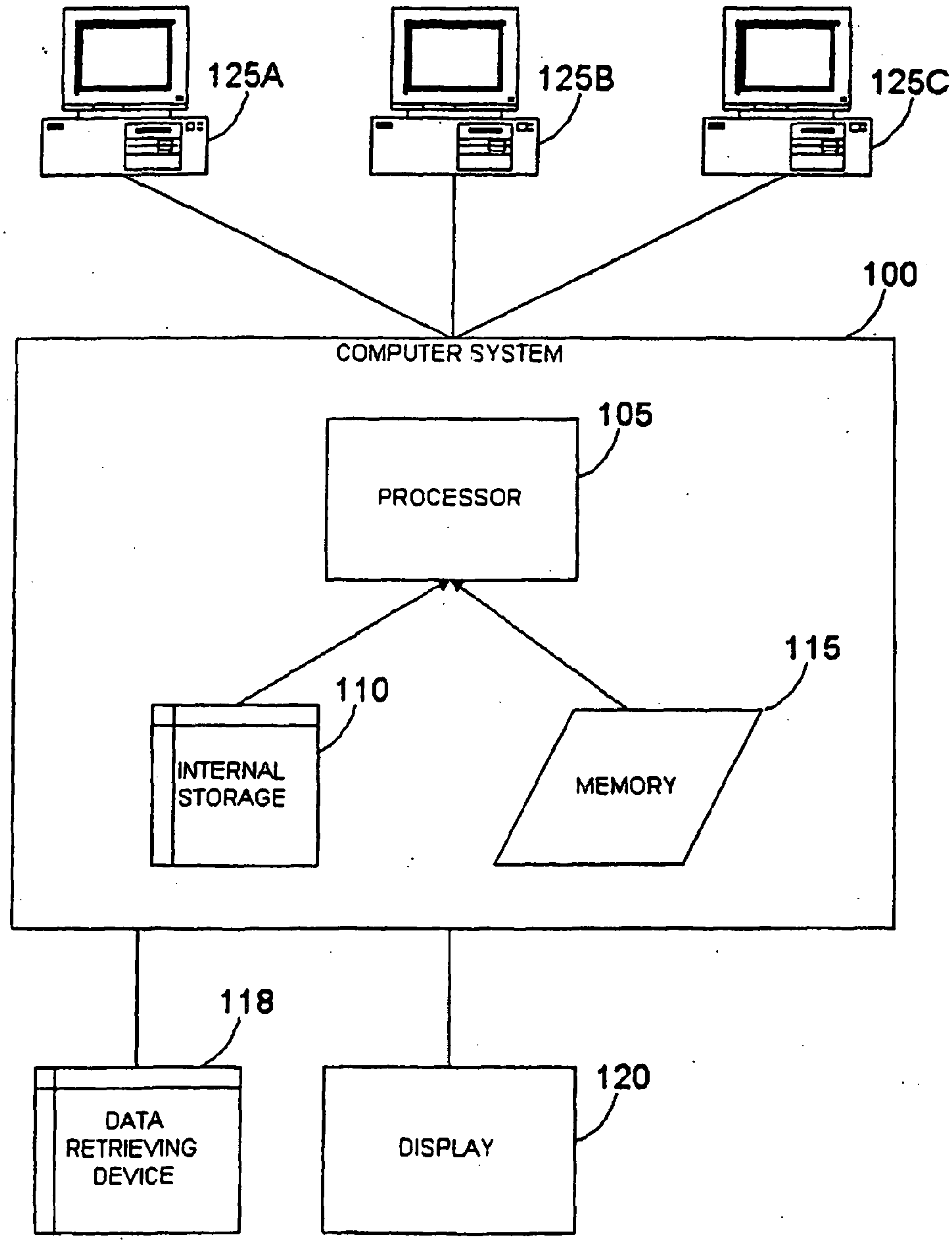
125A
125B
125C
100
COMPUTER SYSTEM
105
PROCESSOR
115
110
INTERNAL STORAGE
MEMORY
118
120
DATA RETRIEVING DEVICE
DISPLAY

FIG.17

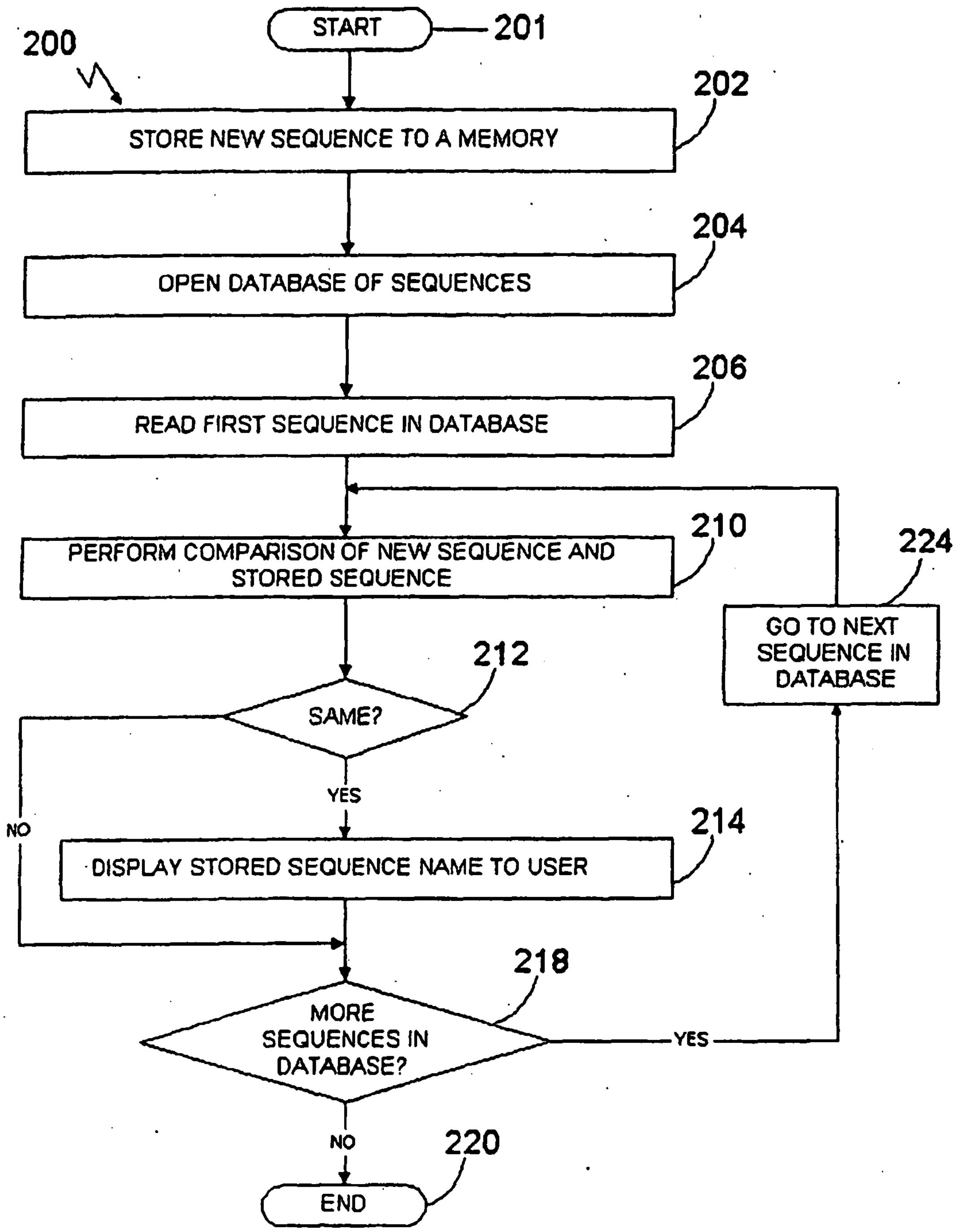
200
START
201
STORE NEW SEQUENCE TO A MEMORY
202
OPEN DATABASE OF SEQUENCES
204
READ FIRST SEQUENCE IN DATABASE
206
PERFORM COMPARISON OF NEW SEQUENCE AND STORED SEQUENCE
210
GO TO NEXT SEQUENCE IN DATABASE
224
SAME?
212
YES
NO
DISPLAY STORED SEQUENCE NAME TO USER
214
MORE SEQUENCES IN DATABASE?
218
YES
NO
END
220

FIG.18

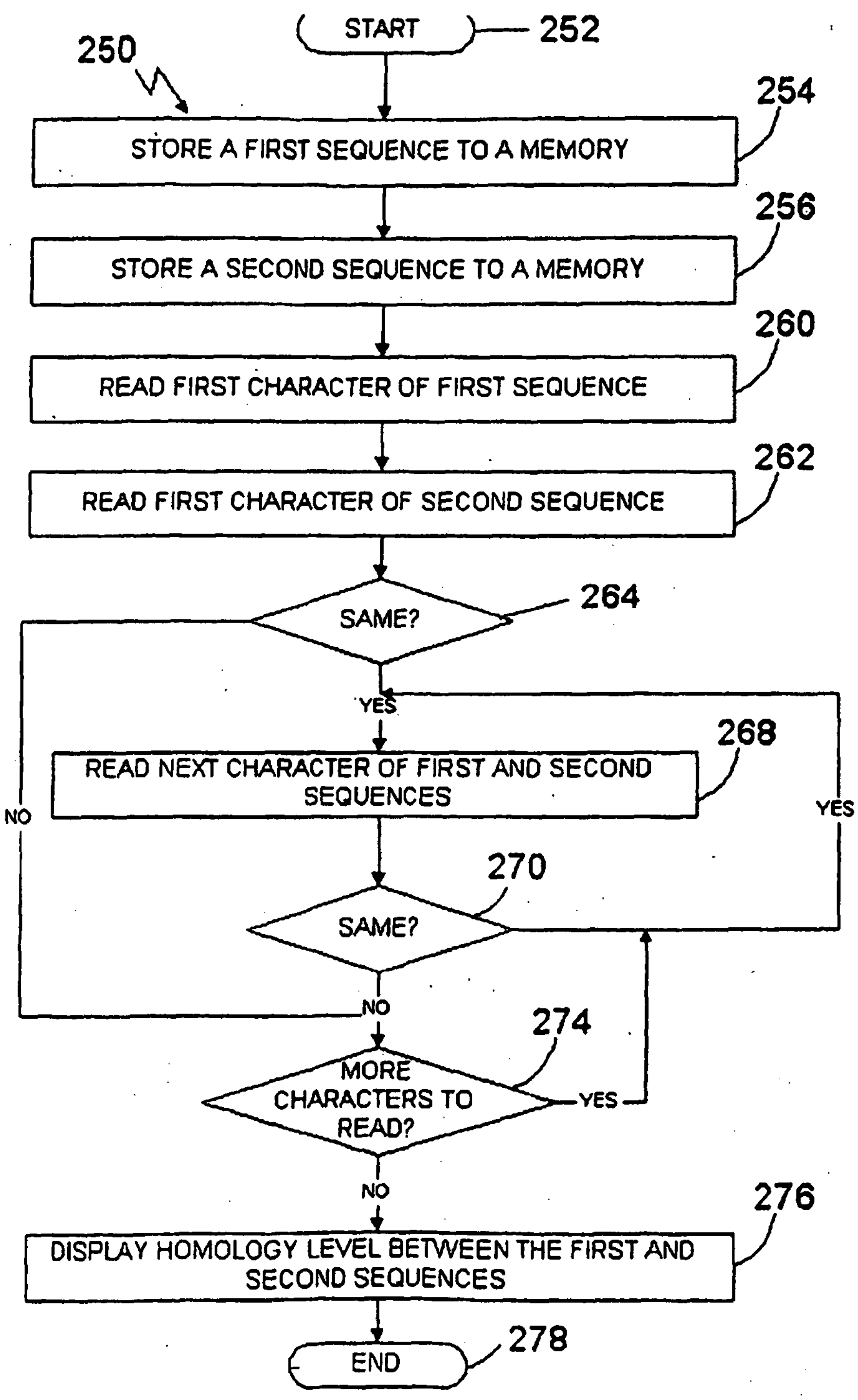
START
252
250
254
STORE A FIRST SEQUENCE TO A MEMORY
256
STORE A SECOND SEQUENCE TO A MEMORY
260
READ FIRST CHARACTER OF FIRST SEQUENCE
262
READ FIRST CHARACTER OF SECOND SEQUENCE
264
SAME?
YES
NO
268
READ NEXT CHARACTER OF FIRST AND SECOND SEQUENCES
YES
270
SAME?
NO
274
MORE CHARACTERS TO READ?
YES
NO
276
DISPLAY HOMOLOGY LEVEL BETWEEN THE FIRST AND SECOND SEQUENCES
END
278

FIG.19

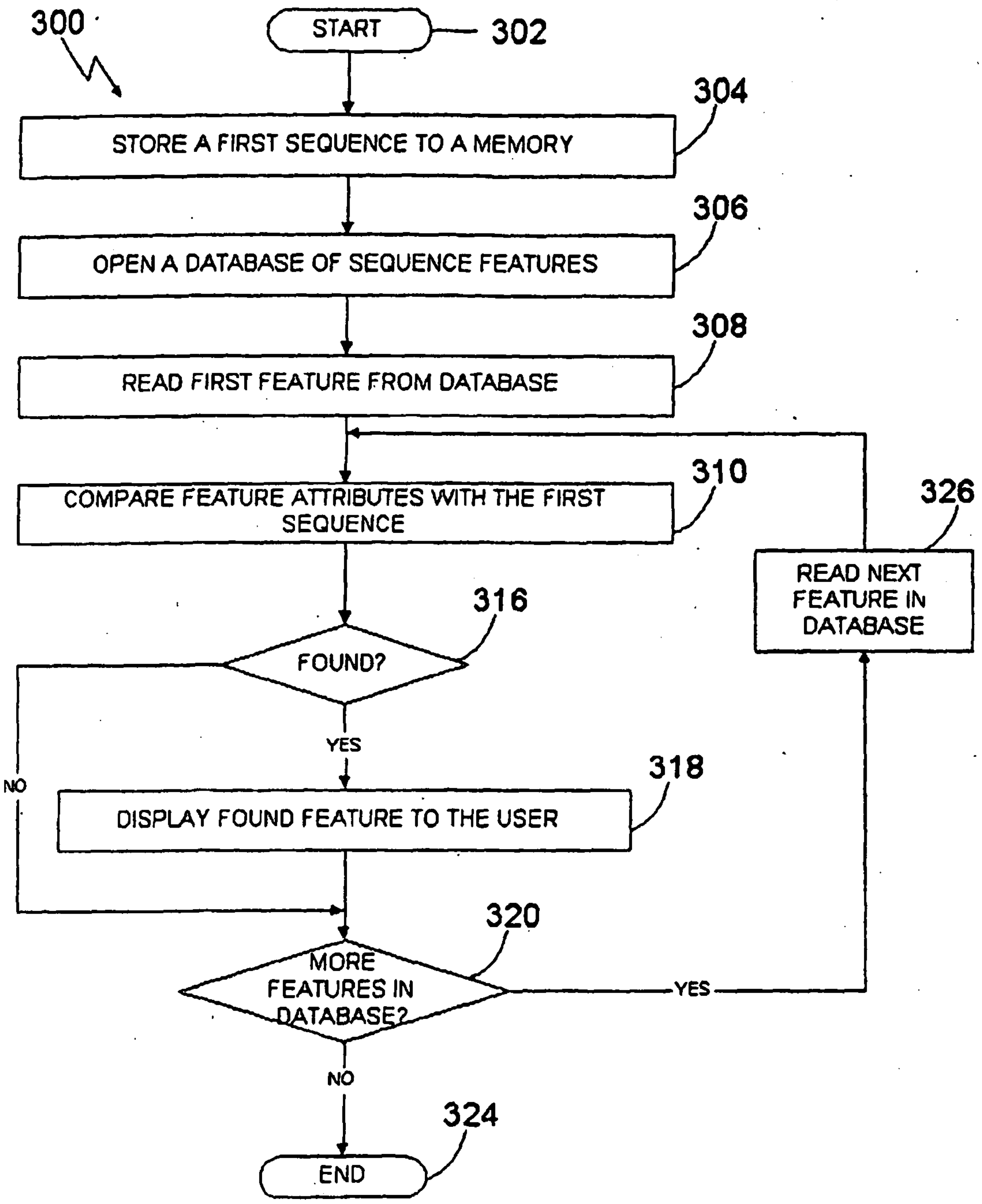
300
START
302
304
STORE A FIRST SEQUENCE TO A MEMORY
306
OPEN A DATABASE OF SEQUENCE FEATURES
308
READ FIRST FEATURE FROM DATABASE
310
COMPARE FEATURE ATTRIBUTES WITH THE FIRST SEQUENCE
326
READ NEXT FEATURE IN DATABASE
316
FOUND?
YES
NO
318
DISPLAY FOUND FEATURE TO THE USER
320
MORE FEATURES IN DATABASE?
YES
NO
324
END

FIG.20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9504064 A
- WO 8403564 A
- WO 8403506 A
- US 5143854 A
- WO 9015070 A
- WO 9210092 A
- US 5412087 A
- WO 9511995 A
- WO 9412305 A
- WO 9411530 A
- WO 9729212 A
- WO 9731256 A
- WO 9717359 A
- US 5374544 A
- EP 582796 A
- US 5698389 A
- US 5643746 A
- US 5502176 A
- US 5266488 A
- EP 0036776 A
- WO 9424298 A
- WO 9325234 A
- WO 9406920 A
- WO 9011092 A
- WO 9511307 A
- US 4873191 A
- US 5464764 A
- US 5789215 A
- US 5082767 A, Hatfield
- US 09058746 B
- EP 320308 A
- EP 439182 A
- EP 4544610 A
- EP 684315 A
- WO 9322461 A
- WO 9320227 A
- US 5322770 A
- US 4683195 A
- US 4683202 A
- US 4965188 A
- EP 0707592 A
- US 4656127 A
- EP 412883 A
- WO 9215712 A
- WO 9001069 A
- WO 9521271 A
- WO 9220702 A
- US 5185444 A
- US 5034506 A
- US 5142047 A
- US 04906193 A
- EP 785280 A
- US 5424186 A
- US 5589136 A
- WO 9820165 A
- US 082614 P
- US 5436850 A, Eisenberg
- US 5557535 A, Srinivasan
- US 60126269 B
- US 60131961 B


### Non-patent literature cited in the description


- **HARDMAN J.G. ; GOODMAN, GILMAN A. ; LIMBIRD L.E.** Goodman & Gilman's The Pharmacological Basis of Therapeutics. McGraw-Hill, 1996
- **ANDERSON M.E.** *Advances in Pharmacology,* 1997, vol. 38, 65-74
- **BALLANTYNE, B. ; MARRS T. ; TURNER P.** General & Applied Toxicology. Stockton Press, 1993
- **STENKE et al.** *Acta Oncologica,* 1987, vol. 27, 803-805
- **PETRIC et al.** *Biochim. Biophys. Acta,* 1995, vol. 1254, 207-215
- **JAKOBSSON et al.** *Journal of Biological Chemistry,* 1996, vol. 271, 22203-22210
- **WEBER W.W.** Pharmacogenetics. Oxford University Press, 1997
- **WEBER W.W.** Pharmacogenctics. Oxford University Press, 1997
- **H. MARIO GEYSEN et al.** *Proc. Natl. Acad. Sci. U.S.A.,* 1984, vol. 81, 3998-4002
- **STRYER, L.** Biochemistry. 1995
- **PEARSON ; LIPMAN.** *Proc. Natl. Acad. Sci.,* 1988, vol. 85 (8), 2444-2448
- **ALTSCHUL et al.** *J. Mol. Biol.,* 1990, vol. 215 (3), 403-410
- **THOMPSON et al.** *Nucleic Acids Res.,* 1994, vol. 22 (2), 4673-4680
- **HIGGINS et al.** *Methods Enzymol,* 1996, vol. 266, 383-402

- **ALTSCHUL et al.** *Nature Genetics,* 1993, vol. 3, 266-272
- **KARLIN ; ALTSCHUL.** *Proc. Natl. Acad. Sci. USA,* 1990, vol. 87, 2267-2268
- **ALTSCHUL et al.** *Nuc. Acids Res.,* 1997, vol. 25, 3389-3402
- **GONNET et al.** *Science,* 1992, vol. 256, 1443-1445
- **HENIKOFF ; HENIKOFF.** *Proteins,* 1993, vol. 17, 49-61
- Matrices for Detecting Distance Relationships: Atlas of Protein Sequence and Structure. Washington:National Biomedical Research Foundation. 1978
- **HAMES ; HIGGINS.** NucleicAcid Hybridization: A Practical Approach. IRL Press, 1985
- **SAMBROOK et al.** Molecular Cloning: A Laboratory Manual. Cold Spring Harbor Laboratory, 1989
- **FODOR et al.** *Science,* 1991, vol. 251, 767-777
- **SAMBROOK et al.** Molecular Cloning A Laboratory Manual. Cold Spring Harbor Laboratory, 1989
- **COLES et al.** *Hum. Mol. Genet.,* 1998, vol. 7, 791-800
- **GOSSEN et al.** *Science,* 1995, vol. 268, 1766-1769
- **THOMAS et al.** *Cell,* 1986, vol. 44, 419-428
- **TE RIELE et al.** *Nature,* 1990, vol. 348, 649-651
- **VAN DER LUGT et al.** *Gene,* 1991, vol. 105, 263-267
- **REID et al.** *Proc. Natl. Acad Sci. USA,* 1990, vol. 87, 4299-4303
- **NADA et al.** *Cell,* 1993, vol. 73, 1125-1135
- **YAGI et al.** *Proc. Natl; Acad. Sci. USA,* 1990, vol. 87, 9918-9922
- **MANSOUR et al.** *Nature,* 1988, vol. 336, 348-352
- **KOLLER et al.** *Ann. Rev. Immunol.,* 1992, vol. 10, 705-730
- **HOESS et al.** *Nucleic Acids Res.,* 1986, vol. 14, 2287-2300
- **GU et al.** *Cell,* 1993, vol. 73, 1155-1164
- **ARAKI et al.** *Proc. Natl; Acad. Sci. USA,* 1995, vol. 92, 160-164
- **BAUBONIS et al.** *Nucleic Acids Res.,* 1993, vol. 21, 2025-2029
- **SAUER et al.** *Proc. Natl; Acad. Sci. USA,* 1988, vol. 85, 5166-5170
- **GU et al.** *Science,* 1994, vol. 265, 103-106
- **ZOU et al.** *Curr. Biol.,* 1994, vol. 4, 1099-1103
- **ANTON et al.** *J. Virol.,* 1995, vol. 69, 4600-4606
- **KANEGAE et al.** *Nucleic Acids Res.,* 1995, vol. 23, 3816-3821
- **STERNBERG.** *Mamm. Genome,* 1994, vol. 5, 397-404
- **LINTON et al.** *J. Clin. Invest.,* 1993, vol. 92, 3029-3037
- **MCCORMICK et al.** *Genet. Anal. Tech. Appl.,* 1994, vol. 11, 158-164
- **CHAI et al.** *Biotech. Appl. Blochem.,* 1993, vol. 18, 259-273
- **VLASAK et al.** *Eur. J. Biochem.,* 1983, vol. 135, 123-126
- **LENHARD et al.** *Gene,* 1996, vol. 169, 187-190
- **ROTH et al.** *Nature Medicine,* 1996, vol. 2, 985-991
- **MURYCZKA et al.** *Current Topics in Microbiol. Immunol.,* 1992, vol. 158, 97-129
- **MCLAUGHLIN et al.** *Am. J. Hum. Genet.,* 1989, vol. 59, 561-569
- **KIM et al.** *Genomics,* 1996, vol. 34, 213-218
- **CHEN et al.** *Proc. Natl. Acad Sci. USA,* 1987, vol. 94, 10756-10761
- **GOPAL.** *Mol. Cell. Biol.,* 1985, vol. 5, 1188-1190
- **TUR-KASPA et al.** *Mol. Cell. Biol.,* vol. 6, 716-7181986
- **KLEIN et al.** *Nature,* 1987, vol. 327, 70-73
- Targeting of liposomes to hepatocytes. **GHOSH ; BACCHAWAT.** Liver Diseases, Targeted diagnosis and therapy using specific rceptors and ligands. Marcel Dekeker, 1991, 87-104
- **WONG et al.** *Gene,* 1980, vol. 10, 87-94
- **NICOLAU et al.** *Biochim. Biophys. Acta,* 1982, vol. 721, 185-190
- **SCHEDL et al.** *Nucleic Acids Res,* 1993, vol. 21, 4783-4787
- **ABBONDANZO et al.** Methods in Enzymology. Academic Press, 1993, 803-823
- Embryo-Derived StemCell Lines. **ROBERTSON.** Teratocarcinomas and Embrionic Stem Cells: A Practical Approach. IRL Press, 1987, 71
- **PEASE ; WILLIAMS.** *Exp. Cell. Res.,* 1990, vol. 190, 09-211
- **THOMAS et al.** *Cell,* 1997, vol. 51, 503-512
- Production and Analysis of Chimaeric Mice. **BRADLEY.** Teratocarcinomas and embryonic stem cells: A practical approach. IRL Press, 1987, 113
- **WOOD et al.** *Proc. Natl. Acad Sci. U.S.A.,* 1993, vol. 90, 4582-4585
- **NAGY et al.** *Proc. Natl. Acad. Sci. USA.,* 1993, vol. 90, 8424-8428
- **CHOU.** *Mol. Endocrinol.,* 1989, vol. 3, 1511-1514
- **SHAY et al.** *Biochem. Biophys. Acta.,* 1991, vol. 1072, 1-7
- **DAVIS et al.** Basic Methods in Molecular Biology. Elsevier Press, 1986
- Manual of Clinical Immunology. **FISHER, D.** Amer. Soc. For Microbiol. 1980

- **MANIATIS et al.** Molecular Cloning, A Laboratory Manual. Cold Spring Harbor press, 1989
- **CHEE et al.** *Science,* 1996, vol. 274, 610
- **WOLCOTT, M.J.** *Clin. Mcrobiol. Rev.,* vol. 5, 370-386
- **GUATELLI J.C. et al.** *Proc. Natl. Acad Sci. USA,* 1990, vol. 87, 1874-1878
- **COMPTON J.** *Nature,* 1991, vol. 350, 91-92
- **WALKER et al.** *Clin. Chem.,* 1996, vol. 42, 9-13
- **MARSHALL R.L. et al.** *PCR Methods and Applications,* 1994, vol. 4, 80-84
- Molecular Cloning to Genetic Engineering. Methods in Molecular Biology. Humana Press, 1997, 67
- PCR Methods and Applications. Cold Spring Harbor Laboratory Press, 1991
- **NARANG S.A. et al.** *Methods Enzymol.,* 1979, vol. 68, 90-98
- **BROWN E.L. et al.** *Methods Enzymol,* 1979, vol. 68, 109-151
- **BEAUCAGE et al.** *Tetrahedron Lett,* 1981, vol. 22, 1859-1862
- **ORITA et al.** *Proc. Natl. Acad. Sci. U.S.A,* 1989, vol. 86, 27776-2770
- **SHEFFIELD, V.C. et al.** *Proc. Natl. Acad Sci. USA,* 1991, vol. 49, 699-706
- **WHITE et al.** *Genomics,* 1992, vol. 12, 301-306
- **GROMPE, M. et al.** *Proc. Natl. Acad Sci. USA,* 1989, vol. 86, 5855-5892
- **GROMPE, M.** *Nature Genetics,* 1993, vol. 5, 111-117
- **CHEN ; KWOK.** *Nucleic Acids Research,* 1997, vol. 25, 347-353
- **CHEN et al.** *Proc. Natl. Acad. Sci. USA,* 1997, vol. 94/20, 10756-10761
- **HAFF L.A. ; SMIRNOV I.P.** *Genome Research,* 1997, vol. 7, 378-388
- **SYVÄNEN.** *Clinica Chimica Acta,* 1994, vol. 226, 225-236
- **LIVAK ; HAINER.** *Human Mutation,* 1994, vol. 3, 379-385
- **HARJU et al.** *Clin. Chem.,* 1993, vol. 39, 112282-2287
- **NYREN et al.** *Analytical Biochemistry,* 1993, vol. 208, 171-175
- **PASTINEN et al.** *Genome research,* 1997, vol. 7, 606-614
- **NICKERSON D.A. et al.** *Proc. Natl. Acad. Sci. U.S.A.,* 1990, vol. 87, 8923-8927
- **SAMBROOK et al.** Molecular Cloning - A Laboratory Manual. Cold Spring Harbor Press, 1989
- **AUSUBEL et al.** Current Protocols in Molecular Biology. Green Publishing Associates and Wiley Interscience, 1989
- **LANDEGREN U. et al.** *Genome Research,* vol. 8, 769-776
- **LIVAK et al.** *Nature Genetics,* 1995, vol. 9, 341-342
- **TYAGI et al.** *Nature Biotechnology,* 1998, vol. 16, 49-53
- **HACIA et al.** *Nature Genetics,* 1996, vol. 14 (4), 441-447
- **SHOEMAKER et al.** *Nature Genetics,* 1996, vol. 14 (4), 450-456
- **KOZAL et al.** *Nature Medicine,* 1996, vol. 2, 753-759
- **LANDER ; SCHORK.** *Science,* 1994, vol. 265, 2037-2048
- **KHOURY J. et al.** Fundamentals of Genetic Epidemiology. Oxford University Press, 1993
- **WEIR, B.S.** Genetic data Analysis II: Methods for Discrete population genetic Data. Sinauer Assoc., Inc, 1996
- **MORTON N.E.** *Am.J. Hum. Genet.,* 1955, vol. 7, 277-318
- **OTT J.** Analysis of Human Genetic Linkage. John Hopkins University Press, 1991
- **RICH, N. ; MERIKANGAS, K.** *Science,* 1996, vol. 273, 1516-1517
- **ZHAO et al.** *Am. J. Hum. Genet.,* 1998, vol. 63, 225-240
- **PERLIN et al.** *Am. J. Hum. Genet.,* 1994, vol. 55, 777-787
- **NEWTON et al.** *Nucleic Acids Res.,* 1989, vol. 17, 2503-2516
- **WU et al.** *Proc. Natl. Acad Sci. USA,* 1989, vol. 86, 2757
- **RUANO et al.** *Proc. Natl. Acad Sci. USA,* 1990, vol. 87, 6296-6300
- **SARKAR, G. ; SOMMER S.S.** *Biotechniques,* 1991
- **CLARK A.G.** *Mol. Biol. Evol.,* 1990, vol. 7, 111-122
- **DEMPSTER et al.** *J. R. Stat. Soc.,* 1977, vol. 39B, 1-38
- **EXCOFFIER L. ; SLATKIN M.** *Mol. Biol. Evol.,* 1995, vol. 12 (5), 921-927
- **AJIOKA R.S. et al.** *Am. J. Hum. Genet.,* 1997, vol. 60, 1439-1447
- **SPIELMANN S. et al.** *Am. J. Hum. Genet.,* 1993, vol. 52, 506-516
- **SCHAID D.J. et al.** *Genet. Epidemiol.,* 1996, vol. 13, 423-450
- **SPIELMANN S. ; EWENS W.J.** *Am. J. Hum. Genet.,* 1998, vol. 62, 450-458
- **TERWILLIGER J.D. ; OTT J.** Handbook of Human Genetic Linkage. John Hopkins University Press, 1994
- **LANGE K.** Mathematical and Statistical Methods for Genetic Analysis. Springer, 1997
- **HAWLEY M.E. et al.** *Am. J. Phys. Anthropol.,* 1994, vol. 18, 104
- **SCHNEIDER et al.** *Arlequin: a software for population genetics data analysis,* 1997

- **SMITH.** *Ann. Hum. Genet.,* 1957, vol. 21, 254-276
- **WEIR B.S.** Genetic Data Analysis. 1996
- **RANG H.P. ; RITTER J.M. ; DALE M.M.** *Pharmacology,* 1995
- **STRYER, LUBERT.** Biochemistry. W. H Freeman & Co,
- **SOWDHAMINI et al.** *Protein Engineering,* 1997, vol. 10 (207), 215
- **ASZÒDI et al.** *Proteins: Structure, Function, and Genetics,* 1997, vol. 1, 38-42
- **ALTSCHUL et al.** *J. Mol. Biol,* 1990, vol. 215, 403
- **PEARSON ; LIPMAN.** *Proc. Natl. Acad. Sci. USA,* 1988, vol. 85, 2444
- **BRUTLAG et al.** *Comp. App. Biosci.,* 1990, vol. 6, 237-245
- **EXCOFFIER ; SLATKIN.** *Mol. Biol. Evol.,* 1995, vol. 12, 921-927
- **HAWLEY et al.** *Am. J. Phys. Anthropol.,* 1994, vol. 18, 104
- **KOHLER ; MILSTEIN.** *Nature,* 1975, vol. 256, 495
- **HARLOW ; LANE.** Antibodies A Laboratory Manual. Cold Spring Harbor Laboratory, 1988, 53-242
- **ENGVALL, E.** *Meth. Enzymol.,* 1980, vol. 70, 419
- **DAVIS, L. et al.** Basic Methods in Molecular Biology. Elsevier
- **VAITUKAITIS, J. et al.** *J. Clin. Endocrinol. Metab.,* 1971, vol. 33, 988-991
- **OUCHTERLONY, O. et al.** Handbook of Experimental Immunology. Blackwell, 1973